(19) Europäisches Patentamt
European Patent Office
Office européen des brevets

(11) EP 2 765 192 A1

(12) **EUROPEAN PATENT APPLICATION**
published in accordance with Art. 153(4) EPC

(43) Date of publication:
**13.08.2014 Bulletin 2014/33**

(21) Application number: **12838341.1**

(22) Date of filing: **05.10.2012**

(51) Int Cl.:
***C12N 15/09*** (2006.01)
***A61K 39/00*** (2006.01)
***A61K 39/395*** (2006.01)
***C07K 16/18*** (2006.01)
***C12P 21/02*** (2006.01)
***C12P 21/08*** (2006.01)
***C12Q 1/02*** (2006.01)

(86) International application number:
**PCT/JP2012/006453**

(87) International publication number:
**WO 2013/051294 (11.04.2013 Gazette 2013/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.10.2011 JP 2011221400**

(71) Applicant: **Chugai Seiyaku Kabushiki Kaisha**
**Kita-ku**
**Tokyo 115-8543 (JP)**

(72) Inventors:
- **IGAWA, Tomoyuki**
  **Gotemba-shi**
  **Shizuoka 412-8513 (JP)**
- **TAMBA, Shigero**
  **Gotemba-shi**
  **Shizuoka 412-8513 (JP)**
- **KAWAZOE, Meiri**
  **Gotemba-shi**
  **Shizuoka 412-8513 (JP)**

(74) Representative: **Woods, Geoffrey Corlett**
**J A Kemp**
**14 South Square**
**Gray's Inn**
**London WC1R 5JJ (GB)**

(54) **ANTIGEN-BINDING MOLECULE FOR PROMOTING CLEARANCE FROM PLASMA OF ANTIGEN COMPRISING SACCHARIDE CHAIN RECEPTOR-BINDING DOMAIN**

(57) Disclosed are an antigen-binding molecule containing a sugar chain receptor-binding domain and having a weak antigen-binding activity in the pH of early-stage endosome compared to the antigen-binding activity in the pH of plasma; a pharmaceutical composition containing the antigen-binding molecule; and a method for producing these. Use of the antigen-binding molecule of the invention enables to promote uptake of an antigen into a cell and increase the number of antigens that a single antibody molecule can bind. Administration of the antibody enables to reduce the number of antigens in plasma more and more and improve pharmacokinetics of the antibody.



Printed by Jouve, 75001 PARIS (FR)

SEQUENCE LISTING

<110>  Chugai Seiyaku Kabushiki Kaisha

<120>  Antigen binding molecule comprising sugar receptor binding domain that stimulate plasma clearance of antigen

<130>  PCG-9037WO

<150>  JP 2011-221400

<151>  2011-10-05

<160>  79

<170>  PatentIn version 3.1

<210>  1

<211>  468

<212>  PRT

<213>  homo sapiens

<400>  1

Met Leu Ala Val Gly Cys Ala Leu Leu Ala Ala Leu Leu Ala Ala Pro
1               5                   10                  15
Gly Ala Ala Leu Ala Pro Arg Arg Cys Pro Ala Gln Glu Val Ala Arg
            20                  25                  30
Gly Val Leu Thr Ser Leu Pro Gly Asp Ser Val Thr Leu Thr Cys Pro
        35                  40                  45
Gly Val Glu Pro Glu Asp Asn Ala Thr Val His Trp Val Leu Arg Lys
    50                  55                  60
Pro Ala Ala Gly Ser His Pro Ser Arg Trp Ala Gly Met Gly Arg Arg
65                  70                  75                  80
Leu Leu Leu Arg Ser Val Gln Leu His Asp Ser Gly Asn Tyr Ser Cys
                85                  90                  95
Tyr Arg Ala Gly Arg Pro Ala Gly Thr Val His Leu Leu Val Asp Val
            100                 105                 110
Pro Pro Glu Glu Pro Gln Leu Ser Cys Phe Arg Lys Ser Pro Leu Ser
        115                 120                 125
Asn Val Val Cys Glu Trp Gly Pro Arg Ser Thr Pro Ser Leu Thr Thr
    130                 135                 140
Lys Ala Val Leu Leu Val Arg Lys Phe Gln Asn Ser Pro Ala Glu Asp
145                 150                 155                 160
Phe Gln Glu Pro Cys Gln Tyr Ser Gln Glu Ser Gln Lys Phe Ser Cys
                165                 170                 175
Gln Leu Ala Val Pro Glu Gly Asp Ser Ser Phe Tyr Ile Val Ser Met
            180                 185                 190
Cys Val Ala Ser Ser Val Gly Ser Lys Phe Ser Lys Thr Gln Thr Phe
        195                 200                 205
Gln Gly Cys Gly Ile Leu Gln Pro Asp Pro Pro Ala Asn Ile Thr Val
    210                 215                 220
Thr Ala Val Ala Arg Asn Pro Arg Trp Leu Ser Val Thr Trp Gln Asp
225                 230                 235                 240
Pro His Ser Trp Asn Ser Ser Phe Tyr Arg Leu Arg Phe Glu Leu Arg
                245                 250                 255
Tyr Arg Ala Glu Arg Ser Lys Thr Phe Thr Thr Trp Met Val Lys Asp
            260                 265                 270
Leu Gln His His Cys Val Ile His Asp Ala Trp Ser Gly Leu Arg His
        275                 280                 285
Val Val Gln Leu Arg Ala Gln Glu Glu Phe Gly Gln Gly Glu Trp Ser
    290                 295                 300
Glu Trp Ser Pro Glu Ala Met Gly Thr Pro Trp Thr Glu Ser Arg Ser
305                 310                 315                 320
Pro Pro Ala Glu Asn Glu Val Ser Thr Pro Met Gln Ala Leu Thr Thr
                325                 330                 335
Asn Lys Asp Asp Asp Asn Ile Leu Phe Arg Asp Ser Ala Asn Ala Thr
            340                 345                 350
Ser Leu Pro Val Gln Asp Ser Ser Ser Val Pro Leu Pro Thr Phe Leu
        355                 360                 365
Val Ala Gly Gly Ser Leu Ala Phe Gly Thr Leu Leu Cys Ile Ala Ile
    370                 375                 380

Val Leu Arg Phe Lys Lys Thr Trp Lys Leu Arg Ala Leu Lys Glu Gly
385 390 395 400
Lys Thr Ser Met His Pro Pro Tyr Ser Leu Gly Gln Leu Val Pro Glu
405 410 415
Arg Pro Arg Pro Thr Pro Val Leu Val Pro Leu Ile Ser Pro Pro Val
420 425 430
Ser Pro Ser Ser Leu Gly Ser Asp Asn Thr Ser Ser His Asn Arg Pro
435 440 445
Asp Ala Arg Asp Pro Arg Ser Pro Tyr Asp Ile Ser Asn Thr Asp Tyr
450 455 460
Phe Phe Pro Arg
465

<210> 2
<211> 1407
<212> PRT
<213> homo sapiens
<400> 2

Ala Thr Gly Cys Thr Gly Gly Cys Cys Gly Thr Cys Gly Gly Cys Thr
1 5 10 15
Gly Cys Gly Cys Gly Cys Thr Gly Cys Thr Gly Gly Cys Thr Gly Cys
20 25 30
Cys Cys Thr Gly Cys Thr Gly Gly Cys Cys Gly Cys Gly Cys Cys Gly
35 40 45
Gly Gly Ala Gly Cys Gly Gly Cys Gly Cys Thr Gly Gly Cys Cys Cys
50 55 60
Cys Ala Ala Gly Gly Cys Gly Cys Thr Gly Cys Cys Cys Thr Gly Cys
65 70 75 80
Gly Cys Ala Gly Gly Ala Gly Gly Thr Gly Gly Cys Gly Ala Gly Ala
85 90 95
Gly Gly Cys Gly Thr Gly Cys Thr Gly Ala Cys Cys Ala Gly Thr Cys
100 105 110
Thr Gly Cys Cys Ala Gly Gly Ala Gly Ala Cys Ala Gly Cys Gly Thr
115 120 125
Gly Ala Cys Thr Cys Thr Gly Ala Cys Cys Thr Gly Cys Cys Cys Gly
130 135 140
Gly Gly Gly Gly Thr Ala Gly Ala Gly Cys Cys Gly Gly Ala Ala Gly
145 150 155 160
Ala Cys Ala Ala Thr Gly Cys Cys Ala Cys Thr Gly Thr Thr Cys Ala
165 170 175
Cys Thr Gly Gly Gly Thr Gly Cys Thr Cys Ala Gly Gly Ala Ala Gly
180 185 190
Cys Cys Gly Gly Cys Thr Gly Cys Ala Gly Gly Cys Thr Cys Cys Cys
195 200 205
Ala Cys Cys Cys Cys Ala Gly Cys Ala Gly Ala Thr Gly Gly Gly Cys
210 215 220
Thr Gly Gly Cys Ala Thr Gly Gly Gly Ala Ala Gly Gly Ala Gly Gly
225 230 235 240
Cys Thr Gly Cys Thr Gly Cys Thr Gly Ala Gly Gly Thr Cys Gly Gly
245 250 255
Thr Gly Cys Ala Gly Cys Thr Cys Cys Ala Cys Gly Ala Cys Thr Cys
260 265 270
Thr Gly Gly Ala Ala Ala Cys Thr Ala Thr Thr Cys Ala Thr Gly Cys
275 280 285
Thr Ala Cys Cys Gly Gly Gly Cys Cys Gly Gly Cys Cys Gly Cys Cys
290 295 300
Cys Ala Gly Cys Thr Gly Gly Gly Ala Cys Thr Gly Thr Gly Cys Ala
305 310 315 320
Cys Thr Thr Gly Cys Thr Gly Gly Thr Gly Gly Ala Thr Gly Thr Thr
325 330 335
Cys Cys Cys Cys Cys Cys Gly Ala Gly Gly Ala Gly Cys Cys Cys Cys
340 345 350
Ala Gly Cys Thr Cys Thr Cys Cys Thr Gly Cys Thr Thr Cys Cys Gly
355 360 365

Gly Ala Ala Gly Ala Gly Cys Cys Cys Cys Cys Thr Cys Ala Gly Cys
    370                 375                 380
Ala Ala Thr Gly Thr Thr Gly Thr Thr Thr Gly Thr Gly Ala Gly Thr
385                 390                 395                 400
Gly Gly Gly Gly Thr Cys Cys Thr Cys Gly Gly Ala Gly Cys Ala Cys
                405                 410                 415
Cys Cys Cys Ala Thr Cys Cys Cys Thr Gly Ala Cys Gly Ala Cys Ala
            420                 425                 430
Ala Ala Gly Gly Cys Thr Gly Thr Gly Cys Thr Cys Thr Thr Gly Gly
        435                 440                 445
Thr Gly Ala Gly Gly Ala Ala Gly Thr Thr Thr Cys Ala Gly Ala Ala
    450                 455                 460
Cys Ala Gly Thr Cys Cys Gly Gly Cys Cys Gly Ala Ala Gly Ala Cys
465                 470                 475                 480
Thr Thr Cys Cys Ala Gly Gly Ala Gly Cys Cys Gly Thr Gly Cys Cys
                485                 490                 495
Ala Gly Thr Ala Thr Thr Cys Cys Cys Ala Gly Gly Ala Gly Thr Cys
            500                 505                 510
Cys Cys Ala Gly Ala Ala Gly Thr Thr Cys Thr Cys Cys Thr Gly Cys
        515                 520                 525
Cys Ala Gly Thr Thr Ala Gly Cys Ala Gly Thr Cys Cys Cys Gly Gly
    530                 535                 540
Ala Gly Gly Gly Ala Gly Ala Cys Ala Gly Cys Thr Cys Thr Thr Thr
545                 550                 555                 560
Cys Thr Ala Cys Ala Thr Ala Gly Thr Gly Thr Cys Cys Ala Thr Gly
                565                 570                 575
Thr Gly Cys Gly Thr Cys Gly Cys Cys Ala Gly Thr Ala Gly Thr Gly
            580                 585                 590
Thr Cys Gly Gly Gly Ala Gly Cys Ala Ala Gly Thr Thr Cys Ala Gly
        595                 600                 605
Cys Ala Ala Ala Ala Cys Thr Cys Ala Ala Ala Cys Cys Thr Thr Thr
    610                 615                 620
Cys Ala Gly Gly Gly Thr Thr Gly Thr Gly Gly Ala Ala Thr Cys Thr
625                 630                 635                 640
Thr Gly Cys Ala Gly Cys Cys Thr Gly Ala Thr Cys Cys Gly Cys Cys
                645                 650                 655
Thr Gly Cys Cys Ala Ala Cys Ala Thr Cys Ala Cys Ala Gly Thr Cys
            660                 665                 670
Ala Cys Thr Gly Cys Cys Gly Thr Gly Gly Cys Cys Ala Gly Ala Ala
        675                 680                 685
Ala Cys Cys Cys Cys Cys Gly Cys Thr Gly Gly Cys Thr Cys Ala Gly
    690                 695                 700
Thr Gly Thr Cys Ala Cys Cys Thr Gly Gly Cys Ala Ala Gly Ala Cys
705                 710                 715                 720
Cys Cys Cys Cys Ala Cys Thr Cys Cys Thr Gly Gly Ala Ala Cys Thr
                725                 730                 735
Cys Ala Thr Cys Thr Thr Thr Cys Thr Ala Cys Ala Gly Ala Cys Thr
            740                 745                 750
Ala Cys Gly Gly Thr Thr Thr Gly Ala Gly Cys Thr Cys Ala Gly Ala
        755                 760                 765
Thr Ala Thr Cys Gly Gly Gly Cys Thr Gly Ala Ala Cys Gly Gly Thr
    770                 775                 780
Cys Ala Ala Ala Gly Ala Cys Ala Thr Thr Cys Ala Cys Ala Ala Cys
785                 790                 795                 800
Ala Thr Gly Gly Ala Thr Gly Gly Thr Cys Ala Ala Gly Gly Ala Cys
                805                 810                 815
Cys Thr Cys Cys Ala Gly Cys Ala Thr Cys Ala Cys Thr Gly Thr Gly
            820                 825                 830
Thr Cys Ala Thr Cys Cys Ala Cys Gly Ala Cys Gly Cys Cys Thr Gly
        835                 840                 845
Gly Ala Gly Cys Gly Gly Cys Cys Thr Gly Ala Gly Gly Cys Ala Cys
    850                 855                 860
Gly Thr Gly Gly Thr Gly Cys Ala Gly Cys Thr Thr Cys Gly Thr Gly

```
865                 870                 875                 880
Cys Cys Cys Ala Gly Gly Ala Gly Gly Ala Gly Thr Thr Cys Gly Gly
                885                 890                 895
Gly Cys Ala Ala Gly Gly Cys Gly Ala Gly Thr Gly Gly Ala Gly Cys
            900                 905                 910
Gly Ala Gly Thr Gly Gly Ala Gly Cys Cys Cys Gly Gly Ala Gly Gly
        915                 920                 925
Cys Cys Ala Thr Gly Gly Gly Cys Ala Cys Gly Cys Cys Thr Thr Gly
    930                 935                 940
Gly Ala Cys Ala Gly Ala Ala Thr Cys Cys Ala Gly Gly Ala Gly Thr
945                 950                 955                 960
Cys Cys Thr Cys Cys Ala Gly Cys Thr Gly Ala Gly Ala Ala Cys Gly
                965                 970                 975
Ala Gly Gly Thr Gly Thr Cys Cys Ala Cys Cys Cys Cys Cys Ala Thr
            980                 985                 990
Gly Cys Ala Gly Gly Cys Ala Cys  Thr Thr Ala Cys Thr  Ala Cys Thr
        995                 1000                1005
Ala Ala  Thr Ala Ala Ala Gly  Ala Cys Gly Ala Thr  Gly Ala Thr
    1010                 1015                 1020
Ala Ala  Thr Ala Thr Thr Cys  Thr Cys Thr Thr Cys  Ala Gly Ala
    1025                 1030                 1035
Gly Ala  Thr Thr Cys Thr Gly  Cys Ala Ala Ala Thr  Gly Cys Gly
    1040                 1045                 1050
Ala Cys  Ala Ala Gly Cys Cys  Thr Cys Cys Cys Ala  Gly Thr Gly
    1055                 1060                 1065
Cys Ala  Ala Gly Ala Thr Thr  Cys Thr Thr Cys Thr  Thr Cys Ala
    1070                 1075                 1080
Gly Thr  Ala Cys Cys Ala Cys  Thr Gly Cys Cys Cys  Ala Cys Ala
    1085                 1090                 1095
Thr Thr  Cys Cys Thr Gly Gly  Thr Thr Gly Cys Thr  Gly Gly Ala
    1100                 1105                 1110
Gly Gly  Gly Ala Gly Cys Cys  Thr Gly Gly Cys Cys  Thr Thr Cys
    1115                 1120                 1125
Gly Gly  Ala Ala Cys Gly Cys  Thr Cys Cys Thr Cys  Thr Gly Cys
    1130                 1135                 1140
Ala Thr  Thr Gly Cys Cys Ala  Thr Thr Gly Thr Thr  Cys Thr Gly
    1145                 1150                 1155
Ala Gly  Gly Thr Thr Cys Ala  Ala Gly Ala Ala Gly  Ala Cys Gly
    1160                 1165                 1170
Thr Gly  Gly Ala Ala Gly Cys  Thr Gly Cys Gly Gly  Gly Cys Thr
    1175                 1180                 1185
Cys Thr  Gly Ala Ala Gly Gly  Ala Ala Gly Gly Cys  Ala Ala Gly
    1190                 1195                 1200
Ala Cys  Ala Ala Gly Cys Ala  Thr Gly Cys Ala Thr  Cys Cys Gly
    1205                 1210                 1215
Cys Cys  Gly Thr Ala Cys Thr  Cys Thr Thr Thr Gly  Gly Gly Gly
    1220                 1225                 1230
Cys Ala  Gly Cys Thr Gly Gly  Thr Cys Cys Cys Gly  Gly Ala Gly
    1235                 1240                 1245
Ala Gly  Gly Cys Cys Thr Cys  Gly Ala Cys Cys Cys  Ala Cys Cys
    1250                 1255                 1260
Cys Cys  Ala Gly Thr Gly Cys  Thr Thr Gly Thr Thr  Cys Cys Thr
    1265                 1270                 1275
Cys Thr  Cys Ala Thr Cys Thr  Cys Cys Cys Cys Ala  Cys Cys Gly
    1280                 1285                 1290
Gly Thr  Gly Thr Cys Cys Cys  Cys Cys Ala Gly Cys  Ala Gly Cys
    1295                 1300                 1305
Cys Thr  Gly Gly Gly Gly Thr  Cys Thr Gly Ala Cys  Ala Ala Thr
    1310                 1315                 1320
Ala Cys  Cys Thr Cys Gly Ala  Gly Cys Cys Ala Cys  Ala Ala Cys
    1325                 1330                 1335
Cys Gly  Ala Cys Cys Ala Gly  Ala Thr Gly Cys Cys  Ala Gly Gly
    1340                 1345                 1350
```

```
Gly Ala  Cys Cys Cys Ala Cys  Gly Gly Ala Gly Cys  Cys Cys Thr
    1355                1360                1365
Thr Ala  Thr Gly Ala Cys Ala  Thr Cys Ala Gly Cys  Ala Ala Thr
    1370                1375                1380
Ala Cys  Ala Gly Ala Cys Thr  Ala Cys Thr Thr Cys  Thr Thr Cys
    1385                1390                1395
Cys Cys  Cys Ala Gly Ala Thr  Ala Gly
    1400                1405
<210>  3
<211>  19
<212>  PRT
<213>  Artificial Sequence
<220>
<223>  signal sequence
<400>  3
Met Gly Trp Ser Cys Ile Ile Leu Phe Leu Val Ala Thr Ala Thr Gly
1               5                   10                  15
Val His Ser

<210>  4
<211>  365
<212>  PRT
<213>  homo sapiens
<400>  4
Met Gly Val Pro Arg Pro Gln Pro Trp Ala Leu Gly Leu Leu Leu Phe
1               5                   10                  15
Leu Leu Pro Gly Ser Leu Gly Ala Glu Ser His Leu Ser Leu Leu Tyr
            20                  25                  30
His Leu Thr Ala Val Ser Ser Pro Ala Pro Gly Thr Pro Ala Phe Trp
        35                  40                  45
Val Ser Gly Trp Leu Gly Pro Gln Gln Tyr Leu Ser Tyr Asn Ser Leu
    50                  55                  60
Arg Gly Glu Ala Glu Pro Cys Gly Ala Trp Val Trp Glu Asn Gln Val
65                  70                  75                  80
Ser Trp Tyr Trp Glu Lys Glu Thr Thr Asp Leu Arg Ile Lys Glu Lys
                85                  90                  95
Leu Phe Leu Glu Ala Phe Lys Ala Leu Gly Gly Lys Gly Pro Tyr Thr
            100                 105                 110
Leu Gln Gly Leu Leu Gly Cys Glu Leu Gly Pro Asp Asn Thr Ser Val
        115                 120                 125
Pro Thr Ala Lys Phe Ala Leu Asn Gly Glu Glu Phe Met Asn Phe Asp
    130                 135                 140
Leu Lys Gln Gly Thr Trp Gly Gly Asp Trp Pro Glu Ala Leu Ala Ile
145                 150                 155                 160
Ser Gln Arg Trp Gln Gln Gln Asp Lys Ala Ala Asn Lys Glu Leu Thr
                165                 170                 175
Phe Leu Leu Phe Ser Cys Pro His Arg Leu Arg Glu His Leu Glu Arg
            180                 185                 190
Gly Arg Gly Asn Leu Glu Trp Lys Glu Pro Pro Ser Met Arg Leu Lys
        195                 200                 205
Ala Arg Pro Ser Ser Pro Gly Phe Ser Val Leu Thr Cys Ser Ala Phe
    210                 215                 220
Ser Phe Tyr Pro Pro Glu Leu Gln Leu Arg Phe Leu Arg Asn Gly Leu
225                 230                 235                 240
Ala Ala Gly Thr Gly Gln Gly Asp Phe Gly Pro Asn Ser Asp Gly Ser
                245                 250                 255
Phe His Ala Ser Ser Ser Leu Thr Val Lys Ser Gly Asp Glu His His
            260                 265                 270
Tyr Cys Cys Ile Val Gln His Ala Gly Leu Ala Gln Pro Leu Arg Val
        275                 280                 285
Glu Leu Glu Ser Pro Ala Lys Ser Ser Val Leu Val Val Gly Ile Val
    290                 295                 300
Ile Gly Val Leu Leu Leu Thr Ala Ala Ala Val Gly Gly Ala Leu Leu
```

```
305                 310                 315                 320
Trp Arg Arg Met Arg Ser Gly Leu Pro Ala Pro Trp Ile Ser Leu Arg
            325                 330                 335
Gly Asp Asp Thr Gly Val Leu Leu Pro Thr Pro Gly Glu Ala Gln Asp
            340                 345                 350
Ala Asp Leu Lys Asp Val Asn Val Ile Pro Ala Thr Ala
        355                 360                 365
<210>  5
<211>  119
<212>  PRT
<213>  homo sapiens
<400>  5
Met Ser Arg Ser Val Ala Leu Ala Val Leu Ala Leu Leu Ser Leu Ser
1               5                   10                  15
Gly Leu Glu Ala Ile Gln Arg Thr Pro Lys Ile Gln Val Tyr Ser Arg
            20                  25                  30
His Pro Ala Glu Asn Gly Lys Ser Asn Phe Leu Asn Cys Tyr Val Ser
        35                  40                  45
Gly Phe His Pro Ser Asp Ile Glu Val Asp Leu Leu Lys Asn Gly Glu
    50                  55                  60
Arg Ile Glu Lys Val Glu His Ser Asp Leu Ser Phe Ser Lys Asp Trp
65                  70                  75                  80
Ser Phe Tyr Leu Leu Tyr Tyr Thr Glu Phe Thr Pro Thr Glu Lys Asp
                85                  90                  95
Glu Tyr Ala Cys Arg Val Asn His Val Thr Leu Ser Gln Pro Lys Ile
            100                 105                 110
Val Lys Trp Asp Arg Asp Met
        115
<210>  6
<211>  107
<212>  PRT
<213>  Artificial Sequence
<220>
<223>  modified antibody fragment
<400>  6
Glu Thr Thr Leu Thr Gln Ser Pro Ala Phe Met Ser Ala Thr Pro Gly
1               5                   10                  15
Asp Lys Val Asn Ile Ser Cys Lys Ala Ser Gln Asp Ile Asp Asp Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro Tyr
                85                  90                  95
Thr Phe Gly Gln Gly Thr Lys Leu Glu Ile Lys
            100                 105
<210>  7
<211>  121
<212>  PRT
<213>  Artificial Sequence
<220>
<223>  modified antibody fragment
<400>  7
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Tyr Met His Trp Val Arg Gln Ala Pro Gly Gln Gly Leu Glu Trp Met
        35                  40                  45
Gly Ile Ile Asn Pro Ser Gly Gly Ser Thr Ser Tyr Ala Gln Lys Phe
```

50 55 60
Gln Gly Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Asp Asp Pro Gly Gly Gly Glu Tyr Tyr Phe Asp Tyr Trp Gly
100 105 110
Gln Gly Thr Leu Val Thr Val Ser Ser
115 120

<210> 8
<211> 126
<212> PRT
<213> Artificial Sequence
<220>
<223> modified antibody fragment
<400> 8

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Asp Ala Pro Tyr Tyr Tyr Asp Ser Ser Gly Tyr Thr Asp Ala
100 105 110
Phe Asp Ile Trp Gly Gln Gly Thr Met Val Thr Val Ser Ser
115 120 125

<210> 9
<211> 330
<212> PRT
<213> Artificial Sequence
<220>
<223> modified antibody fragment
<400> 9

Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Ile Cys Asn Val Asn His Lys Pro Ser Asn Thr Lys Val Asp Lys
85 90 95
Lys Val Glu Pro Lys Ser Cys Asp Lys Thr His Thr Cys Pro Pro Cys
100 105 110
Pro Ala Pro Glu Leu Leu Gly Gly Pro Ser Val Phe Leu Phe Pro Pro
115 120 125
Lys Pro Lys Asp Thr Leu Met Ile Ser Arg Thr Pro Glu Val Thr Cys
130 135 140
Val Val Val Asp Val Ser His Glu Asp Pro Glu Val Lys Phe Asn Trp
145 150 155 160
Tyr Val Asp Gly Val Glu Val His Asn Ala Lys Thr Lys Pro Arg Glu
165 170 175
Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205
Lys Ala Leu Pro Ala Pro Ile Glu Lys Thr Ile Ser Lys Ala Lys Gly
210 215 220
Gln Pro Arg Glu Pro Gln Val Tyr Thr Leu Pro Pro Ser Arg Asp Glu
225 230 235 240
Leu Thr Lys Asn Gln Val Ser Leu Thr Cys Leu Val Lys Gly Phe Tyr
245 250 255
Pro Ser Asp Ile Ala Val Glu Trp Glu Ser Asn Gly Gln Pro Glu Asn
260 265 270
Asn Tyr Lys Thr Thr Pro Pro Val Leu Asp Ser Asp Gly Ser Phe Phe
275 280 285
Leu Tyr Ser Lys Leu Thr Val Asp Lys Ser Arg Trp Gln Gln Gly Asn
290 295 300
Val Phe Ser Cys Ser Val Met His Glu Ala Leu His Asn His Tyr Thr
305 310 315 320
Gln Lys Ser Leu Ser Leu Ser Pro Gly Lys
325 330

<210> 10
<211> 326
<212> PRT
<213> Artificial Sequence
<220>
<223> modified antibody fragment
<400> 10

Ala Ser Thr Lys Gly Pro Ser Val Phe Pro Leu Ala Pro Cys Ser Arg
1 5 10 15
Ser Thr Ser Glu Ser Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Asn Phe Gly Thr Gln Thr
65 70 75 80
Tyr Thr Cys Asn Val Asp His Lys Pro Ser Asn Thr Lys Val Asp Lys
85 90 95
Thr Val Glu Arg Lys Cys Cys Val Glu Cys Pro Pro Cys Pro Ala Pro
100 105 110
Pro Val Ala Gly Pro Ser Val Phe Leu Phe Pro Pro Lys Pro Lys Asp
115 120 125
Thr Leu Met Ile Ser Arg Thr Pro Glu Val Thr Cys Val Val Val Asp
130 135 140
Val Ser His Glu Asp Pro Glu Val Gln Phe Asn Trp Tyr Val Asp Gly
145 150 155 160
Val Glu Val His Asn Ala Lys Thr Lys Pro Arg Glu Glu Gln Phe Asn
165 170 175
Ser Thr Phe Arg Val Val Ser Val Leu Thr Val Val His Gln Asp Trp
180 185 190
Leu Asn Gly Lys Glu Tyr Lys Cys Lys Val Ser Asn Lys Gly Leu Pro
195 200 205
Ala Pro Ile Glu Lys Thr Ile Ser Lys Thr Lys Gly Gln Pro Arg Glu
210 215 220
Pro Gln Val Tyr Thr Leu Pro Pro Ser Arg Glu Glu Met Thr Lys Asn
225 230 235 240
Gln Val Ser Leu Thr Cys Leu Val Lys Gly Phe Tyr Pro Ser Asp Ile
245 250 255
Ala Val Glu Trp Glu Ser Asn Gly Gln Pro Glu Asn Asn Tyr Lys Thr
260 265 270
Thr Pro Pro Met Leu Asp Ser Asp Gly Ser Phe Phe Leu Tyr Ser Lys
275 280 285
Leu Thr Val Asp Lys Ser Arg Trp Gln Gln Gly Asn Val Phe Ser Cys
290 295 300

Ser Val Met His Glu Ala Leu His Asn His Tyr Thr Gln Lys Ser Leu
305 310 315 320
Ser Leu Ser Pro Gly Lys
325

<210> 11
<211> 377
<212> PRT
<213> Artificial Sequence
<220>
<223> modified antibody fragment
<400> 11

Ala Ser Thr Lys Gly Pro Ser Val Phe Pro Leu Ala Pro Cys Ser Arg
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Thr Cys Asn Val Asn His Lys Pro Ser Asn Thr Lys Val Asp Lys
85 90 95
Arg Val Glu Leu Lys Thr Pro Leu Gly Asp Thr Thr His Thr Cys Pro
100 105 110
Arg Cys Pro Glu Pro Lys Ser Cys Asp Thr Pro Pro Pro Cys Pro Arg
115 120 125
Cys Pro Glu Pro Lys Ser Cys Asp Thr Pro Pro Pro Cys Pro Arg Cys
130 135 140
Pro Glu Pro Lys Ser Cys Asp Thr Pro Pro Pro Cys Pro Arg Cys Pro
145 150 155 160
Ala Pro Glu Leu Leu Gly Gly Pro Ser Val Phe Leu Phe Pro Pro Lys
165 170 175
Pro Lys Asp Thr Leu Met Ile Ser Arg Thr Pro Glu Val Thr Cys Val
180 185 190
Val Val Asp Val Ser His Glu Asp Pro Glu Val Gln Phe Lys Trp Tyr
195 200 205
Val Asp Gly Val Glu Val His Asn Ala Lys Thr Lys Pro Arg Glu Glu
210 215 220
Gln Tyr Asn Ser Thr Phe Arg Val Val Ser Val Leu Thr Val Leu His
225 230 235 240
Gln Asp Trp Leu Asn Gly Lys Glu Tyr Lys Cys Lys Val Ser Asn Lys
245 250 255
Ala Leu Pro Ala Pro Ile Glu Lys Thr Ile Ser Lys Thr Lys Gly Gln
260 265 270
Pro Arg Glu Pro Gln Val Tyr Thr Leu Pro Pro Ser Arg Glu Glu Met
275 280 285
Thr Lys Asn Gln Val Ser Leu Thr Cys Leu Val Lys Gly Phe Tyr Pro
290 295 300
Ser Asp Ile Ala Val Glu Trp Glu Ser Ser Gly Gln Pro Glu Asn Asn
305 310 315 320
Tyr Asn Thr Thr Pro Pro Met Leu Asp Ser Asp Gly Ser Phe Phe Leu
325 330 335
Tyr Ser Lys Leu Thr Val Asp Lys Ser Arg Trp Gln Gln Gly Asn Ile
340 345 350
Phe Ser Cys Ser Val Met His Glu Ala Leu His Asn Arg Phe Thr Gln
355 360 365
Lys Ser Leu Ser Leu Ser Pro Gly Lys
370 375

<210> 12
<211> 327
<212> PRT
<213> Artificial Sequence

<220>
<223> modified antibody fragment
<400> 12
Ala Ser Thr Lys Gly Pro Ser Val Phe Pro Leu Ala Pro Cys Ser Arg
1 5 10 15
Ser Thr Ser Glu Ser Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Lys Thr
65 70 75 80
Tyr Thr Cys Asn Val Asp His Lys Pro Ser Asn Thr Lys Val Asp Lys
85 90 95
Arg Val Glu Ser Lys Tyr Gly Pro Pro Cys Pro Ser Cys Pro Ala Pro
100 105 110
Glu Phe Leu Gly Gly Pro Ser Val Phe Leu Phe Pro Pro Lys Pro Lys
115 120 125
Asp Thr Leu Met Ile Ser Arg Thr Pro Glu Val Thr Cys Val Val Val
130 135 140
Asp Val Ser Gln Glu Asp Pro Glu Val Gln Phe Asn Trp Tyr Val Asp
145 150 155 160
Gly Val Glu Val His Asn Ala Lys Thr Lys Pro Arg Glu Glu Gln Phe
165 170 175
Asn Ser Thr Tyr Arg Val Val Ser Val Leu Thr Val Leu His Gln Asp
180 185 190
Trp Leu Asn Gly Lys Glu Tyr Lys Cys Lys Val Ser Asn Lys Gly Leu
195 200 205
Pro Ser Ser Ile Glu Lys Thr Ile Ser Lys Ala Lys Gly Gln Pro Arg
210 215 220
Glu Pro Gln Val Tyr Thr Leu Pro Pro Ser Gln Glu Glu Met Thr Lys
225 230 235 240
Asn Gln Val Ser Leu Thr Cys Leu Val Lys Gly Phe Tyr Pro Ser Asp
245 250 255
Ile Ala Val Glu Trp Glu Ser Asn Gly Gln Pro Glu Asn Asn Tyr Lys
260 265 270
Thr Thr Pro Pro Val Leu Asp Ser Asp Gly Ser Phe Phe Leu Tyr Ser
275 280 285
Arg Leu Thr Val Asp Lys Ser Arg Trp Gln Glu Gly Asn Val Phe Ser
290 295 300
Cys Ser Val Met His Glu Ala Leu His Asn His Tyr Thr Gln Lys Ser
305 310 315 320
Leu Ser Leu Ser Leu Gly Lys
325

<210> 13
<211> 324
<212> PRT
<213> Artificial Sequence
<220>
<223> modified antibody fragment
<400> 13
Ala Ser Thr Lys Gly Pro Ser Val Phe Pro Leu Ala Pro Cys Ser Arg
1 5 10 15
Ser Thr Ser Glu Ser Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Asn Phe Gly Thr Gln Thr
65 70 75 80
Tyr Thr Cys Asn Val Asp His Lys Pro Ser Asn Thr Lys Val Asp Lys

85 90 95
Thr Val Glu Arg Lys Cys Cys Val Glu Cys Pro Pro Cys Pro Ala Pro
100 105 110
Pro Val Ala Gly Pro Ser Val Phe Leu Phe Pro Pro Lys Pro Lys Asp
115 120 125
Thr Leu Met Ile Ser Arg Thr Pro Glu Val Thr Cys Val Val Val Asp
130 135 140
Val Ser His Glu Asp Pro Glu Val Gln Phe Asn Trp Tyr Val Asp Gly
145 150 155 160
Val Glu Val His Asn Ala Lys Thr Lys Pro Arg Glu Glu Gln Phe Asn
165 170 175
Ser Thr Phe Arg Val Val Ser Val Leu Thr Val Val His Gln Asp Trp
180 185 190
Leu Asn Gly Lys Glu Tyr Lys Cys Lys Val Ser Asn Lys Gly Leu Pro
195 200 205
Ala Pro Ile Glu Lys Thr Ile Ser Lys Thr Lys Gly Gln Pro Arg Glu
210 215 220
Pro Gln Val Tyr Thr Leu Pro Pro Ser Arg Glu Glu Met Thr Lys Asn
225 230 235 240
Gln Val Ser Leu Thr Cys Leu Val Lys Gly Phe Tyr Pro Ser Asp Ile
245 250 255
Ala Val Glu Trp Glu Ser Asn Gly Gln Pro Glu Asn Asn Tyr Lys Thr
260 265 270
Thr Pro Pro Met Leu Asp Ser Asp Gly Ser Phe Phe Leu Tyr Ser Lys
275 280 285
Leu Thr Val Asp Lys Ser Arg Trp Gln Gln Gly Asn Val Phe Ser Cys
290 295 300
Ser Val Met His Glu Ala Leu His Asn His Tyr Thr Gln Lys Ser Leu
305 310 315 320
Ser Leu Ser Pro

<210> 14
<211> 324
<212> PRT
<213> Artificial Sequence
<220>
<223> modified antibody fragment
<400> 14
Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Asn Phe Gly Thr Gln Thr
65 70 75 80
Tyr Thr Cys Asn Val Asp His Lys Pro Ser Asn Thr Lys Val Asp Lys
85 90 95
Thr Val Glu Arg Lys Ser Cys Val Glu Cys Pro Pro Cys Pro Ala Pro
100 105 110
Pro Val Ala Gly Pro Ser Val Phe Leu Phe Pro Pro Lys Pro Lys Asp
115 120 125
Thr Leu Met Ile Ser Arg Thr Pro Glu Val Thr Cys Val Val Val Asp
130 135 140
Val Ser Gln Glu Asp Pro Glu Val Gln Phe Asn Trp Tyr Val Asp Gly
145 150 155 160
Val Glu Val His Asn Ala Lys Thr Lys Pro Arg Glu Glu Gln Phe Asn
165 170 175
Ser Thr Phe Arg Val Val Ser Val Leu Thr Val Val His Gln Asp Trp
180 185 190
Leu Asn Gly Lys Glu Tyr Lys Cys Lys Val Ser Asn Lys Gly Leu Pro

```
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Gln Glu Glu Met Thr Lys Asn
225                 230                 235                 240
Gln Val Ser Leu Thr Cys Leu Val Lys Gly Phe Tyr Pro Ser Asp Ile
                245                 250                 255
Ala Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Glu Gly Asn Val Phe Ser Cys
    290                 295                 300
Ser Val Met His Glu Ala Leu His Asn His Tyr Thr Gln Lys Ser Leu
305                 310                 315                 320
Ser Leu Ser Pro
```

<210> 15
<211> 326
<212> PRT
<213> Artificial Sequence
<220>
<223> modified antibody fragment
<400> 15

```
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg Lys Cys Cys Val Glu Cys Pro Pro Cys Pro Ala Pro
            100                 105                 110
Pro Val Ala Gly Pro Ser Val Phe Leu Phe Pro Pro Lys Pro Lys Asp
        115                 120                 125
Thr Leu Met Ile Ser Arg Thr Pro Glu Val Thr Cys Val Val Val Asp
    130                 135                 140
Val Ser His Glu Asp Pro Glu Val Gln Phe Asn Trp Tyr Val Asp Gly
145                 150                 155                 160
Val Glu Val His Asn Ala Lys Thr Lys Pro Arg Glu Glu Gln Phe Asn
                165                 170                 175
Ser Thr Phe Arg Val Val Ser Val Leu Thr Val Val His Gln Asp Trp
            180                 185                 190
Leu Asn Gly Lys Glu Tyr Lys Cys Lys Val Ser Asn Lys Gly Leu Pro
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Arg Glu Glu Met Thr Lys Asn
225                 230                 235                 240
Gln Val Ser Leu Thr Cys Leu Val Lys Gly Phe Tyr Pro Ser Asp Ile
                245                 250                 255
Ala Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Gln Gly Asn Val Phe Ser Cys
    290                 295                 300
Ser Val Met His Glu Ala Leu His Ala His Tyr Thr Gln Lys Ser Leu
```

305 310 315 320
Ser Leu Ser Pro Gly Lys
325

<210> 16
<211> 324
<212> PRT
<213> Artificial Sequence
<220>
<223> modified antibody fragment
<400> 16

Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Asn Phe Gly Thr Gln Thr
65 70 75 80
Tyr Thr Cys Asn Val Asp His Lys Pro Ser Asn Thr Lys Val Asp Lys
85 90 95
Thr Val Glu Arg Lys Ser Cys Val Glu Cys Pro Pro Cys Pro Ala Pro
100 105 110
Pro Val Ala Gly Pro Ser Val Phe Leu Phe Pro Pro Lys Pro Lys Asp
115 120 125
Thr Leu Met Ile Ser Arg Thr Pro Glu Val Thr Cys Val Val Val Asp
130 135 140
Val Ser Gln Glu Asp Pro Glu Val Gln Phe Asn Trp Tyr Val Asp Gly
145 150 155 160
Val Glu Val His Asn Ala Lys Thr Lys Pro Arg Glu Glu Gln Phe Asn
165 170 175
Ser Thr Phe Arg Val Val Ser Val Leu Thr Val Val His Gln Asp Trp
180 185 190
Leu Asn Gly Lys Glu Tyr Lys Cys Lys Val Ser Asn Lys Gly Leu Pro
195 200 205
Ala Pro Ile Glu Lys Thr Ile Ser Lys Thr Lys Gly Gln Pro Arg Glu
210 215 220
Pro Gln Val Tyr Thr Leu Pro Pro Ser Gln Glu Glu Met Thr Lys Asn
225 230 235 240
Gln Val Ser Leu Thr Cys Leu Val Lys Gly Phe Tyr Pro Ser Asp Ile
245 250 255
Ala Val Glu Trp Glu Ser Asn Gly Gln Pro Glu Asn Asn Tyr Lys Thr
260 265 270
Thr Pro Pro Met Leu Asp Ser Asp Gly Ser Phe Phe Leu Tyr Ser Lys
275 280 285
Leu Thr Val Asp Lys Ser Arg Trp Gln Glu Gly Asn Val Phe Ser Cys
290 295 300
Ser Val Met His Glu Ala Leu His Ala His Tyr Thr Gln Lys Ser Leu
305 310 315 320
Ser Leu Ser Pro

<210> 17
<211> 182
<212> PRT
<213> homo sapiens
<400> 17

Pro Pro Gly Glu Asp Ser Lys Asp Val Ala Ala Pro His Arg Gln Pro
1 5 10 15
Leu Thr Ser Ser Glu Arg Ile Asp Lys Gln Ile Arg Tyr Ile Leu Asp
20 25 30
Gly Ile Ser Ala Leu Arg Lys Glu Thr Cys Asn Lys Ser Asn Met Cys
35 40 45

Glu Ser Ser Lys Glu Ala Leu Ala Glu Asn Asn Leu Asn Leu Pro Lys
    50                  55                  60
Met Ala Glu Lys Asp Gly Cys Phe Gln Ser Gly Phe Asn Glu Glu Thr
65                  70                  75                  80
Cys Leu Val Lys Ile Ile Thr Gly Leu Leu Glu Phe Glu Val Tyr Leu
                85                  90                  95
Glu Tyr Leu Gln Asn Arg Phe Glu Ser Ser Glu Glu Gln Ala Arg Ala
            100                 105                 110
Val Gln Met Ser Thr Lys Val Leu Ile Gln Phe Leu Gln Lys Lys Ala
        115                 120                 125
Lys Asn Leu Asp Ala Ile Thr Thr Pro Asp Pro Thr Thr Asn Ala Ser
    130                 135                 140
Leu Leu Thr Lys Leu Gln Ala Gln Asn Gln Trp Leu Gln Asp Met Thr
145                 150                 155                 160
Thr His Leu Ile Leu Arg Ser Phe Lys Glu Phe Leu Gln Ser Ser Leu
                165                 170                 175
Arg Ala Leu Arg Gln Met
            180

<210> 18
<211> 4
<212> PRT
<213> Artificial Sequence
<220>
<223> linker
<400> 18
Gly Gly Gly Ser
1

<210> 19
<211> 4
<212> PRT
<213> Artificial Sequence
<220>
<223> linker
<400> 19
Ser Gly Gly Gly
1

<210> 20
<211> 5
<212> PRT
<213> Artificial Sequence
<220>
<223> linker
<400> 20
Gly Gly Gly Gly Ser
1               5

<210> 21
<211> 5
<212> PRT
<213> Artificial Sequence
<220>
<223> linker
<400> 21
Ser Gly Gly Gly Gly
1               5

<210> 22
<211> 6
<212> PRT
<213> Artificial Sequence
<220>
<223> linker
<400> 22
Gly Gly Gly Gly Gly Ser
1               5

<210> 23
<211> 6
<212> PRT
<213> Artificial Sequence
<220>
<223> linker
<400> 23
Ser Gly Gly Gly Gly Gly
1 5
<210> 24
<211> 7
<212> PRT
<213> Artificial Sequence
<220>
<223> linker
<400> 24
Gly Gly Gly Gly Gly Gly Ser
1 5
<210> 25
<211> 7
<212> PRT
<213> Artificial Sequence
<220>
<223> linker
<400> 25
Ser Gly Gly Gly Gly Gly Gly
1 5
<210> 26
<211> 447
<212> PRT
<213> Artificial Sequence
<220>
<223> modified antibody fragment
<400> 26
Gln Val Gln Leu Gln Glu Ser Gly Pro Gly Leu Val Lys Pro Ser Glu
1 5 10 15
Thr Leu Ser Leu Thr Cys Ala Val Ser Gly Tyr Ser Ile Ser Asp Asp
20 25 30
Gln Ala Trp Ser Trp Val Arg Gln Pro Pro Gly Glu Gly Leu Glu Trp
35 40 45
Ile Gly Tyr Ile Ser Tyr Ser Gly Ile Thr Asn Tyr Asn Pro Ser Leu
50 55 60
Lys Gly Arg Val Thr Ile Ser Arg Asp Thr Ser Lys Asn Gln Phe Ser
65 70 75 80
Leu Lys Leu Ser Ser Val Thr Ala Ala Asp Thr Ala Ala Tyr Tyr Cys
85 90 95
Ala Arg Ser Leu Ala Arg Thr Thr Ala Met Asp Tyr Trp Gly Glu Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro

225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285
Ala Lys Thr Lys Pro Arg Glu Glu Gln Tyr Asn Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro
435 440 445

<210> 27
<211> 214
<212> PRT
<213> Artificial Sequence
<220>
<223> modified antibody fragment
<400> 27

Asp Ile Gln Met Thr Gln Ser Pro Ser Ser Leu Ser Ala Ser Val Gly
1 5 10 15
Asp Ser Val Thr Ile Thr Cys Gln Ala Ser Gln Asp Ile Ser Ser Tyr
20 25 30
Leu Asn Trp Tyr Gln Gln Lys Pro Gly Lys Ala Pro Glu Leu Leu Ile
35 40 45
Tyr Tyr Gly Ser Glu Leu His Ser Gly Val Pro Ser Arg Phe Ser Gly
50 55 60
Ser Gly Ser Gly Thr Asp Phe Thr Phe Thr Ile Ser Ser Leu Glu Ala
65 70 75 80
Glu Asp Ala Ala Thr Tyr Tyr Cys Gln Gln Gly Asn Ser Leu Pro Tyr
85 90 95
Thr Phe Gly Gln Gly Thr Lys Val Glu Ile Glu Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 28

<211> 447
<212> PRT
<213> Artificial Sequence
<220>
<223> modified antibody fragment
<400> 28

```
Gln Val Gln Leu Gln Glu Ser Gly Pro Gly Leu Val Lys Pro Ser Glu
1               5                   10                  15
Thr Leu Ser Leu Thr Cys Ala Val Ser Gly His Ser Ile Ser His Asp
            20                  25                  30
His Ala Trp Ser Trp Val Arg Gln Pro Pro Gly Glu Gly Leu Glu Trp
        35                  40                  45
Ile Gly Phe Ile Ser Tyr Ser Gly Ile Thr Asn Tyr Asn Pro Ser Leu
    50                  55                  60
Gln Gly Arg Val Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Ser Leu Ala Arg Thr Thr Ala Met Asp Tyr Trp Gly Glu Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro
        435                 440                 445
```

<210> 29

<211> 214
<212> PRT
<213> Artificial Sequence
<220>
<223> modified antibody fragment
<400> 29

```
Asp Ile Gln Met Thr Gln Ser Pro Ser Ser Leu Ser Ala Ser Val Gly
1               5                   10                  15
Asp Ser Val Thr Ile Thr Cys Gln Ala Ser Thr Asp Ile Ser Ser His
            20                  25                  30
Leu Asn Trp Tyr Gln Gln Lys Pro Gly Lys Ala Pro Glu Leu Leu Ile
        35                  40                  45
Tyr Tyr Gly Ser His Leu Leu Ser Gly Val Pro Ser Arg Phe Ser Gly
    50                  55                  60
Ser Gly Ser Gly Thr Asp Phe Thr Phe Thr Ile Ser Ser Leu Glu Ala
65                  70                  75                  80
Glu Asp Ala Ala Thr Tyr Tyr Cys Gly Gln Gly Asn Arg Leu Pro Tyr
                85                  90                  95
Thr Phe Gly Gln Gly Thr Lys Val Glu Ile Glu Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 30
<211> 328
<212> PRT
<213> Artificial Sequence
<220>
<223> modified antibody fragment
<400> 30

```
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
```

Glu Gln Tyr Ala Ser Thr Tyr Arg Val Val Ser Val Leu Thr Val Leu
180 185 190
His Gln Asp Trp Leu Asn Gly Lys Glu Tyr Lys Cys Lys Val Ser Asn
195 200 205
Lys Ala Leu Pro Ala Pro Ile Glu Lys Thr Ile Ser Lys Ala Lys Gly
210 215 220
Gln Pro Arg Glu Pro Gln Val Tyr Thr Leu Pro Pro Ser Arg Asp Glu
225 230 235 240
Leu Thr Lys Asn Gln Val Ser Leu Thr Cys Leu Val Lys Gly Phe Tyr
245 250 255
Pro Ser Asp Ile Ala Val Glu Trp Glu Ser Asn Gly Gln Pro Glu Asn
260 265 270
Asn Tyr Lys Thr Thr Pro Pro Val Leu Asp Ser Asp Gly Ser Phe Phe
275 280 285
Leu Tyr Ser Lys Leu Thr Val Asp Lys Ser Arg Trp Gln Gln Gly Asn
290 295 300
Val Phe Ser Cys Ser Val Met His Glu Ala Leu His Asn His Tyr Thr
305 310 315 320
Gln Lys Ser Leu Ser Leu Ser Pro
325

<210> 31
<211> 447
<212> PRT
<213> Artificial Sequence
<220>
<223> modified antibody fragment
<400> 31

Gln Val Gln Leu Gln Glu Ser Gly Pro Gly Leu Val Lys Pro Ser Glu
1 5 10 15
Thr Leu Ser Leu Thr Cys Ala Val Ser Gly His Ser Ile Ser His Asp
20 25 30
His Ala Trp Ser Trp Val Arg Gln Pro Pro Gly Glu Gly Leu Glu Trp
35 40 45
Ile Gly Phe Ile Ser Tyr Ser Gly Ile Thr Asn Tyr Asn Pro Ser Leu
50 55 60
Gln Gly Arg Val Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Arg Ser Leu Ala Arg Thr Thr Ala Met Asp Tyr Trp Gly Glu Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285

Ala Lys Thr Lys Pro Arg Glu Glu Gln Tyr Ala Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro
435 440 445

<210> 32
<211> 447
<212> PRT
<213> Artificial Sequence
<220>
<223> modified antibody fragment
<400> 32

Gln Val Gln Leu Gln Glu Ser Gly Pro Gly Leu Val Lys Pro Ser Glu
1 5 10 15
Thr Leu Ser Leu Thr Cys Ala Val Ser Gly His Ser Ile Ser His Asp
20 25 30
His Ala Trp Ser Trp Val Arg Gln Pro Pro Gly Glu Gly Leu Glu Trp
35 40 45
Ile Gly Phe Ile Ser Tyr Ser Gly Ile Thr Asn Tyr Asn Pro Ser Leu
50 55 60
Gln Gly Arg Val Thr Ile Ser Arg Asp Asn Ser Asn Asn Thr Leu Tyr
65 70 75 80
Leu Gln Met Asn Ser Leu Arg Ala Glu Asp Thr Ala Val Tyr Tyr Cys
85 90 95
Ala Arg Ser Leu Ala Arg Thr Thr Ala Met Asp Tyr Trp Gly Glu Gly
100 105 110
Thr Leu Val Thr Val Ser Ser Ala Ser Thr Lys Gly Pro Ser Val Phe
115 120 125
Pro Leu Ala Pro Ser Ser Lys Ser Thr Ser Gly Gly Thr Ala Ala Leu
130 135 140
Gly Cys Leu Val Lys Asp Tyr Phe Pro Glu Pro Val Thr Val Ser Trp
145 150 155 160
Asn Ser Gly Ala Leu Thr Ser Gly Val His Thr Phe Pro Ala Val Leu
165 170 175
Gln Ser Ser Gly Leu Tyr Ser Leu Ser Ser Val Val Thr Val Pro Ser
180 185 190
Ser Ser Leu Gly Thr Gln Thr Tyr Ile Cys Asn Val Asn His Lys Pro
195 200 205
Ser Asn Thr Lys Val Asp Lys Lys Val Glu Pro Lys Ser Cys Asp Lys
210 215 220
Thr His Thr Cys Pro Pro Cys Pro Ala Pro Glu Leu Leu Gly Gly Pro
225 230 235 240
Ser Val Phe Leu Phe Pro Pro Lys Pro Lys Asp Thr Leu Met Ile Ser
245 250 255
Arg Thr Pro Glu Val Thr Cys Val Val Val Asp Val Ser His Glu Asp
260 265 270
Pro Glu Val Lys Phe Asn Trp Tyr Val Asp Gly Val Glu Val His Asn
275 280 285

Ala Lys Thr Lys Pro Arg Glu Glu Gln Tyr Ala Ser Thr Tyr Arg Val
290 295 300
Val Ser Val Leu Thr Val Leu His Gln Asp Trp Leu Asn Gly Lys Glu
305 310 315 320
Tyr Lys Cys Lys Val Ser Asn Lys Ala Leu Pro Ala Pro Ile Glu Lys
325 330 335
Thr Ile Ser Lys Ala Lys Gly Gln Pro Arg Glu Pro Gln Val Tyr Thr
340 345 350
Leu Pro Pro Ser Arg Asp Glu Leu Thr Lys Asn Gln Val Ser Leu Thr
355 360 365
Cys Leu Val Lys Gly Phe Tyr Pro Ser Asp Ile Ala Val Glu Trp Glu
370 375 380
Ser Asn Gly Gln Pro Glu Asn Asn Tyr Lys Thr Thr Pro Pro Val Leu
385 390 395 400
Asp Ser Asp Gly Ser Phe Phe Leu Tyr Ser Lys Leu Thr Val Asp Lys
405 410 415
Ser Arg Trp Gln Gln Gly Asn Val Phe Ser Cys Ser Val Met His Glu
420 425 430
Ala Leu His Asn His Tyr Thr Gln Lys Ser Leu Ser Leu Ser Pro
435 440 445

<210> 33
<211> 214
<212> PRT
<213> Artificial Sequence
<220>
<223> modified antibody fragment
<400> 33

Asp Ile Gln Met Thr Gln Ser Pro Ser Ser Leu Ser Ala Ser Val Gly
1 5 10 15
Asp Asn Val Thr Ile Thr Cys Gln Ala Ser Thr Asp Ile Ser Ser His
20 25 30
Leu Asn Trp Tyr Gln Gln Lys Pro Gly Lys Ala Pro Glu Leu Leu Ile
35 40 45
Tyr Tyr Gly Ser His Leu Leu Ser Gly Val Pro Ser Arg Phe Ser Gly
50 55 60
Ser Gly Ser Gly Thr Asp Phe Thr Phe Thr Ile Ser Ser Leu Glu Ala
65 70 75 80
Glu Asp Ala Ala Thr Tyr Tyr Cys Gly Gln Gly Asn Arg Leu Pro Tyr
85 90 95
Thr Phe Gly Gln Gly Thr Lys Val Glu Ile Glu Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 34
<211> 214
<212> PRT
<213> Artificial Sequence
<220>
<223> modified antibody fragment
<400> 34

Asp Ile Gln Met Thr Gln Ser Pro Ser Ser Leu Ser Ala Ser Val Gly

1 5 10 15
Asp Ser Val Asn Ile Thr Cys Gln Ala Ser Thr Asp Ile Ser Ser His
20 25 30
Leu Asn Trp Tyr Gln Gln Lys Pro Gly Lys Ala Pro Glu Leu Leu Ile
35 40 45
Tyr Tyr Gly Ser His Leu Leu Ser Gly Val Pro Ser Arg Phe Ser Gly
50 55 60
Ser Gly Ser Gly Thr Asp Phe Thr Phe Thr Ile Ser Ser Leu Glu Ala
65 70 75 80
Glu Asp Ala Ala Thr Tyr Tyr Cys Gly Gln Gly Asn Arg Leu Pro Tyr
85 90 95
Thr Phe Gly Gln Gly Thr Lys Val Glu Ile Glu Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 35
<211> 214
<212> PRT
<213> Artificial Sequence
<220>
<223> modified antibody fragment
<400> 35

Asp Ile Gln Met Thr Gln Ser Pro Ser Ser Leu Ser Ala Ser Val Gly
1 5 10 15
Asp Ser Val Thr Ile Thr Cys Asn Ala Ser Thr Asp Ile Ser Ser His
20 25 30
Leu Asn Trp Tyr Gln Gln Lys Pro Gly Lys Ala Pro Glu Leu Leu Ile
35 40 45
Tyr Tyr Gly Ser His Leu Leu Ser Gly Val Pro Ser Arg Phe Ser Gly
50 55 60
Ser Gly Ser Gly Thr Asp Phe Thr Phe Thr Ile Ser Ser Leu Glu Ala
65 70 75 80
Glu Asp Ala Ala Thr Tyr Tyr Cys Gly Gln Gly Asn Arg Leu Pro Tyr
85 90 95
Thr Phe Gly Gln Gly Thr Lys Val Glu Ile Glu Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 36

<211> 214
<212> PRT
<213> Artificial Sequence
<220>
<223> modified antibody fragment
<400> 36

```
Asp Ile Gln Met Thr Gln Ser Pro Ser Ser Leu Ser Ala Ser Val Gly
1               5                   10                  15
Asp Asn Val Thr Ile Thr Cys Asn Ala Ser Thr Asp Ile Ser Ser His
            20                  25                  30
Leu Asn Trp Tyr Gln Gln Lys Pro Gly Lys Ala Pro Glu Leu Leu Ile
        35                  40                  45
Tyr Tyr Gly Ser His Leu Leu Ser Gly Val Pro Ser Arg Phe Ser Gly
    50                  55                  60
Ser Gly Ser Gly Thr Asp Phe Thr Phe Thr Ile Ser Ser Leu Glu Ala
65                  70                  75                  80
Glu Asp Ala Ala Thr Tyr Tyr Cys Gly Gln Gly Asn Arg Leu Pro Tyr
                85                  90                  95
Thr Phe Gly Gln Gly Thr Lys Val Glu Ile Glu Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 37
<211> 449
<212> PRT
<213> Artificial Sequence
<220>
<223> modified antibody fragment
<400> 37

```
Gln Val Gln Leu Gln Glu Ser Gly Pro Gly Leu Val Arg Pro Ser Gln
1               5                   10                  15
Thr Leu Ser Leu Thr Cys Thr Val Ser Gly Tyr Ser Ile Thr Ser Asp
            20                  25                  30
His Ala Trp Ser Trp Val Arg Gln Pro Pro Gly Arg Gly Leu Glu Trp
        35                  40                  45
Ile Gly Tyr Ile Ser Tyr Ser Gly Ile Thr Thr Tyr Asn Pro Ser Leu
    50                  55                  60
Lys Ser Arg Val Thr Met Leu Arg Asp Thr Ser Lys Asn Gln Phe Ser
65                  70                  75                  80
Leu Arg Leu Ser Ser Val Thr Ala Ala Asp Thr Ala Val Tyr Tyr Cys
                85                  90                  95
Ala Arg Ser Leu Ala Arg Thr Thr Ala Met Asp Tyr Trp Gly Gln Gly
            100                 105                 110
Ser Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
```

```
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210>  38
<211>  214
<212>  PRT
<213>  Artificial Sequence
<220>
<223>  modified antibody fragment
<400>  38
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Ser Tyr
            20                  25                  30
Leu Asn Trp Tyr Gln Gln Lys Pro Gly Lys Ala Pro Lys Leu Leu Ile
        35                  40                  45
Tyr Tyr Thr Ser Arg Leu His Ser Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Gly Asn Thr Leu Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
```

Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 39
<211> 217
<212> PRT
<213> Artificial Sequence
<220>
<223> modified antibody fragment
<400> 39

Gln Ser Val Leu Thr Gln Pro Pro Ser Val Ser Gly Ala Pro Gly Gln
1 5 10 15
Arg Val Thr Ile Ser Cys Thr Gly Ser Arg Ser Asn Met Gly Ala Gly
20 25 30
Tyr Asp Val His Trp Tyr Gln Leu Leu Pro Gly Ala Ala Pro Lys Leu
35 40 45
Leu Ile Ser His Asn Thr His Arg Pro Ser Gly Val Pro Asp Arg Phe
50 55 60
Ser Gly Ser Lys Ser Gly Ala Ser Ala Ser Leu Ala Ile Thr Gly Leu
65 70 75 80
Gln Ala Glu Asp Glu Ala Asp Tyr Tyr Cys Gln Ser His Asp Ser Ser
85 90 95
Leu Ser Ala Val Val Phe Gly Gly Gly Thr Lys Leu Thr Val Leu Ser
100 105 110
Gln Pro Lys Ala Ala Pro Ser Val Thr Leu Phe Pro Pro Ser Ser Glu
115 120 125
Glu Leu Gln Ala Asn Lys Ala Thr Leu Val Cys Leu Ile Ser Asp Phe
130 135 140
Tyr Pro Gly Ala Val Thr Val Ala Trp Lys Ala Asp Ser Ser Pro Val
145 150 155 160
Lys Ala Gly Val Glu Thr Thr Thr Pro Ser Lys Gln Ser Asn Asn Lys
165 170 175
Tyr Ala Ala Ser Ser Tyr Leu Ser Leu Thr Pro Glu Gln Trp Lys Ser
180 185 190
His Arg Ser Tyr Ser Cys Gln Val Thr His Glu Gly Ser Thr Val Glu
195 200 205
Lys Thr Val Ala Pro Thr Glu Cys Ser
210 215

<210> 40
<211> 125
<212> PRT
<213> Artificial Sequence
<220>
<223> modified antibody fragment
<400> 40

Gln Val Gln Leu Val Gln Ser Gly Ala Glu Val Lys Lys Pro Gly Ser
1 5 10 15
Ser Val Lys Val Ser Cys Lys Ala Ser Gly Gly Thr Phe Ser Ser Tyr
20 25 30
Ala Ile Ser Trp Val Arg Gln Ala Pro Gly Gln Gly Leu Glu Trp Met
35 40 45
Gly Gly Ile Ile Pro Ile Phe Gly Thr Ala Asn Tyr Ala Gln Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Glu Arg Asp Tyr Tyr Asp Ser Ser Gly Tyr Tyr Asp Ala Phe

100 105 110
Asp Ile Trp Gly Gln Gly Thr Met Val Thr Val Ser Ser
115 120 125

<210> 41
<211> 107
<212> PRT
<213> Artificial Sequence
<220>
<223> modified antibody fragment
<400> 41

Glu Thr Thr Val Thr Gln Ser Pro Ser Phe Leu Ser Ala Ser Val Gly
1 5 10 15
Asp Arg Val Thr Ile Thr Cys Ile Thr Thr Thr Asp Ile Asp Asp Asp
20 25 30
Met Asn Trp Phe Gln Gln Glu Pro Gly Lys Ala Pro Lys Leu Leu Ile
35 40 45
Ser Glu Gly Asn Ile Leu Arg Pro Gly Val Pro Ser Arg Phe Ser Ser
50 55 60
Ser Gly Tyr Gly Thr Asp Phe Thr Leu Thr Ile Ser Lys Leu Gln Pro
65 70 75 80
Glu Asp Phe Ala Thr Tyr Tyr Cys Leu Gln Ser Asp Asn Leu Pro Phe
85 90 95
Thr Phe Gly Gln Gly Thr Lys Leu Glu Ile Lys
100 105

<210> 42
<211> 107
<212> PRT
<213> Artificial Sequence
<220>
<223> modified antibody fragment
<400> 42

Arg Thr Val Ala Ala Pro Ser Val Phe Ile Phe Pro Pro Ser Asp Glu
1 5 10 15
Gln Leu Lys Ser Gly Thr Ala Ser Val Val Cys Leu Leu Asn Asn Phe
20 25 30
Tyr Pro Arg Glu Ala Lys Val Gln Trp Lys Val Asp Asn Ala Leu Gln
35 40 45
Ser Gly Asn Ser Gln Glu Ser Val Thr Glu Gln Asp Ser Lys Asp Ser
50 55 60
Thr Tyr Ser Leu Ser Ser Thr Leu Thr Leu Ser Lys Ala Asp Tyr Glu
65 70 75 80
Lys His Lys Val Tyr Ala Cys Glu Val Thr His Gln Gly Leu Ser Ser
85 90 95
Pro Val Thr Lys Ser Phe Asn Arg Gly Glu Cys
100 105

<210> 43
<211> 106
<212> PRT
<213> Artificial Sequence
<220>
<223> modified antibody fragment
<400> 43

Glu Ile Val Leu Thr Gln Ser Pro Ala Thr Leu Ser Leu Ser Pro Gly
1 5 10 15
Glu Arg Ala Thr Leu Ser Cys Arg Ala Ser Gln Ser Val Ser Ser Tyr
20 25 30
Leu Ala Trp Tyr Gln Gln Lys Pro Gly Gln Ala Pro Arg Leu Leu Ile
35 40 45
Phe Asp Ala Ser Asn Arg Ala Ala Gly Ile Pro Ala Arg Phe Ser Gly
50 55 60
Ser Gly Ser Gly Thr Asp Phe Thr Leu Thr Ile Ser Ser Leu Glu Pro
65 70 75 80
Glu Asp Phe Ala Val Tyr Tyr Cys Gln Gln Phe Asp Lys Trp Val Thr

```
                85                  90                  95
Phe Gly Gly Gly Thr Thr Val Glu Ile Arg
            100                 105
```

<210> 44
<211> 454
<212> PRT
<213> Artificial Sequence
<220>
<223> modified antibody fragment
<400> 44

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Glu Met Asn Trp Val Arg Gln Ala Pro Gly Lys Gly Leu Glu Trp Val
        35                  40                  45
Ser Tyr Ile Ser Ser Ser Gly Ser Thr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Ala Pro Tyr Tyr Tyr Asp Ser Ser Gly Tyr Thr Asp Ala
            100                 105                 110
Phe Asp Ile Trp Gly Gln Gly Thr Met Val Thr Val Ser Ser Ala Ser
        115                 120                 125
Thr Lys Gly Pro Ser Val Phe Pro Leu Ala Pro Ser Ser Lys Ser Thr
    130                 135                 140
Ser Gly Gly Thr Ala Ala Leu Gly Cys Leu Val Lys Asp Tyr Phe Pro
145                 150                 155                 160
Glu Pro Val Thr Val Ser Trp Asn Ser Gly Ala Leu Thr Ser Gly Val
                165                 170                 175
His Thr Phe Pro Ala Val Leu Gln Ser Ser Gly Leu Tyr Ser Leu Ser
            180                 185                 190
Ser Val Val Thr Val Pro Ser Ser Ser Leu Gly Thr Gln Thr Tyr Ile
        195                 200                 205
Cys Asn Val Asn His Lys Pro Ser Asn Thr Lys Val Asp Lys Lys Val
    210                 215                 220
Glu Pro Lys Ser Cys Asp Lys Thr His Thr Cys Pro Pro Cys Pro Ala
225                 230                 235                 240
Pro Glu Leu Leu Gly Gly Pro Ser Val Phe Leu Phe Pro Pro Lys Pro
                245                 250                 255
Lys Asp Thr Leu Met Ile Ser Arg Thr Pro Glu Val Thr Cys Val Val
            260                 265                 270
Val Asp Val Ser His Glu Asp Pro Glu Val Lys Phe Asn Trp Tyr Val
        275                 280                 285
Asp Gly Val Glu Val His Asn Ala Lys Thr Lys Pro Arg Glu Glu Gln
    290                 295                 300
Tyr Asn Ser Thr Tyr Arg Val Val Ser Val Leu Thr Val Leu His Gln
305                 310                 315                 320
Asp Trp Leu Asn Gly Lys Glu Tyr Lys Cys Lys Val Ser Asn Lys Ala
                325                 330                 335
Leu Pro Ala Pro Ile Glu Lys Thr Ile Ser Lys Ala Lys Gly Gln Pro
            340                 345                 350
Arg Glu Pro Gln Val Tyr Thr Leu Pro Pro Ser Arg Asp Glu Leu Thr
        355                 360                 365
Lys Asn Gln Val Ser Leu Thr Cys Leu Val Lys Gly Phe Tyr Pro Ser
    370                 375                 380
Asp Ile Ala Val Glu Trp Glu Ser Asn Gly Gln Pro Glu Asn Asn Tyr
385                 390                 395                 400
Lys Thr Thr Pro Pro Val Leu Asp Ser Asp Gly Ser Phe Phe Leu Tyr
                405                 410                 415
Ser Lys Leu Thr Val Asp Lys Ser Arg Trp Gln Gln Gly Asn Val Phe
```

420 425 430
Ser Cys Ser Val Met His Glu Ala Leu His Asn His Tyr Thr Gln Lys
435 440 445
Ser Leu Ser Leu Ser Pro
450

<210> 45
<211> 213
<212> PRT
<213> Artificial Sequence
<220>
<223> modified antibody fragment
<400> 45

Glu Ile Val Leu Thr Gln Ser Pro Ala Thr Leu Ser Leu Ser Pro Gly
1 5 10 15
Glu Arg Ala Thr Leu Ser Cys Arg Ala Ser Gln Ser Val Ser Ser Tyr
20 25 30
Leu Ala Trp Tyr Gln Gln Lys Pro Gly Gln Ala Pro Arg Leu Leu Ile
35 40 45
Phe Asp Ala Ser Asn Arg Ala Ala Gly Ile Pro Ala Arg Phe Ser Gly
50 55 60
Ser Gly Ser Gly Thr Asp Phe Thr Leu Thr Ile Ser Ser Leu Glu Pro
65 70 75 80
Glu Asp Phe Ala Val Tyr Tyr Cys Gln Gln Phe Asp Lys Trp Val Thr
85 90 95
Phe Gly Gly Gly Thr Thr Val Glu Ile Arg Arg Thr Val Ala Ala Pro
100 105 110
Ser Val Phe Ile Phe Pro Pro Ser Asp Glu Gln Leu Lys Ser Gly Thr
115 120 125
Ala Ser Val Val Cys Leu Leu Asn Asn Phe Tyr Pro Arg Glu Ala Lys
130 135 140
Val Gln Trp Lys Val Asp Asn Ala Leu Gln Ser Gly Asn Ser Gln Glu
145 150 155 160
Ser Val Thr Glu Gln Asp Ser Lys Asp Ser Thr Tyr Ser Leu Ser Ser
165 170 175
Thr Leu Thr Leu Ser Lys Ala Asp Tyr Glu Lys His Lys Val Tyr Ala
180 185 190
Cys Glu Val Thr His Gln Gly Leu Ser Ser Pro Val Thr Lys Ser Phe
195 200 205
Asn Arg Gly Glu Cys
210

<210> 46
<211> 454
<212> PRT
<213> Artificial Sequence
<220>
<223> modified antibody fragment
<400> 46

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Ala Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Asp Ala Pro Tyr Tyr Tyr Asp Ser Ser Gly Tyr Thr Asp Ala
100 105 110
Phe Asp Ile Trp Gly Gln Gly Thr Met Val Thr Val Ser Ser Ala Ser
115 120 125

Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro
450

<210> 47
<211> 454
<212> PRT
<213> Artificial Sequence
<220>
<223> modified antibody fragment
<400> 47

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Ala Ala Pro Tyr Tyr Tyr Asp Ser Ser Gly Tyr Thr Asp Ala
100 105 110

Phe Asp Ile Trp Gly Gln Gly Thr Met Val Thr Val Ser Ser Ala Ser
115 120 125
Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro
450

<210> 48
<211> 454
<212> PRT
<213> Artificial Sequence
<220>
<223> modified antibody fragment
<400> 48

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Asp Ser Val
50 55 60
Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95

Ala Arg Asp Ala Pro Tyr Tyr Tyr Ala Ser Ser Gly Tyr Thr Asp Ala
100 105 110
Phe Asp Ile Trp Gly Gln Gly Thr Met Val Thr Val Ser Ser Ala Ser
115 120 125
Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro
450

<210> 49
<211> 454
<212> PRT
<213> Artificial Sequence
<220>
<223> modified antibody fragment
<400> 49

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Asp Ser Val
50 55 60
Lys Gly Arg Phe Thr Ile Ser Arg Asp Asn Ala Lys Asn Ser Leu Tyr
65 70 75 80

Leu Gln Met Asn Ser Leu Arg Ala Glu Asp Thr Ala Val Tyr Tyr Cys
85 90 95
Ala Arg Asp Ala Pro Tyr Tyr Tyr Asp Ser Ser Gly Tyr Thr Ala Ala
100 105 110
Phe Asp Ile Trp Gly Gln Gly Thr Met Val Thr Val Ser Ser Ala Ser
115 120 125
Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro
450

<210> 50
<211> 454
<212> PRT
<213> Artificial Sequence
<220>
<223> modified antibody fragment
<400> 50

Glu Val Gln Leu Val Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Glu Met Asn Trp Val Arg Gln Ala Pro Gly Lys Gly Leu Glu Trp Val
35 40 45
Ser Tyr Ile Ser Ser Ser Gly Ser Thr Ile Tyr Tyr Ala Asp Ser Val
50 55 60

Lys Gly Arg Phe Thr Ile Ser Arg Asp Asn Ala Lys Asn Ser Leu Tyr
65 70 75 80
Leu Gln Met Asn Ser Leu Arg Ala Glu Asp Thr Ala Val Tyr Tyr Cys
85 90 95
Ala Arg Asp Ala Pro Tyr Tyr Tyr Asp Ser Ser Gly Tyr Thr Asp Ala
100 105 110
Phe Ala Ile Trp Gly Gln Gly Thr Met Val Thr Val Ser Ser Ala Ser
115 120 125
Thr Lys Gly Pro Ser Val Phe Pro Leu Ala Pro Ser Ser Lys Ser Thr
130 135 140
Ser Gly Gly Thr Ala Ala Leu Gly Cys Leu Val Lys Asp Tyr Phe Pro
145 150 155 160
Glu Pro Val Thr Val Ser Trp Asn Ser Gly Ala Leu Thr Ser Gly Val
165 170 175
His Thr Phe Pro Ala Val Leu Gln Ser Ser Gly Leu Tyr Ser Leu Ser
180 185 190
Ser Val Val Thr Val Pro Ser Ser Ser Leu Gly Thr Gln Thr Tyr Ile
195 200 205
Cys Asn Val Asn His Lys Pro Ser Asn Thr Lys Val Asp Lys Lys Val
210 215 220
Glu Pro Lys Ser Cys Asp Lys Thr His Thr Cys Pro Pro Cys Pro Ala
225 230 235 240
Pro Glu Leu Leu Gly Gly Pro Ser Val Phe Leu Phe Pro Pro Lys Pro
245 250 255
Lys Asp Thr Leu Met Ile Ser Arg Thr Pro Glu Val Thr Cys Val Val
260 265 270
Val Asp Val Ser His Glu Asp Pro Glu Val Lys Phe Asn Trp Tyr Val
275 280 285
Asp Gly Val Glu Val His Asn Ala Lys Thr Lys Pro Arg Glu Glu Gln
290 295 300
Tyr Asn Ser Thr Tyr Arg Val Val Ser Val Leu Thr Val Leu His Gln
305 310 315 320
Asp Trp Leu Asn Gly Lys Glu Tyr Lys Cys Lys Val Ser Asn Lys Ala
325 330 335
Leu Pro Ala Pro Ile Glu Lys Thr Ile Ser Lys Ala Lys Gly Gln Pro
340 345 350
Arg Glu Pro Gln Val Tyr Thr Leu Pro Pro Ser Arg Asp Glu Leu Thr
355 360 365
Lys Asn Gln Val Ser Leu Thr Cys Leu Val Lys Gly Phe Tyr Pro Ser
370 375 380
Asp Ile Ala Val Glu Trp Glu Ser Asn Gly Gln Pro Glu Asn Asn Tyr
385 390 395 400
Lys Thr Thr Pro Pro Val Leu Asp Ser Asp Gly Ser Phe Phe Leu Tyr
405 410 415
Ser Lys Leu Thr Val Asp Lys Ser Arg Trp Gln Gln Gly Asn Val Phe
420 425 430
Ser Cys Ser Val Met His Glu Ala Leu His Asn His Tyr Thr Gln Lys
435 440 445
Ser Leu Ser Leu Ser Pro
450

<210> 51
<211> 213
<212> PRT
<213> Artificial Sequence
<220>
<223> modified antibody fragment
<400> 51

Glu Ile Val Leu Thr Gln Ser Pro Ala Thr Leu Ser Leu Ser Pro Gly
1 5 10 15
Glu Arg Ala Thr Leu Ser Cys Arg Ala Ser Gln Ser Val Ser Ser Tyr
20 25 30
Leu Ala Trp Tyr Gln Gln Lys Pro Gly Gln Ala Pro Arg Leu Leu Ile
35 40 45

Phe Ala Ala Ser Asn Arg Ala Ala Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Phe Asp Lys Trp Val Thr
                85                  90                  95
Phe Gly Gly Gly Thr Thr Val Glu Ile Arg Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

<210> 52
<211> 213
<212> PRT
<213> Artificial Sequence
<220>
<223> modified antibody fragment
<400> 52

Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Phe Asp Ala Ser Asn Arg Ala Ala Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Phe Ala Lys Trp Val Thr
                85                  90                  95
Phe Gly Gly Gly Thr Thr Val Glu Ile Arg Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

<210> 53
<211> 107
<212> PRT
<213> Artificial Sequence
<220>
<223> modified antibody fragment

<400> 53
Asp Ile Gln Met Thr Gln Ser Pro Ser Ser Leu Ser Ala Ser Val Gly
1 5 10 15
Asp Arg Val Thr Ile Thr Cys Arg Ala Ser Gln Ser Ile Ser Ser Tyr
20 25 30
Leu Asn Trp Tyr Gln Gln Lys Pro Gly Lys Ala Pro Lys Leu Leu Ile
35 40 45
Tyr Ala Ala Ser Ser Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Ser Tyr Ser Thr Pro Phe
85 90 95
Thr Phe Gly Pro Gly Thr Lys Val Asp Ile Lys
100 105

<210> 54
<211> 112
<212> PRT
<213> Artificial Sequence
<220>
<223> modified antibody fragment
<400> 54
Asp Ile Val Met Thr Gln Ser Pro Leu Ser Leu Pro Val Thr Pro Gly
1 5 10 15
Glu Pro Ala Ser Ile Ser Cys Arg Ser Ser Gln Ser Leu Leu His Ser
20 25 30
Asn Gly Asp Asn Tyr Leu Asp Trp Tyr Leu Gln Lys Pro Gly Gln Ser
35 40 45
Pro Gln Leu Leu Ile Tyr Leu Gly Ser Asn Arg Ala Ser Gly Val Pro
50 55 60
Asp Arg Phe Ser Gly Ser Gly Ser Gly Thr Asp Phe Thr Leu Lys Ile
65 70 75 80
Ser Arg Val Glu Ala Glu Asp Val Gly Val Tyr Tyr Cys Met Gln Val
85 90 95
Leu Arg Asn Pro Leu Thr Phe Gly Gly Gly Thr Lys Val Glu Ile Gln
100 105 110

<210> 55
<211> 107
<212> PRT
<213> Artificial Sequence
<220>
<223> modified antibody fragment
<400> 55
Glu Ile Val Met Thr Gln Ser Pro Ala Thr Leu Ser Leu Ser Pro Gly
1 5 10 15
Glu Arg Ala Thr Leu Ser Cys Arg Ala Ser Gln Ser Val Ser Ser Tyr
20 25 30
Leu Ala Trp Tyr Gln Gln Lys Pro Gly Gln Ala Pro Arg Leu Leu Ile
35 40 45
Tyr Asp Ala Ser Asn Arg Ala Thr Gly Ile Pro Ala Arg Phe Ser Gly
50 55 60
Ser Gly Ser Gly Thr Asp Phe Thr Leu Thr Ile Ser Ser Leu Glu Pro
65 70 75 80
Glu Asp Phe Ala Val Tyr Tyr Cys Gln Gln Arg Ser Asn Trp Pro Pro
85 90 95
Thr Phe Gly Gln Gly Thr Lys Val Glu Ile Lys
100 105

<210> 56
<211> 112
<212> PRT
<213> Artificial Sequence
<220>
<223> modified antibody fragment

<400> 56
Asp Ile Val Met Thr Gln Ser Pro Glu Ser Leu Val Leu Ser Leu Gly
1 5 10 15
Gly Thr Ala Thr Ile Asn Cys Arg Ser Ser Gln Ser Val Leu Tyr Ser
20 25 30
Ser Asn Asn Lys Asn Tyr Leu Thr Trp Tyr Gln Gln Lys Pro Gly Gln
35 40 45
Pro Pro Thr Leu Leu Phe Ser Trp Ala Ser Ile Arg Asp Ser Gly Val
50 55 60
Pro Asp Arg Phe Ser Ala Ser Gly Ser Gly Thr Glu Phe Thr Leu Thr
65 70 75 80
Ile Ser Asp Leu Gln Ala Glu Asp Ala Ala Val Tyr Tyr Cys Gln Gln
85 90 95
Tyr Tyr Arg Ala Pro Ser Phe Gly Gln Gly Thr Lys Leu Gln Ile Lys
100 105 110

<210> 57
<211> 128
<212> PRT
<213> Artificial Sequence
<220>
<223> modified antibody fragment
<400> 57
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Ser Tyr
20 25 30
Tyr Met His Trp Val Arg Gln Ala Pro Gly Gln Gly Leu Glu Trp Met
35 40 45
Gly Ile Ile Asn Pro Ser Gly Gly Ser Thr Ser Tyr Ala Gln Lys Phe
50 55 60
Gln Gly Arg Val Thr Met Thr Arg Asp Thr Ser Thr Ser Thr Val Tyr
65 70 75 80
Met Glu Leu Ser Ser Leu Arg Ser Glu Asp Thr Ala Val Tyr Tyr Cys
85 90 95
Ala Arg Asp Gly Thr Leu Tyr Asp Phe Trp Ser Gly Tyr Tyr Ser Tyr
100 105 110
Asp Ala Phe Asp Ile Trp Gly Gln Gly Thr Met Val Thr Val Ser Ser
115 120 125

<210> 58
<211> 122
<212> PRT
<213> Artificial Sequence
<220>
<223> modified antibody fragment
<400> 58
Gln Met Gln Leu Val Gln Ser Gly Ala Glu Val Lys Lys Pro Gly Ser
1 5 10 15
Ser Val Lys Val Ser Cys Lys Ala Ser Gly Gly Thr Phe Ser Ser Tyr
20 25 30
Ala Ile Ser Trp Val Arg Gln Ala Pro Gly Gln Gly Leu Glu Trp Met
35 40 45
Gly Gly Ile Ile Pro Ile Phe Gly Thr Ala Asn Tyr Ala Gln Lys Phe
50 55 60
Gln Gly Arg Val Thr Ile Thr Ala Asp Glu Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Asp Leu Asp Thr Gly Pro Tyr Tyr Tyr Gly Met Asp Val Trp
100 105 110
Gly Gln Gly Thr Met Val Thr Val Ser Ser
115 120

<210> 59
<211> 124

<212> PRT
<213> Artificial Sequence
<220>
<223> modified antibody fragment
<400> 59

```
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met
        35                  40                  45
Gly Gly Ile Ile Pro Ile Phe Gly Thr Ala Asn Tyr Ala Gln Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Ser Pro Val Pro Gly Val Tyr Tyr Tyr Tyr Gly Met Asp
            100                 105                 110
Val Trp Gly Gln Gly Thr Met Val Thr Val Ser Ser
        115                 120
```

<210> 60
<211> 119
<212> PRT
<213> Artificial Sequence
<220>
<223> modified antibody fragment
<400> 60

```
Gln Val Gln Leu Val Gln Ser Gly Ala Glu Val Lys Lys Pro Gly Glu
1               5                   10                  15
Ser Leu Lys Ile Ser Cys Lys Gly Ser Gly Tyr Ser Phe Thr Ser Tyr
            20                  25                  30
Trp Ile Gly Trp Val Arg Gln Met Pro Gly Lys Gly Leu Glu Trp Met
        35                  40                  45
Gly Ile Ile Tyr Pro Gly Asp Ser Asp Thr Arg Tyr Ser Pro Ser Phe
    50                  55                  60
Gln Gly Gln Val Thr Ile Ser Ala Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg His Arg Ala Gly Asp Leu Gly Gly Asp Tyr Trp Gly Gln Gly
            100                 105                 110
Thr Leu Val Thr Val Ser Ser
        115
```

<210> 61
<211> 121
<212> PRT
<213> Artificial Sequence
<220>
<223> modified antibody fragment
<400> 61

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Gly Tyr
            20                  25                  30
Ile Met Asn Trp Val Arg Gln Ala Pro Gly Gln Gly Leu Glu Trp Met
        35                  40                  45
Gly Leu Ile Asn Pro Tyr Asn Gly Gly Thr Asp Tyr Asn Pro Gln Phe
    50                  55                  60
Gln Asp Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
```

Ala Arg Asp Gly Tyr Asp Asp Gly Pro Tyr Thr Leu Glu Thr Trp Gly
100 105 110
Gln Gly Thr Leu Val Thr Val Ser Ser
115 120

<210> 62
<211> 214
<212> PRT
<213> Artificial Sequence
<220>
<223> modified antibody fragment
<400> 62

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Asp Asp Asp
20 25 30
Met Asn Trp Tyr Gln Gln Lys Pro Gly Glu Ala Ala Ile Phe Ile Ile
35 40 45
Gln Glu Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80
Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 63
<211> 445
<212> PRT
<213> Artificial Sequence
<220>
<223> modified antibody fragment
<400> 63

Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Gly Tyr
20 25 30
Ile Met Asn Trp Val Arg Gln Ala Pro Gly Gln Gly Leu Glu Trp Met
35 40 45
Gly Leu Ile Asn Pro Tyr Asn Gly Gly Thr Asp Tyr Asn Pro Gln Phe
50 55 60
Gln Asp Arg Val Thr Ile Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Asp Gly Tyr Asp Asp Gly Pro Tyr Thr Leu Glu Thr Trp Gly
100 105 110
Gln Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala

130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Asn Phe Gly Thr Gln Thr Tyr Thr Cys Asn Val Asp His
195 200 205
Lys Pro Ser Asn Thr Lys Val Asp Lys Thr Val Glu Arg Lys Ser Cys
210 215 220
Val Glu Cys Pro Pro Cys Pro Ala Pro Pro Val Ala Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser Gln Glu Asp Pro Glu
260 265 270
Val Gln Phe Asn Trp Tyr Val Asp Gly Val Glu Val His Asn Ala Lys
275 280 285
Thr Lys Pro Arg Glu Glu Gln Phe Asn Ser Thr Phe Arg Val Val Ser
290 295 300
Val Leu Thr Val Val His Gln Asp Trp Leu Asn Gly Lys Glu Tyr Lys
305 310 315 320
Cys Lys Val Ser Asn Lys Gly Leu Pro Ala Pro Ile Glu Lys Thr Ile
325 330 335
Ser Lys Thr Lys Gly Gln Pro Arg Glu Pro Gln Val Tyr Thr Leu Pro
340 345 350
Pro Ser Gln Glu Glu Met Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Met Leu Asp Ser
385 390 395 400
Asp Gly Ser Phe Phe Leu Tyr Ser Lys Leu Thr Val Asp Lys Ser Arg
405 410 415
Trp Gln Glu Gly Asn Val Phe Ser Cys Ser Val Met His Glu Ala Leu
420 425 430
His Asn His Tyr Thr Gln Lys Ser Leu Ser Leu Ser Pro
435 440 445

<210> 64
<211> 107
<212> PRT
<213> Artificial Sequence
<220>
<223> modified antibody fragment
<400> 64

Asp Ile Gln Met Thr Gln Ser Pro Ser Ser Leu Ser Ala Ser Val Gly
1 5 10 15
Asp Arg Val Thr Ile Thr Cys Lys Ala Ser Gln Asp Ile Asp Asp Asp
20 25 30
Met Asn Trp Tyr Gln Gln Lys Pro Gly Lys Ala Pro Lys Leu Leu Ile
35 40 45
Tyr Glu Ala Thr Thr Leu Val Pro Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Leu Gln His Asp Asn Phe Pro Tyr
85 90 95
Thr Phe Gly Gln Gly Thr Lys Val Glu Ile Lys
100 105

<210> 65
<211> 107
<212> PRT

<213> Artificial Sequence
<220>
<223> modified antibody fragment
<400> 65
Asp Ile Val Met Thr Gln Ser Pro Leu Ser Leu Pro Val Thr Pro Gly
1 5 10 15
Glu Pro Ala Ser Ile Ser Cys Lys Ala Ser Gln Asp Ile Asp Asp Asp
20 25 30
Met Asn Trp Tyr Leu Gln Lys Pro Gly Gln Ser Pro Gln Leu Leu Ile
35 40 45
Tyr Glu Ala Thr Thr Leu Val Pro Gly Val Pro Asp Arg Phe Ser Gly
50 55 60
Ser Gly Ser Gly Thr Asp Phe Thr Leu Lys Ile Ser Arg Val Glu Ala
65 70 75 80
Glu Asp Val Gly Val Tyr Tyr Cys Leu Gln His Asp Asn Phe Pro Tyr
85 90 95
Thr Phe Gly Gln Gly Thr Lys Leu Glu Ile Lys
100 105
<210> 66
<211> 107
<212> PRT
<213> Artificial Sequence
<220>
<223> modified antibody fragment
<400> 66
Glu Ile Val Leu Thr Gln Ser Pro Gly Thr Leu Ser Leu Ser Pro Gly
1 5 10 15
Glu Arg Ala Thr Leu Ser Cys Lys Ala Ser Gln Asp Ile Asp Asp Asp
20 25 30
Met Asn Trp Tyr Gln Gln Lys Pro Gly Gln Ala Pro Arg Leu Leu Ile
35 40 45
Tyr Glu Ala Thr Thr Leu Val Pro Gly Ile Pro Asp Arg Phe Ser Gly
50 55 60
Ser Gly Ser Gly Thr Asp Phe Thr Leu Thr Ile Ser Arg Leu Glu Pro
65 70 75 80
Glu Asp Phe Ala Val Tyr Tyr Cys Leu Gln His Asp Asn Phe Pro Tyr
85 90 95
Thr Phe Gly Gln Gly Thr Lys Val Glu Ile Lys
100 105
<210> 67
<211> 107
<212> PRT
<213> Artificial Sequence
<220>
<223> modified antibody fragment
<400> 67
Asp Ile Val Met Thr Gln Ser Pro Asp Ser Leu Ala Val Ser Leu Gly
1 5 10 15
Glu Arg Ala Thr Ile Asn Cys Lys Ala Ser Gln Asp Ile Asp Asp Asp
20 25 30
Met Asn Trp Tyr Gln Gln Lys Pro Gly Gln Pro Pro Lys Leu Leu Ile
35 40 45
Tyr Glu Ala Thr Thr Leu Val Pro Gly Val Pro Asp Arg Phe Ser Gly
50 55 60
Ser Gly Ser Gly Thr Asp Phe Thr Leu Thr Ile Ser Ser Leu Gln Ala
65 70 75 80
Glu Asp Val Ala Val Tyr Tyr Cys Leu Gln His Asp Asn Phe Pro Tyr
85 90 95
Thr Phe Gly Gln Gly Thr Lys Leu Glu Ile Lys
100 105
<210> 68
<211> 214
<212> PRT

<213> Artificial Sequence
<220>
<223> modified antibody fragment
<400> 68
Glu Thr Thr Leu Thr Gln Ser Pro Ala Phe Met Ser Ala Thr Pro Gly
1               5                   10                  15
Asp Lys Val Thr Ile Ser Cys Lys Ala Ser Gln Asp Ile Ala Asp Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro Tyr
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210> 69
<211> 214
<212> PRT
<213> Artificial Sequence
<220>
<223> modified antibody fragment
<400> 69
Glu Thr Thr Leu Thr Gln Ser Pro Ala Phe Met Ser Ala Thr Pro Gly
1               5                   10                  15
Asp Lys Val Thr Ile Ser Cys Lys Ala Ser Gln Asp Ile Asp Ala Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro Tyr
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
195 200 205
Phe Asn Arg Gly Glu Cys
210

<210> 70
<211> 214
<212> PRT
<213> Artificial Sequence
<220>
<223> modified antibody fragment
<400> 70

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Asp Asp Ala
20 25 30
Met Asn Trp Tyr Gln Gln Lys Pro Gly Glu Ala Ala Ile Phe Ile Ile
35 40 45
Gln Glu Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80
Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 71
<211> 214
<212> PRT
<213> Artificial Sequence
<220>
<223> modified antibody fragment
<400> 71

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Asp Asp Asp
20 25 30
Met Asn Trp Tyr Gln Gln Lys Pro Gly Glu Ala Ala Ile Phe Ile Ile
35 40 45
Gln Ala Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80
Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala

130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 72
<211> 214
<212> PRT
<213> Artificial Sequence
<220>
<223> modified antibody fragment
<400> 72

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Ala Asp Ala
20 25 30
Met Asn Trp Tyr Gln Gln Lys Pro Gly Glu Ala Ala Ile Phe Ile Ile
35 40 45
Gln Glu Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80
Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 73
<211> 214
<212> PRT
<213> Artificial Sequence
<220>
<223> modified antibody fragment
<400> 73

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Ala Asp Asp
20 25 30
Met Asn Trp Tyr Gln Gln Lys Pro Gly Glu Ala Ala Ile Phe Ile Ile
35 40 45
Gln Ala Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80

Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 74
<211> 214
<212> PRT
<213> Artificial Sequence
<220>
<223> modified antibody fragment
<400> 74

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Ala Asp Ala
20 25 30
Met Asn Trp Tyr Gln Gln Lys Pro Gly Glu Ala Ala Ile Phe Ile Ile
35 40 45
Gln Ala Ala Thr Thr Leu Val Pro Gly Ile Ser Pro Arg Phe Ser Gly
50 55 60
Ser Gly Tyr Gly Thr Asp Phe Thr Leu Thr Ile Asn Asn Ile Glu Ser
65 70 75 80
Glu Asp Ala Ala Tyr Tyr Phe Cys Leu Gln His Asp Asn Phe Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 75
<211> 214
<212> PRT
<213> Artificial Sequence
<220>
<223> modified antibody fragment
<400> 75

Glu Thr Thr Leu Thr Gln Ser Pro Ala Phe Met Ser Ala Thr Pro Gly
1 5 10 15
Asp Lys Val Thr Ile Ser Cys Lys Ala Ser Gln Asp Ile Asp Asp Asp

```
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Ala Asn Phe Pro Tyr
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210>  76
<211>  214
<212>  PRT
<213>  Artificial Sequence
<220>
<223>  modified antibody fragment
<400>  76
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Asp Asp Asp
            20                  25                  30
Leu Asn Trp Tyr Gln Gln Lys Pro Gly Lys Ala Pro Lys Leu Leu Ile
        35                  40                  45
Tyr Glu Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Asp Ser Thr Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
<210>  77
<211>  214
<212>  PRT
```

<213> Artificial Sequence
<220>
<223> modified antibody fragment
<400> 77

```
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Tyr Ser Thr Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 78
<211> 214
<212> PRT
<213> Artificial Sequence
<220>
<223> modified antibody fragment
<400> 78

```
Glu Thr Thr Leu Thr Gln Ser Pro Ala Phe Met Ser Ala Thr Pro Gly
1               5                   10                  15
Asp Lys Val Asn Ile Ser Cys Lys Ala Ser Gln Asp Ile Asp Asp Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro Tyr
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
```

```
Ala Cys Glu Val Thr His Gln Gly Leu Ser Ser Pro Val Thr Lys Ser
        195                 200                 205
Phe Asn Arg Gly Glu Cys
    210
<210>  79
<211>  214
<212>  PRT
<213>  Artificial Sequence
<220>
<223>  modified antibody fragment
<400>  79
Glu Thr Thr Leu Thr Gln Ser Pro Ala Phe Met Ser Ala Thr Pro Gly
1               5                   10                  15
Asp Lys Val Asn Ile Ser Cys Lys Ala Ser Gln Asp Ile Glu Asp Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Ser Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro Leu
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

**Description**

Technical Field

Related Application

The present application claims the priority based on Japanese Patent Application Nos. 2011-221400 (filed on Oct. 5, 2011), the contents of which are incorporated herein by reference in their entirety.

Technical Field

The present invention relates to an antigen-binding molecule for promoting uptake of an antigen into a cell, an antigen-binding molecule capable of promoting a decrease of the antigen concentration in plasma, an antigen-binding molecule capable of binding to an antigen a plurality of times, an antigen-binding molecule improved in pharmacokinetics, a pharmaceutical composition containing such an antigen-binding molecule and a method for producing the same.

Background Art

Antibodies have received attention as pharmaceutical agents because of their high stability in plasma and few adverse reactions. Among others, many IgG antibody drugs have already been launched, and a large number of antibody drugs are still under development (Non Patent Literatures 1 and 2). Meanwhile, various techniques applicable to second-generation antibody drugs have been developed. For example, techniques of improving effector functions, antigen-binding ability, pharmacokinetics, or stability or of reducing the risk of immunogenicity have been reported (Non Patent Literature 3). Possible problems of such antibody drugs are the difficult preparation of subcutaneous administration preparations (this is because the antibody drugs are generally administered at very high doses), high production cost, etc. Methods for improving the pharmacokinetics of antibodies and methods for improving the affinity of antibodies for their antigens may be used for reducing the doses of the antibody drugs.

The artificial substitution of amino acids in constant regions has been reported as a method for improving the pharmacokinetics of antibodies (Non Patent Literatures 4 and 5). Previously reported affinity maturation, a technique of enhancing antigen-binding ability and antigen-neutralizing ability (Non Patent Literature 6), involves mutating amino acids in, for example, CDR regions of variable regions, to thereby achieve enhanced antigen-binding activity. Such enhancement in antigen-binding ability can improve biological activity *in vitro* or reduce doses and can further improve drug efficacy *in vivo* (Non Patent Literature 7).

The amount of an antigen that can be neutralized by one antibody molecule depends on affinity. Stronger affinity allows the antibody in a smaller amount to neutralize the antigen. The antibody affinity can be enhanced by various methods (Non Patent Literature 6). An antibody capable of covalently binding to an antigen with infinite affinity would be able to neutralize, by one molecule, one antigen molecule (or two antigens in the case of a divalent antibody). Previous methods, however, have a stoichiometric limitation of neutralization reaction up to one antigen molecule (or two antigens in the case of a divalent antibody) per antibody molecule and are unable to completely neutralize an antigen using an antibody in an amount below the amount of the antigen. In short, these methods have the limited effect of enhancing affinity (Non Patent Literature 9). A given duration of the neutralizing effect of a neutralizing antibody requires administering the antibody in an amount above the amount of an antigen produced *in vivo* for the period. Only the above-mentioned technique for improvement in the pharmacokinetics of antibodies or affinity maturation is not sufficient for reducing the necessary antibody doses. In this respect, one antibody must neutralize a plurality of antigens in order to sustain its antigen-neutralizing effect for the period of interest in an amount below the amount of the antigen. In order to attain this object, an antibody binding to an antigen in a pH-dependent manner has been reported recently as a novel approach (Patent Literature 1). This pH-dependent antigen-binding antibody is strongly associated with an antigen under the neutral condition in plasma and dissociated from the antigen under the acidic condition in endosome. Thus, the pH-dependent antigen-binding antibody can be dissociated from the antigen in endosome. The pH-dependent antigen-binding antibody thus dissociated from the antigen can be reassociated with an antigen after being recycled into plasma by FcRn. This allows one antibody to bind to a plurality of antigens repeatedly.

Retentivity of an antigen in plasma is extremely short, compared to an antibody, which binds to FcRn and is repeatedly used. When such an antibody having long plasma retentivity binds to an antigen thereof, the retentivity of an antibody-antigen complex in plasma becomes long like the antibody. Therefore, an antigen acquires long plasma retentivity by binding to an antibody, with the result that the concentration of the antigen in plasma increases. In this case, even if the affinity of an antibody for an antigen is improved, clearance of the antigen from plasma cannot be promoted. The aforementioned pH-dependent antigen-binding antibody is reported to be effective as a method for promoting clearance of an antigen from plasma compared to general antibodies (Patent Literature 1).

As described above, the pH-dependent antigen-binding antibody solely binds to a plurality of antigen molecules and thus can promote clearance of the antigen molecules from plasma, compared to general antibodies. Therefore, the pH-dependent antigen-binding antibody has a function that cannot be attained by general antibodies. However, up until now, antibody-engineering techniques for further improving an effect of a pH-dependent antigen-binding antibody in repeatedly binding to an antigen and an effect of an antibody in promoting clearance of an antigen from plasma have not yet been reported.

In the meantime, a glycoprotein present in plasma binds to a sugar chain specific receptor and disappears from the plasma (Non Patent Literature 10). At this time, the glycoprotein binds to a sugar chain receptor present in a cell surface and is taken up into a cell. In the cell, the glycoprotein is dissociated from the sugar chain receptor and decomposed by lysosome; whereas, the sugar chain receptor is known to return to the cell surface for recycle use. More specifically, a glycoprotein and a sugar chain receptor strongly bind under a neutral condition in plasma and dissociate under an acidic condition within an endosome. In short, the sugar chain receptor has a pH-dependent binding ability (Non Patent Literature 11, Non Patent Literature 12). In such pH-dependent binding between a glycoprotein and a sugar chain receptor, a sugar chain added to the glycoprotein is involved. Examples of such combination of a sugar chain and a sugar chain receptor include a combination of a sugar chain such as N-linked sugar chain having galactose at a terminal and an asialoglycoprotein receptor (Non Patent Literature 11); and a combination of a sugar chain having mannose at a terminal and a mannose receptor (Non Patent Literature 12).

From the properties of such an N-linked sugar chain, it has been considered unfavorable to add an N-linked sugar chain, which is not functionally required for a biotechnology-based pharmaceutical product containing an antibody, in view of maintaining its efficacy or retentivity in plasma (Non Patent Literature 13, 14). Such an N-linked sugar chain has not yet been actually used in practice for further improving an effect of promoting clearance of an antigen from plasma.

Reference documents cited in the specification are as follows. The contents described in these documents are all incorporated herein by reference. However, these documents are not always admitted to correspond to prior art documents to the specification of the present application.

Citation List

Patent Literature

Patent Literature 1: WO2009/125825

Non Patent Literature

Non Patent Literature 1: Clark J Rosensweig, Laura B Faden & Matthew C Dewitz, Nature Biotechnology (2005) 23, 1073-1078

Non Patent Literature 2: Pavlou AK, Belsey MJ., Eur J Pharm Biopharm. (2005) 59 (3), 389-96

Non Patent Literature 3: Kim SJ, Park Y, Hong HJ., Mol Cells. (2005) 20 (1), 17-29

Non Patent Literature 4: Hinton PR, Xiong JM, Johlfs MG, Tang MT, Keller S, Tsurushita N., J Immunol. (2006) 176 (1), 346-56

Non Patent Literature 5: Ghetie V, Popov S, Borvak J, Radu C, Matesoi D, Medesan C, Ober RJ, Ward ES., Nat Biotechnol. (1997) 15 (7), 637-40

Non Patent Literature 6: Rajpal A, Beyaz N, Haber L, Cappuccilli G, Yee H, Bhatt RR, Takeuchi T, Lerner RA, Crea R., Proc Natl Acad Sci U S A. (2005) 102 (24), 8466-71

Non Patent Literature 7: Wu H, Pfarr DS, Johnson S, Brewah YA, Woods RM, Patel NK, White WI, Young JF, Kiener PA., J Mol Biol. (2007) 368, 652-665

Non Patent Literature 8: Hanson CV, Nishiyama Y, Paul S., (2005) 16 (6), 631-6

Non Patent Literature 9: Rathanaswami P, Roalstad S, Roskos L, Su QJ, Lackie S, Babcook J., Biochem Biophys Res Commun. (2005) 334 (4), 1004-13

Non Patent Literature 10: Spiess M., Biochemistry (1990) 29 (43), 10009-18

Non Patent Literature 11: Wragg S. and Drickamer K., J Biol Chem (1999) 274 (50), 35400-6

Non Patent Literature 12: Kim JJ, Olson LJ, Dahms NM., Curr Opin Struct Biol. (2009) 19 (5), 534-42

Non Patent Literature 13: Coloma MJ, Trinh RK, Martinez AR, Morrison SL., J Immunol (1999) 162, 2162-2170

Non Patent Literature 14: Igawa T, Tsunoda H, Kuramochi T, Sampei Z, Ishii S, Hattori K. MAbs. (2011) 3, 243-52

Summary of Invention

Solution to Problem

The present inventors conducted intensive studies on a method for promoting uptake of an antigen into a cell by an antigen-binding molecule having an antigen-binding activity; a method for allowing an antigen-binding molecule to bind to an antigen a plurality of times; a method for promoting a decrease of an antigen concentration in plasma by administering an antigen-binding molecule; and a method for improving retentivity of an antigen-binding molecule in plasma. As a result, the present inventors found that an antigen-binding molecule, which has an antigen-binding domain, a binding domain to FcRn (particularly human FcRn) and a domain binding to a sugar chain receptor, and which has a weak binding ability (binding to a sugar-chain receptor) in the ion-concentration condition of an endosome compared to the ion-concentration condition of plasma, can promote uptake of an antigen into a cell; that an antigen-binding molecule having an antigen-binding domain, whose antigen-binding activity in the ion-concentration condition of early-stage endosome is weak compared to the antigen-binding activity of the ion-concentration condition in plasma, can further promote uptake of an antigen into a cell by the antigen-binding molecule; that the number of antigens, to which a single antigen-binding molecule can bind, can be increased; that a decrease of the antigen concentration in plasma is promoted by administering an antigen-binding molecule; and that pharmacokinetics of an antigen-binding molecule can be improved.

More specifically, the present invention relates to a method for promoting uptake of an antigen into a cell by an antigen-binding molecule; a method for increasing the number of antigens to which a single antigen-binding molecule can bind; a method for promoting a decrease of the antigen concentration in plasma by administration of the antigen-binding molecule; a method for improving the pharmacokinetics of an antigen-binding molecule; an antigen-binding molecule promoting uptake of an antigen into a cell; an antigen-binding molecule capable of binding a larger number of antigens; an antigen-binding molecule capable of promoting a decrease of the antigen concentration in plasma by administration of the antigen-binding molecule; an antigen-binding molecule improved in pharmacokinetics; a pharmaceutical composition containing such an antigen-binding molecule; and a method for producing the same. More specifically, the present invention provides:

[1] A method for producing an antigen-binding molecule, including the following steps:

(a) a step of providing a polypeptide sequence of an antigen-binding molecule containing an antigen-binding domain and an FcRn binding domain,
(b) a step of identifying an amino acid sequence serving as a candidate for a motif for a sugar chain receptor-binding domain in the polypeptide sequence,
(c) a step of designing the motif for a sugar chain receptor-binding domain containing an amino acid sequence having at least one amino acid different from the amino acid sequence identified in the step (b),
(d) a step of preparing a gene encoding a polypeptide of an antigen-binding molecule containing the motif for the sugar chain receptor-binding domain designed in the step (c), and
(e) a step of recovering the antigen-binding molecule from a culture fluid of a host cell transformed with the gene obtained in the step (d).

[2] The method according to [1], further including a step of treating the antigen-binding molecule obtained in the step (e) with an enzyme.
[3] The method according to [1] or [2], in which a binding activity of the antigen-binding domain to an antigen changes depending upon an ion-concentration condition.
[4] The method according to [3], in which the ion-concentration condition is a pH condition.
[5] The method according to [4], in which the antigen-binding domain has a higher binding activity to an antigen under a neutral pH range condition than a binding activity to the antigen under an acidic pH range condition.
[6] The method according to [5], in which the antigen-binding domain having a higher binding activity to the antigen under a neutral pH range condition than a binding activity to the antigen under an acidic pH range condition is provided by substituting at least one amino acid of the antigen-binding domain with an amino acid whose side chain has a pKa of 4.0-8.0 or by inserting at least one amino acid whose side chain has a pKa of 4.0-8.0 into the antigen-binding domain.
[7] The method according to [3], in which the ion-concentration condition is a calcium-ion concentration condition.
[8] The method according to [7], in which the antigen-binding domain has a higher binding activity to the antigen under a high calcium-ion concentration condition than a binding activity to the antigen under a low calcium-ion concentration condition.
[9] The method according to [8], in which the antigen-binding domain having a higher binding activity to the antigen under a high calcium-ion concentration condition than a binding activity to the antigen under a low calcium-ion

concentration condition is provided by substituting at least one amino acid of the antigen-binding domain with a calcium-binding motif or by inserting a calcium-binding motif into the antigen-binding domain.

[10] The method according to any one of [1] to [9], in which the antigen-binding domain contains a variable region of an antibody.

[11] The method according to any one of [1] to [10], in which the FcRn binding domain contains an Fc region of an antibody.

[12] The method according to [11], in which the antibody is an IgG antibody.

[13] The method according to [12], in which the IgG antibody is IgG1, IgG2, IgG3 or IgG4.

[14] The method according to any one of [1] to [12], in which the binding activity of the sugar chain receptor-binding domain to a sugar chain receptor changes depending upon an ion-concentration condition.

[15] The method according to [14], in which the ion-concentration condition is a pH condition.

[16] The method according to any one of [1] to [15], in which a binding activity of the sugar chain receptor-binding domain to the sugar chain receptor under a neutral pH range condition is higher than a binding activity to the sugar chain receptor under an acidic pH range condition.

[17] The method according to [14], in which the ion-concentration condition is a calcium-ion concentration condition.

[18] The method according to [17], in which a binding activity of the sugar chain receptor-binding domain to the sugar chain receptor under a high calcium-ion concentration condition is higher than a binding activity to the sugar chain receptor under a low calcium-ion concentration condition.

[19] The method according to any one of [1] to [18], in which the sugar chain receptor-binding domain is a sugar chain.

[20] The method according to [19], in which the sugar chain is an O-linked sugar chain.

[21] The method according to [19], in which the sugar chain is an N-linked sugar chain.

[22] The method according to [21], in which designing a motif for the sugar chain receptor-binding domain include designing a motif to which an N-linked sugar chain is added.

[23] The method according to [21] or [22], in which a terminal of the N-linked sugar chain contains galactose.

[24] The method according to [23], in which a terminal of the N-linked sugar chain contains three or more galactoses.

[25] The method according to any one of [21] to [24], in which the sugar chain receptor is an asialoglycoprotein receptor.

[26] The method according to [21] or [22], in which a terminal of the N-linked sugar chain contains mannose.

[27] The method according to [26], in which the sugar chain receptor is a mannose receptor.

[28] An antigen-binding molecule prepared by the method according to any one of [1] to [27].

[29] An antigen-binding molecule containing an FcRn binding domain, an antigen-binding domain whose binding activity to an antigen changes depending upon an ion-concentration condition, and one or more sugar chain receptor-binding domains whose binding activity to a sugar chain receptor changes depending upon an ion-concentration condition.

[30] The antigen-binding molecule according to [29], in which a binding activity of the antigen-binding domain to the antigen changes depending upon a pH condition.

[31] The antigen-binding molecule according to [30], in which a binding activity of the antigen-binding domain to the antigen under a neutral pH range condition is higher than a binding activity to the antigen under an acidic pH range condition.

[32] The antigen-binding molecule according to [31], in which at least one amino acid of the antigen-binding domain includes at least one amino acid whose side chain has a pKa of 4.0-8.0.

[33] The antigen-binding molecule according to [29], in which a binding activity of the antigen-binding domain to the antigen changes depending upon the calcium-ion concentration condition.

[34] The antigen-binding molecule according to [33], in which a binding activity of the antigen-binding domain to the antigen under a high calcium-ion concentration condition is higher than a binding activity to the antigen under a low calcium-ion concentration condition.

[35] The antigen-binding molecule according to [34], in which at least one amino acid of the antigen-binding domain includes a calcium-binding motif.

[36] The antigen-binding molecule according to any one of [29] to [35] in which the antigen-binding domain contains a variable region of an antibody.

[37] The antigen-binding molecule according to any one of [29] to [36], in which the FcRn binding domain contains an Fc region of an antibody.

[38] The antigen-binding molecule according to [37], in which the antibody is an IgG antibody.

[39] The antigen-binding molecule according to [38], in which the IgG antibody is IgG1, IgG2, IgG3 or IgG4.

[40] The antigen-binding molecule according to any one of [29] to [39], in which a binding activity of the sugar chain receptor-binding domain to a sugar chain receptor changes depending upon an ion-concentration condition.

[41] The antigen-binding molecule according to [40], in which the ion-concentration condition is a pH condition.

[42] The antigen-binding molecule according to [41], in which a binding activity of the sugar chain receptor-binding

domain to a sugar chain receptor under a neutral pH range condition is higher than a binding activity to the sugar chain receptor under an acidic pH range condition.

[43] The antigen-binding molecule according to [40], in which the ion-concentration condition is the calcium-ion concentration condition.

[44] The antigen-binding molecule according to [43], in which a binding activity of the sugar chain receptor-binding domain to a sugar chain receptor under a high calcium-ion concentration condition is higher than a binding activity to the sugar chain receptor under a low calcium-ion concentration condition.

[45] The antigen-binding molecule according to any one of [29] to [44], in which the sugar chain receptor-binding domain is a sugar chain.

[46] The antigen-binding molecule according to [45], in which the sugar chain is an O-linked sugar chain or an N-linked sugar chain.

[47] The antigen-binding molecule according to [46], in which the sugar chain receptor-binding domain includes a motif to which an N-linked sugar chain is bound.

[48] The antigen-binding molecule according to [46] or [47], in which a terminal of the N-linked sugar chain contains galactose.

[49] The antigen-binding molecule according to [48], in which a terminal of the N-linked sugar chain contains c.

[50] The antigen-binding molecule according to any one of [47] to [49], in which the sugar chain receptor is an asialoglycoprotein receptor.

[51] The antigen-binding molecule according to [46] or [47], in which a terminal of the N-linked sugar chain contains mannose.

[52] The antigen-binding molecule according to [46] or [47], in which the sugar chain receptor is a mannose receptor.

[53] The antigen-binding molecule according to any one of [28] to [52], in which the antigen-binding molecule is an antibody.

[54] The antigen-binding molecule according to any one of [28] to [53], in which the sugar chain receptor-binding domain is contained in the antigen-binding domain.

[55] The antigen-binding molecule according to any one of [28] to [53], in which the sugar chain receptor-binding domain is contained in the FcRn binding domain.

[56] A pharmaceutical composition containing an antigen-binding molecule according to any one of [28] to [55].

[57] A method for allowing a cell expressing a sugar chain receptor to take up an antigen-binding molecule according to any one of [28] to [55] in to the cell, comprising bringing the antigen-binding molecule into contact with the cell *in-vivo* or *ex-vivo.*

[58] A method for allowing a cell expressing a sugar chain receptor to take up an antigen bound to an antigen-binding molecule according to any one of [28] to [55], comprising bringing the antigen-binding molecule into contact with the cell *in-vivo* or *ex-vivo.*

[59] A method for increasing the number of antigens to which a single antigen-binding molecule according to any one of [28] to [55] binds, comprising bringing the antigen-binding molecule into contact with a cell expressing a sugar chain receptor, *in-vivo* or *ex-vivo.*

[60] A method for decreasing the number of an antigen being present in an extracellular space, comprising bringing an antigen-binding molecule according to any one of [28] to [55] into contact with a cell expressing a sugar chain receptor, *in-vivo* or *ex-vivo.*

[61] The method according to [58], in which the extracellular space is plasma.

[62] A method for improving the pharmacokinetics of an antigen-binding molecule according to any one of [28] to [55], comprising bringing the antigen-binding molecule into contact with a cell expressing a sugar chain receptor, *in-vivo.*

[63] A method for promoting dissociation of an antigen bound extracellularly to an antigen-binding molecule according to any one of [28] to [55] from the antigen-binding molecule, comprising bringing the antigen-binding molecule into contact with a cell expressing a sugar chain receptor, *in-vivo* or *ex-vivo.*

[64] A method selected from the following methods:

(i) a method for promoting uptake of an antigen-binding molecule into a cell expressing the sugar chain receptor, *in-vivo* or *ex-vivo;*

(ii) a method for promoting uptake of an antigen bound to an antigen-binding molecule into a cell expressing a sugar chain receptor, *in-vivo* or *ex-vivo;*

(iii) a method for increasing the number of an antigen to which a single antigen-binding molecule binds, *in-vivo* or *ex-vivo;*

(iv) a method for enhancing potency of an antigen-binding molecule to clear an antigen, *in-vivo* or *ex-vivo;*

(v) a method for improving the pharmacokinetics of an antigen-binding molecule; or

(vi) a method for promoting dissociation of an antigen which has bound extracellularly to an antigen-binding

molecule;

the method comprising, in an antigen-binding molecule containing an antigen-binding domain, an FcRn binding domain and one or more binding domains to a sugar chain receptor, increasing the number of the binding domains to the sugar chain receptor.

[65] The method according to [64], in which a binding activity of the antigen-binding molecule to an antigen changes depending upon an ion-concentration condition.

[66] The method according to [64], in which a binding activity of the antigen-binding domain to an antigen changes depending upon a pH condition.

[67] The method according to [66], in which a binding activity of the antigen-binding domain to an antigen under a neutral pH range condition is higher than a binding activity to the antigen under an acidic pH range condition.

[68] The method according to [67], in which at least one amino acid of the antigen-binding domain includes at least one amino acid whose side chain has a pKa of 4.0-8.0.

[69] The method according to [64], in which a binding activity of the antigen-binding domain to an antigen changes depending upon a calcium-ion concentration condition.

[70] The method according to [69], in which a binding activity of the antigen-binding domain to an antigen under a high calcium-ion concentration condition is higher than a binding activity to the antigen under a low calcium-ion concentration condition.

[71] The method according to [70], in which at least one amino acid of the antigen-binding domain includes a calcium-binding motif.

[72] The method according to any one of [64] to [71], in which the antigen-binding domain contains a variable region of an antibody.

[73] The method according to any one of [64] to [72], in which the FcRn binding domain contains an Fc region of an antibody.

[74] The method according to [73], in which the antibody is an IgG antibody.

[75] The method according to [74], in which the IgG antibody is IgG1, IgG2, IgG3 or IgG4.

[76] The method according to any one of [64] to [75], in which a binding activity of the sugar chain receptor-binding domain to a sugar chain receptor changes depending upon an ion-concentration condition.

[77] The method according to [76], in which the ion-concentration condition is a pH condition.

[78] The method according to [76], in which a binding activity of the sugar chain receptor-binding domain to a sugar chain receptor under a neutral pH range condition is higher than a binding activity to the sugar chain receptor under an acidic pH range condition.

[79] The method according to [76], in which the ion-concentration condition is a calcium-ion concentration condition.

[80] The method according to [79], in which a binding activity of the sugar chain receptor-binding domain to a sugar chain receptor under a high calcium-ion concentration condition is higher than a binding activity to the sugar chain receptor under a low calcium-ion concentration condition.

[81] The method according to any one of [64] to [80], in which the sugar chain receptor-binding domain is a sugar chain.

[82] The method according to [81], in which the sugar chain is an O-linked sugar chain.

[83] The method according to [81], in which the sugar chain is an N-linked sugar chain.

[84] The method according to [83], in which the sugar chain receptor-binding domain contains a motif to which an N-linked sugar chain is bound.

[85] The method according to [83] or [84], in which a terminal of the N-linked sugar chain contains galactose.

[86] The method according to [85], in which a terminal of the N-linked sugar chain contains three or more terminal galactoses.

[87] The method according to any one of [84] to [86], in which the sugar chain receptor is an asialoglycoprotein receptor.

[88] The method according to [83] or [84], in which a terminal of the N-linked sugar chain contains mannose.

[89] The method according to [83] or [84], in which the sugar chain receptor is a mannose receptor.

[90] The method according to any one of [64] to [89], in which the antigen-binding molecule is an antibody.

[91] The method according to any one of [64] to [90], in which the sugar chain receptor-binding domain is contained in the antigen-binding domain.

[92] The method according to any one of [64] to [90], in which the sugar chain receptor-binding domain is contained in the FcRn binding domain.

Brief Description of Drawings

[Figure 1] Figure 1 is a schematic diagram showing that an IgG antibody molecule is dissociated from a soluble antigen in an endosome, accelerates clearance of the antigen and binds again to a new antigen.

[Figure 2] Figure 2 is a schematic diagram showing that an IgG antibody molecule binds to a sugar chain receptor in a blood vessel and is taken up into a cell, in which the IgG antibody molecule dissociates from a soluble antigen together with the sugar chain receptor in an endosome, thereby accelerating clearance of the antigen and binds again to a new antigen.

[Figure 3] Figure 3 is an electrophoretogram of reducing SDS-PAGE in which a heavy chain and a light chain are detected.

[Figure 4] Figure 4 is a chromatogram before and after a neuraminidase treatment obtained by anion exchange chromatography.

[Figure 5] Figure 5 is a mass chromatogram of reduced GL-M111 analyzed by RP-LC/ESI-MS in which an N-linked sugar chain added to a light chain is observed.

[Figure 6] Figure 6 is an anion exchange chromatography in which a neuraminidase activity is observed.

[Figure 7] Figure 7 is an electrophoretogram of a sample of an antibody having a constant region back to IgG1, analyzed by reducing SDS-PAGE, in which a heavy chain and a light chain are detected.

[Figure 8] Figure 8 is a graph showing a change in plasma concentration of an antibody with time in a normal mouse.

[Figure 9] Figure 9 is a graph showing a change of the concentration of a soluble human IL-6 receptor in plasma with time in a normal mouse.

[Figure 10] Figure 10 is a graph showing a change of the antibody concentration in plasma with time in a normal mouse.

[Figure 11] Figure 11 is a graph showing a change of concentration of a soluble human IL-6 receptor in plasma with time in a normal mouse.

[Figure 12] Figure 12 is an electrophoretogram of reducing SDS-PAGE in which a heavy chain and a light chain are detected.

[Figure 13] Figure 13 is a mass chromatogram of reduced GL5-G1_kif+ analyzed by RP-LC/ESI-MS analysis, in which an N-linked sugar chain added to a light chain is observed.

[Figure 14] Figure 14 is a graph showing a change of antibody concentration in plasma with time in a normal mouse.

[Figure 15] Figure 15 is a graph showing a change of the concentration of a soluble human IL-6 receptor in plasma with time in a normal mouse.

[Figure 16] Figure 16 is a Biacore sensorgram showing interaction of H54/L28-IgG1 with a soluble human IL-6 receptor in (Ca2+ 2 mM) and (Ca2+ 3 μM).

[Figure 17] Figure 17 is a Biacore sensorgram showing interaction of FH4-IgG1 with a soluble human IL-6 receptor in (Ca2+ 2 mM) and (Ca2+ 3 μM).

[Figure 18] Figure 18 is a Biacore sensorgram showing interaction of 6RL#9-IgG with a soluble human IL-6 receptor in (Ca2+ 2 mM) and Ca2+ 3 μM).

[Figure 19] Figure 19 is a graph showing a change of antibody (H54/L28-IgG1, FH4-IgG1 and 6RL#9-IgG1) concentration in plasma with time in normal mice.

[Figure 20] Figure 20 is a graph showing a change of concentration of a soluble human IL-6 receptor (hsIL-6R) to HS4/L28-IgG1, FH4-IgG1 and 6RL#9-IgG1 in plasma in normal mice.

[Figure 21] Figure 21 is an illustration showing the structure of Fab-fragment heavy chain CDR3 of 6RL#9 antibody determined by X-ray crystal structure analysis.

[Figure 22] Figure 22 is an ion exchange chromatogram of an antibody containing a human Vk5-2 sequence and an antibody containing h Vk5-2_L65 sequence obtained by modifying the glycosylation sequence of human Vk5-2 sequence, in which a continuous line indicates the chromatogram of an antibody containing human Vk5-2 sequence (heavy chain: CIM_H, SEQ ID NO: 63 and light chain: hVk5-2, fusion molecule of SEQ ID NO: 6 and SEQ ID NO: 42); a broken line indicates a chromatogram of an antibody having human hVk5-2_L65 sequence (heavy chain: CIM_H (SEQ ID NO: 63), light chain: hVk5-2_L65 (SEQ ID NO. 62)).

Description of Embodiments

The following definitions and detailed descriptions are provided to promote understanding of the present invention described in the specification.

Amino acid

Each amino acid is indicated herein by single-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, and Val/V.

And/Or

The term "and/or" described herein is meant to include every combination appropriately represented by "and" and "or". Specifically, for example, the phrase "amino acids 33, 55, and/or 96 are substituted" includes the following variations of amino acid modification: (a) position 33, (b) position 55, (c) position 96, (d) positions 33 and 55, (e) positions 33 and 96, (f) positions 55 and 96, and (g) positions 33, 55, and 96.

Antigen

The "antigen" described herein is not limited by a particular structure as long as the antigen comprises an epitope to which the antigen-binding domain binds. In another sense, the antigen may be inorganic matter or may be organic matter. As the antigen-binding molecule improved in pharmacokinetics by the method of the present invention, antigen-binding molecules recognizing a membrane antigen such as receptor proteins (membrane bound receptor, soluble receptor) and cell surface markers, and antigen-binding molecules recognizing a soluble antigen such as cytokines are preferably mentioned. Examples of the antigen can include the following molecules: 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic factor, av/b3 integrin, Axl, b2M, B7-1, B7-2, B7-H, B-lymphocyte stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer-associated antigens, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, botulinum toxin, *Clostridium perfringens* toxin, CKb8-1, CLC, CMV, CMV UL, CNTF, CNTN-1, COX, C-Ret, CRG-2, CT-1, CTACK, CTGF, CTLA-4, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor-associated antigens, DAN, DCC, DcR3, DC-SIGN, decay accelerating factor, des (1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast-activating protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle-stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha 1, GFR-alpha 2, GFR-alpha 3, GITR, glucagon, Glut4, glycoprotein IIb/IIIa (GPIIb/IIIa), GM-CSF, gp130, gp72, GRO, growth hormone-releasing factor, hapten (NP-cap or NIP-cap), HB-EGF, HCC, HCMV gB envelope glycoprotein, HCMV gH envelope glycoprotein, HCMVUL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high-molecular-weight melanoma-associated antigen (HMW-MAA), HIV gp120, HIV IIIB gp 120 V3 loop, HLA, HLA-DR, HM1.24, HMFG PEM, HRG, Hrk, human heart myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF-binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-23, interferon (INF)-alpha, INF-beta, INF-gamma, inhibin, iNOS, insulin chain A, insulin chain B, insulin-like growth factor 1, integrin alpha 2, integrin alpha 3, integrin alpha 4, integrin alpha 4/beta 1, integrin alpha 4/beta 7, integrin alpha 5 (alpha V), integrin alpha 5/beta 1, integrin alpha 5/beta 3, integrin alpha 6, integrin beta 1, integrin beta 2, interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF,

LECT2, lefty, Lewis-Y antigen, Lewis-Y-related antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, metalloproteases, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), P1GF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T cell receptor (e.g., T cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta 1, TGF-beta 2, TGF-beta 3, TGF-beta 4, TGF-beta 5, thrombin, thymus Ck-1, thyroid stimulating hormone, Tie, TIMP, TIQ, tissue factor, TMEFF2, Tmpo, TMPRSS2, TNF, TNF-alpha, TNF-alpha/beta, TNF-beta 2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAILR3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK RFN14), TNFRSF13B (TACI), TNFRSF13C (BAFF R), TNFRSF14 (HVEM ATAR, HveA, LIGHT R, TR2), TNFRSF16 (NGFRp75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TSG, TSLP, tumor-associated antigen CA125, tumor-associated antigen-expressing Lewis-Y-related carbohydrate, TWEAK, TXB2, Ung, uPAR, uPAR-1, urokinase, VCAM, VCAM-1, VECAD, VE-cadherin, VE-cadherin-2, VEFGR-1 (flt-1), VEGF, VEGFR, VEGFR-3 (flt-4), VEGI, VIM, viral antigens, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, chromogranin A, chromogranin B, tau, VAP1, high-molecular-weight kininogen, IL-31, IL-31R, Nav1.1, Nav1.2, Nav1.3, Nav1.4, Nav1.5. Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, syndecan-1, syndecan-2, syndecan-3, syndecan-4, LPA, S1P, acetylcholine receptor, AdipoR1, AdipoR2, ADP ribosyl cyclase-1, alpha-4/beta-7 integrin, alpha-5/beta-1 integrin, alpha-v/beta-6 integrin, alpha-v/beta-1 integrin, angiopoietin ligand-2, Angpt12, Anthrax, cadherin, carbonic anhydrase-IX, CD105, CD155, CD158a, CD37, CD49b, CD51, CD70, CD72, Claudin 18, *Clostridium difficile* toxin, CS1, delta-like protein ligand 4, DHICA oxidase, Dickkopf-1 ligand, dipeptidyl peptidase IV, EPOR, F protein of RSV, factor Ia, FasL, folate receptor alpha, glucagon receptor, glucagon-like peptide 1 receptor, glutamate carboxypeptidase II, GMCSFR, hepatitis C virus E2 glycoprotein, hepcidin, IL-17 receptor, IL-22 receptor, IL-23 receptor, IL-3 receptor, Kit tyrosine kinase, leucine rich alpha-2-glycoprotein 1 (LRG1), lysosphingolipid receptor, membrane glycoprotein OX2, mesothelin, MET, MICA, MUC-16, myelin associated glycoprotein, neuropilin-1, neuropilin-2, Nogo receptor, PLXNA1, PLXNA2, PLXNA3, PLXNA4A, PLXNA4B, PLXNB1, PLXNB2, PLXNB3, PLXNC1, PLXND1, programmed cell death ligand 1, proprotein convertase PC9, P-selectin glycoprotein ligand-1, RAGE, reticulon 4, RF, RON-8, SEMA3A, SEMA3B, SEMA3C, SEMA3D, SEMA3E, SEMA3F, SEMA3G, SEMA4A, SEMA4B, SEMA4C, SEMA4D, SEMA4F, SEMA4G, SEMA5A, SEMA5B, SEMA6A, SEMA6B, SEMA6C, SEMA6D, SEMA7A, Shiga like toxin II, sphingosine-1-phosphate receptor-1, ST2, Staphylococcal lipoteichoic acid,

tenascin, TG2, thymic stromal lymphopoietin receptor, TNF superfamily receptor 12A, transmembrane glycoprotein NMB, TREM-1, TREM-2, trophoblast glycoprotein, TSH receptor, TTR, tubulin, ULBP2, and receptors for hormones or growth factors.

The epitope, which means an antigenic determinant, contained in the antigen means a site on the antigen to which the antigen-binding domain in the antigen-binding molecule disclosed herein binds. Accordingly, for example, the epitope can be defined by its structure. Alternatively, the epitope may be defined by the antigen-binding activity of the antigen-binding molecule that recognizes the epitope. The epitope in an antigenic peptide or polypeptide may be determined by amino acid residues constituting the epitope. Alternatively, the epitope composed of a sugar chain may be determined by a particular sugar chain structure.

A linear epitope refers to an epitope comprising an epitope that is recognized via its primary sequence of amino acids. The linear epitope comprises typically at least 3 and most commonly at least 5, for example, approximately 8 to approximately 10 or 6 to 20 amino acids, in a unique sequence.

In contrast to the linear epitope, a conformational epitope refers to an epitope that is contained in a primary sequence of amino acids comprising a component other than the single defined component of the epitope to be recognized (e.g., an epitope whose primary sequence of amino acids may not be recognized by an antibody that determines the epitope). The conformational epitope may contain an increased number of amino acids, compared with the linear epitope. An antibody recognizes the conformational epitope by recognizing the three-dimensional structure of the antigenic peptide or protein. For example, the protein molecule may be folded to form a three-dimensional structure. In such a case, certain amino acids and/or polypeptide backbone constituting the conformational epitope are arranged in parallel to allow the antibody to recognize the epitope. The conformation of the epitope is determined by a method including, for example, but not limited to, X-ray crystallography, two-dimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

## Binding activity

A method for confirming the binding of a test antigen-binding molecule containing an antigen-binding domain to IL-6R, to an epitope is mentioned below. A method for confirming the binding of a test antigen-binding molecule containing an antigen-binding molecule to an antigen other than IL-6R to an epitope can be appropriately performed in accordance with the following example.

For example, recognizing a linear epitope present in an IL-6R molecule by a test antigen-binding molecule containing an antigen-binding domain to IL-6R can be confirmed, for example, as follows. A linear peptide consisting of an amino acid sequence, which constitutes an extracellular domain of IL-6R, is synthesized for the above purpose. The peptide can be chemically synthesized or by genetic engineering method using a region encoding an amino acid sequence corresponding to the extracellular domain in cDNA of IL-6R. Then, the binding activity between the linear peptide (which consists of an amino acid sequence constituting the extracellular domain), and a test antigen-binding molecule, which contains an antigen-binding domain to IL-6R, is evaluated. For example, the binding activity of the antigen-binding molecule to the peptide can be evaluated by ELISA using the linear peptide immobilized as an antigen. Alternatively, the binding activity to the linear peptide is determined based on the level of inhibition by the linear peptide against the binding of the antigen-binding molecule to an IL-6R expression cell. By these tests, the binding activity of the antigen-binding molecule to the linear peptide can be determined.

Furthermore, recognizing a steric epitope by a test antigen-binding molecule containing an antigen-binding domain to IL-6R can be confirmed as follows. First, a cell expressing IL-6R is prepared for the above purpose. When the test antigen-binding molecule (containing an antigen-binding domain to IL-6R) is brought into contact with the IL-6R expression cell, if the test antigen-binding molecule strongly binds to the cell but the test antigen-binding molecule does not substantially bind to a linear peptide (which consists of an amino acid sequence constituting the extracellular domain of IL-6R and immobilized), recognition is made. Herein, the expression "does not substantially bind" means the case where the binding activity is 80% or less, usually 50% or less, preferably 30% or less, and particularly preferably 15% or less of the binding activity to a human IL-6R expression cell.

As a method for measuring the binding activity of a test antigen-binding molecule (containing an antigen-binding domain to IL-6R) to the IL-6R expression cell, for example, the method described in the Antibodies A Laboratory Manual (Ed Harlow, David Lane, Cold Spring Harbor Laboratory (1988) 359-420) is mentioned. More specifically, the binding activity can be evaluated based on the principle of ELISA and FACS (fluorescence activated cell sorting) using an IL-6R expression cell as an antigen.

In the ELISA format, the binding activity of a test antigen-binding molecule (containing an antigen-binding domain to IL-6R) to an IL-6R expression cell is quantitatively evaluated by comparing a signal level produced by an enzyme reaction. To explain more specifically, a test antigen-binding molecule is added to an ELISA plate, to which an IL-6R expression cell is immobilized. The test antigen-binding molecule bound to the cell is detected by an enzyme

labeled antibody capable of recognizing the test antigen-binding molecule. In contrast, in FACS, a test antigen-binding molecule is serially diluted and the titer against the IL-6R expression cell is determined. In this manner, the binding activity of the test antigen-binding molecule to the IL-6R expression cell can be compared.

The binding of a test antigen-binding molecule to an antigen expressed on the surface of a cell suspended in a buffer, etc. can be detected by a flow cytometer. As the flow cytometer, for example, the following apparatuses are known.

FACSCanto™II
FACSAria™
FACSArray™
FACSVantage™SE
FACSCalibur™ (all are trade names of products manufactured by BD Biosciences)
EPICS ALTRA HyPerSort
Cytomics FC 500
EPICS XL-MCL ADC EPICS XL ADC
Cell Lab Quanta /Cell Lab Quanta SC (all are trade names of products manufactured by Beckman Coulter)

As a preferable method for measuring the binding activity of a test antigen-binding molecule (containing an antigen-binding domain to IL-6R) to an antigen, for example, the following method is mentioned. First, a test antigen-binding molecule is reacted with a cell expressing IL-6R and then stained by FITC-labeled secondary antibody recognizing the test antigen-binding molecule. If the test antigen-binding molecule is appropriately diluted with a preferable buffer, the antigen-binding molecule can be used in a desired concentration. For example, the antigen-binding molecule can be used in any concentration from 10 μg/ml to 10 ng/ml. Subsequently, fluorescent intensity and the number of cells are measured by FACSCalibur (manufactured by BD). The binding amount of antibody to the cells is analyzed by CELL QUEST Software (manufactured by BD). The binding amount is reflected in the fluorescent intensity obtained, in other words, a Geometric Mean value. To explain more specifically, the binding activity of a test antigen-binding molecule, which is represented by the binding amount of test antigen-binding molecule, can be measured by obtaining the Geometric Mean value.

Whether a test antigen-binding molecule (containing an antigen-binding domain to IL-6R) shares an epitope with another antigen-binding molecule, can be confirmed by a competition test between both antigen-binding molecules to the same epitope. The competition between the antigen-binding molecules is detected by a crossover blocking assay, etc. For example, a competitive ELISA assay is a preferable crossover blocking assay.

The crossover blocking assay is specifically performed as follows. IL-6R protein is applied onto the wells of a microtiter plate and pre-incubated in the presence or absence of a competitive antigen-binding molecule serving as a candidate. Thereafter, a test antigen-binding molecule is added. The amount of test antigen-binding molecule bound to IL-6R protein in a well indirectly correlates with the binding ability of the candidate-competitive antigen-binding molecule, which competitively binds to the same epitope. In other words, the larger the affinity of a competitive antigen-binding molecule to the same epitope, the lower the binding activity of the test antigen-binding molecule to IL-6R protein applied to a well.

The amount of test antigen-binding molecule bound to a well via an IL-6R protein can be easily measured by labelling the test antigen-binding molecule in advance. For example, an antigen-binding molecule labeled with biotin is measured by using an avidin-peroxidase conjugate and an appropriate substrate. A crossover blocking assay using an enzyme label such as peroxidase is particularly called as a competitive ELISA assay. The test antigen-binding molecule may be labeled with another detectable or measurable labeling substance. For example, a radio label or a fluorescent label is known in the art.

Relative to the binding activity obtained in a control test, which is performed in the absence of a candidate competitive antigen-binding molecule, if the binding of a test antigen-binding molecule (containing an antigen-binding domain to IL-6R) can be blocked by the competitive antigen-binding molecule in a percentage of at least 20%, preferably at least 20-50%, and further preferably at least 50%, it is determined that the test antigen-binding molecule binds to substantially the same epitope or is a competitive antigen-binding molecule binding to the same epitope.

When the structure of the epitope, to which a test antigen-binding molecule (containing an antigen-binding domain to IL-6R) is bound, is identified, sharing the same epitope by a test antigen-binding molecule and a control antigen-binding molecule can be evaluated by comparing the binding activities of both antigen-binding molecules to a peptide, which is prepared by introducing an amino acid mutation to the peptide constituting the epitope.

Such a binding activity can be measured, for example, if an ELISA format is used, by a method of comparing binding activities of the test antigen-binding molecule and the control antigen-binding molecule to a linear peptide having a mutation introduced therein. Other than ELISA, the binding activity can be measured by a method of determining the binding activities of a test antigen-binding molecule and a control antigen-binding molecule to the mutant peptide by passing these antigen biding molecules through a column, to which a mutant peptide as mentioned above is bound, and quantifying each of the antigen-binding molecules released in an eluant. The mutant peptide is fused with e.g., GST and adsorbed to a column as a fusion peptide is known in the art.

Furthermore, when the epitope identified is a steric epitope, sharing the epitope by a test antigen-binding molecule as well as a control antigen-binding molecule can be evaluated by the following method. First, a cell expressing IL-6R and a cell expressing IL-6R having an epitope to which a mutation is introduced are prepared. These cells are suspended in an appropriate buffer such as PBS. To the cell suspension solution, the test antigen-binding molecule and the control antigen-binding molecule are added. Then, the cell suspension solution is appropriately washed with a buffer. To the cell suspension solution, an FITC-labeled antibody capable of recognizing the test antigen-binding molecule and the control antigen-binding molecule, is added. The fluorescent intensity and the number of cells stained with the labeled antibody are measured by FACSCalibur (manufactured by BD). The test antigen-binding molecule and the control antigen-binding molecule are appropriately diluted with a preferable buffer to prepare solutions having a desired concentration and then put in use. The concentration to be used falls within the range, for example, from 10 $\mu$g/ml to 10 ng/ml. The binding amount of labelled antibody to the cells is reflected in the fluorescent intensity, more specifically, the Geometric Mean value, which is obtained by analyzing by CELLQUEST Software (manufactured by BD). In other words, the binding activities of the test antigen-binding molecule and the control antigen-binding molecule (which are expressed by the binding amounts of labelled antibody) can be measured by obtaining the Geometric Mean value.

In this method, for example "substantially not binding to a mutant IL-6R expression cell" can be determined by the following method. First, a test antigen-binding molecule and a control antigen-binding molecule bound to a cell expressing mutant IL-6R are stained with a labelled antibody. Then, the fluorescent intensity of the cell is detected. When fluorescence is detected by use of FACSCalibur for flow cytometry, the obtained fluorescent intensity can be analyzed by CELL QUEST Software. Based on the Geometric Mean values obtained in the presence and absence of the antigen-binding molecule, the comparison value ($\Delta$Geo-Mean) is calculated based on the following computation expression. In this way, the ratio of fluorescent intensity increased by binding of the antigen-binding molecule can be obtained.

$$\Delta\text{Geo-Mean} = \text{Geo-Mean (in the presence of antigen-binding molecule)} / \text{Geo-Mean (in the absence of antigen-binding molecule)}$$

The Geometric Mean comparison value (mutant IL-6R Molecule $\Delta$Geo-Mean value), in which the binding amount of the test antigen-binding molecule to the mutant IL-6R expression cell (obtained by analysis) is reflected, is compared with the $\Delta$Geo-Mean comparison value, in which the binding amount of test antigen-binding molecule to the IL-6R expression cell is reflected. In this case, it is particularly preferable that the concentrations of the test antigen-binding molecules to be used for obtaining $\Delta$Geo-Mean comparison value with respect to the mutant IL-6R expression cell and the IL-6R expression cell are controlled to be the same or substantially the same with each other. The antigen-binding molecule, which is previously confirmed to recognize the epitope of IL-6R, is used as a control antigen-binding molecule.

If the $\Delta$Geo-Mean comparison value of a test antigen-binding molecule to a mutant IL-6R expression cell relative to the $\Delta$Geo-Mean comparison value of the test antigen-binding molecule to an IL-6R expression cell is at least 80%, preferably 50%, further preferably 30%, particularly preferably smaller than 15%, the antigen-binding molecule is determined as "substantially not binding to a mutant IL-6R expression cell". The computation expression for obtaining a Geo-Mean value (Geometric Mean) is described in the CELL QUEST Software User's Guide (BD biosciences). If the binding amounts are substantially equivalent by comparing the $\Delta$Geo-Mean comparison values, it can be evaluated that the test antigen-binding molecule and the control antigen-binding molecule bind to the same epitope.

<u>Antigen-binding domain</u>

In the specification, as the "antigen-binding domain", a domain of any structure can be used as long as it binds to a desired antigen. Preferable examples of such a domain include a variable region of a heavy chain or light chain of an antibody; a module (WO2004/044011, WO2005/040229) of approximately 35 amino acids called A domain and contained in Avimer (a cell membrane protein present *in vivo*); Adnectin (WO2002/032925) containing a 10Fn3 domain, which is a domain binding to a protein in fibronectin (a glycoprotein expressed in cell membrane); Affibody (WO1995/001937), composed of a three-helix bundle consisting of 58 amino acids of Protein A and based on the scaffold of an IgG binding domain; DARPins (Designed Ankyrin Repeat proteins) (WO2002/020565), which is a region exposed in the molecular surface of ankyrin repeat (AR) containing 33 amino acid residues and having a structure formed by repeatedly laminating a subunit consisting of a turn, two antiparallel helixes and a loop; Anticalin, etc. (WO2003/029462), which is a four-loop regions supporting one side of a barrel structure in which 8 highly-conserved antiparallel strands are twisted toward the center axis, in a lipocalin molecule such as neutrophil gelatinase-associated lipocalin (NGAL); and a depressed region (WO2008/016854) of a parallel sheet structure within a horseshoe-shaped structure, which is

formed by repeatedly laminating a leucine-rich-repeat (LRR) module of a variable lymphocyte receptor (VLR) having no immunoglobulin structure, as the acquired immune system of a jawless vertebrate such as lamprey and hagfish. As a preferable example of the antigen-binding domain used in the present invention, an antigen-binding domain containing a variable region of a heavy chain and a light chain of an antibody is mentioned. As an example of such an antigen-binding domain, e.g., "scFv (single chain Fv)", " single chain antibody", "Fv", "scFv2 (single chain Fv 2)", "diabody", "Fab", "F(ab')2", domain antibody (dAb) (WO2004/058821, WO2003/002609), scFv-sc (WO2005/037989) or Fc fusion protein is preferably mentioned. In the molecule containing an Fc region, the Fc region can be used as a binding domain for FcRn (particularly human FcRn). Also, a molecule prepared by fusing such a molecule with a human FcRn binding domain can be used.

Antigen-binding domains in the antigen-binding molecules of the present invention can be bound to the same epitope. The same epitope used herein can be present in the protein consisting of an amino acid sequence represented by, for example, SEQ ID NO: 1 (IL-6R_PP; NP_000556.1); more specifically, can be present in the protein consisting of 20th to 365th amino acids of the amino acid sequence represented by SEQ ID NO: 1. Alternatively, an antigen-binding domain in the antigen-binding molecule of the present invention can bind to mutually different epitopes. The different epitopes herein can be present in the protein consisting of, for example, the amino acid sequence represented by SEQ ID NO: 1; more specifically, can be present in the protein consisting of 20th to 365th amino acid of the amino acid sequence represented by SEQ ID NO: 1. For this purpose, an antigen-binding domain contained in a bispecific antibody can be appropriately used. The bispecific antibody refers to an antibody having variable regions recognizing different epitopes in the same antibody molecule. The bispecific antibody may be an antibody recognizing two or more different antigens and may be an antibody recognizing two or more different epitopes present on the same antigen.

As the antigen-binding domain of the present invention, a domain of a receptor protein binding to a target (antigen) and involved in binding to the target(antigen) can be preferably used. More specifically, the antigen-binding molecule may be a protein prepared by fusing a binding domain to FcRn (in particular, FcRn) and a sugar chain receptor-binding domain with a receptor protein binding to a target (antigen). Examples of such an antigen-binding molecule include TNFR-Fc fusion protein, IL1R-Fc fusion protein, VEGFR-Fc fusion protein and CTLA4-Fc fusion protein (Nat Med. (2003) 9 (1), 47-52, BioDrugs. (2006) 20 (3), 151-160). Even if the antigen-binding molecule of the present invention is a fusion protein formed of such a receptor protein, a binding domain to FcRn (particularly human FcRn), as long as the antigen-binding molecule changes the binding activity to a target molecule depending upon the ion-concentration condition and as long as the antigen-binding molecule has the binding activity to a sugar chain receptor and to FcRn (particularly human FcRn), the antigen-binding molecule can promote uptake of an antigen into a cell. Furthermore, the antigen-binding molecule, if it is administered, can promote a decrease of the antigen concentration in plasma. Moreover, the pharmacokinetics of such an antigen-binding molecule is improved and the number of antigens, to which a single antigen-binding molecule can bind, can be increased.

Furthermore, as the antigen-binding domain of the present invention, a domain of a natural or artificial ligand binding to a target and involved in binding to the target can be preferably used. More specifically, the antigen-binding molecule may also be a molecule prepared by fusing an FcRn binding domain (particularly, FcRn) and a sugar chain receptor-binding domain with a natural or artificial protein ligand, which binds to a target and has an antagonist activity and a neutralization effect. Of these antigen-binding domains, as an artificial ligand, for example, an artificial ligand such as mutant IL-6 (EMBO J. (1994) 13 (24), 5863-70) is mentioned. Even if the antigen-binding molecule of the present invention is a fusion molecule with such an artificial ligand, as long as the antigen-binding molecule changes the binding activity to a target molecule depending upon the ion-concentration condition and as long as the antigen-binding molecule has the binding activity to a sugar chain receptor and to FcRn (particularly human FcRn), the antigen-binding molecule can promote uptake of an antigen into a cell. Furthermore, the antigen-binding molecule, if it is administered, can promote a decrease of the antigen concentration in plasma. Moreover, the pharmacokinetics of such an antigen-binding molecule is improved and the number of antigens to which a single antigen-binding molecule can bind can be increased.

Specificity

Specificity refers to the state where one of the molecules specifically bind to each other does not show a significant binding to any other molecule except the other bonding partner(s). Alternatively, in the case where an antigen-binding domain is specific to a certain epitope among a plurality of epitopes contained in a certain antigen, the term "specificity" is used. In the case where an epitope (to which an antigen-binding domain binds) is contained in a plurality of different antigens, an antigen-binding molecule having the antigen-binding domain can bind to various antigens containing the epitope.

Antibody

In the specification, the antibody refers to a natural or partial or completely synthesized immunoglobulin. The

antibody can be isolated from a natural source in which a natural antibody is present, such as plasma and serum, from the supernatant of a culture of a hybridoma cell producing an antibody, or can be partially or completely synthesized by a genetic recombination technique, etc. As examples of the antibody, immunoglobulin isotypes and subclasses of these isotypes are preferably mentioned. As the human immunoglobulins, 9 classes (isotypes) are known, which include IgG1, IgG2, IgG3, IgG4, IgA1, IgA2, IgD, IgE and IgM, Of these isotypes, IgG1, IgG2, IgG3 and IgG4 can be included in the antibodies of the present invention.

A method for preparing an antibody having a desired binding activity is known to those skilled in the art. A method for preparing an antibody (anti-IL-6R antibody) binding to IL-6R will be illustrated. An antibody binding to an antigen except IL-6R can be appropriately prepared in accordance with the method illustrated below.

An anti-IL-6R antibody can be obtained as a polyclonal or a monoclonal antibody by use of a known means. As the anti-IL-6R antibody, a monoclonal antibody derived from a mammalian animal is preferably prepared. The monoclonal antibody derived from a mammalian animal includes a monoclonal antibody produced from a hybridoma and a monoclonal antibody produced from a host cell transformed by an expression vector containing an antibody gene in accordance with a genetic engineering method. Note that a "humanized antibody" and a "chimeric antibody" are included in the monoclonal antibody of the present invention.

A hybridoma producing a monoclonal antibody can be prepared by use of a technique known in the art, for example, as follows. First, a mammalian animal is immunized with an IL-6R protein used as a sensitizing antigen in accordance with a conventional immunization method. The obtained immunocyte is fused with a parent cell known in the art in accordance with a conventional cell fusion method. Then, a monoclonal antibody-producing cell is screened by a conventional screening method. In this manner, a hybridoma producing an anti-IL-6R antibody can be selected.

Specifically, a monoclonal antibody is prepared, for example, as follows. First, an IL-6R gene, whose nucleotide sequence is disclosed in SEQ ID NO: 2 (IL-6R_PN; NM_000565.3), is allowed to be expressed to obtain IL-6R protein (represented by SEQ ID NO: 1) to be used as a sensitizing antigen for obtaining an antibody. To describe more specifically, an appropriate host cell is transformed by inserting an IL-6R encoding gene sequence into a known expression vector. A desired human IL-6R protein is purified by a known method from the host cell or the culture supernatant. To obtain soluble IL-6R from the culture supernatant, for example, soluble IL-6R as described in Mullberg et al. (J. Immunol. (1994) 152 (10), 4958-4968), i.e., a protein consisting of 1st to 357th amino acids of the IL-6R polypeptide sequence represented by SEQ ID NO: 1, is expressed in place of the IL-6R protein represented by SEQ ID NO: 1. Also, a purified natural IL-6R protein can be used as a sensitizing antigen.

As a sensitizing antigen for use in immunization of a mammalian animal, the purified IL-6R protein can be used. Also, a partial IL-6R peptide can be used as a sensitizing antigen. At this time, the partial peptide can be also obtained by chemical synthesis based on the amino acid sequence of human IL-6R or obtained by inserting a part of an IL-6R gene into an expression vector and expressing the vector. The region and size of the IL-6R peptide used as a partial peptide are not particularly limited. As a preferable region, an arbitrary sequence that can be selected from the amino acid sequence corresponding to 20-357th amino acids of the amino acid sequence (represented by SEQ ID NO: 1) can be used. The number of amino acids constituting a peptide serving as a sensitizing antigen is preferably at least 5 or more, for example, 6 or more, or 7 or more; more specifically, a peptide having 8 to 50 residues and preferably 10 to 30 residues can be used as a sensitizing antigen.

A fusion protein prepared by fusing a desired partial polypeptide or peptide of an IL-6R protein with a different polypeptide can be used as a sensitizing antigen. To produce the fusion protein to be used as a sensitizing antigen, for example, an Fc fragment of an antibody and a peptide tag can be preferably used. The vector for expressing the fusion protein can be prepared by fusing genes encoding desired two types or more polypeptide fragments in frame and inserting the fusion gene in an expression vector as described above. A method for preparing a fusion protein is described in Molecular Cloning 2nd ed. (Sambrook, J et al., Molecular Cloning 2nd ed., 9.47-9.58 (1989) Cold Spring Harbor Lab. press). A method for obtaining IL-6R used as a sensitizing antigen and an immunization method using IL-6R are specifically described in WO2003/000883, WO2004/022754 and WO2006/006693, etc.

The mammalian animal to be immunized with the sensitizing antigen is not limited to a specific animal and is preferably selected in view of compatibility to the parent cell to be used in cell fusion. Generally, an animal belonging to the rodent family such as a mouse, a rat and a hamster or an animal such as a rabbit or a monkey is preferably used.

An animal as mentioned above is immunized by a sensitizing antigen in accordance with a known method. A mammalian animal is immunized by a general method such as intraperitoneally or subcutaneously injection of a sensitizing antigen. To describe more specifically, a sensitizing antigen is diluted with e.g., PBS (Phosphate-Buffered Saline) or physiological saline at an appropriate dilution rate, if desired, a conventional adjuvant, for example, Freund complete adjuvant, is added thereto, and emulsified, and thereafter, administered to a mammalian animal several times every 4 to 21 days. In immunization with a sensitizing antigen, an appropriate carrier can be used. Particularly when a small molecular-weight partial peptide is used as a sensitizing antigen, the sensitizing antigen is joined to a carrier protein, such as albumin and keyhole limpet hemocyanin, to prepare a sensitizing antigen peptide, which is desirably used for immunization.

Furthermore, a hybridoma producing a desired antibody can be also prepared by use of DNA immunization in accordance with the following manner. DNA immunization is an immunization method of giving immunostimulation by administering vector DNA (which is constructed such that an antigen protein-encoding gene is expressed in an immunized animal) to the animal to be immunized and allowing a sensitizing antigen to express in the immunized animal, *in-vivo.* Compared to a general immunization method of administering a protein antigen to an animal to be immunized, the DNA immunization has the following advantages:

- immunostimulation can be given while maintaining the structure of a membrane protein such as IL-6R.
- Purification of an immunizing antigen is not required.

To obtain the monoclonal antibody of the present invention by DNA immunization, first, DNA expressing an IL-6R protein is administered to the animal to be immunized. IL-6R-encoding DNA can be synthesized by a known method such as PCR. The obtained DNA is inserted to an appropriate expression vector and then administered to the animal to be immunized. As the expression vector, for example, a commercially available expression vector such as pcDNA3.1 can be preferably used. As a method for administering a vector to a living body, a method commonly used can be employed. For example, gold particles on which an expression vector is adsorbed are introduced by a gene gun into animal cells to be immunized. In this manner, DNA immunization is performed. An antibody recognizing IL-6R can be also prepared by use of a method described in International Publication WO2003/104453.

As described above, a mammalian animal is immunized. After an increase of titer of an antibody binding to IL-6R in the serum is confirmed, immunocytes are taken from the mammalian animal and subjected to cell fusion. As preferable immunocytes, particularly, spleen cells can be used.

As the cells to be fused with the immunocytes, mammalian myeloma cells are used. It is preferable that the myeloma cells have an appropriate selection marker for screening. The selection marker refers to a trait that can be maintained (or cannot be maintained) in specific culture conditions. As a selection marker, e.g., hypoxanthine-guanine-phosphoribosyltransferase defective (hereinafter abbreviated as HGPRT defective) marker or a thymidine kinase defective (hereinafter abbreviated as TK defective) marker is known. A HGPRT or TK defective cell has hypoxanthine-aminopterin-thymidine sensitivity (hereinafter abbreviated as HAT sensitivity). A HAT sensitive cell cannot synthesize DNA in a HAT selection medium and die; however, if the HAT sensitive cell is fused with a normal cell, DNA synthesis can be continued by use of salvage pathway of the normal cell. With this mechanism, the HAT sensitive cell comes to grow even in a HAT selection medium.

HGPRT defective and TK defective cells can be selected in mediums containing 6 thioguanine and 8 azaguanine (hereinafter abbreviated as 8AG), respectively or in a medium containing 5' bromodeoxyuridine. Normal cells, which can take these pyrimidine analogues into DNA, die, whereas cells, which lack enzymes and thus cannot take these pyrimidine analogues, can survive in a selection medium. Other than these, a selection marker called a G418 resistant is resistant against 2-deoxystreptamine antibiotic substance (gentamicin analogue) due to the presence of a neomycin resistant gene. Various myeloma cells suitably used for cell fusion are known in the art.

Examples of such myeloma cells that can be preferably used include P3 (P3x63Ag8.653) (J. Immunol. (1979) 123 (4), 1548-1550), P3x63Ag8U.1 (Current Topics in Microbiology and Immunology (1978) 81, 1-7), NS-1 (C. Eur. J. Immunol. (1976) 6 (7), 511-519), MPC-11 (Cell (1976) 8 (3), 405-415), SP2/0 (Nature (1978) 276 (5685), 269-270), FO (J. Immunol. Methods (1980) 35 (1-2), 1-21), S194/5.XX0.BU.1 (J. Exp. Med. (1978) 148 (1), 313-323) and R210 (Nature (1979) 277 (5692), 131-133).

An immunocyte as mentioned above and a myeloma cell are fused basically in accordance with a known method, for example, a method of Kohler and Milstein, et al. (Methods Enzymol. (1981)73, 3-46). More specifically, the cell fusion can be performed in a general nutrition culture solution in the presence of, e.g., a cell fusion accelerator. Examples of the fusion accelerator that can be used include polyethylene glycol (PEG) and Sendai virus (HVJ). To further increase a fusion efficiency, if desired, an adjuvant such as dimethylsulfoxide is added.

The ratio of the immunocytes and myeloma cells to be used can be set arbitrarily. For example, the ratio of immunocytes relative to the myeloma cells is preferably 1 to 10. As the culture solution to be used for the cell fusion, for example, RPMI1640 culture solution and MEM culture solution suitable for proliferation of the myeloma cell strain and other conventional culture solutions for use in culturing such fusion cells, are used. Furthermore, a serum complemental liquid such as fetal bovine serum (FCS), can be preferably added.

Cell fusion is performed as follows. First, predetermined amounts of immunocytes and myeloma cells are mixed well in the culture solution. To the mixture, a PEG solution (for example, average molecular weight of approximately 1000 to 6000) previously heated to approximately 37°C and usually having a concentration of 30 to 60% (w/v), is added. The solution mixture is gently mixed to form a desired fusion cell (hybridoma). Then, an appropriate culture solution as mentioned above is sequentially added. The mixture is centrifuged and the supernatant is removed. This operation is repeated to remove a cell fusion agent, etc., which are unfavorable for hybridoma growth.

The hybridoma thus obtained can be selected by culturing it in a conventional selective culture solution such

as a HAT culture solution (culture solution containing hypoxanthine, aminopterin and thymidine). The culture can be continued in the HAT culture solution for a sufficient time (usually, several day to several weeks) until cells (nonfusion cells) except a desired hybridoma die. Subsequently, screening and monocloning of a hybridoma producing a desired antibody are performed by conventional limiting dilution method.

The hybridoma thus obtained can be selected by use of a selective culture solution in accordance with the selection marker that the myeloma used in cell fusion has. For example, cells defective in e.g., HGPRT and TK can be screened by culturing them in a HAT culture solution (culture solution containing hypoxanthine, aminopterin and thymidine). In other words, when a HAT sensitive myeloma cell is used in cell fusion, the cell successively fused with a normal cell can selectively grow in the HAT culture solution. The culture is continued for a sufficient time until cells (nonfusion cells) except a desired hybridoma die in the HAT culture solution. More specifically, a desired hybridoma can be generally selected by culturing for several days to several weeks. Subsequently, screening and monocloning of a hybridoma producing a desired antibody are carried out by a conventional limiting dilution method.

Screening and monocloning of a desired antibody can be preferably performed by a screening method based on an antigen antibody reaction known in the art. For example, a monoclonal antibody binding to IL-6R can be bound to IL-6R expressed on a cell surface. Such a monoclonal antibody can be screened, for example, by FACS (fluorescence activated cell sorting). FACS is a system, which can analyze binding of an antibody to a cell surface, by bringing cells into contact with a fluorescent antibody, applying laser light to the cells, and measuring fluorescence emitted from individual cells.

To screen a hybridoma producing the monoclonal antibody of the present invention by FACS, first, a cell expressing IL-6R is prepared. A preferable cell for screening is a mammalian animal cell in which IL-6R is forcibly expressed. By using a mammalian animal cell (serving as a host cell) not transformed as a control, the binding activity of an antibody against IL-6R on a cell surface can be selectively detected. More specifically, a hybridoma producing an antibody, which does not bind to a host cell but binds to the cell forcibly expressing IL-6R, is selected to obtain a hybridoma producing an IL-6R monoclonal antibody.

The binding activity of an antibody against IL-6R expression cell (immobilized) can be evaluated based on the principle of ELISA. For example, an IL-6R expression cell is immobilized to wells of an ELISA plate. The supernatant of a hybridoma culture is brought into contact with the immobilized cell in the wells. In this manner, an antibody bound to the immobilized cell is detected. When the monoclonal antibody is derived from a mouse, the antibody bound to the cell can be detected by an anti-mouse immunoglobulin antibody. The hybridoma producing a desired antibody having a binding ability to an antigen and selected by the screening can be cloned by a limiting dilution method, etc.

The hybridoma producing a monoclonal antibody and prepared as mentioned above can be subcultured in a conventional culture solution. Furthermore, the hybridoma can be stored in liquid nitrogen for a long time.

The hybridoma is cultured in accordance with a conventional method. From the culture supernatant, a desired monoclonal antibody can be obtained. Alternatively, the hybridoma is administered to a mammalian animal having a compatibility with it and grown therein. From the ascitic fluid, a monoclonal antibody can be obtained. The former method is preferable to obtain a high-purity antibody.

<u>Recombinant antibody</u>

An antibody encoded by an antibody gene, which is cloned from an antibody-producing cell such as the hybridoma, can be preferably used. The antibody gene cloned is incorporated into an appropriate vector and then introduced into a host. In this manner, the antibody encoded by the gene is expressed. The methods for isolating an antibody gene, for introducing the antibody gene into a vector, and for transforming a host cell have been already established, for example, by Vandamme et al. (Eur. J. Biochem. (1990)192 (3), 767-775). Also, a method for producing a recombinant antibody is known in the art, as described below.

To describe more specifically, cDNA encoding a variable region (V region) of the anti-IL-6R antibody is obtained from a hybridoma cell producing an anti-IL-6R antibody. For this purpose, usually, total RNA is first extracted from the hybridoma. As a method for extracting mRNA from a cell, for example, the following methods can be used.

- Guanidine ultracentrifugation method (Biochemistry (1979) 18 (24), 5294-5299)
- AGPC method (Anal. Biochem. (1987) 162 (1), 156-159)

The mRNA extracted can be purified by an mRNA Purification Kit (manufactured by GE Healthcare), etc. Alternatively, mRNA can be obtained from a hybridoma by use of a commercially available kit (for directly extracting total mRNA from a cell) such as QuickPrep mRNA Purification Kit (manufactured by GE Healthcare). From the obtained mRNA, cDNA encoding an antibody V region can be synthesized by using a reverse transcriptase. Such cDNA can be synthesized by use of an AMV Reverse Transcriptase First-strand cDNA Synthesis kit (manufactured by SEIKAGAKU CORPORATION). For synthesis and amplification of cDNA, a SMART RACE cDNA amplification kit (manufactured by

Clontech) and 5'-RACE method using a PCR (Proc. Natl. Acad. Sci. USA (1988) 85 (23), 8998-9002, Nucleic Acids Res. (1989) 17 (8), 2919-2932) can be appropriately used. Furthermore, in the process for synthesizing cDNA, appropriate restriction enzyme sites (described later) can be introduced into both terminals of the cDNA.

From the obtained PCR product, a desired cDNA fragment is purified and then ligated to vector DNA. A recombinant vector is prepared in this manner and introduced in *Escherichia coli,* etc. After a colony is selected, a desired recombinant vector can be prepared from *Escherichia coli* forming the colony. Subsequently, whether the recombinant vector has the nucleotide sequence of a desired cDNA or not is determined by a known method, for example, a dideoxynucleotide chain termination method, etc.

To obtain a variable region-encoding gene, it is convenient to use 5'-RACE method using a variable region gene amplification primer. First, cDNA is synthesized by using RNA extracted from a hybridoma cell as a template, and then, 5'-RACE cDNA library is obtained. For constructing the 5'-RACE cDNA library, a commercially available kit such as a SMART RACE cDNA amplification kit, is appropriately used.

Using the obtained 5'-RACE cDNA library as a template, an antibody gene is amplified by a PCR method. Based on a known antibody gene sequence, primers for amplifying a mouse antibody gene can be designed. The nucleotide sequences of the primers vary depending upon the subclass of immunoglobulin. Therefore, it is desirable to previously determine the subclass by use of a commercially available kit such as Iso Strip mouse monoclonal antibody isotyping kit (Roche Diagnostics).

To describe it more specifically, in order to obtain, for example, a mouse IgG-encoding gene, the primer capable of amplifying genes encoding $\gamma1$, $\gamma2a$, $\gamma2b$ and $\gamma3$ as heavy chains, and $\kappa$ chain and $\lambda$ chain as light chains can be used. In order to amplify an IgG variable-region gene, a primer annealing to a portion corresponding to a constant region near a variable region is generally used as a 3'-side primer. In contrast, as a 5'-side primer, a primer attached to a 5' RACE cDNA library preparation kit is used.

Using the PCR product thus amplified, an immunoglobulin formed of a combination of heavy chains and light chains can be reconstituted. A desired antibody can be screened based on the binding activity of the reconstituted immunoglobulin to IL-6R. For example, when an antibody against IL-6R is desired, it is further preferable that the binding of the antibody to IL-6R is specific. The antibody binding to IL-6R can be screened, for example, as follows:

(1) a step of bringing an antibody, which contains a V region encoded by the cDNA obtained from a hybridoma, into contact with an IL-6R expression cell,
(2) a step of detecting binding between the IL-6R expression cell and the antibody, and
(3) a step of selecting the antibody binding to the IL-6R expression cell.

A method for detecting the binding between the antibody and the IL-6R expression cell is known in the art. More Specifically, the binding between the antibody and the IL-6R expression cell can be detected by a method such as FACS as mentioned above. To evaluate the binding activity of the antibody, a preparation on which IL-6R expression cells are fixed can be appropriately used.

As a method for screening an antibody based on the binding activity, a panning method using a phage vector is preferably used. When an antibody gene is obtained as a library of a subclass such as a heavy chain and a light chain from a polyclonal antibody expression cells, a screening method using a phage vector is advantageously used.

If genes encoding variable regions of a heavy chain and a light chain are ligated via an appropriate linker sequence, a single chain Fv (scFv) (Nat. Biotechnol. (2005) 23 (9), 1126-1136) can be formed. If the scFv-encoding gene is inserted to a phage vector, a phage expressing a scFv on the surface can be obtained. After the phage is brought into contact with a desired antigen, the phage bound to the antigen is recovered to obtain scFv-encoding DNA having a desired binding activity. This operation is repeated, if necessary, to concentrate scFv having a desired binding activity.

After cDNA encoding a V region of a desired anti-IL-6R antibody is obtained, the cDNA is digested with a restriction enzyme(s), which recognize the restriction enzyme sites inserted to both terminals of the cDNA. A restriction enzyme preferably recognizes and digests a nucleotide sequence that less frequently emerges in the nucleotide sequence constituting an antibody gene. In order to insert a single copy digestion fragment into a vector in a right direction, use of a restriction enzyme providing sticky ends is preferable. An antibody expression vector can be obtained by inserting cDNA encoding a V region of the anti-IL-6R antibody digested as mentioned above into an appropriate expression vector. At this time, if an antibody constant region (C region)-encoding gene and a gene encoding the V region are fused in frame, a chimeric antibody is obtained. The chimeric antibody herein refers to an antibody having a constant region and a variable region derived from different origins. Accordingly, in addition to a xenogeneic chimeric antibody such as a mouse-human chimeric antibody, a human-human homogeneous chimeric antibody is included in the chimeric antibody defined in the present invention. By inserting the V region gene into an expression vector already having a constant region, a chimeric antibody expression vector can be constructed. To explain it more specifically, for example, on the 5' side of the expression vector having DNA encoding a desired antibody constant region (C region), the sequence recognized by a restriction enzyme digesting the V region gene can be appropriately arranged. Both fragments digested

by the same combination of restriction enzymes are fused in frame to construct a chimeric antibody expression vector.

To produce an anti-IL-6R monoclonal antibody, the antibody gene is inserted into an expression vector such that the antibody gene can be expressed under the control of an expression control region. The expression control region for expressing the antibody contains, for example, an enhancer and a promoter. In addition, an appropriate signal sequence can be added to the amino terminal so as to secrete an expressed antibody out of the cell. In Examples (described later), a peptide having an amino acid sequence MGWSCIILFLVATATGVHS (SEQ ID NO: 3) is used as a signal sequence. Other than this, an appropriate signal sequence is added. The polypeptide expressed is cleaved at the carboxyl terminal of the above sequence and the cleaved polypeptide can be secreted as a mature polypeptide out of the cell. Subsequently, an appropriate host cell is transformed by the expression vector to obtain a recombinant cell expressing an anti-IL-6R antibody-encoding DNA.

To express an antibody gene, DNAs encoding an antibody heavy chain (H-chain) and an antibody light chain (L-chain), respectively, are integrated into different expression vectors. A host cell is simultaneously transformed (co-transfected) with the vectors having the H-chain and the L-chain, respectively integrated therein to express an antibody molecule having the H-chain and L-chain. Alternatively, DNAs respectively encoding a H-chain and a L-chain are integrated into a single expression vector and then introduced to a host cell. In this manner, the host cell can be transformed (see, International Publication WO 1994/011523).

For preparing antibodies by introducing an isolated antibody gene into an appropriate host, various combinations of host cells and expression vectors are known in the art. These expression systems all can be applied to isolating the antigen-binding domain of the present invention. When an eukaryotic cell is used as a host cell, an animal cell, a plant cell or a fungus cell can be appropriately used. As the animal cell, for example, the following cells can be illustrated

(1) Mammalian cells: CHO, COS, myeloma, BHK (baby hamster kidney), Hela, Vero, HEK (human embryonic kidney) 293, etc.
(2) Amphibian cells: Xenopus oocyte, etc.
(3) Insect cells: sf9, sf21, Tn5, etc.

Alternatively, antibody gene expression systems using cells derived from the genus *Nicotiana* (e.g., *Nicotiana tabacum*) as the plant cells are known in the art. Cultured callus cells can be appropriately used for the plant cell transformation.

The following cells can be used as the fungus cells:

- cells derived from yeasts of the genus *Saccharomyces* (e.g., *Saccharomyces cerevisiae*) and the genus *Pichia* (e.g., *Pichia pastoris*), and
- cells derived from filamentous fungi of the genus *Aspergillus* (e.g., *Aspergillus niger*).

Also, antibody gene expression systems using prokaryotic cells are known in the art. In the case of using, for example, bacterial cells, cells of bacteria such as *E. coli* and *Bacillus subtilis* can be appropriately used. The expression vectors comprising the antibody gene of interest are transferred into these cells by transformation. The transformed cells are cultured *in vitro,* and the desired antibody can be obtained from the resulting cultures of the transformed cells.

In addition to the host cells, transgenic animals may be used for the recombinant antibody production. Specifically, the desired antibody can be obtained from animals transfected with the gene encoding this antibody. For example, the antibody gene can be inserted in frame into genes encoding proteins specifically produced in milk to construct fusion genes. For example, goat $\beta$ casein can be used as the proteins secreted into milk. DNA fragments comprising the fusion genes having the antibody gene inserted are injected into goat embryos, which are in turn introduced into female goats. From milk produced by transgenic goats (or progeny thereof) brought forth by the goats that have received the embryos, the desired antibody can be obtained as a fusion protein with the milk protein. In addition, hormone can be administered to the transgenic goats in order to increase the amount of milk containing the desired antibody produced from the transgenic goats (Bio/Technology (1994), 12 (7), 699-702).

Humanized antibody and human antibody

In the case of administering the antigen-binding molecule described herein to humans, an antigen-binding domain derived from a genetically recombinant antibody that has been engineered artificially can be appropriately adopted as an antigen-binding domain for the antigen-binding molecule, for example, for the purpose of reducing heteroantigenicity in humans. The genetically recombinant antibody encompasses, for example, humanized antibodies in addition to the chimera antibodies described above. These engineered antibodies are appropriately produced using a method known in the art.

Each antibody variable region used for preparing the antigen-binding domain in the antigen-binding molecule

described herein is typically composed of three complementarity-determining regions (CDRs) flanked by four framework regions (FRs). The CDRs are regions that substantially determine the binding specificity of the antibody. The CDRs have diverse amino acid sequences. On the other hand, the FRs are mostly constituted by amino acid sequences that are highly identical even among antibodies differing in binding specificity. Therefore, it is considered that in general, the binding specificity of a certain antibody can be transplanted to other antibodies through CDR grafting.

The humanized antibodies are also called reshaped human antibodies. Specifically, for example, a humanized antibody consisting of a non-human animal (e.g., mouse) antibody CDR-grafted human antibody is known in the art. General gene recombination approaches are also known for obtaining the humanized antibodies. Specifically, for example, overlap extension PCR is known in the art as a method for grafting mouse antibody CDRs to human FRs. In the overlap extension PCR, a nucleotide sequence encoding each mouse antibody CDR to be grafted is added to primers for human antibody FR synthesis. The primers are prepared with respect to each of the four FRs. For grafting the mouse CDRs to the human FRs, it is generally regarded as advantageous to select human FRs highly identical to mouse FRs, in order to maintain the CDR functions. Specifically, in general, human FRs comprising amino acid sequences highly identical to those of FRs adjacent to the mouse CDRs to be grafted are preferably used.

The nucleotide sequences to be linked are designed so that the sequences are connected in frame with each other. The human FR-encoding nucleotide sequences are individually synthesized using their respective primers. The resulting products contain the mouse CDR-encoding DNA added to each human FR-encoding sequence. The mouse CDR-encoding nucleotide sequences are designed so that the nucleotide sequence in each product overlaps with another. Subsequently, the overlapping CDR portions in the products synthesized with human antibody genes as templates are annealed to each other for complementary strand synthesis reaction. Through this reaction, the human FR sequences are linked via the mouse CDR sequences.

Finally, the full-length sequence of the gene of the V region comprising three CDRs and four FRs linked is amplified using primers that each anneal to the 5' and 3' ends thereof and have an added recognition sequence for an appropriate restriction enzyme. The DNA thus obtained and a human antibody C region-encoding DNA can be inserted into expression vectors such that these DNAs are fused in frame to prepare vectors for human-type antibody expression. These vectors having the inserts are transferred to hosts to establish recombinant cells. Then, the recombinant cells are cultured for the expression of the humanized antibody-encoding DNA to produce the humanized antibodies into the cultures of the cultured cells (See European Patent Publication EP239400 and International Publication WO1996002576).

The humanized antibodies thus prepared can be evaluated for their antigen-binding activity by qualitative or quantitative assay to thereby select suitable human antibody FRs that allow CDRs to form a favorable antigen-binding site when linked via the CDRs. If necessary, FR amino acid residue(s) may be substituted such that the CDRs of the resulting reshaped human antibody form an appropriate antigen-binding site. For example, the amino acid sequence of FR can be mutated by the application of the PCR method used in the mouse CDR grafting to the human FRs. Specifically, a mutation of a partial nucleotide sequence can be introduced to the primers annealing to a FR nucleotide sequence. The FR nucleotide sequence synthesized using such primers contains the mutation thus introduced. Such variant antibodies having the substituted amino acid(s) can be evaluated for their antigen-binding activity by the same assay as above to thereby select variant FR sequences having the desired properties (Cancer Res., (1993) 53, 851-856).

Furthermore, a desired human antibody can be obtained through DNA immunization using a transgenic animal having all repertories of a human antibody gene (see, International Publication WO1993/012227, WO1992/003918, WO1994/002602, WO1994/025585, WO1996/034096, WO1996/033735) as an immunized animal.

Moreover, a technique for obtaining a human antibody by panning using a human antibody library is also known in the art. For example, a human-antibody V region is expressed on the surface of a phage as a single-chain antibody (scFv) by a phage display method. A phage expressing a scFv binding to an antigen can be selected. A DNA sequence encoding a human antibody V region binding to the antigen can be determined by analyzing the gene of the selected phage. After the DNA sequence of the scFv binding to the antigen is determined, the V region sequence is fused with the sequence of a desired human antibody C region in frame and inserted into an appropriate expression vector. In this manner, the expression vector can be prepared. The expression vector is introduced in a preferable expression cell as mentioned above and a gene encoding the human antibody is allowed to express to obtain the human antibody. These methods are already known in the art (see, International Publication WO1992/001047, WO1992/020791, WO1993/006213, WO1993/011236, WO1993/019172, WO1995/001438, WO1995/015388).

As a method for obtaining an antibody gene, a B cell cloning method described in Bernasconi et al. (Science (2002) 298, 2199-2202) or described in WO2008/081008 (identification, cloning, isolation of individual antibody coding sequences and use thereof for constructing expression vectors for preparing individual antibodies (particularly, IgG1, IgG2, IgG3 or IgG4) etc.) can be appropriately used other than those mentioned above.

<u>EU numbering and Kabat numbering</u>

According to the method used in the present invention, amino acid positions assigned for CDR and FR of an

antibody are defined in accordance with Kabat (Sequences of Proteins of Immunological Interest (National Institute of Health, Bethesda, Md., 1987 and 1991). In the specification, when an antigen-binding molecule is an antibody or an antigen binding fragment, the amino acids in a variable region are indicated in accordance with the Kabat numbering, whereas amino acids in a constant region are indicated by the EU numbering in accordance with the Kabat amino acid positions.

FcRn

Unlike an Fcγ receptor belonging to an immunoglobulin superfamily, a human FcRn is structurally analogous to a polypeptide of a major histoincompatible complex (MHC) class I and has a sequence homology of 22 to 29% with class I MHC molecules (Ghetie et al., Immunol. Today (1997) 18 (12), 592-598). FcRn is expressed as a hetero dimer, which is a complex formed of soluble β chain or a light chain (β2 microglobulin) and a transmembrane α chain or a heavy chain. Like MHC, the α chain of FcRn consists of 3 extracellular domains (α1, α2, α3), and a short cytoplasmic domain plays a role of anchoring a protein on a cell surface. The α1 and α2 domains interact with an FcRn binding domain in the Fc region of an antibody (Raghavan et al. (Immunity (1994) 1, 303-315)).

FcRn is expressed in the placenta or yolk sac of a mammalian animal and involved in IgG transfer from a mother to a fetus. In addition, in the small intestine of a new-bone baby rodent in which FcRn is expressed, FcRn is involved in transferring a maternal IgG from ingested first milk or milk across through the brush border epithelium. FcRn is expressed in various tissues other than the aforementioned tissue and various endothelial cells in a wide variety of species. FcRn is also expressed in human adult vascular endothelial cells, muscular and blood vessel system and liver vasis sinusoideum. FcRn binds to IgG and recycles it to the serum. FcRn is thus considered to play a role in maintaining the concentration of IgG in plasma. FcRn binds to an IgG molecule usually definitely depending upon pH and optimal binding is observed in an acidic pH range of less than 7.0.

Human FcRn, which is obtained from a polypeptide containing a signal sequence represented by SEQ ID NO: 4 (FcRn; NP_004098.1) as a precursor (the polypeptide containing a signal sequence is described in SEQ ID NO: 5 (beta2-microglobulin; NP_004039.1)), forms a complex with human β2-microglobulin *in-vivo.* As shown in Examples later, a soluble human FcRn, which forms a complex with β2-microglobulin, is produced by use of a conventional recombinant expression method. The binding activity of the Fc region of the present invention to such soluble human FcRn (which forms a complex with β2-microglobulin) can be evaluated. In the present invention, unless otherwise specified, human FcRn refers to a human FcRn having a structure capable of binding to the Fc region of the present invention. Examples thereof include a complex of human FcRn and human β2-microglobulin.

FcRn binding domain

The antigen-binding molecule of the present invention has an FcRn binding domain. The FcRn binding domain is not particularly limited as long as the antigen-binding molecule has a binding activity to FcRn in an acidic pH range. The FcRn binding domain may be a domain having an activity to directly or indirectly bind to FcRn. As such a domain, the Fc region of an IgG immunoglobulin having an activity to directly bind to FcRn, albumin, albumin domain 3, an anti-FcRn antibody, an anti-FcRn peptide and an anti-FcRn scaffold (Scaffold) molecule are preferably mentioned; or an IgG having an activity to indirectly bind to FcRn and a molecule binding to albumin are mentioned. In the present invention, a domain having a binding activity to FcRn in an acidic pH range and a neutral pH range is preferable. The domain, as long as it already has a binding activity to FcRn in an acidic pH range, can be preferably used as it is. When the domain has no binding activity or a weak binding activity to FcRn in an acidic pH range, it is possible to provide a binding activity to FcRn by modifying the amino acids of the antigen-binding molecule. Alternatively, the FcRn-binding activity may be enhanced by modifying the amino acids of the domain already having a binding activity to FcRn in an acidic pH range. In modifying the amino acids of the FcRn binding domain, a desired modification can be found out by comparing the binding activities to FcRn in an acidic pH range before and after amino acid modification.

The FcRn binding domain is preferably a region which directly binds to FcRn. As a preferable example of the FcRn binding domain, the Fc region of an antibody can be mentioned. However, a region, which can bind to a polypeptide having a binding activity to FcRn, such as albumin and IgG, can indirectly bind to FcRn via albumin or IgG, etc. Accordingly, as the FcRn binding region in the present invention, a region, which can bind to the polypeptide having a binding activity to FcRn, can be preferably used. The Fc region contains an amino acid sequence derived from a constant region of an antibody heavy chain. The Fc region is a portion of a heavy-chain constant region of an antibody ranging from the N terminal of a hinge region (papain cleavage portion) including the hinge, CH2 and CH3 domains and consisting of approximately 216 amino acids represented by the EU numbering.

In the present invention, the binding activity of the FcRn binding domain to FcRn (particularly human FcRn), can be measured by a method known to those skilled in the art, as described in the section of "Binding activity". Conditions other than pH can be appropriately determined by those skilled in the art. The antigen-binding activity and human FcRn-binding activity of an antigen-binding molecule are evaluated based on e.g., KD (Dissociation constant), apparent KD (Apparent dissociation constant), kd (Dissociation rate) or apparent kd (Apparent dissociation rate). They can be measured by methods known to those skilled in the art such as Biacore (GE healthcare), Scatchard plot and flow cytometer.

In measuring the binding activity of an FcRn binding domain to FcRn, conditions other than pH can be appropriately determined by those skilled in the art and are not particularly limited. The binding activity can be measured in MES buffer at 37°C, as is described in WO2009/125825. In the present invention, the binding activity of an FcRn binding domain to FcRn can be measured by a method known to those skilled in the art, such as Biacore (GE Healthcare). The binding activity of an FcRn binding domain to FcRn can be measured and evaluated by feeding FcRn, or an FcRn binding domain or the antigen-binding molecule of the present invention containing an FcRn binding domain as an analyte, to chips on which an FcRn binding domain or the antigen-binding molecule of the present invention containing an FcRn binding domain or FcRn is immobilized.

The acidic pH range used as a condition, in which the binding activity of the FcRn binding domain contained in the antigen-binding molecule of the present invention to FcRn is obtained, usually refers to a region of pH 4.0 to pH 6.5, preferably pH 5.5 to pH 6.5, and particularly preferably, pH 5.8 to pH 6.0 which is close to pH value within the early-stage endosome *in-vivo.* The binding affinity of an FcRn binding domain for FcRn may be evaluated at any measurement temperature from 10°C to 50°C. Preferably, the binding affinity of an FcRn binding domain for human FcRn is determined at a temperature of 15°C to 40°C. More preferably, the binding affinity of an FcRn binding domain for FcRn is determined at any temperature from 20°C to 35°C, such as 20, 21, 22, 23, 24, 25, 26, 27, 28, 29, 30, 31, 32, 33, 34, and 35°C. A temperature of 25°C is a non-limiting example of the present invention.

According to Yeung et al. (J. Immunol. (2009) 182, 7663-7671), the binding activity of natural human IgG1 to human FcRn in an acidic pH range (pH 6.0) is KD 1.7 μM; however, the activity cannot be virtually detected in a neutral pH range. Accordingly, in a preferable aspect, it is possible to use the antigen-binding molecule of the present invention having a binding activity to human FcRn under the acidic pH range condition, which includes an antigen-binding molecule whose binding activity to human FcRn under an acidic pH range condition is KD 20 μM or stronger and whose binding activity to human FcRn under a neutral pH range condition is the same as that of a natural human IgG. In more preferable aspect, the antigen-binding molecule of the present invention including an antigen-binding molecule whose human FcRn-binding activity under the acidic pH range condition is KD 2.0 μM or stronger, can be used. In further more preferable aspect, an antigen-binding molecule whose human FcRn-binding activity under the acidic pH range condition is KD 0.5 μM or stronger can be used. The aforementioned KD value is determined in accordance with the method described in The Journal of Immunology (2009) 182: 7663-7671 (an antigen-binding molecule is immobilized to a chip, to which human FcRn is fed as an analyte).

In the present invention, an Fc region having a binding activity to FcRn under an acidic pH range condition is preferable. As the Fc domain, any Fc domain can be used if the domain is an Fc region already having a binding activity to FcRn under an acidic pH range condition, the domain can be used as it is. If the domain has no or weak binding activity to FcRn under an acidic pH range condition, an Fc region having a desired binding activity to FcRn can be obtained by modifying an amino acid in an antigen-binding molecule. Also, an Fc region having a desired or enhanced binding activity to FcRn under an acidic pH range condition can be preferably obtained by modifying an amino acid in the Fc region. The amino acid modification in an Fc region by which such a desired binding activity is brought, can be found by comparing binding activities to FcRn before and after modification of an amino acid under the acidic pH range condition. Those skilled in the art can appropriately modify an amino acid by use of a method known in the art as described in the section of "Modification of amino acid" in the specification.

The Fc region (contained in the antigen-binding molecule of the present invention) having a binding activity to FcRn under the acidic pH range condition, can be obtained by any method. To describe it more specifically, an FcRn binding domain having a binding activity to FcRn or an enhanced binding activity to FcRn under an acidic pH range condition can be obtained by modification of an amino acid of a human IgG immunoglobulin used as a starting Fc region. As a preferable Fc region of the IgG immunoglobulin to be modified, for example, the Fc region of a human IgG (IgG1, IgG2, IgG3, or IgG4, and a modified form of them) are mentioned. Other than these, an amino acid at any position can be modified as long as the binding activity to FcRn is obtained under an acidic pH range condition or the binding activity to human FcRn under the acidic pH range can be enhanced. If an antigen-binding molecule contains human IgG1 Fc region as an Fc region, it is preferable to contain a modification by which an effect of enhancing binding to FcRn under an acidic pH range condition is brought compared to the binding activity of a starting Fc region of human IgG1. Preferable examples of the amino acid that can be modified as mentioned above include amino acids (represented by the EU numbering) at position 238, position 252, position 253, position 254, position 255, position 256, position 265, position 272, position 286, position 288, position 303, position 305, position 307, position 309, position 311, position 312, position 317, position 340, position 356, position 360, position 362, position 376, position 378, position 380, position 382, position 386, position 388, position 400, position 413, position 415, position 424, position 433, position 434, position 435, position 436, position 439 and/or position 447 (as described in WO2000/042072). Similarly, preferable examples of such amino acid that can be modified as mentioned above amino acids (represented by the EU numbering) at position 251, position

252, position 254, position 255, position 256, position 308, position 309, position 311, position 312, position 385, position 386, position 387, position 389, position 428, position 433, position 434, and/or position 436 (described in WO2002/060919). Furthermore, preferable examples of such amino acid that can be modified as mentioned above include amino acids (represented by the EU numbering) at position 250, position 314, and position 428 (described in WO2004/092219). Moreover, preferable examples of such amino acid that can be modified as mentioned above include amino acids (represented by the EU numbering) at position 251, position 252, position 307, position 308, position 378, position 428, position 430, position 434, and/or position 436 (described in WO2010/045193). The binding of the Fc region of an IgG immunoglobulin to FcRn under an acidic pH range condition is enhanced by modification of these amino acids.

The Binding of the Fc region of IgG to FcRn under an acidic pH range condition can be enhanced by using, for example, these modifications of amino acids singly or in combination. The modification of amino acid to be introduced is not particularly limited, and any amino acid modification may be introduced as long as a plasma retentivity improving effect is produced.

According to a non-limiting aspect of the present invention, as an FcRn binding domain, an antigen-binding domain having a binding activity to FcRn, particularly human FcRn, can be also appropriately used. As described above, as the antigen-binding molecule of the present invention, a bispecific antibody can be appropriately used. Of them, a bispecific antibody recognizing two epitopes, one of which is present in a desired antigen except FcRn and the other of which is present in FcRn, is preferably mentioned. The structure of the bispecific antibody is not particularly limited to a specific structure as long as it contains a bivalent binding domain (binding to a desired antigen and FcRn) and a sugar chain receptor-binding domain. For example, a structure of an antibody such as an IgG antibody, in which Fc regions are linked, can be used. Besides, e.g., "scFv2 (single chain Fv 2)", "diabody", or "F(ab')2" can be preferably used. When a structure of an antibody such as an IgG antibody is used, a sugar chain binding domain can be contained not only in an Fc region but also in an FcRn binding domain and/or an antigen-binding domain. Furthermore, when a structure of "scFv2 (single chain Fv 2)", "diabody", or "F(ab')2" is used, a sugar chain binding domain can be also contained in an FcRn binding domain and/or an antigen-binding domain.

When such a bispecific antibody is used, as an FcRn binding domain, an FcRn binding domain, whose binding to FcRn changes depending upon the ion-concentration condition (as described later in the section of "Conditions of ion concentration") can be appropriately used. More specifically, according to a non-limiting aspect of the present invention, an FcRn binding domain, whose binding to FcRn changes depending upon the condition of metal ion concentration and pH condition, can be used (as later described in the section of "Conditions of ion concentration").

<u>Condition of ion concentration</u>

<u>(1) Condition of metal-ion concentration</u>

In a non-limiting aspect of the present invention, the ion concentration refers to a metal-ion concentration. The "metal ion" refers to an ion of a metal element belonging to Group I including alkali metals except hydrogen and coppers; Group II including alkali earth metals and zincs; Group III except boron; Group IV except carbon and silicon; Group VIII including iron and platinum; elements belonging to a sub A group of each of Group V, VI and VII, and metal elements such as antimony, bismuth and polonium. A metal atom is disposed to release an atomic electron(s) into a cation. This property is called ionization tendency. A metal having large ionization tendency is said to be chemically highly active.

In the present invention, a calcium ion is mentioned as a preferable example of such a metal ion. The calcium ion is involved in control of many life phenomena, more specifically involved in contraction of muscle such as skeleton muscle, smooth muscle and cardiac muscle, activation of movement and phagocytosis of leucocytes, activation of deformation and secretion of platelets, activation of lymphocytes, activation of mast cells such as secretion of histamine, cellular response via a catecholamine $\alpha$ receptor and an acetylcholine receptor, exocytosis, release of a transmittance from a neuron terminal and axonal flow of a neuron. As an intracellular calcium ion receptor, Troponin C, calmodulin, parvalbumin and myosin light-chain, etc. are known, which have a plurality of calcium ion-binding sites and are presumably derived from the same origin in view of molecular evolution. Many binding motifs of them are also known. For example, a cadherin domain, EF hand contained in calmodulin, a C2 domain contained in Protein kinase C, a Gla domain contained in blood coagulation protein, Factor IX, a C type lectin contained in an asialoglycoprotein receptor and a mannose receptor, an A domain contained in an LDL receptor, annexin, a thrombospondin type III domain and an EGF-like domain are known well.

In the present invention, when the metal ion is a calcium ion, as a calcium-ion concentration condition, the low calcium-ion concentration condition and a high calcium-ion concentration condition are mentioned. The expression: "the binding activity changes depending upon a calcium-ion concentration condition" means that the binding activity of an antigen-binding molecule to an antigen changes depending upon the difference in conditions between a low calcium-ion concentration and a high calcium-ion concentration. For example, a case where the binding activity of an antigen-binding molecule to an antigen is higher under a high calcium-ion concentration condition than the binding activity of the

antigen-binding molecule to the antigen under a low calcium-ion concentration condition, is mentioned. Alternatively, a case where the binding activity of an antigen-binding molecule to an antigen under a low calcium-ion concentration condition is higher than (the binding activity of the antigen-binding molecule to the antigen) under a high calcium-ion concentration condition, is mentioned.

In the specification, the high calcium-ion concentration is not limited to a single numerical value and can be preferably a concentration selected from the range between 100 μM to 10 mM. In another aspect, the high calcium-ion concentration can be a concentration selected from the range between 200 μM to 5 mM. In another aspect, the high calcium-ion concentration can be a concentration selected from the range between 500 μM to 2.5 mM, and in another aspect, from 200 μM to 2 mM, and further from 400 μM to 1.5 mM. In particular, a concentration selected from the range between 500 μM to 2.5 mM, which is close to the calcium-ion concentration of *in-vivo* plasma (in blood), is preferably mentioned.

In the specification, the low calcium-ion concentration is not limited to a single numerical value and can be preferably a concentration selected from the range between 0.1 μM to 30 μM. In another aspect, the low calcium-ion concentration can be a concentration selected from the range between 0.2 μM to 20 μM. In another aspect, the low calcium-ion concentration can be a concentration selected from the range between from 0.5 μM to 10 μM, and in another aspect, from 1 μM to 5 μM, and further from 2 μM to 4 μM. In particular, a concentration selected from the range between 1 μM to 5 μM, which is close to the calcium-ion concentration of an early-stage endosome *in vivo,* is preferably mentioned.

In the present invention, the expression: the binding activity to an antigen under a low calcium-ion concentration condition is lower than the binding activity to the antigen under a high calcium-ion concentration condition, means that the binding activity of an antigen-binding molecule to an antigen in a calcium-ion concentration selected from the range between 0.1 μM to 30 μM is weaker than the binding activity to the antigen in a calcium-ion concentration selected from the range between 100 μM to 10 mM. Preferably, the binding activity of an antigen-binding molecule to an antigen in a calcium-ion concentration selected from the range between 0.5 μM to 10 μM is weaker than (the binding activity of the antigen-binding molecule to the antigen) in a calcium-ion concentration selected from the range between 200 μM to 5 mM. Particularly preferably, the antigen-binding activity in a calcium-ion concentration within the early-stage endosome *in vivo* is weaker than (the antigen-binding activity) in a calcium-ion concentration within plasma *in-vivo.* More specifically, the binding activity of an antigen-binding molecule to an antigen in a calcium-ion concentration selected from the range between 1 μM to 5 μM is weaker than the binding activity to the antigen in a calcium-ion concentration selected from the range between 500 μM to 2.5 mM.

Whether the binding activity of the antigen-binding domain of the present invention to an antigen changes or not depending upon the metal ion-concentration condition can be determined by use of a measurement method known in the art (as described, for example, in the above section of "Binding activity"). For example, to determine that the binding activity of an antigen-binding molecule containing the antigen-binding domain of the present invention to an antigen under a high calcium-ion concentration condition changes in a higher level than the binding activity of the antigen-binding molecule containing the antigen-binding domain to the antigen under a low calcium-ion concentration condition, the binding activity of the antigen-binding molecule containing the antigen-binding domain to an antigen is compared between under the low calcium-ion concentration condition and under a high calcium-ion concentration condition.

In the present invention, the expression: "the binding activity to an antigen under the low calcium-ion concentration condition is lower than the binding activity to the antigen under a high calcium-ion concentration condition" can be rephrased by the expression that the binding activity of an antigen-binding molecule to an antigen under a high calcium-ion concentration condition is higher than the binding activity to the antigen under a low calcium-ion concentration condition. Note that, in the present invention "the binding activity to an antigen under a low calcium-ion concentration condition is lower than the binding activity to the antigen under a high calcium-ion concentration condition" is sometimes described as "the binding ability to an antigen under a low calcium-ion concentration condition is weaker than the binding ability to the antigen under a high calcium-ion concentration condition"; and "the antigen-binding activity to an antigen under a low calcium-ion concentration condition is lowered than the antigen-binding activity under a high calcium-ion concentration condition" is sometimes described as "the binding ability to an antigen under a low calcium-ion concentration condition is weaken than the antigen binding ability under a high calcium-ion concentration condition".

Conditions for measuring the binding activity to an antigen other than the calcium-ion concentration can be appropriately selected by those skilled in the art and are not particularly limited. For example, measurement can be performed in a HEPES buffer at 37°C. For example, measurement can be performed by use of Biacore (GE Healthcare), etc. The binding activity of an antigen-binding molecule containing an antigen-binding domain to an antigen, in the case the antigen is a soluble antigen, can be measured and evaluated by feeding the antigen-binding molecule containing an antigen-binding domain as an analyte to a chip having the antigen immobilized thereto. If the antigen is a membrane antigen, the binding activity to the membrane antigen can be evaluated by feeding the antigen-binding molecule containing an antigen-binding domain as an analyte to a chip having the antigen immobilized thereto.

In the antigen-binding molecule of the present invention, as long as the binding activity to an antigen in a low calcium-ion concentration condition is weaker than the binding activity to the antigen in a high calcium-ion concentration

condition, the ratio of the binding activity to the antigen under the low calcium-ion concentration condition relative to the binding activity to the antigen under the high calcium-ion concentration condition is not particularly limited; however, the ratio is expressed by the ratio of KD (Dissociation constant) of the antigen in the low calcium-ion concentration condition relative to KD in the high calcium-ion concentration condition, i.e., a value of KD (Ca 3 μM)/KD (Ca 2 mM). This value (KD (Ca 3 μM)/KD (Ca 2 mM)) is preferably 2 or more, more preferably 10 or more and further preferably is 40 or more. The upper limit of the value of KD ((Ca 3 μM)/KD (Ca 2 mM)) is not particularly limited as long as preparation can be made by technique of those skilled in the art. Any value such as 400, 1000, 10000 can be employed. Furthermore, the ratio can be specified by a value of KD (Ca3 μM)/KD (Ca 1.2 mM). The value of KD (Ca 3 μM)/KD (Ca 1.2 mM) is 2 or more, more preferably 10 or more and further preferably 40 or more. The upper limit of the value of KD (Ca 3 μM)/KD (Ca 1.2 mM) is not particularly limited. As long as preparation can be made by technique of those skilled in the art, any value such as 400, 1000, 10000 can be employed.

As a value showing the binding activity to an antigen, KD (dissociation constant) can be used if the antigen is a soluble antigen; however, if the antigen is a membrane antigen, an apparent KD (Apparent dissociation constant) can be used. The KD (dissociation constant) and apparent KD (apparent dissociation constant) can be determined by a method known to those skilled in the art, for example, determined by use of Biacore (GE healthcare), Scatchard plot and a flow cytometer.

Furthermore, as another index showing the ratio of the binding activity of the antigen-binding molecule of the present invention to an antigen in a low-calcium concentration condition relative to the binding activity in a high-calcium concentration condition, for example, a dissociation rate constant, i.e., kd (Dissociation rate constant), is further preferably used. When kd (dissociation rate constant) is used in place of KD (dissociation constant) as the index showing the ratio of the binding activity, the ratio of kd (dissociation rate constant) in a low-calcium concentration condition to an antigen relative to kd (dissociation rate constant) in a high-calcium concentration condition, i.e., a value of kd (low-calcium concentration condition)/kd (high-calcium concentration condition), is preferably 2 or more, more preferably 5 or more, further preferably 10 or more and still more preferably 30 or more. The upper limit of the value of kd (low-calcium concentration condition)/kd (high-calcium concentration condition) is not particularly limited. As long as preparation can be made by common technical knowledge of those skilled in the art, any value such as 50, 100 and 200 may be used.

As the value of an antigen-binding activity, kd (dissociation rate constant) can be used if the antigen is a soluble antigen; however, if the antigen is a membrane antigen, an apparent kd (Apparent dissociation rate constant) can be used. The kd (dissociation rate constant) and apparent kd (apparent dissociation rate constant) can be measured by a method known to those skilled in the art, such as Biacore (GE healthcare), Scatchard plot and a flow cytometer. Note that, in the present invention, when the binding activity of an antigen-binding molecule to an antigen is measured in different calcium-ion concentrations, conditions other than the calcium concentrations are preferably the same.

For example, according to an aspect of the present invention, an antigen-binding domain or antibody whose binding activity to an antigen in a low calcium-ion concentration condition is lower than the binding activity to the antigen in a high calcium-ion concentration condition, can be obtained by a screening for the antigen-binding domain or the antibody comprising the following steps (a) to (c):

(a) a step of obtaining the antigen-binding activity of each of antigen-binding domains or antibodies in a low-calcium concentration condition,
(b) a step of obtaining the antigen-binding activity of each of the antigen-binding domains or antibodies in a high-calcium concentration condition, and
(c) a step of selecting an antigen-binding domain or antibody having a lower antigen-binding activity in the condition of the low-calcium concentration condition than (the antigen-binding activity) in the high-calcium concentration condition.

According to an aspect of the present invention, an antigen-binding domain or antibody whose binding activity to an antigen in a low calcium-ion concentration condition is lower than the binding activity to the antigen in a high calcium-ion concentration condition, can be obtained by a screening for the antigen-binding domain, the antibody or libraries of these comprising the following steps (a) to (c):

(a) a step of bringing antigen-binding domains, antibodies or libraries of them into contact with an antigen in a high-calcium concentration condition,
(b) a step of placing the antigen-binding domains or antibodies that bind to the antigen in the step (a), under a low-calcium concentration condition, and
(c) a step of isolating an antigen-binding domain or antibody that dissociates in the step (b).

According to an aspect of the present invention, an antigen-binding domain or antibody whose binding activity to an antigen in a low calcium-ion concentration condition is lower than the binding activity to the antigen in a high

calcium-ion concentration condition can be obtained by a screening for the antigen-binding domain or antibody or libraries of them comprising the following steps (a) to (d):

(a) a step of bringing antigen-binding domains or an antibody library into contact with an antigen in a low-calcium concentration condition,
(b) a step of selecting the antigen-binding domain or antibody that does not bind to the antigen in the step (a),
(c) a step of binding the antigen-binding domain or antibody selected in the step (b) to the antigen under a high-calcium concentration condition, and
(d) a step of isolating the antigen-binding domain or antibody that binds to the antigen in the step (c).

According to an aspect of the present invention, an antigen-binding domain or antibody whose binding activity to an antigen in a low calcium-ion concentration condition is lower than the binding activity to the antigen in a high calcium-ion concentration condition, can be obtained by a screening process comprising the following steps (a) to (c):

(a) a step of bringing antigen-binding domains or an antibody library into contact with a column having the antigen immobilized thereto in a high-calcium concentration condition,
(b) a step of releasing the antigen-binding domain or antibody that binds to the column in the step (a) by elution from the column in a low-calcium concentration condition, and
(c) a step of isolating the antigen-binding domain or antibody released by elution in the step (b).

According to an aspect of the present invention, an antigen-binding domain or antibody whose binding activity to an antigen in a low calcium-ion concentration condition is lower than the binding activity to the antigen in a high calcium-ion concentration condition, can be obtained by a screening process comprising the following steps (a) to (d):

(a) a step of passing antigen-binding domains or an antibody library through a column having an antigen immobilized thereto, in a low-calcium concentration condition,
(b) a step of recovering the antigen-binding domain or antibody that is released by elution without binding to the column in the step (a),
(c) a step of binding the antigen-binding domain or antibody recovered in the step (b) to the antigen in a high-calcium concentration condition, and
(d) a step of isolating the antigen-binding domain or antibody that binds to the antigen in the step (c).

According to an aspect of the present invention, an antigen-binding domain or antibody whose binding activity to an antigen in a low calcium-ion concentration condition is lower than the binding activity to the antigen in a high calcium-ion concentration condition, can be obtained by a screening process comprising the following steps (a) to (d):

(a) a step of bringing antigen-binding domains or an antibody library into contact with an antigen in a high-calcium concentration condition,
(b) a step of obtaining antigen-binding domains or antibodies that bind to the antigen in the step (a),
(c) a step of placing the antigen-binding domain or antibody obtained in the step (b) under a low-calcium concentration condition, and
(d) a step of isolating the antigen-binding domain or antibody whose antigen-binding activity in the step (c) is weaker than the standard selected in the step (b).

Note that the aforementioned steps may be repeated twice or more. Thus, according to the present invention, an antigen-binding domain or antibody, whose binding activity to an antigen in a low calcium-ion concentration condition is lower than the binding activity to the antigen in a high calcium-ion concentration condition, obtained by any one of the aforementioned screening processes further comprising a step of repeating the steps (a) to (c) or (a) to (d), twice or more, is provided. The number of repeats of (a) to (c) or (a) to (d) steps is not particularly limited; however, the number of repeats is usually within 10 times.

In the screening method of the present invention, the antigen-binding activity of an antigen-binding domain or an antibody in a low-calcium concentration condition is not particularly limited, as long as it is the antigen-binding activity obtained at a calcium-ion concentration between 0.1 μM and 30 μM. The antigen-binding activity obtained at a more preferable calcium-ion concentration between 0.5 μM and 10 μM is mentioned. As a more preferable calcium-ion concentration, a calcium-ion concentration within early-stage endosome *in vivo* is mentioned. More specifically, the antigen-binding activity obtained at a calcium-ion concentration of 1 μM to 5 μM can be mentioned. In contrast, the antigen-binding activity of an antigen-binding domain or an antibody under a high-calcium concentration condition is not particularly limited, as long as it is the antigen-binding activity obtained at a calcium-ion concentration between 100 μM and 10 mM.

The antigen-binding activity obtained at a preferable calcium-ion concentration between 200 μM and 5 mM is mentioned. As a more preferable calcium-ion concentration, a calcium-ion concentration within plasma *in-vivo* can be mentioned. More specifically, the antigen-binding activity obtained at a calcium-ion concentration of 0.5 mM to 2.5 mM can be mentioned.

The binding activity of an antigen-binding domain or an antibody can be measured by a method known to those skilled in the art. Conditions other than a calcium-ion concentration can be appropriately determined by those skilled in the art. The antigen-binding activity of an antigen-binding domain or an antibody can be evaluated based on e.g., KD (Dissociation constant), apparent KD (Apparent dissociation constant), a dissociation rate, kd (Dissociation rate) or apparent kd (Apparent dissociation). These can be measured by a method known to those skilled in the art such as Biacore (GE healthcare), Scatchard plot, FACS.

In the present invention, the step of selecting an antigen-binding domain or antibody, whose antigen-binding activity under a high-calcium concentration condition is higher than (the antigen-binding activity) under a low-calcium concentration condition, is equivalent to a step of selecting an antigen-binding domain or antibody whose antigen-binding activity under a low-calcium concentration condition is lower than (the antigen-binding activity) under a high-calcium concentration condition.

As long as the antigen-binding activity under a high-calcium concentration condition is higher than (the antigen-binding activity) under a low-calcium concentration condition, difference between the antigen-binding activity under a high-calcium concentration condition and the antigen-binding activity under a low-calcium concentration condition is not particularly limited; however, the antigen-binding activity under a high-calcium concentration condition is preferably twice or more as high as the antigen-binding activity under a low-calcium concentration condition, more preferably, 10 fold or more and further preferably 40 fold or more.

The antigen-binding domain or antibody of the present invention to be screened by any one of the screening methods mentioned above is not particularly limited. For example, any one of the aforementioned antigen-binding domains or antibodies can be screened. For example, an antigen-binding domain or an antibody having a natural sequence can be screened or an antigen-binding domain or antibody whose amino acid sequence is substituted, may be screened.

<u>(2) Amino acid that changes the binding activity of antigen-binding domain to antigen depending upon the calcium-ion concentration condition</u>

The antigen-binding domain or antibody of the present invention to be screened by any one of the screening methods as mentioned above may be prepared in any manner. For example, if a metal ion is a calcium ion, antibodies already exist; libraries (phage library, etc.) already exist; antibodies or libraries prepared from hybridomas prepared by immunization of animals or the B cells of immunized animals; and antibodies or libraries prepared by introducing an amino acid (e.g., aspartic acid and glutamine acid) capable of chelating calcium and a non-natural amino acid mutation into these antibodies, (a library prepared by introducing an amino acid (e.g., aspartic acid and glutamine acid) capable of chelating calcium, a library whose non-natural amino acid content is increased or a library prepared by introducing an amino acid (e.g., aspartic acid and glutamine acid) capable of chelating calcium or a non-natural amino acid mutation into a specific site), can be used.

As the amino acid that changes the binding activity of an antigen-binding molecule to an antigen depending upon the ion-concentration condition as described above, if a metal ion is a calcium ion, as long as it is an amino acid forming a calcium-binding motif, the type of amino acid is not limited. The calcium-binding motifs are known to those skilled in the art and more specifically described in the literatures: (for example, Springer et al. (Cell (2000) 102, 275-277), Kawasaki and Kretsinger (Protein Prof. (1995) 2, 305-490), Moncrief, et al. (J. Mol. Evol. (1990) 30, 522-562), Chauvaux, et al. (Biochem. J. (1990) 265, 261-265), Bairoch and Cox (FEBS Lett. (1990) 269, 454-456), Davis (New Biol. (1990) 2, 410-419), Schaefer, et al. (Genomics (1995) 25, 638 to 643), Economou, et al. (EMBO J. (1990) 9, 349-354), Wurzburg, et al. (Structure. (2006) 14, 6, 1049-1058)). More specifically, any known calcium-binding motifs such as C type lectins including ASGPR, CD23, MBR and DC-SIGN can be contained in the antigen-binding molecule of the present invention. As a preferable example of such a calcium-binding motif other than the aforementioned ones, a calcium-binding motif contained in the antigen-binding domain represented by SEQ ID NO: 6 can be mentioned.

As the amino acid that changes the binding activity of an antigen-binding molecule to an antigen depending upon the calcium-ion concentration condition, an amino acid having a metal chelating function is also preferably used. Preferable examples of the amino acid having a metal chelating function include serine (Ser (S)), threonine (Thr (T)), asparagine (Asn (N)), glutamine (Gln (Q)), aspartic acid (Asp (D)) and glutamine acid (Glu (E)).

The position of the antigen-binding domain containing an amino acid is not particularly limited to a specific position. As long as the binding activity of an antigen-binding molecule to an antigen is changed depending upon the calcium-ion concentration condition, any position in a heavy-chain variable region or a light-chain variable region forming the antigen-binding domain may be acceptable. More specifically, the antigen-binding domain of the present invention can be obtained from a library mainly consisting of antigen-binding molecules mutually different in sequence and containing an amino acid that changes the binding activity of an antigen-binding molecule to an antigen depending upon the calcium-ion concentration condition in a heavy chain antigen-binding domain. According to another non-limiting aspect, the antigen-binding domain of the present invention can be obtained from a library mainly consisting of antigen-binding molecules mutually different in sequence and containing the amino acid in a heavy chain CDR3. According to another non-limiting aspect, the antigen-binding domain of the present invention can be obtained from a library mainly consisting of antigen-binding molecules mutually different in sequence and containing the amino acid in heavy chain CDR3 at the position 95, position 96, position 100a, and/or position 101 (represented by Kabat numbering).

In a non-limiting aspect of the present invention, the antigen-binding domain of the present invention can be obtained from a library mainly consisting of antigen-binding molecules mutually different in sequence and containing an amino acid that changes the binding activity of an antigen-binding molecule to an antigen depending upon the calcium-ion concentration condition, in a light chain antigen-binding domain. In another aspect, the antigen-binding domain of the present invention can be obtained from a library mainly consisting of antigen-binding molecules mutually different in sequence and containing the amino acid in light chain CDR1. In another aspect, the antigen-binding domain of the present invention can be obtained from a library mainly consisting of antigen-binding molecules mutually different in sequence and containing the amino acid in light chain CDR1 at the position 30, position 31, and/or position 32 (represented by Kabat numbering).

In another non-limiting aspect, the antigen-binding domain of the present invention can be obtained from a library mainly consisting of antigen-binding molecules mutually different in sequence and containing the amino acid residue in light chain CDR2. In another aspect, a library mainly consisting of antigen-binding molecules mutually different in sequence and containing the amino acid residue in light chain CDR2, at the position 50 (represented by Kabat numbering) is provided.

In another non-limiting aspect, the antigen-binding domain of the present invention can be obtained from a library mainly consisting of antigen-binding molecules mutually different in sequence and containing the amino acid residue in light chain CDR3. In another aspect, the antigen-binding domain of the present invention can be obtained from a library mainly consisting of antigen-binding molecules mutually different in sequence and containing the amino acid residue in light chain CDR3 at the position 92 (represented by Kabat numbering).

The antigen-binding domain of the present invention can be obtained from a library mainly consisting of antigen-binding molecules mutually different in sequence and containing the amino acid residue in a 2 or 3 CDRs selected from light chain CDR1, CDR2 and CDR3 mentioned above, as a different aspect of the present invention. Furthermore, the antigen-binding domain of the present invention can be obtained from a library mainly consisting of antigen-binding molecules mutually different in sequence and containing the amino acid residue in a light chain at at least one position of the position 30, position 31, position 32, position 50, and/or position 92 (represented by Kabat numbering).

As long as these amino acid residues form a calcium-binding motif and/or as long as the binding activity of an antigen-binding molecule to an antigen changes depending upon the calcium-ion concentration condition, these amino acid residues can be contained alone and in combination of two or more. Furthermore, troponin C, calmodulin, parvalbumin and myosin light-chain, etc., which have a plurality of calcium ion-binding sites and are presumably derived from the same origin in view of molecular evolution, are known and light chain CDR1, CDR2 and/or CDR3 can be designed so as to contain its binding motif(s). For example, a cadherin domain, EF hand contained in calmodulin, a C2 domain contained in Protein kinase C, a Gla domain contained in a blood coagulation protein, Factor IX, a C type lectin contained in an asialoglycoprotein receptor and a mannose receptor, an A domain contained in an LDL receptor, Annexin, thrombospondin type III domain and EGF-like domain are appropriately used for the purpose described above.

In one aspect of the present invention, the antigen-binding domain of the present invention can be obtained from a library containing a plurality of antigen-binding molecules of the present invention having mutual different sequences and obtained by using a heavy-chain variable region (which is selected as a framework sequence already containing "at least one amino acid residue that changes the binding activity of an antigen-binding molecule to an antigen depending upon the ion-concentration condition") in combination with a light-chain variable region (which is prepared as a randomized variable region sequence library). As a non-limiting example thereof, in the case where the ion concentration is a calcium-ion concentration, a library, which is obtained by using a heavy-chain variable region sequence represented by, for example, SEQ ID NO: 7 (6RL#9-IgG1) or SEQ ID NO: 8 (6KC4-1#85-IgG1) in combination with a light-chain variable region prepared as a randomized variable region sequence library is preferably mentioned. A library can be also prepared by appropriately selecting a light-chain variable region from the light-chain variable regions having a genital cell lineage sequence in place of the light-chain variable region prepared as the randomized variable region sequence library. For example, a library obtained by using the heavy-chain variable region sequence represented by SEQ ID NO: 7 (6RL#9-IgG1) or SEQ ID NO: 8 (6KC4-1#85-IgG1) in combination with a light-chain variable region having a genital cell lineage sequence is preferably mentioned.

In the specification, the "library" refers to a plurality of antigen-binding molecules or a plurality of fusion polypeptides each containing an antigen-binding molecule or nucleic acids and polynucleotides encoding these sequences. The sequences of the antigen-binding molecules or the fusion polypeptides each containing an antigen-binding molecule

contained in the library are not the same and mutually different. In the library, a method for exposing a fusion polypeptide containing an antibody fragment on the surface of a bacteriophage is known in the art and described, for example, in WO1992001047 and this specification. Other than these, relevant methods are described in WO1992020791, WO1993006213, WO1993011236 and 1993019172. Those skilled in the art can appropriately use these methods. Other documents (H. R. Hoogenboom & G. Winter (1992) J. Mol. Biol. 227, 381-388, WO1993006213 and WO1993011236) disclose identification of antibodies against various antigens exposed on a phage surface by a variable region gene repertory artificially rearranged.

(3) Hydrogen ion-concentration condition

In one aspect of the present invention, the ion-concentration condition refers to a hydrogen ion-concentration condition or a pH condition. In the present invention, the concentration condition of an atomic nucleus of a proton, i.e., a hydrogen atom, is regarded as the same as the condition of hydrogen index (pH). If the active mass of a hydrogen ion in an aqueous solution is represented by $aH^+$, pH is defined as - log10aH+. If the ion intensity of the aqueous solution is low (for example, lower than $10^{-3}$), aH+ is almost equal to hydrogen ion intensity. For example, since the ionic product of water at 25°C under 1 atmospheric pressure, Kw = aH + aOH = 10-14, the ionic product in pure water is aH+ = aOH = 10-7. In this case, pH = 7 is neutral. The aqueous solution having a pH value smaller than 7 is acidic; whereas, the aqueous solution having a pH value larger than 7 is alkaline.

In the present invention, when a pH condition is used as the ion-concentration condition, high hydrogen-ion concentration or low pH, i.e., an acidic pH range condition; a low hydrogen-ion concentration or higher pH, i.e., an neutral pH range condition are used as the pH condition. The expression: "binding activity changes depending upon the pH condition" means that the binding activity of an antigen-binding molecule to an antigen changes depending upon difference in condition, more specifically difference between a high hydrogen-ion concentration or low pH (an acidic pH range condition) and a low hydrogen-ion concentration or high pH (neutral pH range condition). For example, a case where the binding activity of an antigen-binding molecule to an antigen in a neutral pH range condition is higher than (the binding activity of the antigen-binding molecule to the antigen) in an acidic pH range condition, is mentioned. Another case where the binding activity of an antigen-binding molecule to an antigen in an acidic pH range condition is higher than (the binding activity of the antigen-binding molecule to the antigen) in a neutral pH range condition, is also mentioned.

In the specification, the neutral pH range, which is not limited to a single numerical value, can be selected preferably from pH 6.7 to pH 10.0, and selected from pH 6.7 to pH 9.5 in another aspect, pH 7.0 to pH 9.0 in another aspect, and pH 7.0 to pH 8.0 in another aspect. Particularly, pH 7.4, which is close to pH of plasma (in blood) *in-vivo*, is preferably mentioned.

In the specification, the acidic pH range, which is not limited to a single numerical value, can be selected preferably from pH 4.0 to pH 6.5, and selected from pH 4.5 to pH 6.5 in another aspect, pH 5.0 to pH 6.5 in another aspect, and pH 5.5 to pH 6.5 in another aspect. Particularly, pH 5.8, which is close to pH of calcium-ion concentration within early-stage endosome *in-vivo*, is preferably mentioned.

In the present invention, the expression: the binding activity of an antigen-binding molecule to an antigen in a high hydrogen-ion concentration condition or low pH (acidic pH range) is lower than the binding activity to the antigen in a low hydrogen-ion concentration condition or high pH (a neutral pH range), means that the binding activity of an antigen-binding molecule to an antigen at a pH selected from pH 4.0 to pH 6.5 is weaker than the binding activity to the antigen at a pH selected from pH 6.7 to pH 10.0; preferably, means that the binding activity of an antigen-binding molecule to an antigen at a pH selected from pH 4.5 to pH 6.5 is weaker than the binding activity to the antigen at a pH selected from pH 6.7 to pH 9.5; more preferably, means that the binding activity of an antigen-binding molecule to an antigen at a pH selected from pH 5.0 to pH 6.5 is weaker than the binding activity to the antigen at a pH selected from pH 7.0 to pH 9.0; further preferably means that the binding activity of an antigen-binding molecule to an antigen at a pH selected from pH 5.5 to pH 6.5 is weaker than (the binding activity to an antigen) at a pH selected from pH 7.0 to pH 8.0; particularly preferably means that antigen-binding activity at pH within early-stage endosome *in-vivo* is weaker than (the antigen-binding activity) at pH within plasma *in-vivo*; and, to be more specific, means that the binding activity of an antigen-binding molecule to an antigen at pH 5.8 is weaker than the binding activity to the antigen at pH 7.4.

Whether the binding activity of the antigen-binding domain of the present invention to an antigen changes or not depending upon the pH condition, can be determined by use of a measurement method known in the art as described, for example, in the above section "Binding activity". To explain this more specifically, in the measurement method, the binding activity is measured in different pH conditions. For example, to confirm that the binding activity of an antigen-binding molecule containing the antigen-binding domain of the present invention to an antigen under a neutral pH range condition changes in a higher level than (the binding activity of the antigen-binding molecule containing the antigen-binding domain of the present invention) to the antigen under an acidic pH range condition, the binding activity of the antigen-binding molecule to the antigen under the acidic pH range condition is compared to that under the neutral pH range condition.

In the present invention, the expression: "the binding activity to an antigen in a high hydrogen-ion concentration condition (or low pH, i.e., acidic pH range), is lower than the binding activity to the antigen in a low hydrogen-ion concentration condition (or high pH, i.e., a neutral pH range)" can be expressed by the expression "the binding activity to an antigen in a low hydrogen-ion concentration condition (or high pH, i.e., a neutral pH range) is higher than the binding activity to the antigen in a high hydrogen-ion concentration condition (or low pH, i.e., acidic pH range)". Note that, in the present invention, "the binding activity to an antigen in a high hydrogen-ion concentration condition (or low pH, i.e., acidic pH range) is lower than the binding activity to the antigen in a low hydrogen-ion concentration condition (or high pH, i.e., a neutral pH range)" can be sometimes expressed by the expression: "the binding activity to an antigen in a high hydrogen-ion concentration condition (or low pH, i.e., acidic pH range) is weaker than (the binding capacity to the antigen) in a low hydrogen-ion concentration condition (or high pH, i.e., a neutral pH range)"; or the expression: "the binding activity to an antigen in a high hydrogen-ion concentration condition (or low pH, i.e., acidic pH range), is lowered than the binding activity to the antigen in a low hydrogen-ion concentration condition (or high pH, i.e., a neutral pH range)" is sometimes expressed by "the binding activity to an antigen in a high hydrogen-ion concentration condition (or low pH, i.e., acidic pH range), is weakened than (the binding activity to the antigen) in a low hydrogen-ion concentration condition (or high pH, i.e., a neutral pH range).

Conditions other than the hydrogen-ion concentration or pH in measuring the binding activity to an antigen can be appropriately selected by those skilled in the art and are not particularly limited. For example, measurement can be made in HEPES buffer at 37°C and for example by use of Biacore (GE Healthcare), etc. The binding activity of an antigen-binding molecule containing an antigen-binding domain to an antigen, if the antigen is a soluble antigen, can be measured and evaluated by feeding the antigen as an analyte to a chip in which the antigen-binding molecule containing an antigen-binding domain is immobilized. If the antigen is a membrane antigen, the binding activity to the membrane antigen can be evaluated by feeding an antigen-binding molecule containing an antigen-binding domain as an analyte to a chip in which the antigen is immobilized.

In the antigen-binding molecule of the present invention, as long as the binding activity to an antigen in a high hydrogen-ion concentration condition (or low pH, i.e., acidic pH range) is weaker than the binding activity to the antigen in a low hydrogen-ion concentration condition (or high pH, i.e., a neutral pH range), the ratio of the binding activity to the antigen in a high hydrogen-ion concentration condition (or low pH, i.e., acidic pH range) relative to the binding activity to an antigen in a low hydrogen-ion concentration condition (or high pH, i.e., a neutral pH range) is not particularly limited; however, preferably, a KD (pH 5.8)/KD (pH 7.4) value (which is a ratio of KD in a high hydrogen-ion concentration condition (or low pH, i.e., acidic pH range) relative to KD in a low hydrogen-ion concentration condition (or high pH, i.e., a neutral pH range)) is 2 or more; more preferably 10 or more and further preferably 40 or more. The upper limit of the value of KD (pH 5.8)/KD (pH 7.4) value is not particularly limited. As long as preparation can be made by technique of those skilled in the art, any value such as 400, 1000 and 10000 can be employed.

As the value of the binding activity to an antigen, if the antigen is a soluble antigen, KD (dissociation constant) can be used; however, if the antigen is a membrane antigen, an apparent KD (Apparent dissociation constant) can be used. The KD (dissociation constant) and apparent KD (apparent dissociation constant) can be determined by a method known to those skilled in the art, for example, by use of Biacore (GE healthcare), Scatchard plot and a flow cytometer.

As another index showing the ratio of the binding activity to the antigen in a high hydrogen-ion concentration condition (or low pH, i.e., acidic pH range) relative to the binding activity to an antigen in a low hydrogen-ion concentration condition (or high pH, i.e., a neutral pH range), for example, a dissociation rate constant kd (Dissociation rate constant) can be further preferably used. When kd (dissociation rate constant) is used in place of KD (dissociation constant) as an index showing the ratio of the binding activity, a value of kd (acidic pH range)/kd (a neutral pH range) (which is the ratio of kd (dissociation rate constant) in a high hydrogen-ion concentration condition (or low pH, i.e., acidic pH range) relative to kd (dissociation rate constant) in a low hydrogen-ion concentration condition (or high pH, i.e., a neutral pH range)) is preferably 2 or more, more preferably 5 or more, further preferably 10 or more and still more preferably 30 or more. The upper limit of the value of kd (acidic pH range)/kd (a neutral pH range) is not particularly limited. As long as preparation can be made based on common technical knowledge of those skilled in the art, any value such as 50, 100 and 200 can be employed.

As the value of an antigen-binding activity, kd (dissociation rate constant) can be used if the antigen is a soluble antigen; however, if the antigen is a membrane antigen, an apparent kd (Apparent dissociation rate constant) can be used. The kd (dissociation rate constant) and apparent kd (apparent dissociation rate constant) can be determined by a method known to those skilled in the art, for example, by use of Biacore (GE healthcare) and a flow cytometer. Note that, in the present invention, when the binding activity of an antigen-binding molecule to an antigen is measured in different hydrogen-ion concentrations (i.e., pH), conditions other than the hydrogen-ion concentration (i.e., pH) are preferably the same.

For example, according to an aspect of the present invention, an antigen-binding domain or an antibody, whose binding activity to an antigen in a high hydrogen-ion concentration condition (or low pH, i.e., acidic pH range) is lower than the binding activity to the antigen in a low hydrogen-ion concentration condition (or high pH, i.e., a neutral pH range),

can be obtained by a screening for the antigen-binding domain or the antibody, comprising the following steps (a) to (c):

(a) a step of obtaining the antigen-binding activity of each of antigen-binding domains or antibodies in an acidic pH-range condition,
(b) a step of obtaining the antigen-binding activity of each of the antigen-binding domains or antibodies in a neutral pH range condition, and
(c) a step of selecting an antigen-binding domain or antibody having a lower antigen-binding activity in the acidic pH-range condition than (the antigen-binding activity) in the neutral pH range condition.

According to an aspect of the present invention, an antigen-binding domain or an antibody, whose binding activity to an antigen in a high hydrogen-ion concentration condition (or low pH, i.e., acidic pH range) is lower than the binding activity to the antigen in a low hydrogen-ion concentration condition (or high pH, i.e., a neutral pH range), can be obtained by a screening process for the antigen-binding domain, the antibody or a library thereof, comprising the following steps (a) to (c) :

(a) a step of bringing antigen-binding domains, antibodies or a library thereof into contact with an antigen in a neutral pH range condition,
(b) a step of placing the antigen-binding domains or antibodies that bind to the antigen in the step (a) under an acidic pH range condition, and
(c) a step of isolating the antigen-binding domain or antibody that dissociates in the step (b).

According to an aspect of the present invention, an antigen-binding domain or antibody, whose binding activity to an antigen in a high hydrogen-ion concentration condition (or low pH, i.e., acidic pH range) is lower than the binding activity to the antigen in a low hydrogen-ion concentration condition (or high pH, i.e., a neutral pH range), can be obtained by a screening for the antigen-binding domain or antibody or a library thereof, comprising the following steps (a) to (d):

(a) a step of bringing antigen-binding domains or an antibody library into contact with an antigen in an acidic pH-range condition,
(b) a step of selecting the antigen-binding domain or antibody that does not bind to the antigen in the step (a),
(c) a step of binding the antigen-binding domain or antibody selected in the step (b) to the antigen in a neutral pH range condition, and
(d) a step of isolating the antigen-binding domain or antibody that binds to the antigen in the step (c).

According to an aspect of the present invention, an antigen-binding domain or an antibody, whose binding activity to an antigen in a high hydrogen-ion concentration condition (or low pH, i.e., acidic pH range) is lower than the binding activity to the antigen in a low hydrogen-ion concentration condition (or high pH, i.e., a neutral pH range), can be obtained by a screening process, comprising the following steps (a) to (c):

(a) a step of bringing antigen-binding domains or an antibody library into contact with a column having the antigen immobilized thereto in a neutral pH range condition,
(b) a step of releasing the antigen-binding domain or antibody that binds to the column in the step (a) by elution from the column in the acidic pH range, and
(c) a step of isolating the antigen-binding domain or antibody released by elution in the step (b).

According to an aspect of the present invention, an antigen-binding domain or an antibody, whose binding activity to an antigen in a high hydrogen-ion concentration condition (or low pH, i.e., acidic pH range) is lower than the binding activity to the antigen in a low hydrogen-ion concentration condition (or high pH, i.e., a neutral pH range), can be obtained by a screening process, comprising the following steps (a) to (d):

(a) a step of passing antigen-binding domains or an antibody library through a column having an antigen immobilized thereto in an acidic pH range condition,
(b) a step of recovering the antigen-binding domain or antibody that is released by elution without binding to the column in the step (a), and
(c) a step of binding the antigen-binding domain or antibody recovered in the step (b) to the antigen in a neutral pH range condition, and
(d) a step of isolating the antigen-binding domain or antibody bound to the antigen in the step (c).

According to an aspect of the present invention, an antigen-binding domain or an antibody, whose binding

activity to an antigen in a high hydrogen-ion concentration condition (or low pH, i.e., acidic pH range) is lower than the binding activity to the antigen in a low hydrogen-ion concentration condition (or high pH, i.e., a neutral pH range), can be obtained by a screening process, comprising the following steps (a) to (d):

(a) a step of bringing antigen-binding domains or an antibody library into contact with an antigen in a neutral pH range condition,
(b) a step of obtaining the antigen-binding domains or antibodies that bind to the antigen in the step (a),
(c) a step of placing the antigen-binding domain or antibody obtained in the step (b) under the acidic pH range condition, and
(d) a step of isolating the antigen-binding domain or antibody whose antigen-binding activity in the step (c) is weaker than the standard selected in the step (b).

Note that the aforementioned steps may be repeated twice or more. Thus, according to the present invention, an antigen-binding domain or an antibody, whose binding activity to an antigen in an acidic pH-range condition is lower than the binding activity to the antigen in a neutral pH range condition, obtained by any one of the aforementioned screening processes further comprising a step of repeating the steps (a) to (c) or (a) to (d), twice or more is provided. The number of repeats of (a) to (c) or (a) to (d) steps is not particularly limited; however, the number of repeats is usually within 10 times.

In the screening method of the present invention, the binding activity of an antigen-binding domain or an antibody in a high hydrogen-ion concentration condition (or low pH, i.e., acidic pH range), is not particularly limited, as long as the antigen-binding activity is obtained in the pH range between 4.0 to 6.5, and preferably 4.5 to 6.6, still preferably 5.0 to 6.5, further preferably, 5.5 to 6.5, more preferably the pH of early-stage endosome *in-vivo*, more specifically, pH 5.8. Furthermore, the binding activity of an antigen-binding domain or an antibody in a low hydrogen-ion concentration condition (or high pH, i.e., a neutral pH range) is not particularly limited, as long as the antigen-binding activity is obtained in the pH range between 6.7 to 10, and preferably 6.7 to 9.5, still preferably 7.0 to 9.5, further preferably, 7.0 to 8.0, more preferably the pH of plasma *in-vivo*, more specifically, pH 7.4.

The binding activity of an antigen-binding domain or an antibody can be measured by a method known to those skilled in the art. Conditions other than a calcium-ion concentration can be appropriately determined by those skilled in the art. The binding activity of an antigen-binding domain or an antibody can be evaluated based on KD (Dissociation constant), apparent KD (Apparent dissociation constant), a dissociation rate, kd (Dissociation rate) or apparent kd (Apparent dissociation). These can be measured by a method known to those skilled in the art such as Biacore (GE healthcare), Scatchard plot, FACS, etc.

In the present invention, the step of selecting an antigen-binding domain or antibody whose antigen-binding activity in a low hydrogen-ion concentration condition (or high pH, i.e., a neutral pH range) is higher than (the antigen-binding activity) in a high hydrogen-ion concentration condition (or low pH, i.e., acidic pH range) is equivalent to a step of selecting an antigen-binding domain or antibody whose antigen-binding activity in a high hydrogen-ion concentration condition (or low pH, i.e., acidic pH range) is lower than (the antigen-binding activity) in a low hydrogen-ion concentration condition (or high pH, i.e., a neutral pH range).

As long as the antigen-binding activity in a low hydrogen-ion concentration condition (or high pH, i.e., a neutral pH range) is higher than (the antigen-binding activity) in a high hydrogen-ion concentration condition (or low pH, i.e., acidic pH range), difference between the antigen-binding activity in a low hydrogen-ion concentration condition (or high pH, i.e., a neutral pH range) and the antigen-binding activity in a high hydrogen-ion concentration condition (or low pH, i.e., acidic pH range), is not particularly limited; however, preferably the antigen-binding activity in a low hydrogen-ion concentration condition (or high pH, i.e., a neutral pH range) is twice or more as high as the antigen-binding activity in a high hydrogen-ion concentration condition (or low pH, i.e., acidic pH range), more preferably, 10 times or more, and further preferably 40 times or more.

The antigen-binding domain or antibody of the present invention to be screened by the screening method is not particularly limited. For example, the aforementioned antigen-binding domains or antibodies can be screened. For example, antigen-binding domains or antibodies having a natural sequence can be screened. Alternatively, antigen-binding domains or antibodies whose amino acid sequence is substituted may be screened.

The antigen-binding domain or antibody of the present invention to be screened by any one of the screening method mentioned above may be prepared in any manner. For example, antibodies already exist; libraries (phage library, etc.) already exist; antibodies or libraries prepared from hybridomas (prepared by immunization of animals) or the B cells of immunized animals; and antibodies or libraries prepared by introducing an mutation into an amino acid (for example histidine and glutamine acid) or a non-natural amino acid whose side chain has a pKa of 4.0-8.0 to these antibodies and libraries (e.g., a library rich in an amino acid (e.g., histidine and glutamine acid) whose side chain has a pKa of 4.0-8.0 or rich in non-natural amino acids; and a library to which an amino acid (e.g., histidine and glutamine acid) whose side chain has a pKa of 4.0-8.0 or a non-natural amino acid mutation, is introduced into a specific site); can

be used.

As a method for obtaining an antigen-binding domain or antibody having an antigen-binding activity in a low hydrogen-ion concentration condition (or high pH, i.e., a neutral pH range) higher than (the antigen-binding activity) in a high hydrogen-ion concentration condition (or low pH, i.e., acidic pH range), from a hybridoma prepared by immunization of an animal or the B cell of an immunized animal, a method as described, for example, a method described in WO2009/125825 is preferably mentioned, in which such an antigen-binding domain or antibody is obtained in accordance with this method by substituting at least one amino acid of an antigen-binding domain or an antibody with an amino acid whose side chain has a pKa of 4.0-8.0 (for example histidine and glutamine acid) or a non-natural amino acid mutation or by inserting an amino acid whose side chain has a pKa of 4.0-8.0 (for example histidine and glutamine acid) or a non-natural amino acid in an antigen-binding domain or an antibody.

The position, to which an amino acid whose side chain has a pKa of 4.0-8.0 (for example histidine and glutamine acid) or a non-natural amino acid mutation is to be introduced, is not particularly limited. Any position may be accepted as long as the antigen-binding activity in an acidic pH range becomes weaker than (the antigen-binding activity) in a neutral pH range ((the value of (KD (acidic pH range)/KD(a neutral pH range) increases, or the value of kd (acidic pH range)/kd (a neutral pH range) increases) compared to before the substitution or insertion. For example, if the antigen-binding molecule is an antibody, e.g., a variable region and CDR of the antibody are preferably mentioned. The number of amino acids to be substituted with an amino acid (for example histidine and glutamine acid) or a non-natural amino acid whose side chain has a pKa of 4.0-8.0, or the number of amino acids to be inserted can be appropriately determined by those skilled in the art. Substitution can be made by a single amino acid whose side chain has a pKa of 4.0-8.0 (for example histidine and glutamine acid) and a single non-natural amino acid; and insertion can be made by a single amino acid whose side chain has a pKa of 4.0-8.0 (for example histidine and glutamine acid) and a single non-natural amino acid. Substitution can be made by a plurality of amino acids (2 or more) having a pKa of a side chain of 4.0-8.0 (for example histidine and glutamine acid) and non-natural amino acids; and insertion can be made by 2 or more amino acids having a pKa of a side chain of 4.0-8.0 (for example histidine and glutamine acid) and non-natural amino acids. In addition, other than substation or insertion of an amino acid(s) (for example histidine and glutamine acid) or a non-natural amino acid whose side chain has a pKa of 4.0-8.0 (s), deletion, addition, insertion and/or substitution, etc. of other amino acid(s) can be made at the same time. The substation or insertion of an amino acid(s) (for example histidine and glutamine acid) or a non-natural amino acid whose side chain has a pKa of 4.0-8.0 (s) can be made at random by a histidine scanning method, etc., which is a substantially the same method as an alanine scanning method known to those skilled in the art except that alanine is replaced by histidine, etc. From the antigen-binding domains or antibodies, to which a mutation is introduced at random by substation or insertion of an amino acid (for example histidine and glutamine acid) or a non-natural amino acid whose side chain has a pKa of 4.0-8.0, an antigen-binding molecule having a larger KD (acidic pH range)/KD (a neutral pH range) or kd (acidic pH range)/kd (a neutral pH range) value, compared to before introduction of mutation, can be selected.

As a preferable example of the antigen-binding molecule, to which a mutation with an amino acid (for example histidine and glutamine acid) or a non-natural amino acid whose side chain has a pKa of 4.0-8.0, is introduced, and whose antigen-binding activity in the acidic pH range is lower than the antigen-binding activity in the neutral pH range, as described above, an antigen-binding molecule whose antigen-binding activity in the neutral pH range after mutation with an amino acid (for example histidine and glutamine acid) or a non-natural amino acid whose side chain has a pKa of 4.0-8.0 is equivalent to the antigen-binding activity (in the neutral pH range before mutation with an amino acid (for example histidine and glutamine acid) or a non-natural amino acid whose side chain has a pKa of 4.0-8.0), is preferably mentioned. In the present invention, the expression: an antigen-binding molecule after mutation with an amino acid (for example histidine and glutamine acid) or a non-natural amino acid whose side chain has a pKa of 4.0-8.0 has the equivalent antigen-binding activity before mutation with an amino acid (for example histidine and glutamine acid) or a non-natural amino acid whose side chain has a pKa of 4.0-8.0 means that the antigen-binding activity of the antigen-binding molecule after the mutation with an amino acid whose side chain has a pKa of 4.0-8.0 (for example histidine and glutamine acid) or a non-natural amino acid is at least 10% or more, preferably 50% or more, further preferably 80% or more, and more preferably 90% or more, based on the antigen-binding activity of an antigen-binding molecule before mutation with an amino acid (for example histidine and glutamine acid) or a non-natural amino acid whose side chain has a pKa of 4.0-8.0, which is regarded as 100%. The antigen-binding activity after the mutation with an amino acid (for example histidine and glutamine acid) or a non-natural amino acid whose side chain has a pKa of 4.0-8.0 at pH 7.4 may be higher than (the antigen-binding activity) before the mutation (with an amino acid (for example histidine and glutamine acid) or a non-natural amino acid whose side chain has a pKa of 4.0-8.0) at pH 7.4. If the antigen-binding activity of an antigen-binding molecule is lowered by substation or insertion (with an amino acid (for example histidine and glutamine acid) or a non-natural amino acid whose side chain has a pKa of 4.0-8.0), the antigen-binding activity can be increased to the same level as the antigen-binding activity before substitution or insertion (with an amino acid (for example histidine and glutamine acid) or a non-natural amino acid whose side chain has a pKa of 4.0-8.0) by providing substitution, deletion, addition and/or insertion, etc. of a single or a plurality of amino acids to the antigen-binding molecule. The

present invention includes the antigen-binding molecule, which acquires the same binding activity by substitution, deletion, addition and/or insertion, etc. of a single or a plurality of amino acids after substation or insertion with an amino acid (for example histidine and glutamine acid) or a non-natural amino acid whose side chain has a pKa of 4.0-8.0.

When an antigen-binding molecule contains an antibody constant region, as another preferable aspect of the antigen-binding molecule whose antigen-binding activity in an acidic pH range is lower than (the antigen-binding activity) in a neutral pH range, an antigen-binding molecule having a modified antibody constant region is mentioned. As a specific example of the modified antibody constant region, for example, the constant regions represented by SEQ ID NO: 9, 10, 11, or 12 are preferably mentioned.

(4) Amino acid changing the binding activity of an antigen-binding domain to an antigen depending upon the hydrogen ion-concentration condition

The antigen-binding domain or antibody of the present invention to be screened by any one of the above screening methods may be prepared in any manner. For example, if the ion-concentration condition is a hydrogen ion-concentration condition or a pH condition, antibodies already exist; libraries (phage library, etc.) already exist; antibodies or libraries prepared from hybridomas (prepared by immunization of animals) or the B cells of immunized animals; and antibodies or libraries prepared by introducing an amino acid (for example histidine and glutamine acid) or a non-natural amino acid mutation whose side chain has a pKa of 4.0-8.0 to these antibodies and libraries (e.g., library rich in an amino acid and non-natural amino acid whose side chain has a pKa of 4.0-8.0 (for example, histidine and glutamine acid); and a library to which an amino acid (for example histidine and glutamine acid) or a non-natural amino acid mutation whose side chain has a pKa of 4.0-8.0 is introduced in a predetermined site); can be used. As the amino acid having such an electron donating amino acid, not only natural amino acids such as histidine or glutamine acid but also non-natural amino acids such as a histidine analog (US20090035836) or m-NO2-Tyr (pKa 7.45), 3,5-Br2-Tyr (pKa 7.21) or 3,5-I2-Tyr (pKa 7.38) (Bioorg. Med. Chem. (2003) 11 (17), 3761-2768) are preferably illustrated. In the non-natural amino acids, it is known that pKa can be artificially controlled (Angew. Chem. Int. Ed. (2005) 44, 34, Chem Soc Rev. (2004) 33 (7), 422-430, Amino Acids. (1999) 16 (3-4), 345-379).

According to a non-limiting aspect of the present invention, a library containing a plurality of antigen-binding molecules of the present invention mutually different in sequence can be prepared also by using a light-chain variable region "to which at least one amino acid residue capable of changing the binding activity of an antigen-binding molecule to an antigen depending upon the hydrogen ion-concentration condition" is introduced, in combination with a heavy-chain variable region, which is prepared as a randomized variable region sequence library.

As a non-limiting example of the amino acid residue, an amino acid residue contained in CDR1 of a light chain is illustrated. Other than this, as a non-limiting example of the amino acid residue, an amino acid residue contained in CDR2 of a light chain is illustrated. In another non-limiting example of the amino acid residue, an amino acid residue contained in CDR3 of a light chain is also illustrated.

As a non-limiting example of the amino acid residue contained in CDR1 of a light chain as described above, the amino acid residues in CDR1 of a light-chain variable region at the position 24, position 27, position 28, position 31, position 32, and/or position 34 (represented by Kabat numbering) are mentioned. Furthermore, as a non-limiting example of the amino acid residue contained in CDR2 of a light chain, the amino acid residues in CDR2 of a light-chain variable region at the position 50, position 51, position 52, position 53, position 54, position 55, and/or position 56 (represented by Kabat numbering) are mentioned. As a non-limiting example of the amino acid residue contained in CDR3 of a light chain, the amino acid residues in CDR3 of a light-chain variable region at the position 89, position 90, position 91, position 92, position 93, position 94, and/or position 95A (represented by Kabat numbering) are mentioned. As long as these amino acid residues change the binding activity of an antigen-binding molecule to an antigen depending upon the hydrogen ion-concentration condition, these amino acid residues can be contained singly or in combination of two or more.

In the present invention, as non-limiting examples of the site to be substituted with histidine or a non-natural amino acid, for example, the following sites described in WO2009/125825 are mentioned. Note that, the amino acid positions are specified in accordance with the Kabat numbering.

Heavy chain: H27, H31, H32, H33, H35, H50, H58, H59, H61, H62, H63, H64, H65, H99, H100b, H102

Light chain: L24, L27, L28, L32, L53, L54, L56, L90, L92, L94

Of these modification sites, H32, H61, L53, L90 and L94 are considered as highly universal modification sites; however, the modification sites are not limited to these and can be appropriately designed depending upon the purpose.

Although it is not particularly limited, as preferable modification sites when the antigen is an IL-6 receptor (for example, human IL-6 receptor), the following sites can be mentioned.

Heavy chain: H27, H31, H32, H35, H50, H58, H61, H62, H63, H64, H65, H100b, H102

Light chain: L24, L27, L28, L32, L53, L56, L90, L92, L94

When a plurality of sites in combination are substituted by histidine or a non-natural amino acid, specific examples of preferable combinations can include a combination of H27, H31 and H35, a combination of H27, H31, H32, H35, H58,

H62 and H102, a combination of L32 and L53, and a combination of L28, L32 and L53. Furthermore, as an example of a preferable combination of substation sites in a heavy chain and a light chain, a combination of H27, H31, L32 and L53 can be mentioned. One and a plurality of sites of these sites can be substituted with histidine or non-natural amino acid.

When an antigen-binding molecule contains an antibody constant region, as another method for changing the binding of an antigen-binding molecule to an antigen depending upon the ion-concentration condition, a method for modifying an amino acid in an antibody constant region can be also mentioned. Specific examples of such an antibody constant region can include antibody constant regions (SEQ ID NO: 13, SEQ ID NO: 14, SEQ ID NO: 15, SEQ ID NO: 16) having a substitution(s), which is inserted by the method as described in Examples WO2009/125825. As a method for modifying an antibody constant region, for example, a method of investigating a plurality of isotypes (IgG1, IgG2, IgG3, IgG4) of a constant region and selecting an isotype reducing the antigen-binding activity in an acidic pH range (a dissociation rate in the acidic pH range increases) is mentioned. Another method of reducing the antigen-binding activity in an acidic pH range (a dissociation rate in the acidic pH range increases) by introducing an amino acid substitution in a wild type isotype amino acid sequence (wild type IgG1, IgG2, IgG3, IgG4 amino acid sequence) is mentioned. The sequence of the hinge region of an antibody constant region greatly varies between isotypes (IgG1, IgG2, IgG3, IgG4). Since the difference in amino acid sequence of the hinge region has a large effect upon antigen-binding activity, if an isotype is appropriately selected in consideration of the types of antigen and epitope, the antigen-binding activity in an ion-concentration condition, for example, the acidic pH range, can be decreased (dissociation rate in the acidic pH range increases). Furthermore, since the difference in amino acid sequence of the hinge region has a large effect upon antigen-binding activity, as the amino acid substitution site in the amino acid sequence of a wild type isotype, a hinge region is presumably desirable.

An antigen-binding molecule whose binding activity to an antigen changes depending upon the ion-concentration condition, can be prepared by introducing a substitution or insertion of an amino acid(s) in an antigen-binding molecule not having a such a property by use of a method as mentioned above. Another method, a method for directly obtaining an antigen-binding molecule having such a property is mentioned. For example, an antibody having a desired property can be directly obtained by immunizing, an animal (mouse, rat, hamster, rabbit, transgenic mouse with a human immunoglobulin, transgenic rat with a human immunoglobulin, transgenic rabbit with a human immunoglobulin, lama, camel, etc.) with an antigen to obtain antibodies and screening the antibodies based on the binding to the antigen in an ion concentration depending manner. Furthermore, an antibody having a desired property can be directly obtained from an antibody library prepared *in-vitro* by screening based on the binding to the antigen performed in an ion concentration depending manner. Such a method is not particularly limited.

Modification of amino acid

For modification of amino acid(s) of an antigen-binding domain, a method known in the art such as a site-directed mutation inducing method (Kunkel, et al. (Proc. Natl. Acad. Sci. USA (1985) 82, 488-492)) and an Overlap extension PCR can be appropriately employed. As a modification method for substituting with an amino acid(s) except natural amino acid(s), a plurality of known methods can be employed (Annu. Rev. Biophys. Biomol. Struct. (2006) 35, 225-249, Proc. Natl. Acad. Sci. U.S.A. (2003) 100 (11), 6353-6357). For example, an acellular translation system (Clover Direct (Protein Express)) containing tRNA having a non-natural amino acid bound to a complementary amber suppressor tRNA of a termination codon, i.e., UAG codon (amber codon) is preferably used.

Neutralization activity

In a non-limiting aspect of the present invention, a pharmaceutical composition, which contains an antigen-binding molecule containing an FcRn binding domain, an antigen-binding domain whose binding activity to an antigen changes depending upon the ion-concentration condition, and one or more sugar chain receptor-binding domains whose binding activity to a sugar chain receptor changes depending upon the ion-concentration condition, is provided. Generally, the neutralization activity refers to an activity that inhibits the biological activity of a ligand such as a virus and a toxin having a biological activity against cells. More specifically, a substance having a neutralization activity refers to a substance which binds to a ligand or a receptor to which the ligand binds to thereby inhibit binding between the ligand and the receptor. The receptor whose binding with the ligand is inhibited by the neutralization activity cannot exhibit a biological activity via the receptor. If the antigen-binding molecule is an antibody, an antibody having such a neutralization activity is generally called as a neutralizing antibody. The neutralization activity of a test substance is determined by comparing the biological activity in the presence of a ligand and in the presence or absence of the test substance.

For example, as a major ligand for IL-6R, IL-6 represented by SEQ ID NO: 17 is conceivably and preferably mentioned. IL-6R is a type I membrane protein (in which the amino terminal thereof forms an extracellular domain) forms a hetero tetramer together with a gp130 receptor (which is induced to form a dimer by IL-6) (HEINRICH, et al. (Biochem. J. (1998) 334, 297-314)). When the hetero tetramer is formed, Jak associated with the gp130 receptor is activated. Jak

phosphorylates itself and a receptor. The phosphorylated sites of the receptor and Jak each serve as a binding site to a molecule belonging to a Stat family having SH2 such as Stat 3, MAP kinase and PI3/Akt, and other proteins and adaptors having SH2. Subsequently, Stat that binds to a gp130 receptor is phosphorylated by Jak. The phosphorylated Stat forms a dimer and migrates into a nucleus to control transcription of a target gene. Jak or Stat can be involved in a signal cascade via a receptor of another class. The signal cascade of IL-6 out of control is observed in the pathological condition of an autoimmune disease, inflammation, multiple myeloma and cancer such as prostatic cancer. Stat3, which may serve as a cancer gene, is constantly activated in many cancers. In prostatic cancer and multiple myeloma, there is a crosstalk between the signal cascade of IL-6R and the signal cascade of an epithelial growth factor receptor (EGFR) family member (Ishikawa, et al. (J. Clin. Exp. Hematopathol. (2006) 46 (2), 55-66)).

Since such a signal cascade within a cell varies depending upon the type of cell, a target molecule can be appropriately set every desired target cell and is not limited to the forementioned factors. Neutralization activity can be evaluated by measuring activation of an *in-vivo* signal. Also, the activation of the *in-vivo* signal can be detected based on transcription inducing effect on a target gene present downstream of the *in-vivo* signal cascade. A change of transcription activity of a target gene can be detected based on the principle of reporter assay. Specifically, a reporter gene, such as GFP (Green Fluorescence Protein) or luciferase, is arranged downstream of a transcription factor or a promoter region of a target gene and the reporter activity is measured. In this manner, a change of transcription activity can be measured as reporter activity. As a measurement kit for *in-vivo* signal activation, a commercially available kit can be appropriately used (for example, Mercury Pathway Profiling Luciferase System (Clontech), etc.).

The neutralization activity of a ligand for a receptor (such as EGF receptor family) working on a signal cascade (generally activating cell growth) is measured by determining growth activity of a target cell. In this manner, the neutralization activity of the neutralizing antibody can be evaluated. For example, the suppression effect against growth of cells, which is promoted by a growth factor of an EGF family such as HB-EGF, can be evaluated or determined based on the neutralization activity of an anti HB-EGF antibody preferably in accordance with the following method. The cell growth suppression activity can be evaluated or determined *in-vitro* by measuring uptake of thymidine labeled with [3H] and added to a medium by living cells, as an index for determining a DNA replication ability. As a further simple method, a dye-exclusion test and an MTT method are used. The former one is a method of measuring the ability of a cell to exclude a dye such as trypan blue out of the cell under a microscope. The latter one is a method using an ability of a living cell to convert a tetrazolium salt, i.e., MTT (3-(4,5-dimethylthiazol-2-yl)-2,5-diphenyltetrazolium bromide) into formazan emitting blue color. To describe it more specifically, a test antibody is added together with a ligand to a culture fluid of a test cell. After the passage of a predetermine time, an MTT solution is added to the culture fluid and allowed to stand still for a predetermined time to allow the cells to take MTT. As a result, MTT (yellow compound) is converted into a blue compound by succinic acid dehydrogenase present within mitochondria in the cell. After the blue product is dissolved to allow the product to emit color, its absorbance is measured and used as an index of the number of living cells. Other than MTT, reagents such as MTS, XTT, WST-1 and WST-8 are commercially available (Nacalai Tesque, etc.) and can be preferably used. In measuring the activity, a binding antibody, which is an antibody having the same isotype as the anti HB-EGF antibody and having no cell growth suppression activity is used as a control antibody similarly to an anti HB-EGF antibody. Activity can be determined if the anti HB-EGF antibody shows a stronger cell growth suppression activity than the control antibody.

As the cells for use in evaluation of the activity, for example, a cell whose growth is promoted by HB-EGF, i.e., ovary cancer cell namely, RMG-1 cell strain, and a mouse Ba/F3 cell, which is transformed with a vector, in which a gene encoding a fusion protein hEGFR/mG-CSFR (human EGFR extracellular domain and a mouse GCSF receptor intracellular domain are fused in frame) is connected so as to express the gene, can be preferably used. As described above, those skilled in the art can appropriately select a cell for use in evaluating the activity and use the cell in measurement of the cell growth activity.

It is not necessary for the antigen-binding molecule provided by the present invention to have a neutralization activity by itself since the antigen-binding molecule can clear an antigen out of plasma. However, it is further preferable that the antigen-binding molecule exhibits the neutralization activity to an antigen to block the action of an antigen present in the plasma until the antigen is taken up into the cell expressing an Fcγ receptor together with the antigen-binding molecule by endocytosis via the Fcγ receptor.

Since the antigen-binding molecule (provided by the present invention) can promote dissociation of an antigen(which binds to the antigen-binding molecule outside the cell) from the antigen-binding molecule within the cell, the antigen dissociated from the antigen-binding molecule within the cell is decomposed in a lysosome. Therefore, it is not always necessary for the antigen-binding molecule itself to have a neutralization activity. However, it is further preferable that the antigen-binding molecule preferably exhibits the neutralization activity to an antigen to block the action of an antigen present in the plasma until the antigen is taken up into the cell expressing a sugar chain receptor, together with the antigen-binding molecule by endocytosis via the sugar chain receptor.

Since the antigen-binding molecule (provided by the present invention) can reduce the total antigen concentration or free antigen concentration in plasma, it is not always necessary for the antigen-binding molecule itself to have

a neutralization activity. However, it is further preferable that the antigen-binding molecule exhibits the neutralization activity to an antigen to block the action of an antigen present in the plasma until the antigen is taken up into the cell expressing a sugar chain receptor together with the antigen-binding molecule by endocytosis via the sugar chain receptor.

Sugar chain receptor

The sugar chain refers to a group of compounds in which various types of sugar molecules are connected via a glycoside linkage. Most of sugar chains are present *in-vivo* as a complex molecule in which a sugar molecule binds to a protein or a lipid, which is generally called a glycoconjugate. Of them, a glycoconjugate, which is a sugar bound to a protein, is a glycoprotein.

As an example of the sugar chain (to be used in the present invention), which is recognized by a sugar chain receptor, more specifically, to which a sugar chain receptor-binding domain binds, a sugar chain constituting a glycoprotein is mentioned. Examples of the sugar chain of a glycoprotein include an O-linked sugar chain and an N-linked sugar chain are mentioned. More preferable example of the sugar chain of a glycoprotein, an N-linked sugar chain is mentioned.

The O-linked sugar chain of a glycoprotein is obtained by connecting a sugar chain to the hydroxy group of a serine or threonine residue of a protein via an O-glycoside linkage. The sugar directly binding to a serine or threonine residue is mostly N-acetylgalactosamine (GalNAc). The core structure thereof consists of 8 elements: (1) galactose (Gal) connected to N-acetylgalactosamine (GalNAc) via a β1-3 linkage, (2) Gal connected to GalNAc via a β1-3 linkage and N-acetylglucosamine (GlcNAc) connected to GalNAc via a β1-6 linkage, (3) GlcNAc connected to GalNAc via a β1-3 linkage, (4) GlcNAc connected to GalNAc via a β1-3 linkage and GlcNAc connected to GalNAc via a β1-6 linkage, (5) GlcNAc connected to GalNAc via a β1-6 linkage, (6) GalNAc connected to GalNAc via a β1-6 linkage, (7) Gal connected to GalNAc via a β1-3 linkage.

N-linked sugar chains of glycoproteins all have a common core unit structure, Man 6 (Manα1-3)Manβ1-4GlcNAcβ1-4GlcNAc. This is called a tri-mannosyl core. N-linked sugar chains are divided into three sub-groups based on the structure and the site of the sugar residue(s) added to the tri-mannosyl core.

Of them, a complex type sugar chain does not contain a mannose residue except a tri-mannosyl core. At the reducing end of a side chain, a GlcNAc residue is present and connected to two α-mannosyl residues of the tri-mannosyl core.

A high-mannose sugar chain contains only α-mannose residues in addition to a tri-mannosyl core. In the sugar chain of this group, seven sugars are contained as core units, like Manα1-6 (Manα1-3)Manα1-6 (Manα1-3)Manβ1-4GlcNAcβ1-4GlcNAc.

In the complex sugar chain, one or two α-mannosyl groups are connected to a Manα1-6 arm of a tri-mannosyl core, in the same manner as in a high mannose, and the same side chain as that of the complex sugar chain is connected to the Manα1-3 arm of the core. The presence or absence of a fucose linkage to the position C-6 of GlcNAc, which is positioned at the reducing end of the tri-mannosyl core and furthermore, the presence or absence of β-GlcNAc linkage (called bisecting GlcNAc) to the position C-4 of a β mannosyl residue contribute to diversity of the structure of a complex sugar chain. Of the three N-linked sugar chain sub groups, a complex type contains the most diversified structures.

The sugar chain receptor refers to a receptor recognizing the aforementioned sugar chain and binding it. As long as it recognizes and binds to the sugar chain, any molecule can be used as a sugar chain receptor. A preferable receptor is a receptor expressed on a cell. For example, it is disclosed that an O-linked sugar chain on CD99 plays an important role in binding with PILR (paired Ig-like type-II receptor) (The Journal of immunology (2008) vol.180 (3), 1686-1693). Furthermore, it is known that an asialoglycoprotein receptor binds to an N-linked sugar chain having galactose at a terminal thereof. Moreover, it is known that a mannose receptor binds to an N-linked sugar chain having mannose at a terminal thereof. These receptors are preferably used as a sugar chain receptor in the present invention. More specifically, in the present invention, the antigen-binding molecule has a domain (sugar chain receptor-binding domain) binding to such a receptor. Thus, if the sugar chain receptor is an asialoglycoprotein receptor, an N-linked sugar chain having galactose at a terminal thereof can be preferably used as a sugar chain receptor-binding domain contained in the antigen-binding molecule of the present invention. Furthermore, if the sugar chain receptor is a mannose receptor, an N-linked sugar chain having mannose at a terminal thereof can be preferably used as a sugar chain receptor-binding domain contained in the antigen-binding molecule of the present invention

Sugar chain receptor-binding domain

The antigen-binding molecule of the present invention has one or more binding domain to a sugar chain receptor (particularly a human sugar chain receptor). The binding domain to a sugar chain receptor (particularly, a human sugar chain receptor) is not particularly limited in type and number, as long as the antigen-binding molecule has a binding activity to the sugar chain receptor (particularly, a human sugar chain receptor) in a neutral pH range and as long as the binding activity to the sugar chain receptor in an acidic pH range is lower than (the binding activity to the sugar chain

receptor) in the neutral pH range. Furthermore, a domain having a binding activity directly or indirectly to a sugar chain receptor (particularly, a human sugar chain receptor) can be used. Examples of such a domain may include a sugar chain having an activity directly binding to a sugar chain receptor (particularly, a human sugar chain receptor); an Fc domain of an IgG immunoglobulin; an antibody against a sugar chain receptor (particularly, a human sugar chain receptor); a peptide that binds to a sugar chain receptor (particularly, a human sugar chain receptor); and a Scaffold molecule to a sugar chain receptor (particularly, a human sugar chain receptor). In the present invention, a sugar chain receptor-binding domain, which has a binding activity to a sugar chain receptor (particularly, a human sugar chain receptor) in a neutral pH range and whose binding activity to the sugar chain receptor in an acidic pH range is lower than (the binding activity to the sugar chain receptor) in the neutral pH range, is preferred. The domain can be used as it is as long as the domain already has the binding activity to a sugar chain receptor (particularly, a human sugar chain receptor) in a neutral pH range and as long as the binding activity to a sugar chain receptor in an acidic pH range is lower than (the binding activity to the sugar chain receptor) in the neutral pH range. The binding activity between a sugar chain receptor-binding domain, which has an N-linked sugar chain having galactose at a terminal thereof, and an asialoglycoprotein receptor (a sugar chain receptor binding to the sugar chain) in an acidic pH range is preferably mentioned as an example where the binding activity is lower than the binding activity in the neutral pH range. Also, the binding activity between a sugar chain receptor-binding domain, which has an N-linked sugar chain having mannose at a terminal thereof, and a mannose receptor (a sugar chain receptor binding to the sugar chain) in an acidic pH range is preferably mentioned as an example where the binding activity is lower than the binding activity in the neutral pH range.

In the case where the binding activity of a sugar chain receptor-binding domain to a sugar chain receptor (particularly, a human sugar chain receptor) in a neutral pH range is zero or weak, the binding activity to the sugar chain receptor (particularly, a human sugar chain receptor) can be acquired by modifying an amino acid in the antigen-binding molecule. Alternatively, the binding activity to the sugar chain receptor (particularly, a human sugar chain receptor), can be enhanced by modifying an amino acid in the domain already having the binding activity to the sugar chain receptor (particularly, a human sugar chain receptor) in a neutral pH range. With respect to modification of an amino acid in the binding domain to a sugar chain receptor (particularly, a human sugar chain receptor), a desirable modification can be found out by comparing the binding activity to the sugar chain receptor (particularly, a human sugar chain receptor) in the neutral pH range between before and after modification of the amino acid.

In the case where the binding activity of a sugar chain receptor-binding domain to a sugar chain receptor (particularly, a human sugar chain receptor) in an acidic pH range is not lower than (the binding activity to a sugar chain receptor (particularly, a human sugar chain receptor)) in a neutral pH range, the binding activity to a sugar chain receptor (particularly, a human sugar chain receptor) in the acidic pH range lower than the binding activity (to a sugar chain receptor (particularly, a human sugar chain receptor)) in a neutral pH range can be acquired by modifying an amino acid in the antigen-binding molecule. With respect to modification of an amino acid in the binding domain to a sugar chain receptor (particularly, a human sugar chain receptor), a desirable modification can be found out by comparing the binding activity to the sugar chain receptor (particularly, a human sugar chain receptor) in the acidic pH range to the binding activity (to a sugar chain receptor (particularly, a human sugar chain receptor)) in the neutral pH range between before and after modification of an amino acid.

In the present invention, a sugar chain receptor-binding domain can be introduced into a site other than the antigen-binding domain and an FcRn binding domain constituting an antigen-binding molecule. Furthermore, as long as binding to an antigen by an antigen-binding domain is not inhibited, a sugar chain receptor-binding domain can be introduced in any site of the structure of the antigen-binding molecule. The site can be introduced into the antigen-binding domain as long as binding to an antigen by the antigen-binding domain is not inhibited and can be introduced into a site other than the antigen-binding domain. Furthermore, in another aspect, as long as a sugar chain receptor-binding domain does not inhibit the binding between the FcRn binding domain of an antigen-binding molecule and FcRn (particularly human FcRn), the sugar chain receptor-binding domain can be introduced into any site of the structure of the antigen-binding molecule. For example, the hinge portion of an IgA antibody can be a candidate for an amino acid sequence of the sugar chain receptor-binding domain for binding an O-linked sugar chain. A motif sequence, to which an N-linked sugar chain (i.e., Asn-X-Ser/Thr where X is amino acid except Pro) is added, can be a candidate for amino acid sequence of the sugar chain receptor-binding domain for binding the N-linked sugar chain. A gene encoding an antigen-binding molecule containing such an amino acid sequence is introduced into a host cell (described later) and cultured. From the culture fluid, the antigen-binding molecule of the present invention containing a sugar chain receptor-binding domain, to which a desired sugar chain is bound, can be produced.

In the present invention, a sugar chain receptor-binding domain may be chemically produced. For example, a chemical ligand such as a galactose derivative, which imitates an N-linked sugar chain having a galactose terminal; a mannose derivative, which imitates a high-mannose sugar chain; and a derivative which imitates sialic acid, may covalently conjugate to an antigen-binding molecule. Examples of such a chemical ligand include the molecules described in Bioorg. Med. Chem. (2011) 19 (8), 2494-2500, Bioorg. Med. Chem. (2009) 17 (20), 7254-7264, Bioorg. Med. Chem. (2008) 16 (9), 5216-5231, J. Am. Chem. Soc. (2004) 126 (33), 10355-10363, J. Pept. Sci. (2003) 9 (6), 375-385, Methods

Enzymol. (2010) 478, 343-363, but not limited to these. Examples of the amino acid at the antigen binding site, to which a chemical ligand is be conjugated, include lysine and cysteine, but not limited to these. A method for conjugating a chemical ligand to a specific site of an antigen-binding molecule, a method known to those skilled in the art can be employed, such as a method of substituting the amino acid at a conjugation site with cysteine, or a method of substituting lysine at a site where no conjugate is desired, with another amino acid. As a conjugation method, a known method to those skilled in the art can be used, such as a method of reacting maleimide and thiol of cysteine and a method of reacting an activated ester and lysine.

Conditions other than pH in measuring the binding activity to an antigen and FcRn (particularly human FcRn) can be appropriately selected by those skilled in the art and a method for measuring the binding activity is not limited to a specific method. For example, the binding activity can be measured in MES buffer at 37°C, as described in WO2009/125825. Furthermore, the antigen-binding activity of an antigen-binding molecule and the binding activity to, FcRn (particularly human FcRn) can be measured by a known method to those skilled in the art, such as Biacore (GE Healthcare). When the antigen is a soluble antigen, the binding activity of an antigen-binding molecule to an antigen is measured and evaluated by feeding the antigen as an analyte to a chip having the antigen immobilized thereto. If the antigen is a membrane antigen, the binding activity to the membrane antigen can be evaluated by feeding an antigen-binding molecule as an analyte to a chip having the antigen immobilized thereto. The binding activity of an antigen-binding molecule to FcRn (particularly human FcRn) is measured and evaluated by feeding FcRn (particularly human FcRn) or the antigen-binding molecule as an analyte to a chip having the antigen-binding molecule or FcRn (particularly human FcRn) immobilized thereto.

In the present invention, the binding activity to FcRn (particularly human FcRn) in an acidic pH range refers to the binding activity to FcRn (particularly human FcRn) at pH 4.0 to pH 6.5. The binding activity to FcRn (particularly human FcRn) in an acidic pH range refers to the binding activity to an antigen preferably at an arbitrary pH from pH 5.5 to pH 6.5, for example, the antigen-binding activity to an antigen at pH selected from pH 5.5, 5.6, 5.7, 5.8, 5.9, 6.0, 6.1, 6.2, 6.3, 6.4, and 6.5, particularly preferably, at an arbitrary pH from pH 5.8 to pH 6.0 close to the pH in early-stage endosome *in-vivo,* for example, pH selected from pH 5.80, 5.81, 5.82, 5.83, 5.84, 5.85, 5.86, 5.87, 5.88, 5.89, 5.90, 5.91, 5.92, 5.93, 5.94, 5.95, 5.96, 5.97, 5.98, 5.99, and 6.00.

At any temperature from 10°C to 50°C as the temperature for measuring the binding activity, the binding activity of the antigen-binding molecule (of the present invention) containing an FcRn binding domain to FcRn (particularly human FcRn) can be evaluated. Preferably, the binding activity of the antigen-binding molecule (of the present invention) containing an FcRn binding domain to FcRn (particularly human FcRn) can be measured at any temperature from 15°C to 40°C. More preferably, the binding activity of the antigen-binding molecule (of the present invention) containing an FcRn binding domain to FcRn (particularly human FcRn) can be measured at any temperature from 20°C to 35°C, for example, selected from 20, 21, 22, 23, 24, 25, 26, 27, 28, 29, 30, 31, 32, 33, 34, and 35°C.

In the present invention, the expression: "the binding to an antigen is not inhibited by an antigen-binding domain" means that the antigen-binding activity of an antigen-binding molecule after introduction of an sugar-chain receptor binding domain is maintained at a level of 20% or more, preferably 50% or more, further preferably 80% or more, more preferably 90% or more of the antigen-binding activity of the antigen-binding molecule before the introduction of the sugar chain receptor-binding domain. When the antigen-binding activity of an antigen-binding molecule decreases by the introduction of the sugar chain receptor-binding domain, the antigen-binding activity can be changed to the same level as the antigen-binding activity before the sugar chain receptor-binding domain is introduced, by substitution, deletion, addition and/or insertion of one or a plurality of amino acids in the antigen-binding molecule. The present invention also includes the antigen-binding molecule that regains the binding activity to the same level as that before the introduction by such a substitution, deletion, addition and/or insertion of one or a plurality of amino acids performed after the introduction of a sugar chain receptor-binding domain. Furthermore, the expression: "the binding to FcRn (particularly human FcRn) is not inhibited by a sugar chain receptor-binding domain" means that the binding activity of an antigen-binding molecule to FcRn (particularly human FcRn) after introduction of an sugar-chain receptor binding domain is maintained at a level of 20% or more, preferably 50% or more, further preferably 80% or more, more preferably 90% or more of the binding activity of the antigen-binding molecule to FcRn (particularly human FcRn) before the introduction of the sugar chain receptor-binding domain. When the binding activity of an antigen-binding molecule to FcRn (particularly human FcRn) decreases by the introduction of a sugar chain receptor-binding domain, the binding activity can be changed to the same level as the binding activity to FcRn (particularly human FcRn) before the sugar chain receptor-binding domain is introduced, by substitution, deletion, addition and/or insertion of one or a plurality of amino acids in the antigen-binding molecule. The present invention also include the antigen-binding molecule that regains the binding activity to the same level as that before the introduction, even if such a substitution, deletion, addition and/or insertion of one or a plurality of amino acids is performed after the introduction of a sugar chain receptor-binding domain. A measurement method and determination method of the binding activity to an antigen or FcRn (particularly human FcRn) will be described later.

Sugar chain

As an example of the sugar chain receptor-binding domain contained in the antigen-binding molecule in the present invention, a sugar chain receptor-binding domain having a desired sugar chain bound thereto is preferably mentioned. As an example of the desired sugar chain, an O-linked sugar chain or an N-linked sugar chain is preferably mentioned. As a method of binding a sugar chain to a sugar chain receptor-binding domain, a method known in the art can be employed. For example, an enzymatic method performed in an acellular system as described in (Japanese Patent Laid-Open No. 2006-141241), which is a method for producing an antibody modified after translation in an acellular protein synthesis system, comprising preparing a cell extract from cultured cells of an immortalized mammalian animal cell strain capable of secreting a protein and adding mRNA encoding an antibody to the extract, can be employed as a method for producing the sugar chain receptor-binding domain having a desired sugar chain bound thereto. Furthermore, in one of such methods known in the art, a gene, to which a motif sequence for adding a desired sugar chain is introduced by a genetic recombination technique, etc., and which encodes a sugar chain receptor-binding domain contained in an antigen-binding molecule present in nature or artificially produced is introduced into a host cell, which is then cultured. From the culture fluid of the host cell, the antigen-binding molecule of the present invention containing a sugar chain receptor-binding domain, to which a desired sugar chain to be bound, can be produced.

When the sugar chain is an O-linked sugar chain, a motif sequence, to which the O-linked sugar chain is added, can be designed by use of database known in the art. An O-linked sugar chain is added to the hinge portion of an IgA antibody. A gene sequence encoding such a sugar chain receptor-binding domain having an O-linked sugar chain added thereto, already known in the art, will be used as a candidate for the motif sequence. When the sugar chain is an N-linked sugar chain, a motif sequence, to which the N-linked sugar chain is added, is known to be a motif consisting of three continuous amino acids: Asn-X-Ser/Thr. Because of this, if a gene, which encodes an antigen-binding molecule containing a sugar chain receptor-binding domain and which is designed so as to encode the motif sequence consisting of Asn-X-Ser/Thr for adding an N-linked sugar chain by a genetic recombination technique, etc. is introduced into a host cell (described later) and cultured, the antigen-binding molecule of the present invention containing the sugar chain receptor-binding domain, to which a desired sugar chain is to be bound, can be produced, from the culture fluid of the host cell.

The antigen-binding molecules produced as mentioned above may have the same sugar chain structure in some cases; whereas, the antigen-binding molecules are produced as a mixture of the antigen-binding molecules having different sugar chains added thereto in other cases. In the present invention, such a mixture can be also preferably used. Also, an antigen-binding molecule having a sugar-chain binding domain to which a specific sugar chain is connected, can be preferably used in the present invention.

As a method for connecting a specific sugar chain to the sugar chain receptor-binding domain contained in the antigen-binding molecule (of the present invention), a plurality of methods known in the art can be employed. As one of the known methods, a method of purifying an antigen-binding molecule having a specific sugar chain by use of a property of a sugar chain of a natural antigen-binding molecule or an artificial antigen-binding molecule (prepared by a genetic recombination method, etc.) is mentioned. An antibody having high-mannose sugar chain is known to be purified by affinity chromatography using ConA-sepharose (Millward (Biologicals (2008) 36, 49-60)). Such a purification method can be used for preparing an antigen-binding molecule having an N-linked sugar chain having mannose at the non-reducing end, in the present invention.

Furthermore, in another aspect, in order to obtain an antigen-binding molecule having a specific sugar chain, an enzyme treatment can be appropriately employed. As described later in Examples, an antigen-binding molecule having an N-linked sugar chain having galactose at the non-reducing end can be prepared from an antigen-binding molecule having a complex sugar chain having sialic acid at the non-reducing end, by a sialidase treatment. Furthermore, an antibody having a high-mannose sugar chain is known to be prepared by sialidase and β galactosidase treatment, by which galactose is removed from the sugar chain (Newkirk (Clin. Exp. Immunol. (1996) 106, 259-264)). Such a preparation method including treatments with sialidase and β galactosidase can be used for preparing an antigen-binding molecule having an N-linked sugar chain having mannose at a non-reducing end, in the present invention.

In another aspect, in order to obtain the antigen-binding molecule having a specific sugar chain of the present invention, a method of recovering the antigen-binding molecule from a culture fluid of a host cell, which is transfected with a recombinant gene encoding the antigen-binding molecule and whose glycosidase activity is modified so as to accumulate a specific sugar chain (by a method including genetic or genetic recombination technique but not limited to these), can be appropriately employed. It is known that an antibody having a high mannose N-linked sugar chain is collected from a culture fluid of a Lec1 mutation strain, which is derived from a CHO cell defective in N-acetylglucosaminyltransferase I activity and transfected with a recombinant gene encoding the antibody (Wright and Morrison (J. Exp. Med. (1994) 180, 1087-1096)). In the present invention, an antigen-binding molecule having an N-linked sugar chain having mannose at the non-reducing end can be collected from a culture fluid of a Lec1 mutation strain transfected with a recombinant gene encoding the antigen-binding molecule.

In another aspect, in order to obtain the antigen-binding molecule having a specific sugar chain of the present invention, a method for collecting an antigen-binding molecule having a specific sugar chain and accumulated in a culture fluid by culturing a cell producing the antigen-binding molecule with the addition of an inhibitor for inhibiting a specific glycosidase reaction, can be appropriately employed. It is known that an antibody having a high mannose N-linked sugar chain having no fucose at its reducing end is collected from a culture fluid of a CHO cell producing the antibody and cultured with the addition of kifunesine (Zhou (Biotechnol. Bioeng. (2008) 99, 652-665)). In the present invention, an antibody having an N-linked sugar chain having no fucose at its reducing end and having mannose at the non-reducing end, can be collected from a culture fluid of a CHO cell transfected with a recombinant gene encoding the molecule and cultured with the addition of kifunesine. Furthermore, the antigen-binding molecule having a specific sugar chain of the present invention can be obtained by culturing a host cell whose glycosidase activity is changed as mentioned above, with the addition of an inhibitor mentioned above. Since it is known that an antibody having a specific sugar chain can be collected by such a combination (Kanda, et al. (Glycobiology (2007) 17, 104-118)), the same combination can be appropriately employed in order to obtain an antigen-binding molecule having a specific sugar chain, in the present invention

A method of expressing a protein having a specific sugar chain structure by genetically modifying a host such as *Pichia pastor* is known (Biochemistry. 2008 Sep 30; 47 (39): 10294-304., J Biotechnol. 2009 Feb 23; 139 (4): 318-25., Nat Biotechnol. 2006 Feb;24 (2): 210-5.). By use of such a method, an antigen-binding molecule having a specific sugar chain structure (for example, N-linked sugar chain having a galactose terminal) uniformly bound to an N-linked glycosylation sequence can be prepared.

An antigen-binding molecule containing a sugar chain receptor-binding domain (particularly an antigen-binding molecule containing a human derived sugar chain receptor binding domain) according to the present invention binds to a sugar chain receptor in a pH-dependent manner and/or having a sugar chain receptor binding activity (particularly a human-derived sugar chain receptor binding activity) in a neutral pH range. If the binding activity to a sugar chain receptor in an acidic pH range can be lowered than (the binding activity to the sugar chain receptor) in a neutral pH range, the uptake of an antigen into a cell by the antigen-binding molecule can be promoted. If such an antigen-binding molecule is injected, the antigen concentration in plasma can be reduced more and more and the pharmacokinetics of the antigen-binding molecule can be improved, with the result that the number of antigens that can be bound by a single antigen-binding molecule can be increased.

Binding activity to the sugar chain receptor

In the present invention, the binding activity to the sugar chain receptor (particularly a human-derived sugar chain receptor) in an acidic pH range refers to the binding activity to the sugar chain receptor (particularly a human-derived sugar chain receptor) in the range pH 4.0 to pH 6.5, preferably at arbitrary pH in the range of pH 5.5 to pH 6.5, for example, at a pH value selected from pH 5.5, 5.6, 5.7, 5.8, 5.9, 6.0, 6.1, 6.2, 6.3, 6.4, and 6.5, and particularly preferably, at arbitrary pH from pH 5.8 to pH 6.0 close to pH in early-stage endosome *in-vivo,* for example, pH selected from pH 5.80, 5.81, 5.82, 5.83, 5.84, 5.85, 5.86, 5.87, 5.88, 5.89, 5.90, 5.91, 5.92, 5.93, 5.94, 5.95, 5.96, 5.97, 5.98, 5.99, and 6.00. Furthermore, in the present invention, the binding activity to the sugar chain receptor (particularly a human-derived sugar chain receptor) in a neutral pH range refers to the binding activity to the sugar chain receptor (particularly a human-derived sugar chain receptor) in the range pH 6.7 to pH 10.0, at arbitrary pH in the range of pH 7.0 to pH 8.0, for example, at a pH value selected from pH 7.0, 7.1, 7.2, 7.3, 7.4, 7.5, 7.6, 7.7, 7.8, 7.9, and 8.0, and particularly preferably, at pH 7.4 close to pH in plasma *in-vivo*.

Conditions other than pH in measuring the binding activity to a sugar chain receptor (particularly a human-derived sugar chain receptor) can be appropriately selected by those skilled in the art and are not particularly limited to specific conditions. The binding activity can be measured in MES buffer at 37°C as described, for example, in WO2009/125825. The binding activity of an antigen-binding molecule to a sugar chain receptor (particularly a human-derived sugar chain receptor) can be measured by a method known to those skilled in the art, for example, by use of Biacore (GE Healthcare), etc. The binding activity of an antigen-binding molecule to a sugar chain receptor (particularly a human-derived sugar chain receptor) can be measured and evaluated by feeding the antigen-binding molecule as an analyte to a chip having a sugar chain receptor (particularly a human-derived sugar chain receptor) immobilized thereto. Conversely, the binding activity to a sugar chain receptor (particularly a human-derived sugar chain receptor) can be evaluated by feeding a solubilized sugar chain receptor (particularly a human-derived sugar chain receptor) as an analyte to a chip having the antigen-binding molecule immobilized thereto.

In the present invention, as long as the binding activity to a sugar chain receptor (particularly a human-derived sugar chain receptor) in an acidic pH range is weaker than (the antigen-binding activity) in a neutral pH range, the ratio of the binding activity to the sugar chain receptor (particularly a human-derived sugar chain receptor) in the acidic pH range relative to the binding activity to the sugar chain receptor (particularly a human-derived sugar chain receptor) in the neutral pH range is not particularly limited. A KD (pH 5.8)/KD (pH 7.4) value, which is a ratio of KD (Dissociation

constant) to a sugar chain receptor (particularly a human-derived sugar chain receptor) at pH 5.8 relative to KD at pH 7.4 is 2 or more, more preferably 10 or more and further preferably 40 or more. The upper limit of the KD (pH 5.8)/KD (pH 7.4) value is not particularly limited. As long as preparation can be made by those skilled in the art, any value such as 400, 1000 and 10000 may be used.

As a value representing the binding activity to a sugar chain receptor (particularly a human-derived sugar chain receptor), KD (dissociation constant) is used. KD (dissociation constant) can be determined by a method known to those skilled in the art. For example, Biacore (GE healthcare), Scatchard plot, a flow cytometer, etc. are preferably used.

In the present invention, as another index representing the ratio of the binding activity to a sugar chain receptor (particularly a human-derived sugar chain receptor) in an acidic pH range relative to the binding activity to a sugar chain receptor (particularly a human-derived sugar chain receptor) in a neutral pH range, for example, a dissociation rate constant, kd (Dissociation rate constant) can be preferably used. When $k_d$ (dissociation rate constant) is used in place of KD (dissociation constant) as the index representing the ratio of the binding activities, a value of $k_d$ (in the acidic pH range)/$k_d$ (in the neutral pH range), which is a ratio of $k_d$ (dissociation rate constant) of a sugar chain receptor (particularly a human-derived sugar chain receptor) in the acidic pH range relative to $k_d$ (dissociation rate constant) in the neutral pH range, is preferably 2 or more, more preferably 5 or more, further preferably 10 or more and further more preferably 30 or more. The upper limit value of $k_d$ (in the acidic pH range)/$k_d$ (in the neutral pH range) is not particularly limited. As long as preparation can be made by common technical knowledge of those skilled in the art, any value such as 50, 100 and 200 may be used.

An asialoglycoprotein receptor (a sugar chain receptor) interacts with galactose in a pH-dependent manner and is known to exhibit a high binding activity in a neutral pH range but a low binding activity in an acidic pH range (J Biol Chem Vol. 274, No. 50, pp. 35400-35406, 1999). Similarly, a mannose receptor (a sugar chain receptor) interacts with galactose in a pH-dependent manner and is known to exhibit a high binding activity in a neutral pH range but low binding activity in an acidic pH range (J Biol Chem. 1994 Nov 11;269 (45): 28405-13.). From this, in the present invention, as a sugar chain/sugar chain receptor, galactose/asialoglycoprotein receptor and mannose/mannose receptor can be preferably used.

The binding activity of the galactose/asialoglycoprotein receptor and mannose/mannose receptor is dependent upon not only pH but also calcium-ion concentration. Since most of the sugar chain receptors are C type lectins, the binding between a sugar chain receptor and a sugar chain has calcium-ion concentration dependency. More specifically, similarly to the binding between an antigen-binding molecule and an antigen, the binding between a sugar chain receptor and a sugar chain may vary depending upon the calcium-ion concentration. It is sufficient that the binding in a high calcium-ion concentration is higher than the binding in a low calcium-ion concentration.

Note that, in the present invention, when the binding activity to a sugar chain receptor (particularly a human-derived sugar chain receptor) is measured in different pH conditions, same conditions other than pH condition are preferably employed.

Antigen-binding molecule

The present invention provides an antigen-binding molecule having an antigen-binding domain, an FcRn binding domain and one or more binding domains to a sugar chain receptor, an antigen-binding molecule increased in number of binding domains to the sugar chain receptor, and an antigen-binding molecule further containing an antigen-binding domain whose binding activity to an antigen in an acidic pH range is lower than the binding activity to the antigen in a neutral pH range. Furthermore, the present invention provides a method for producing the antigen-binding molecule and a pharmaceutical composition containing the antigen-binding molecule. Furthermore, the present invention provides a method for introducing an antigen-binding molecule and/or an antigen, to which the antigen-binding molecule is to be bound, into a cell, a method for increasing the number of antigens to which a single antigen-binding molecule binds, a method of reducing the number of antigens present outside a cell, a method for improving the pharmacokinetics of the antigen-binding molecule, and a method for promoting dissociation of an antigen from the antigen-binding molecule, comprising bringing the antigen-binding molecule into contact with a cell expressing a sugar chain receptor, *in-vivo* or *ex-vivo.* Moreover, the present invention provides a method for promoting uptake of the antigen-binding molecule and/or an antigen having the antigen-binding molecule bound thereto, into a cell, a method for increasing the number of antigens to which a single antigen-binding molecule binds, *in-vivo* or *ex-vivo,* a method of enhancing a potency of the antigen-binding molecule to clear antigens *in-vivo* and *ex-vivo,* a method for improving the pharmacokinetics of the antigen-binding molecule, and a method for promoting dissociation of an antigen from the antigen-binding molecule, comprising, in an antigen-binding molecule having an antigen-binding domain, an FcRn binding domain and one or more binding domains to a sugar chain receptor, increasing the number of binding domains to the sugar-chain receptor.

Method for producing antigen-binding molecule

The present invention provides a method for producing an antigen-binding molecule having an antigen-binding domain, an FcRn, particularly a human FcRn binding domain and one or more sugar chain receptor-binding domains, and having a binding activity to the sugar chain receptor in a neutral pH range, in which the binding activity to the sugar chain receptor in an acidic pH range is lower than (the binding activity to the sugar chain receptor) in the neutral pH range; and the antigen-binding activity in an acidic pH range is lower than (the antigen-binding activity) in a neutral pH range. Also, the present invention provides a method for producing an antigen-binding molecule having an excellent promotion effect in decreasing the antigen concentration in plasma and being excellent in pharmacokinetics. The present invention provides a method for producing an antigen-binding molecule, which is particularly usefully used as a pharmaceutical composition.

Specifically, the present invention provides a method for producing an antigen-binding molecule having the following steps:

(a) a step of providing a polypeptide sequence of the antigen-binding molecule containing an antigen-binding domain and an FcRn binding domain,
(b) a step of identifying an amino acid sequence serving as a candidate for a motif for a sugar chain receptor-binding domain in the polypeptide sequence,
(c) a step of designing the motif for a sugar chain receptor-binding domain containing an amino acid sequence, which contains at least one amino acid different from the amino acid sequence identified in the step (b),
(d) a step of preparing a gene encoding a polypeptide of an antigen-binding molecule containing the motif for the sugar chain receptor-binding domain designed in the step (c), and
(e) a step of recovering the antigen-binding molecule from a culture fluid of a host cell transformed with the gene obtained in the step (d).

Note that the steps (c) and (d) may be repeated two or more. The number of repeating the steps (c) and (d) is not particularly limited but usually within 10 times. As described later, a step of further treating the antigen-binding molecule obtained in the step (e) with an enzyme is also included in the method of the present invention.

The antigen-binding domain contained in the antigen-binding molecule produced by a method provided by the present invention can be provided by the method described in the section: "Antigen-binding domain".

The FcRn binding domain having a binding activity to FcRn (particularly human FcRn) and contained in the antigen-binding molecule produced by the method provided by the present invention can be provided by a method described in the section: "FcRn-binding domain". More specifically, the FcRn binding domain is not particularly limited as long as it has a binding activity to FcRn (particularly human FcRn) in the acidic pH range. Furthermore, a domain having a binding activity directly or indirectly to FcRn (particularly human FcRn) can be used. As such a domain, Fc region of an IgG immunoglobulin, albumin, albumin domain 3, anti-human FcRn antibody, anti-human FcRn peptide, and anti-human FcRn Scaffold molecule, which have a binding activity directly to FcRn, (particularly a human FcRn), or an IgG and albumin, which have a binding activity indirectly to human FcRn, are mentioned. In the present invention, the polypeptide sequence of a domain having a binding activity directly or indirectly to FcRn (particularly, a human FcRn) can be provided as the polypeptide sequence of the antigen-binding domain.

As an example of the sugar chain receptor-binding domain contained in the antigen-binding molecule produced by a method provided by the present invention, the sugar chain receptor-binding domain having a desired sugar chain bound thereto is preferably mentioned. As a desired sugar chain, an O-linked sugar chain or N-linked sugar chain is preferably mentioned. As a method for binding a sugar chain to a sugar chain receptor-binding domain, a known method can be employed. In one of such methods known in the art, a gene, to which a motif sequence (more specifically, a motif for a sugar chain receptor-binding domain) for adding a desired sugar chain is introduced by a genetic recombination technique, etc., and which encodes a sugar chain receptor-binding domain contained in an antigen-binding molecule present in nature or artificially produced, is introduced into a host cell, which is then cultured. From the culture fluid of the host cell, the antigen-binding molecule of the present invention containing a sugar chain receptor-binding domain, to which a desired sugar chain is to be bound, can be produced.

In a step of identifying the amino acid sequence serving as a candidate for the motif for a sugar chain receptor-binding domain in the polypeptide sequence provided as described above, for example, the following method can be used. A motif sequence to which an O-linked sugar chain is added can be identified by use of database known in the art. Furthermore, when the hinge portion of an antibody is contained in the antigen-binding molecule of the present invention, from which isotype of antibody the hinge portion is derived can be identified. Such a hinge portion can be identified as a motif candidate for the O-linked sugar chain receptor-binding domain. When the sugar chain is an N-linked sugar chain, a motif sequence (more specifically, a motif for an N-linked sugar chain receptor-binding domain) to which an N-linked sugar chain is added, is known to consist of three continuous amino acids, i.e., Asn-X-Ser/Thr. Because

of this, in order to design a sugar chain receptor-binding domain so as to encode a motif for an N-linked sugar chain receptor-binding domain, i.e., Asn-X-Ser/Thr, by a genetic recombination technique, etc., the presence or absence of a sequence, which is the equivalent or analogous to Asn-X-Ser/Thr in the polypeptide sequence provided is determined.

A motif for a sugar chain receptor-binding domain containing an amino acid sequence different in at least one amino acid from the amino acid sequence identified as mentioned above can be designed. To describe more specifically, using the database etc. known in the art, an amino acid in the polypeptide sequence serving as a candidate motif sequence to which an O-linked sugar chain added and identified above is substituted to design an O-linked sugar chain motif sequence. Furthermore, when the hinge portion of an antibody is contained in the antigen-binding molecule of the present invention, if a hinge portion derived from an IgA antibody is not contained in the hinge portion, the sequence of the hinge portion is substituted with the sequence derived from an IgA antibody to design an O-linked sugar chain motif sequence. In contrast, when the sugar chain is an N-linked sugar chain, if the sequence, which is equivalent or analogous to Asn-X-Ser/Thr, is not identified in the polypeptide sequence provided, an Asn-X-Ser/Thr sequence is inserted in an appropriate site of the polypeptide sequence provided. In this manner, a new motif for an N-linked sugar chain receptor-binding domain can be added. Furthermore, if the amino acid residue of an analogous sequence to an Asn-X-Ser/Thr sequence found in the polypeptide sequence provided is substituted, the analogous sequence can be modified to the Asn-X-Ser/Thr sequence, which is a motif for an N-linked sugar chain receptor-binding domain.

The antigen-binding molecule of the present invention has one or more binding domains to a sugar chain receptor (particularly, a human sugar chain receptor). The binding domain to a sugar chain receptor (particularly a human sugar chain receptor) is not particularly limited in type and number, as long as the antigen-binding molecule has a binding activity to a sugar chain receptor (particularly, a human sugar chain receptor) in a neutral pH range and as long as the binding activity to the sugar chain receptor in an acidic pH range is lower the binding activity to the sugar chain receptor in a neutral pH range. Furthermore, a domain having a binding activity directly or indirectly to a sugar chain receptor (particularly, a human sugar chain receptor) can be used. Examples of such a domain may include a sugar chain having direct binding activity to a sugar chain receptor (particularly, a human sugar chain receptor); an Fc domain of IgG immunoglobulin; an antibody against a sugar chain receptor (particularly, a human sugar chain receptor); a peptide that binds to a sugar chain receptor (particularly, a human sugar chain receptor); and a Scaffold molecule to a sugar chain receptor (particularly, a human sugar chain receptor). In the present invention, a sugar chain receptor-binding domain having a binding activity to a sugar chain receptor (particularly, a human sugar chain receptor) in a neutral pH range, in which the binding activity to the sugar chain receptor in an acidic pH range is lower than (the binding activity to the sugar chain receptor) in the neutral pH range, is preferred. The domain can be used as it is, if it is a sugar chain receptor-binding domain already having a binding activity to a sugar chain receptor (particularly, a human sugar chain receptor) in a neutral pH range in which the binding activity to the sugar chain receptor in an acidic pH range is lower than (the binding activity to the sugar chain receptor) in the neutral pH range. The binding activity between a sugar chain receptor-binding domain having an N-linked sugar chain having galactose at a terminal and an asialoglycoprotein receptor (sugar chain receptor-binding to the sugar chain) in an acidic pH range is preferably mentioned as an example of the case where the binding activity is lower than (the binding activity) in a neutral pH range. Furthermore, the binding activity between a sugar chain receptor-binding domain having an N-linked sugar chain having mannose at a terminal and a mannose receptor (sugar chain receptor binding to the sugar chain) in an acidic pH range is preferably mentioned as an example of the case where the binding activity is lower than (the binding activity) in a neutral pH range.

When the binding activity of a sugar chain receptor-binding domain to a sugar chain receptor (particularly, a human sugar chain receptor) in a neutral pH range is zero or weak, the binding activity to the sugar chain receptor (particularly, a human sugar chain receptor) can be acquired by modifying an amino acid in the antigen-binding molecule. Alternatively, the binding activity to a sugar chain receptor (particularly, a human sugar chain receptor) can be enhanced by modifying an amino acid in the domain already having the binding activity to the sugar chain receptor (particularly, a human sugar chain receptor) in a neutral pH range. With respect to modification of an amino acid in the binding domain to a sugar chain receptor (particularly, a human sugar chain receptor), a desirable modification can be found out by comparing the binding activity to the sugar chain receptor (particularly, a human sugar chain receptor) in the neutral pH range between before and after modification of an amino acid

The binding activity of a sugar chain receptor-binding domain to a sugar chain receptor (particularly, a human sugar chain receptor) in an acidic pH range is not lower than (the binding activity to the sugar chain receptor) (particularly, a human sugar chain receptor) in a neutral pH range, the binding activity to the sugar chain receptor (particularly, a human sugar chain receptor) in the acidic pH range lower than (the binding activity to the sugar chain receptor) (particularly, a human sugar chain receptor) in the neutral pH range can be acquired by modifying an amino acid in the antigen-binding molecule. With respect to modification of an amino acid in the binding domain to a sugar chain receptor (particularly, a human sugar chain receptor), a desirable modification can be found out by comparing the binding activity to the sugar chain receptor (particularly, a human sugar chain receptor) in the acidic pH range to the binding activity to the sugar chain receptor (particularly, a human sugar chain receptor) in the neutral pH range between before and after modification of an amino acid.

In the present invention, as long as the binding to an antigen by an antigen-binding domain is not inhibited, a sugar chain receptor-binding domain can be introduced at any site of the structure of the antigen-binding molecule. The site can be introduced in the antigen-binding domain as long as it does not inhibit the binding to an antigen by an antigen-binding domain, and can be introduced in other sites. In another aspect, as long as an sugar chain receptor-binding domain does not inhibit the binding of an FcRn binding domain of an antigen-binding molecule to FcRn (particularly human FcRn), the sugar chain receptor-binding domain can be introduced in any site of the structure of the antigen-binding molecule. For example, an IgA antibody hinge portion can be a candidate for the amino acid sequence of a sugar chain receptor-binding domain for binding an O-linked sugar chain. Asn-X-Ser/Thr (motif sequence for adding an N-linked sugar chain) can be a candidate for the amino acid sequence of a sugar chain receptor-binding domain for binding an N-linked sugar chain. If a gene encoding an antigen-binding molecule containing such an amino acid sequence is introduced into a host cell (described later) and cultured. From the culture fluid, the antigen-binding molecule of the present invention containing a sugar chain receptor-binding domain, to which a desired sugar chain is to be bound, can be produced.

In the present invention, the expression: "the binding to an antigen is not inhibited by an antigen-binding domain" means that the antigen-binding activity of an antigen-binding molecule after introduction of an sugar-chain receptor-binding domain is maintained at a level of 20% or more, preferably 50% or more, further preferably 80% or more, more preferably 90% or more of the antigen-binding activity of the antigen-binding molecule before the introduction of the sugar chain receptor-binding domain. When the antigen-binding activity of an antigen-binding molecule decreases by the introduction of a sugar chain receptor-binding domain, the antigen-binding activity can be changed to the same level as the antigen-binding activity before the sugar chain receptor-binding domain is introduced by substitution, deletion, addition and/or insertion of one or a plurality of amino acids in the antigen-binding molecule. The present invention also includes the antigen-binding molecule that regains the binding activity to the same level as that before the introduction by such a substitution, deletion, addition and/or insertion of one or a plurality of amino acids performed after the introduction of a sugar chain receptor-binding domain. Furthermore, the expression: "the binding to FcRn (particularly human FcRn) is not inhibited by a sugar chain receptor-binding domain" means that the binding activity of an antigen-binding molecule to FcRn (particularly human FcRn) after introduction of an sugar-chain receptor-binding domain is maintained at a level of 20% or more, preferably 50% or more, further preferably 80% or more, more preferably 90% or more of the binding activity of the antigen-binding molecule before the introduction of the sugar chain receptor-binding domain. When the binding activity of an antigen-binding molecule to FcRn (particularly human FcRn) decreases by the introduction of a sugar chain receptor-binding domain, the antigen-binding activity can be changed to the same level as the antigen-binding activity to FcRn (particularly human FcRn) before the sugar chain receptor-binding domain is introduced by substitution, deletion, addition and/or insertion of one or a plurality of amino acids in the antigen-binding molecule. The present invention also include the antigen-binding molecule that regains the binding activity to the same level as that before the introduction by such a substitution, deletion, addition and/or insertion of one or a plurality of amino acids performed after the introduction of a sugar chain receptor-binding domain.

Furthermore, a sugar chain receptor-binding domain can be introduced into sites other than an antigen-binding domain and an FcRn binding domain constituting an antigen-binding molecule.

In the present invention, the structure of the target antigen-binding molecule is not particularly limited, and an antigen-binding molecule having any structure can be preferably used. However, as a preferable example of the antigen-binding molecule having an antigen-binding domain, a binding domain to FcRn (particularly a human FcRn domain), and two or more sugar chain receptor domains, an antibody is mentioned. As a preferable example of the antibody of the present invention, an IgG antibody is mentioned. When an IgG antibody is used as the antibody, the type thereof is not limited and IgG isotypes (subclass) such as IgG1, IgG2, IgG3 and IgG4 can be used. Furthermore, a constant region of an antibody can be included in the antigen-binding molecule of the present invention and an amino acid mutation can be introduced in the portion of the constant region. As the amino acid mutation to be introduced, mutations that increase or decrease binding to, for example, an Fcγ receptor (Proc Natl Acad Sci U S A. 1 (2006) 103 (11), 4005-10) is mentioned but the mutation is not limited to these. Furthermore, pH-dependent binding can be changed by selecting a proper constant region such as an IgG2 constant region.

When the antigen-binding molecule targeted by the present invention is an antibody, an antibody derived from any animal such as a mouse antibody, a human antibody, a rat antibody, a rabbit antibody, a goat antibody and a camel antibody, can be used. Furthermore, for example, a chimeric antibody can be used. Of the chimeric antibodies, a modified antibody prepared by substituting an amino acid sequence, such as a humanized antibody, can be preferably used. Moreover, a bispecificity antibody, a modified antibody prepared by binding various types of molecules and a polypeptide containing an antibody fragment, etc., can be used.

The "chimeric antibody" refers to an antibody prepared by combining sequences derived from different animals. As a specific examples of the chimeric antibody, an antibody, which is produced of a mouse antibody heavy chain/light chain variable (V) region and a human antibody heavy chain/light chain constant (C) region, can be mentioned.

The "humanized antibody" is also called as a reshaped human antibody, which is an antibody prepared by

grafting an antibody derived from a mammalian animal except a human, for example, a mouse antibody complementarity determining region (CDR) to human antibody CDR. A method for identifying CDR is known in the art(Kabat et al., Sequence of Proteins of Immunological Interest (1987), National Institute of Health, Bethesda, Md., Chothia et al., Nature (1989) 342, 877). Furthermore, a general genetic recombination technique for this is known in the art(EP125023, WO1996/002576).

The bispecific antibody refers to an antibody having variable regions recognizing different epitopes in the same antibody molecule. The bispecific antibody can be an antibody recognizing two or more different antigens and can be an antibody recognizing two or more different epitopes on the same antigen.

As the polypeptide containing an antibody fragment, for example, an Fab fragment, an F(ab')2 fragment, a scFv (Nat Biotechnol. (2005) 23 (9), 1126-36) domain antibody (dAb) (WO2004/058821, WO2003/002609), scFv-Fc (WO2005/037989), dAb-Fc and an Fc fusion protein are mentioned. In a molecule containing an Fc region, the Fc region can be used as a binding domain to FcRn (particularly a human FcRn domain). Furthermore, a molecule prepared by fusing a human FcRn binding domain to such a molecule can be used.

A gene encoding an antigen-binding domain designed as described above, an FcRn binding domain and a sugar chain receptor-binding domain can be prepared. A method for preparing a gene is known in the art. A gene can be prepared by a chemical synthesis and can be prepared by e.g., PCR method in which nucleotides constituting a polynucleotide are ligated in the presence of a primer (serving as a template) by an enzyme reaction. The antigen-binding domain designed as described above, an FcRn binding domain, and a sugar chain receptor-binding domain can be ligated by expressing individual domains separately in host cells (described later), collecting polypeptides from cultures fluids and performing a chemical reaction in the presence of a crosslinking agent. Examples of a synthesized chemical linker (chemical crosslinking agent) include crosslinking agents usually used in crosslinking peptides such as N-hydroxy succinimide (NHS), disuccinimidyl suberate (DSS), bis(sulfosuccineimidyl) suberate (BS3), dithiobis(succinimidylpropionate) (DSP), dithiobis(sulfosuccinimidylpropionate) (DTSSP), ethylene glycol bis(succinimidyl succinate) (EGS), ethylene glycol bis(sulfosuccinimidylsuccinate) (sulfo-EGS), disuccinimidyl tartrate (DST), disulfosuccinimidyl tartrate (sulfo-DST), bis[2-(oxysuccinimideoxycarbonyloxy)ethyl]sulfone (BSOCOES) and bis[2-(sulfosuccinimideoxycarbonyloxy)ethyl]sulfone (sulfo-BSOCOES). These crosslinking agents are commercially available.

A gene, which is designed so as to link an antigen-binding domain as designed mentioned above, an FcRn binding domain, and a sugar chain receptor-binding domain via a peptide bond in frame, is expressed in a host cell (described later). In this manner, an antibody can be obtained by collecting from a culture fluid, etc. In ligating the domains via a peptide bond in frame, the domains can be directly ligated to each other and ligated via a linker having a specific peptide sequence. As the linker for ligating the domains, any peptide linker can be used as long as it can be introduced in a genetic engineering manner or a synthesized compound linker such as a linker disclosed in the document (see, for example, Protein Engineering (1996) 9 (3), 299-305) can be used. In the present invention, a peptide linker is preferable. The length of the peptide linker, which is not particularly limited, can be appropriately selected by those skilled in the art depending upon the purpose. The length of the peptide is preferably 5 amino acids or more (the upper limit, which is not particularly limited is usually, 30 amino acids or less, preferably 20 amino acids or less) and particularly preferably 15 amino acids.

Examples of the peptide linker include:

Ser
Gly·Ser
Gly·Gly·SerSer·Gly·Gly
Gly·Gly·Gly·Ser (SEQ ID NO: 18)
Ser·Gly·Gly·Gly (SEQ ID NO: 19)
Gly·Gly·Gly·Gly·Ser (SEQ ID NO: 20)
Ser·Gly·Gly·Gly·Gly (SEQ ID NO: 21)
Gly·Gly·Gly·Gly·Gly·Ser (SEQ ID NO: 22)
Ser·Gly·Gly·Gly·Gly·Gly (SEQ ID NO: 23)
Gly·Gly·Gly·Gly·Gly·Gly·Ser (SEQ ID NO: 24)
Ser·Gly·Gly·Gly·Gly·Gly·Gly (SEQ ID NO: 25)
(Gly·Gly·Gly·Gly·Ser (SEQ ID NO: 20))n
(Ser·Gly·Gly·Gly·Gly (SEQ ID NO: 21))n

where n is an integer of 1 or more. However, the length and sequence of a peptide linker to be used in the present invention can be appropriately selected by those skilled in the art.

The gene obtained in the production method of the present invention, is usually inserted in an appropriate vector and introduced in a host cell. The vector is not particularly limited as long as the nucleic acid inserted therein can be stably maintained. For example, if *Escherichia coli* is used as a host, e.g., a pBluescript vector (manufactured by

Stratagene) is preferable as a cloning vector; however various commercially available vectors can be used. In order to produce the antigen-binding molecule of the present invention by use of a vector, an expression vector is particularly useful. The expression vector is not particularly limited as long as it expresses an antigen-binding molecule *in-vitro,* in *Escherichia coli,* a culture cell and an living organism. For example, a pBEST vector (manufactured by Promega KK.) is preferably used *in-vitro*; a pET vector (manufactured by Invitrogen) in *Escherichia coli,* a pME18S-FL3 vector (GenBank Accession No. AB009864) in a culture cell; and a pME18S vector (Mol Cell Biol. 8: 466-472 (1988)) in a living organism; however the expression vector is not limited to these. The gene of the present invention can be inserted into a vector by a conventional method, for example, by a ligase reaction using restriction enzyme sites (Current protocols in Molecular Biology edit. Ausubel et al. (1987) Publish. John Wiley & Sons. Section 11.4-11.11).

The host cell is not particularly limited and various host cells can be used depending upon the purpose. Examples of a cell for expressing an antigen-binding molecule include bacteria cells (example: *Streptococcus, Staphylococcus, Escherichia coli, Streptomyces, Bacillus subtilis*), Eumycetes cells (example: Yeast, *Aspergillus*), insect cells (example: *Drosophila* S2, *Spodoptera* SF9), animal cells (example: CHO, COS, HeLa, C127, 3T3, BHK, HEK293, Bowes melanoma cell) and plant cells. A vector is introduced into a host cell by a method known in the art such as a calcium phosphate precipitation method, electroporation (Current protocols in Molecular Biology edit. Ausubel et al. (1987) Publish. John Wiley & Sons. Section 9.1-9.9), a lipofection method and a micro injection method.

A host cell can be cultured by a method known in the art. For example, when an animal cell is used as a host, for example, DMEM, MEM, RPMI1640, IMDM can be used as a culture solution. At this time, cells can be cultured in a culture using a serum replenisher such as FBS and fetal bovine serum (FCS) in combination or a serum-free medium. The pH during culture can be appropriately selected from approximately 6 to 8 but not limited to this. Various pH values can be appropriately selected depending upon the culture or culturing period/culturing time. Culture is usually performed at approximately 30 to 40°C for approximately 15 to 200 hours and, if necessary, the medium is exchanged and aeration and stirring are performed.

To secrete an antigen-binding molecule expressed in a host cell to a lumen of endoplasmic reticulum, periplasmic space or extracellular environment, an appropriate secretion signal is integrated into a desired polypeptide. The signal may be an endogenic signal or a xenogeneic signal for a desired antigen-binding molecule.

As the system in which a polypeptide is produced, *in vivo,* for example, a production system using an animal and a production system using a plant are mentioned. A desired gene is inserted into a vector, which is introduced into an animal or a plant cell, and then the polypeptide encoded by the gene is expressed and recovered from the body fluid or a living body. The "host" in the present invention includes these animals and plants.

In the production system using an animal as a host, a mammalian animal and an insect are used. Examples of the mammalian animal include goat, pig, sheep, mouse and cow (Vicki Glaser, SPECTRUM Biotechnology Applications (1993)). When a mammalian animal is used, a transgenic animal can be appropriately used.

For example, a polynucleotide encoding the antigen-binding molecule of the present invention is fused with a gene encoding a polypeptide such as goat β casein (which is intrinsically produced in goat milk) to prepare a fusion gene. Subsequently, a polynucleotide fragment containing the fusion gene is injected into a goat embryo and the embryo is grafted in a female goat. The goat to which the embryo is grafted, gives birth a transgenic goat. From the milk of the transgenic goat and its progenies, a desired antigen-binding molecule can be collected. To increase the amount of milk produced from a transgenic goat and containing the antigen-binding molecule, an appropriate hormone can be administered to the transgenic goat (Ebert et al., Bio/Technology (1994) 12, 699-702).

As an insect for producing the antigen-binding molecule of the present invention, for example, a silk worm can be used. When a silk worm is used, a polynucleotide encoding a desired antigen-binding molecule is inserted into a baculovirus and then a silk worm is infected with the baculovirus. A desired antigen-binding molecule can be collected from the body fluid of the silk worm.

When a plant is used for producing the antigen-binding molecule of the present invention, for example, tobacco can be used. When tobacco is used, a polynucleotide encoding a desired antigen-binding molecule is inserted into a plant expression vector such as pMON 530, which is then introduced into a bacterium such as *Agrobacterium tumefaciens.* Tobacco is infected with the bacterium. From the leaves of the tobacco, a desired antigen-binding molecule can be recovered (Ma et al., Eur. J. Immunol. (1994) 24, 131-8). Furthermore, duckweed (*Lemna minor*) is infected with the bacterium. From clone cells of duckweed, a desired antigen-binding molecule can be recovered (Cox KM et al. Nat. Biotechnol. (2006) (12), 1591-1597).

When the sugar chain receptor-binding domain having a desired sugar chain bound thereto is used as a sugar chain receptor-binding domain contained in the antigen-binding molecule in the present invention, a method known in the art can be employed as a method for binding a sugar chain to a sugar chain receptor-binding domain. For example, an enzymatic method performed in an acellular system as described in (Japanese Patent Laid-Open No. 2006-141241), which is a method for producing an antibody modified after translation in an acellular protein synthesis system, comprising preparing a cell extraction solution from cultured cells of an immortalized mammalian animal cell strain capable of secreting a protein and adding mRNA encoding an antibody to the extraction solution, can be employed as a method

for producing the sugar chain receptor-binding domain having a desired sugar chain bound thereto. Such an antigen-binding molecule may be further treated with an enzyme. This step can be included in the method of the present invention.

The antigen-binding molecules produced as mentioned above may have the same sugar chain structure in some cases; whereas, the antigen-binding molecules are produced as a mixture of the antigen-binding molecules having different sugar chains added thereto in other cases. In the present invention, such a mixture can be also preferably used. Furthermore, an antigen-binding molecule having a sugar-chain binding-domain to which a specific sugar chain is connected can be preferably used in the present invention.

As a method for connecting a specific sugar chain to a sugar chain receptor-binding domain contained in the antigen-binding molecule in the present invention, a plurality of methods known in the art can be employed. As one of the known methods, a method of purifying an antigen-binding molecule having a specific sugar chain by use of a property of a sugar chain of a natural antigen-binding molecule or an artificial antigen-binding molecule (prepared by a genetic recombination method, etc.) is mentioned. An antibody having high-mannose sugar chain is known to be purified by affinity chromatography using ConA-sepharose (Millward (Biologicals (2008) 36, 49-60)). Such a purification method can be used for preparing an antigen-binding molecule having an N-linked sugar chain having mannose at the non-reducing end, in the present invention

As necessary, before and after purification of an antigen-binding molecule, an appropriate protein modification enzyme may be used. In this manner, modification can be optionally added or a molecule such as a peptide used for partial or entire modification can be removed. Examples of the protein modification enzyme that can be used include trypsin, chymotrypsin, Lysyl Endopeptidase, protein kinase and glucosidase. The antigen-binding molecule may be further treated with an enzyme. Such an enzymatic treatment step is also included in the method of the present invention.

As a method for ligating a specific sugar chain to a sugar chain receptor-binding domain contained in the antigen-binding molecule of the present invention, a plurality of methods known in the art can be employed. In order to obtain an antigen-binding molecule having a specific sugar chain, an enzyme treatment can be appropriately employed. As described later in Examples, an antigen-binding molecule having an N-linked sugar chain having galactose at a terminal can be prepared from an antigen-binding molecule having a complex sugar chain having sialic acid at a terminal, by a sialidase treatment. Furthermore, it is known that an antibody having a high-mannose sugar chain from which galactose is removed is prepared by sialidase and β galactosidase treatment (Newkirk (Clin. Exp. Immunol. (1996) 106, 259-264)). Such a preparation method including treatments with sialidase and β galactosidase can be used for preparing an antigen-binding molecule having an N-linked sugar chain having mannose at a terminal, in the present invention. The antigen-binding molecule may be further treated with an enzyme. Such an enzymatic treatment step is also included in the production method of the present invention.

Through introduction into a host cell as described later, the antigen-binding molecule of the present invention containing a sugar chain receptor-binding domain (to which a desired sugar chain is to be bound) can be produced from the culture fluid of the host cell.

In a different aspect, in order to obtain an antigen-binding molecule having a specific sugar chain of the present invention, a method including transfecting a host cell with a recombinant gene encoding the antigen-binding molecule, modifying glycosidase activity of the host cell so as to accumulate a specific sugar chain (by a method including genetic or genetic recombination technique but not limited to these), and recovering the antigen-binding molecule from a culture fluid of the host cell, can be appropriately employed. It is known that an antibody having a high mannose N-linked sugar chain is collected from a culture fluid of a Lec1 mutation strain derived from a CHO cell defective in N-acetylglucosaminyltransferase I activity and transfected with a recombinant gene encoding the antibody (Wright and Morrison (J. Exp. Med. (1994) 180, 1087-1096)). In the present invention, an antigen-binding molecule having an N-linked sugar chain (having mannose at the non-reducing end) can be collected from a culture fluid of a Lec1 mutation strain transfected with a recombinant gene encoding the antigen-binding molecule.

In another aspect, in order to obtain an antigen-binding molecule having a specific sugar chain of the present invention, a method including culturing a cell producing the antigen-binding molecule with the addition of an inhibitor for a specific glycosidase reaction and collecting an antigen-binding molecule having a specific sugar chain accumulated in the culture fluid can be appropriately employed. It is known that an antibody having a high mannose N-linked sugar chain without fucose is collected from a culture fluid of a CHO cell producing the antibody cultured with the addition of kifunesine (Zhou (Biotechnol. Bioeng. (2008) 99, 652-665)). 652-665)). In the present invention, an antibody having an N-linked sugar chain having mannose at a terminal without fucose can be collected from a culture fluid of a CHO cell transfected with a recombinant gene encoding the molecule and cultured with the addition of kifunesine. Furthermore, the antigen-binding molecule having a specific sugar chain of the present invention can be obtained by using a method of adding such an inhibitor in combination in culturing a host cell whose glycosidase activity is modified as mentioned above. It is known that an antibody having a specific sugar chain can be collected by such a combination (Kanda, et al. (Glycobiology (2007) 17, 104-118)). In the present invention, in order to obtain an antigen-binding molecule having a specific sugar chain, the same combination can be appropriately employed.

The antigen-binding molecules produced as mentioned above may have the same sugar chain structure in

some cases; whereas, the antigen-binding molecules are produced as a mixture of the antigen-binding molecules having different sugar chains added thereto in other cases. In the present invention, such a case of a sugar chain mixture can be preferably used. Furthermore, an antigen-binding molecule having a sugar-chain binding-domain (to which a specific sugar chain is connected) can be preferably used in the present invention.

The antigen-binding molecule thus obtained is isolated from a host cell or outside the cell (medium, milk, etc.) and can be purified as a substantially pure and homogeneous antigen-binding molecule. The method for separating and purifying used for separation and purification of the antigen-binding molecule can be appropriately selected from conventional separation and purification methods (these methods may be used in combination depending upon the purpose); but is not limited to a specific method. Examples thereof include column chromatography, filtration, ultrafiltration, salting out, solvent deposition, solvent extraction, distillation, immunoprecipitation, SDS-polyacrylamide gel electrophoresis, isoelectric focusing, dialysis and recrystallization. These may be appropriately selected or used in combination for separating and purifying an antigen-binding molecule.

Examples of the chromatography include affinity chromatography, ion exchange chromatography, hydrophobic chromatography, gel filtration, reverse phase chromatography, and adsorption chromatography (Strategies for Protein Purification and Characterization: A Laboratory Course Manual. Ed Daniel R. Marshak et al. (1996) Cold Spring Harbor Laboratory Press). These chromatographic methods can be performed by use of liquid phase chromatography such as HPLC, FPLC. As the column to be used in affinity chromatography, a protein A column and a protein G column are mentioned. For example of a column using protein A, Hyper D, POROS, and Sepharose F. F. (manufactured by Pharmacia), etc. are mentioned.

Whether the antigen-binding molecule prepared as mentioned above has an antigen-binding activity to FcRn (particularly human FcRn) and a desired sugar chain receptor binding activity, etc. or not is evaluated by the method of determining an antigen-binding activity, an FcRn-binding activity or an sugar chain receptor-binding activity as mentioned above. As a result of the evaluation, a step of designing a motif for a sugar chain receptor-binding domain and a step of preparing a gene encoding a polypeptide of an antigen-binding molecule containing a motif for a sugar chain receptor-binding domain are repeated more than once to obtain an antigen-binding molecule having a desired property.

An antigen-binding molecule containing a sugar chain receptor-binding domain (particularly a human derived sugar chain receptor-binding domain) according to the present invention binds to a sugar chain receptor in a pH-dependent manner and/or, having a sugar chain receptor-binding activity (particularly a human-derived sugar chain receptor-binding activity) in a neutral pH range. If the binding activity to a sugar chain receptor in an acidic pH range can be lowered than (the binding activity to the sugar chain receptor) in the neutral pH range property, the uptake of an antigen into a cell by the antigen-binding molecule can be promoted. If such an antigen-binding molecule is administered, a decrease of the antigen concentration in plasma can be promoted and the pharmacokinetics of the antigen-binding molecule can be improved, with the result that the number of antigens that can be bound by a single antigen-binding molecule can be increased.

The antigen-binding molecule produced by the production method of the present invention is an antigen-binding molecule that can promote a decrease of the antigen concentration in plasma by administration. Therefore, the production method of the present invention can be used as a method for producing an antigen-binding molecule, by administration of which the antigen concentration in plasma is promoted.

The antigen-binding molecule produced by the production method of the present invention is an antigen-biding molecule improved in pharmacokinetics. Therefore, the production method of the present invention can be used as a method for producing an antigen-binding molecule improved in pharmacokinetics.

It is considered that the antigen-binding molecule produced by the production method of the present invention can increase the number of antigens that can be bound by a single antigen-binding molecule by administering it to an animal such as a human, a mouse and a monkey. Therefore, the production method of the present invention can be used for producing an antigen-binding molecule capable of binding to a larger number of antigens per molecule.

It is considered that when the antigen-binding molecule produced by the production method of the present invention is administered to an animal such as a human, a mouse and a monkey and binds to an antigen outside the cell, the antigen can be dissociated from the antigen-binding molecule within the cell. Therefore, the production method of the present invention can be used as a method for producing an antigen-binding molecule capable of intracellularly dissociating an antigen extracellularly bound.

It is considered that when the antigen-binding molecule produced by the production method of the present invention is administered to an animal such as a human, a mouse and a monkey and binds to an antigen, the antigen-binding molecule bound to the antigen can be taken up into a cell and then released as a free antibody (without an antigen attached thereto). The production method of the present invention can be used as a method for producing an antigen-binding molecule, which is taken up into a cell while binding to an antigen and released outside the cell as a free antigen-binding molecule (not attached with the antigen).

When such an antigen-binding molecule is administered, it highly effectively reduce the antigen concentration in plasma compared to a conventional antigen-binding molecule. Therefore, it is considered that the antigen-binding

molecule is particularly excellent as a pharmaceutical product. Therefore, the production method of the present invention can be used as a method for producing an antigen-binding molecule for use in a pharmaceutical composition.

Pharmaceutical composition

The present invention also relates to a pharmaceutical composition containing the antigen-binding molecule of the present invention or an antigen-binding molecule produced by the production method of the present invention. The antigen-binding molecule of the present invention or the antigen-binding molecule produced by the production method of the present invention is highly effectively reducing the antigen concentration in plasma by administration thereof, compared to a conventional antigen-binding molecule. Therefore, antigen-binding molecule of the present invention is useful for use in a pharmaceutical composition. The pharmaceutical composition of the present invention can contain a pharmaceutically acceptable carrier.

In the present invention, the pharmaceutical composition generally refers to a medicinal agent for treating or preventing a disease or examination/diagnosis.

The pharmaceutical composition of the present invention can be prepared by a method known to those skilled in the art. The pharmaceutical composition of the present invention is mixed with, for example, water or a pharmaceutically acceptable liquid other than water to prepare an aseptic solution or an injection of a suspension agent and can be parenterally used. For example, the pharmaceutical composition of the present invention is appropriately mixed with a pharmacologically acceptable carrier or a medium, more specifically, appropriately blended with sterile water and physiological saline, a vegetable oil, an emulsifier, a suspending agent, a surfactant, a stabilizer, a flavor agent, an excipient, a vehicle, a preservative and a binder, etc. into a preparation in the form of unit dosage required for preparation practice generally admitted. The amount of active ingredient in the preparation is set so as to obtain an appropriate dose within a determined range.

An aseptic composition for an injection can be formulated in accordance with a conventional preparation practice by use of a vehicle such as distillation water for injection. As an aqueous solution for an injection, for example, an isotonic liquid including physiological saline, glucose and other additives (for example, D-sorbitol, D-mannose, D-mannitol, sodium chloride) is mentioned. An appropriate solubilizing agent such as an alcohol (ethanol, etc.), a polyalcohol (propylene glycol, polyethylene glycol, etc.) and a nonionic surfactant (polysorbate 80 (TM), HCO-50, etc.) can be used in combination.

As an oily liquid, sesame oil and soybean oil are mentioned. As a solubilizing agent, benzyl benzoate and/or benzyl alcohol can be used in combination. Furthermore, a buffer (for example, a phosphate buffer solution and a sodium acetate buffer solution), a soothing agent (for example, procaine hydrochloride), a stabilizer (for example, benzyl alcohol and phenol) and an antioxidant can be added. The injection prepared is usually charged in an appropriate ample.

The pharmaceutical composition of the present invention is preferably parenterally administered. The pharmaceutical composition of the present invention can be prepared, for example, as injectable, transnasal administration, pulmonary administration and transdermal administration compositions. The pharmaceutical composition of the present invention is administered systemically or locally, through e.g., intravenous injection, intramuscular injection, intraperitoneal injection and hypodermic injection.

As the administration method for the pharmaceutical composition of the present invention, a preferable method is appropriately selected depending upon the age and symptom of a patient. The dose of a pharmaceutical composition containing an antigen-binding molecule can be set to fall, for example, within the range of 0.0001 mg to 1000 mg per body weight (1 kg) per time or within the range of 0.001 to 100000 mg per patient. However, the dose of the pharmaceutical composition of the present invention is not always limited to these numerical values. Dose and administration method vary depending upon the weight, age and symptom, etc. of a patient. Those skilled in the art can set an appropriate dose and administration method in consideration of these conditions.

Note that amino acids contained in the amino acid sequence described in the present invention are sometimes modified after translation (for example, N-terminal glutamine is modified into pyroglutamine acid, as is known well to those skilled in the art). Such a modified antigen-binding molecule after translation is included within the range of the antigen-binding molecule specified by the amino acid sequence described in the present invention.

Method for promoting uptake of an antigen-binding molecule or an antigen binding to the antigen-binding molecule, including bringing it into contact with a cell expressing a sugar chain receptor *in-vivo* or *ex-vivo*

The present invention further provides a method for promoting uptake of an antigen-binding molecule or an antigen (binding to the antigen-binding molecule) into a cell, comprising bringing the antigen-binding molecule or the antigen (binding to the antigen-binding molecule) into contact with a cell expressing a sugar chain receptor, which binds to a sugar chain receptor-binding domain contained in the antigen-binding molecule, *in-vivo* or *ex-vivo.*

In the present invention, "uptake ... into a cell" refers to uptake of an antigen-binding molecule or an antigen

(binding to the antigen-binding molecule) into a cell by endocytosis. In the present invention, the expression "promoting uptake ... into a cell" refers to increasing a speed of taking up an antigen-binding molecule, which binds to an antigen outside a cell, into the cell. Accordingly, in the present invention, whether or not uptake of an antigen-binding molecule or an antigen (binding to the antigen-binding molecule) was promoted is determined based on whether or not the uptake speed of the antigen-binding molecule or the antigen (binding to the antigen-binding molecule) into the cell increased or not. The uptake speed of an antigen into a cell can be calculated, for example, by adding an antigen-binding molecule and an antigen to a culture fluid containing a sugar chain receptor expression cell; and measuring a decrease of concentration of the antigen-binding molecule or the antigen (binding to the antigen-binding molecule) in the culture fluid with the passage of time, or, measuring the amount of antigen-binding molecule or antigen (binding to the antigen-binding molecule) taken up into a sugar chain receptor expression cell versus time. The contact between a cell expressing a sugar chain receptor and an antigen-binding molecule and an antigen (binding to the antigen-binding molecule) may also occur *ex-vivo* as mentioned above and occurs *in vivo* by administering an antigen-binding molecule.

The clearance speed of an antigen in plasma can be increased by the method (provided by the present invention) for increasing an uptake speed of an antigen-binding molecule and an antigen (binding to the antigen-binding molecule) into a cell, comprising bringing the antigen-binding molecule and the antigen (binding to the antigen-binding molecule) into contact with a cell expressing a sugar chain receptor, which binds to the sugar chain receptor-binding domain contained in the antigen-binding molecule, *in-vivo* or *ex-vivo;* more specifically by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use and returning the plasma containing a free antigen-binding molecule (not bound to the antigen) into the living body; or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body, can be also used. Accordingly, whether or not uptake of an antigen-binding molecule or an antigen (binding to the antigen-binding molecule) into a cell was promoted can be confirmed also by determining whether or not the clearance speed of the antigen present in the plasma was accelerated, compared to the case where the antigen-binding molecule is not administered or by determining whether or not the concentration of the antigen in plasma was reduced by the *ex-vivo* method or administration of an antigen-binding molecule.

Whether uptake is promoted or not can be confirmed by determining whether or not the clearance speed of an antigen in plasma (which is determined by the above (1) and (2) methods) is accelerated compared to the clearance speed of an antigen in plasma, which is determined by the same method as above except that a human natural IgG (particularly human natural IgG1) is used in place of the antigen-binding molecule.

The present invention further provides a method of promoting uptake of an antigen binding to an antigen-binding molecule, into a cell, comprising, bringing the antigen-binding molecule into contact with a cell expressing a sugar chain receptor (which binds to a sugar chain receptor-binding domain contained in the antigen-binding molecule) containing an antigen-binding domain whose binding activity to the antigen changes depending upon the ion-concentration condition, *in-vivo* or *ex-vivo.* The present invention further provides a method of promoting uptake of an antigen binding to an antigen-binding molecule into a cell, comprising, bringing the antigen-binding molecule into contact with a cell expressing a sugar chain receptor which binds to a sugar chain receptor-binding domain contained in the antigen-binding molecule containing an antigen-binding domain (whose binding activity to the antigen has been changed depending upon the ion-concentration condition), *in-vivo* or *ex-vivo.* The present invention further provides a method of promoting uptake of an antigen binding to an antigen-binding molecule into a cell, comprising bringing the antigen-binding molecule (in which at least one amino acid of an antigen-binding domain is an amino acid which changes the binding activity of the antigen-binding domain to the antigen depending upon the calcium-ion concentration condition or pH condition) into contact with a cell expressing a sugar chain receptor (which binds to a sugar chain receptor-binding domain contained in the antigen-binding molecule) *in-vivo* or *ex-vivo.*

In the present invention, as the method for "changing the binding activity of the antigen-binding domain to the antigen depending upon the ion-concentration condition", a plurality of methods described as a method for producing an antigen-binding molecule in the specification may be appropriately used alone or in combination.

In the present invention, as the cell expressing a sugar chain receptor binding to a sugar chain receptor-binding domain and contained in the antigen-binding molecule, any cell can be used as long as the cell expresses a desired sugar chain receptor. The cell is not limited to a specific cell. To specify the cell expressing a desired sugar chain receptor, database known in the art, such as Human Protein Atlas (http://www.proteinatlas.org/), can be used. Furthermore, whether the cell, which is to be brought into contact with the antigen-binding molecule of the present invention, expresses a sugar chain receptor or not can be confirmed by a technique for determining expression of a gene encoding a desired sugar chain receptor and an immunological technique using an antibody binding to a desired sugar chain receptor. These techniques are also known in the art. Since the cell expressing a sugar chain receptor can be brought into contact

with an antigen-binding molecule and an antigen (binding to the antigen-binding molecule) not only outside a living body but also inside a living body, the expression: bringing the antigen-binding molecule into contact with the cell expressing a sugar chain receptor in the present invention, includes administering the antigen-binding molecule to a living body. The contact time is, for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

<u>Method for increasing the number of antigens to which a single antigen-binding molecule binds, comprising bringing an antigen-binding molecule into contact with a cell expressing a sugar chain receptor, *in-vivo* or *ex-vivo*</u>

The present invention provides a method of increasing the number of antigens to which a single antigen-binding molecule binds, comprising bringing an antigen-binding molecule having an antigen-binding domain, an FcRn binding domain and one or more sugar chain receptor-binding domains into contact with a cell expressing a sugar chain receptor binding to the sugar chain receptor-binding domain contained in the antigen-binding molecule, *in-vivo* or *ex-vivo.*

In the present invention, the expression "the number of antigens to which a single antigen-binding molecule binds" refers to the number of antigens to which the antigen-binding molecule can bind until it is decomposed and cleared. In the present invention, "increasing the number of antigens to which a single antigen-binding molecule can bind" refers to increasing the binding times of an antigen-binding molecule which repeats dissociation from an antigen molecule and association with another antigen molecule. The antigen molecule binding to an antigen-binding molecule may be the same antigen molecule or a different antigen molecule in a reaction system where both molecules are present. In other words, the binding times refers to the total binding times of an antigen-binding molecule to an antigen in the reaction system. In other expression, provided that a process where the antigen-binding molecule bound to an antigen is taken up into a cell, dissociates the antigen in an endosome and returns outside the cell is regarded as one cycle, the number of cycle repeats until the antigen-binding molecule is decomposed and cleared is referred to the binding times. The antigen-binding molecule of the present invention having a binding activity to a sugar chain receptor in a neutral pH range binds to the sugar chain receptor and then is taken up into the cell expressing the sugar chain receptor by endocytosis. The antigen-binding molecule of the present invention is released from the sugar chain receptor in an acidic range, binds to FcRn (particularly human FcRn) in the acidic range and is recycled outside the cell. The antigen-binding molecule of the present invention, from which an antigen is dissociated in the acidic range, is recycled outside the cell. Therefore, the antigen-binding molecule can bind again to an antigen. Accordingly, whether the number of cycles increases can be determined based on whether "uptake into a cell is promoted" or not, or whether "pharmacokinetics is improved"(described later) or not.

The clearance speed of an antigen in plasma can be increased by the method (provided by the present invention) for increasing the number of antigens to which a single antigen-binding molecule binds, comprising bringing an antigen-binding molecule and an antigen (binding to the antigen-binding molecule) into contact with to a cell expressing a sugar chain receptor (binding to a sugar chain receptor-binding domain contained in the antigen-binding molecule), more specifically, by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body, or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used. Accordingly, whether or not the number of antigens to which a single antigen-binding molecule binds was increased can be confirmed also by determining whether or not the clearance speed of the antigen present in plasma has been accelerated compared to the case where the antigen-binding molecule is not administered or by determining whether or not the concentration of the antigen in plasma has been reduced by the *ex-vivo* method or administration of the antigen-binding molecule.

Whether the number of antigens to which a single antigen-binding molecule binds is increased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

The present invention further provides a method of increasing the number of antigens, to which a single antigen-binding molecule can bind, comprising, bringing an antigen-binding molecule, whose binding activity to an antigen changes depending upon the ion-concentration condition and thus capable of binding a larger number of antigens (in terms of the number of antigens to which a single antigen-binding molecule can bind) into contact with a cell expressing a sugar chain receptor (binding to a sugar chain receptor-binding domain contained in the antigen-binding molecule), *in-vivo* or *ex-vivo.* The present invention further provides a method of increasing the number of antigens, to which a single antigen-binding molecule can bind, comprising, bringing an antigen-binding molecule containing an antigen-binding domain (whose binding activity to the antigen has been changed depending upon the ion-concentration condition and thus capable of binding a larger number of antigens (in terms of the number of antigens to which a single antigen-binding molecule can bind)), into contact with a cell expressing a sugar chain receptor (binding to a sugar chain receptor-binding domain) contained in the antigen-binding molecule, *in-vivo* or *ex-vivo.* The present invention further provides a method of increasing the number of antigens, to which a single antigen-binding molecule can bind, comprising bringing an antigen-binding molecule (in which at least one amino acid of the antigen-binding domain is an amino acid which changes the binding activity to an antigen depending upon the calcium-ion concentration condition or pH condition) increased in the number of antigens to which a single antigen-binding molecule can bind into contact with a cell expressing a sugar chain receptor (binding to a sugar chain receptor-binding domain) contained in the antigen-binding molecule, *in-vivo* or *ex-vivo.*

In the present invention, as the method of "changing the binding activity of an antigen-binding domain to an antigen depending upon the ion-concentration condition", a plurality of methods described as a method for producing an antigen-binding molecule in the specification may be appropriately used alone or in combination.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell (which is brought into contact with the antigen-binding molecule of the present invention) expresses a sugar chain receptor or not can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) not only outside a living body but also inside a living body, the expression: bringing the antigen-binding molecule into contact with the cell expressing a sugar chain receptor in the present invention, includes administering the antigen-binding molecule to a living body. The contact time is, for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

<u>Method for decreasing the number of antigens present outside a cell, comprising bringing an antigen-binding molecule into contact with a cell expressing a sugar chain receptor *in-vivo* or *ex-vivo*</u>

The present invention provides a method of decreasing the number of antigens present outside a cell, comprising bringing an antigen-binding molecule having an antigen-binding domain, an FcRn binding domain and one or more sugar chain receptor-binding domains into contact with a cell expressing a sugar chain receptor (binding to a sugar chain receptor-binding domain contained in the antigen-binding molecule) *in-vivo* or *ex-vivo.*

The expression: the antigen present outside a cell, refers to an antigen present outside the cell expressing a sugar chain receptor. When the cell expressing a sugar chain receptor is present inside a living body, an antigen present outside the cells of a body fluid such as blood, plasma, serum, urine, lymph fluid, saliva and tear can be mentioned as an example (of the antigen present outside a cell). However, as long as the antigen-binding molecule of the present invention administered to a living body can bind to the antigen outside the cell expressing a sugar chain receptor, the antigen present outside a cell is not limited to the antigen present in these body fluids. Particularly preferable example of the antigen present outside a cell is an antigen present in plasma. When a cell expressing a sugar chain receptor is present outside a living body, not only the antigen present in a body fluid taken out from a living body, such as blood, plasma, serum, urine, lymph fluid, saliva and tear, but also an antigen present in a culture fluid of the cell can be mentioned as an example of the antigen present outside a cell.

The antigen-binding molecule of the present invention having a binding activity to a sugar chain receptor in a neutral pH range, binds to the sugar chain receptor and thereafter is taken up into the cell expressing the sugar chain receptor by endocytosis. The antigen-binding molecule (of the present invention) is released from the sugar chain receptor in an acidic range and binds to FcRn (particularly human FcRn) in the acidic range. In this manner, the antigen-binding molecule of the present invention is returned again outside the cell for recycle use. The antigen-binding molecule of the present invention (from which an antigen dissociates in the acidic range) returned outside the cell for recycle use can bind again to an antigen. In this case, the antigen dissociated is decomposed in a lysosome within a cell. Therefore, every time the antigen-binding molecule of the present invention is recycled, the antigen, which is present outside the cell expressing a sugar chain receptor (which is to be bound to the antigen-binding molecule of the present invention) is decomposed in a lysosome within the cell (expressing a sugar chain receptor). As a result of such decomposition of the antigen, the number of antigens present outside the cell presumably decreases. Whether the number of antigens present outside a cell decreased or not can be evaluated by measuring the amount of antigen extracellularly present out of a body fluid such as blood, plasma, serum, urine, lymph fluid, saliva and tear if a cell (expressing a sugar chain receptor) is present within a living body, and evaluated by measuring the amount of antigen present in not only a body fluid taken out of a living body, such as blood, plasma, serum, urine, lymph fluid, saliva and tear but also a culture fluid of the cell, if the cell (expressing a sugar chain receptor) is present outside a living body.

The number of antigens present outside a cell can be decreased by the method (provided by the present invention) for decreasing the number of antigens present outside a cell, comprising bringing an antigen-binding molecule and an antigen (binding to the antigen-binding molecule) into contact with a cell expressing a sugar chain receptor (binding to a sugar chain receptor-binding domain contained in the antigen-binding molecule), *in-vivo* or *ex-vivo,* more specifically by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body; or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used. Accordingly, whether or not the number of antigens present outside a cell decreased can be confirmed also by determining whether or not the amount of antigen present in the plasma has decreased compared to the case where the antigen-binding molecule is not administered or by determining whether or not the concentration of the antigen in plasma has been reduced by the *ex-vivo* method or administration of an antigen-binding molecule.

Whether the amount of antigen in plasma decreased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

The present invention further provides a method of decreasing the number of antigens present outside a cell, comprising, bringing the antigen-binding molecule of the present invention containing an antigen-binding domain, whose binding activity to an antigen changes depending upon the ion-concentration condition, into contact with a cell expressing a sugar chain receptor (binding to a sugar chain receptor-binding domain contained in the antigen-binding molecule), *in-vivo* or *ex-vivo.* The present invention further provides a method of decreasing the number of antigens present outside a cell, comprising, bringing the antigen-binding molecule of the present invention containing an antigen-binding domain, whose binding activity to an antigen has been changed depending upon the ion-concentration condition, into contact with a cell expressing a sugar chain receptor (binding to a sugar chain receptor-binding domain contained in the antigen-binding molecule), *in-vivo* or *ex-vivo.* The present invention further provides a method of decreasing the number of antigens present outside a cell, comprising, bringing the antigen-binding molecule of the present invention into contact with a cell expressing a sugar chain receptor (binding to a sugar chain receptor-binding domain contained in the antigen-binding molecule), *in-vivo* or *ex-vivo,* (in which at least one amino acid of the antigen-binding domain is an amino acid which changes the binding activity to an antigen depending upon the calcium-ion concentration condition or pH condition).

In the present invention, as the method for "changing the binding activity of an antigen-binding domain to an antigen depending upon the ion-concentration condition", a plurality of methods described as a method for producing an antigen-binding molecule in the specification may be appropriately used alone or in combination.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell (which is brought into contact with the antigen-binding molecule of the present invention) expresses a sugar chain receptor or not can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) not only outside a living body

but also inside a living body, the expression: bringing the antigen-binding molecule into contact with the cell expressing a sugar chain receptor in the present invention, includes administering the antigen-binding molecule to a living body. The contact time is, for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

Method for improving the pharmacokinetics of an antigen-binding molecule, comprising bringing an antigen-binding molecule into contact with a cell expressing a sugar chain receptor, *in-vivo*

The present invention provides a method of improving the pharmacokinetics of an antigen-binding molecule, comprising bringing an antigen-binding molecule (having an antigen-binding domain, an FcRn binding domain and one or more sugar chain receptor-binding domains) into contact with a cell expressing a sugar chain receptor (binding to a sugar chain receptor-binding domain contained in the antigen-binding molecule) *in-vivo* or ex-*vivo.*

In the present invention, "improvement of pharmacokinetics", "enhancement of pharmacokinetics" or "excellent pharmacokinetics" can be rephrased by "improvement of retentivity in plasma (in blood)", " enhancement of retentivity in plasma (in blood)", "excellent retentivity in plasma (in blood)" or "extending retentivity in plasma (in blood)". The meanings of these expressions are all the same.

In the present invention, "pharmacokinetics is improved" refers to not only extending the time period from administration of an antigen-binding molecule to a human or an animal (such as mouse, rat, monkey, rabbit and dog) up to clearance from plasma (for example, until the antigen-binding molecule is, e.g., decomposed in a cell and cannot return to the plasma) but also extending the time period during which the antigen-binding molecule remains in plasma while keeping the state capable of binding to an antigen (e.g., the state of an antigen-binding molecule not binding to an antigen) from administration of an antigen-binding molecule up to clearance from plasma by decomposition. In short, extending the time period until an antigen-binding molecule unbound to an antigen (antigen-unbound antigen-binding molecule) disappears by decomposition is included. Even if an antigen-binding molecule is present in plasma, if an antigen is already bound to the antigen-binding molecule, the antigen-binding molecule cannot bind to another antigen. Thus, if the time period during which an antigen-binding molecule is not bound to an antigen becomes long, the time period during which the antigen-binding molecule can bind to another antigen becomes long (chance to bind another antigen is increased). As a result, the time period during which an antigen is not bound to an antigen-binding molecule *in vivo* can be reduced and the time period during which an antigen binds to an antigen-binding molecule can be extended. If clearance of an antigen from plasma can be accelerated by administration of an antigen-binding molecule, the plasma concentration of the antigen-unbound antigen-binding molecule increases and the time period during which an antigen is bound to an antigen-binding molecule increases. In short, in the present invention, the expression: "improvement of the pharmacokinetics of an antigen-binding molecule" refers to improvement of any one of pharmacokinetics parameters of an antigen-unbound antigen-binding molecule (any one of the parameters: an increase of half-life period in plasma, an increase of average retention time in plasma and decrease of clearance in plasma), or extension of time period during which an antigen is bound to an antigen-binding molecule after the antigen-binding molecule is administered, or acceleration of antigen clearance from plasma by an antigen-binding molecule. Determination can be made by measuring any one of parameters (understanding of pharmacokinetics, by practice (Nanzando Co., Ltd.)): half-life period of an antigen-binding molecule or an antigen-unbound antigen-binding molecule in plasma, average retention time in plasma and clearance in plasma. For example, when an antigen-binding molecule is administered to a mouse, rat, monkey, rabbit, dog and human, etc. the concentration of antigen-binding molecule (or antigen-unbound antigen-binding molecule) in plasma is measured and individual parameters are calculated. For example, if the half-life period in plasma extends or if the average retention time in plasma extends, it is evaluated that the pharmacokinetics of the antigen-binding molecule is improved. These parameters can be measured by a method known to those skilled in the art and can be appropriately evaluated by Noncompartmental analysis using, e.g., pharmacokinetics analysis soft WinNonlin (Pharsight) in accordance with the accompanying protocol. The concentration of an antigen-unbound antigen-binding molecule in plasma can be measured by a method known to those skilled in the art. For example, a measurement method employed in Clin Pharmacol. (2008) 48 (4) 406-17 can be used.

In the present invention, the concept: "pharmacokinetics is improved" includes extending the time period during which antigen binds to an antigen-binding molecule after administration of the antigen-binding molecule. Whether the time period during which an antigen binds to an antigen-binding molecule after administration of the antigen-binding molecule is extended or not can be determined by measuring the concentration of an antigen-binding molecule-unbound antigen (antigen not binding to an antigen-binding molecule) in plasma, more specifically, based on the time until the concentration of antigen-binding molecule-unbound antigen in plasma or the rate of the concentration of an antigen-binding molecule-unbound antigen relative to total antigen concentration increases.

The concentration of antigen-binding molecule-unbound antigen in plasma or the rate of the concentration of an antigen-binding molecule-unbound antigen relative to total antigen concentration can be determined by a method known to those skilled in the art. For example, the measurement method described in Pharm Res. (2006) 23 (1) 95-103 can be used. When an antigen plays some role *in-vivo,* whether or not the antigen has bound to an antigen-binding molecule (antagonist molecule), which neutralizes the function of the antigen, can be evaluated by determining whether or not the function of the antigen has been neutralized. Whether or not the function of the antigen is neutralized can be evaluated by measuring any one of *in-vivo* markers which reflect the function of the antigen. Whether or not an antigen binds to an antigen-binding molecule (agonist molecule) activating the function of the antigen, can be evaluated by measuring an *in-vivo* marker which reflects the function of the antigen.

Measurement, such as measurement of the concentration of plasma of an unbound antigen, measurement of the ratio of the amount of unbound antigen relative to the total antigen amount and measurement of *in-vivo* marker, is not particularly limited and preferably performed after a lapse of predetermined time from administration of an antigen-binding molecule. In the present invention, the time period expressed by the phrase: after a lapse of a predetermined time from administration of an antigen-binding molecule is not particularly limited, and can be appropriately determined by those skilled in the art in consideration of the properties of the antigen-binding molecule to be administered. For example, one day, 3 days, 7 days, 14 days and 28 days after an antigen-binding molecule is administered are mentioned.

In the present invention, it is preferable that pharmacokinetics in a human is improved. If it is difficult to measure retentivity in plasma in a human, retentivity in plasma in a human can be estimated based on retentivity in plasma of a mouse (for example, normal mouse, human antigen expression transgenic mouse, human FcRn expressing transgenic mouse 32 lineage or 276 lineage (Jackson Laboratories) (Methods Mol Biol. (2010) 602, 93-104) etc., transgenic mouse, etc.) and a monkey (for example, crab-eating monkey).

Furthermore, the present invention provides a method of improving pharmacokinetics of an antigen-binding molecule, comprising administering the antigen-binding molecule, *in-vivo,* whose pharmacokinetics is improved as mentioned above and which contains an antigen-binding domain, whose binding activity to an antigen changes depending upon the ion-concentration condition. The present invention further provides a method of improving pharmacokinetics of an antigen-binding molecule, comprising administering an antigen-binding molecule, *in-vivo,* whose pharmacokinetics is improved as mentioned above and which contains an antigen-binding domain, whose binding activity to an antigen has been changed depending upon the ion-concentration condition. The present invention further provides a method of improving pharmacokinetics of an antigen-binding molecule, comprising administering the antigen-binding molecule (in which at least one amino acid of the antigen-binding domain is an amino acid which changes the binding activity to an antigen depending upon the calcium-ion concentration condition or pH condition and whose pharmacokinetics is improved) *in-vivo.*

In the present invention, as a method for "changing the binding activity of the antigen-binding domain to an antigen depending upon the ion-concentration condition", a plurality of methods described as a method for producing an antigen-binding molecule in the specification may be appropriately used alone or in combination.

Method for promoting dissociation of an antigen from antigen-binding molecule within a cell, including bringing antigen-binding molecule to a cell expressing a sugar chain receptor, *in-vivo* or *ex-vivo*

The present invention provides a method for promoting dissociation of an antigen (bound to an antigen-binding molecule outside a cell) from the antigen-binding molecule within the cell, comprising bringing an antigen-binding molecule (having an antigen-binding domain, an FcRn binding domain and one or more sugar chain receptor-binding domains) into contact with a cell expressing a sugar chain receptor (binding to a sugar chain receptor-binding domain contained in the antigen-binding molecule) *in-vivo* or *ex-vivo.*

In the present invention, as a site at which an antigen is dissociated from an antigen-binding molecule, any site may be used as long as the site is within a cell and preferably, a site within early-stage endosome. In the present invention, the expression: "dissociation of an antigen (bound to an antigen-binding molecule outside a cell) from the antigen-binding molecule within the cell" refers to dissociation of an antigen from an antigen-binding molecule as a result that the antigen (bound to an antigen-binding molecule outside a cell) is brought into contact with a cell expressing a sugar chain receptor (binding to the sugar chain receptor-binding domain contained in the antigen-binding molecule) *in-vivo* or *ex-vivo.* It is not necessary to dissociate all antigens taken up into a cell from antigen-binding molecules within the cell. It is sufficient if the rate of antigens from which antigen-binding molecules are dissociated (or antigen-binding molecules from which an antigen is dissociated) in the cell is high.

The antigen-binding molecule of the present invention having a binding activity to a sugar chain receptor in a neutral pH range, binds to the sugar chain receptor and then is taken up into the cell expressing a sugar chain receptor by endocytosis. In an acidic range, the antigen-binding molecule of the present invention is released from the sugar chain receptor, binds to FcRn (particularly human FcRn) in the acidic range and is recycled outside the cell. The antigen-binding molecule of the present invention (from which an antigen dissociates in the acidic range) recycled outside the cell, can bind again to an antigen. In this case, the antigen dissociated is decomposed in a lysosome within a cell. Therefore, every time the antigen-binding molecule of the present invention is recycled, the antigen present outside the cell expressing a sugar chain receptor (which bound to the antigen-binding molecule of the present invention) is decomposed in a lysosome within the cell (expressing a sugar chain receptor). As a result of the decomposition of the antigen, the number of antigens present outside a cell presumably decreases. Whether the number of antigens decreased or not can be evaluated by measuring the amount of antigen extracellularly present out of a body fluid such as blood, plasma, serum, urine, lymph fluid, saliva and tear when a cell expressing a sugar chain receptor is present within a living body, and evaluated by measuring the amount of antigen present in not only a body fluid taken out of a living body, such as blood, plasma, serum, urine, lymph fluid, saliva and tear but also a culture fluid of the cell, if the cell (expressing a sugar chain receptor) is present outside a living body.

Dissociation of an antigen (bound to an antigen-binding molecule outside a cell) from an antigen-binding molecule within the cell, can be promoted by the method (provided by the present invention) for promoting dissociation of an antigen (bound to an antigen-binding molecule outside a cell) from an antigen-binding molecule within the cell, comprising bringing an antigen-binding molecule and an antigen (binding to the antigen-binding molecule) into contact with a cell expressing a sugar chain receptor (binding to a sugar chain receptor-binding domain contained in the antigen-binding molecule) *in-vivo* or *ex-vivo,* more specifically by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body, or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used. Accordingly, whether or not the number of antigens present outside the cell decreased can be confirmed also by determining whether or not the amount of antigen present in plasma has decreased compared to the case where the antigen-binding molecule is not administered, or by determining whether or not the concentration of the antigen in plasma has been reduced by the *ex-vivo* method or administration of the antigen-binding molecule.

Whether the amount of antigen in plasma decreased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell, which is to be brought into contact with the antigen-binding molecule of the present invention expresses a sugar chain receptor or not can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) inside a living body, the expression: bringing the antigen-binding molecule into contact with the cell expressing a sugar chain receptor in the present invention, includes administering the antigen-binding molecule to a living body. The contact time is for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

The present invention provides a method for promoting dissociation of an antigen (bound to an antigen-binding molecule outside a cell) from the antigen-binding molecule within the cell, comprising bringing the antigen-binding molecule of the present invention containing an antigen-binding domain whose binding activity to the antigen changes depending upon the ion-concentration condition, into contact with a cell expressing a sugar chain receptor (binding to a sugar chain receptor-binding domain contained in the antigen-binding molecule). The present invention further provides a method for promoting dissociation of an antigen (bound to an antigen-binding molecule outside a cell) from the antigen-binding molecule within the cell, comprising bringing the antigen-binding molecule of the present invention containing

an antigen-binding domain whose binding activity to the antigen has been changed depending upon the ion-concentration condition, into contact with a cell expressing a sugar chain receptor (binding to a sugar chain receptor-binding domain contained in the antigen-binding molecule). The present invention further provides a method for promoting dissociation of an antigen (bound to an antigen-binding molecule outside a cell) from the antigen-binding molecule within the cell, comprising, bringing the antigen-binding molecule containing an antigen-binding domain (in which at least one amino acid changing the binding activity of the antigen-binding domain to the antigen depending upon the calcium-ion concentration condition or pH condition) into contact with a cell expressing a sugar chain receptor (binding to a sugar chain receptor-binding domain contained in the antigen-binding molecule).

In the present invention, as the method for "changing the binding activity of an antigen-binding domain to an antigen depending upon the ion-concentration condition", a plurality of methods described as a method for producing an antigen-binding molecule in the specification may be appropriately used alone or in combination.

<u>Method for promoting uptake of antigen-binding molecule or an antigen binding to an antigen-binding molecule</u>

The present invention provides a method for promoting uptake of an antigen-binding molecule or an antigen (binding to an antigen-binding molecule) into a cell expressing a sugar chain receptor, *in-vivo* or ex-*vivo,* comprising, in an antigen-binding molecule containing an antigen-binding domain, an FcRn binding domain and two or more binding domains to a sugar chain receptor, increasing the number of binding domains to the sugar chain receptor.

In the present invention, "uptake ... into a cell" refers to uptake of an antigen-binding molecule or an antigen (binding to the antigen-binding molecule) into a cell by endocytosis. In the present invention, the expression "promoting uptake ... into a cell" refers to increasing a speed of taking up an antigen-binding molecule, which binds to an antigen outside a cell, into the cell. Accordingly, in the present invention, whether uptake of an antigen-binding molecule or (an antigen binding to the antigen-binding molecule) was promoted or not is determined based on the uptake speed of the antigen-binding molecule or the antigen (binding to the antigen-binding molecule) into the cell has been increased or not. The uptake speed of an antigen into a cell can be calculated, for example, by adding an antigen-binding molecule and an antigen to a culture fluid containing a sugar chain receptor expression cell; and measuring a decrease in concentration of the antigen-binding molecule or the antigen (binding to the antigen-binding molecule) in the culture fluid with the passage of time, or measuring the amount of antigen-binding molecule or antigen (binding to the antigen-binding molecule) taken up into a sugar chain receptor expression cell with the passage of time.

In the present invention, as the method "for increasing the number of binding domains to the sugar chain receptor", a plurality of methods described as a method for producing an antigen-binding molecule in the specification may be appropriately used alone or in combination.

Uptake of the antigen-binding molecule of the present invention into a cell expressing a sugar chain receptor can be performed not only *ex-vivo* but also *in vivo* by administering the antigen-binding molecule. More specifically, in the present invention, whether or not uptake of an antigen-binding molecule or an antigen (binding to the antigen-binding molecule) into a cell has been promoted can be confirmed by determining whether or not the clearance speed of an antigen present in plasma was promoted (whether or not the speed has been accelerated compared to the case where the antigen-binding molecule is not administered) or whether or not the antigen concentration in plasma is lowered by administration of the antigen-binding molecule, which is determined, for example, by (1) a method (called an ex-*vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body, or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used.

Whether uptake is promoted or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell (which is brought into contact with the antigen-binding molecule of the present invention) expresses a sugar chain receptor or not can be confirmed by a technique for determining expression of a gene encoding a desired sugar chain receptor and an immunological technique using an

antibody binding to a desired sugar chain receptor. These techniques are known in the art. Since the cell expressing a sugar chain receptor can be brought into contact with an antigen-binding molecule and an antigen (binding to the antigen-binding molecule) inside a living body, the expression: bringing the antigen-binding molecule into contact with the cell expressing a sugar chain receptor in the present invention, includes administering the antigen-binding molecule to a living body. The contact time is, for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

The present invention provides a method for increasing the number of binding domains to a sugar chain receptor contained in an antigen-binding molecule (containing an antigen-binding domain, an FcRn binding domain and two or more binding domains to a sugar chain receptor) to the sugar chain receptor; at the same time, for promoting uptake of an antigen-binding molecule or an antigen (binding to the antigen-binding molecule) containing an antigen-binding domain whose binding activity changes to an antigen depending upon the ion-concentration condition. The present invention further provides a method for increasing the number of binding domains, which are contained in an antigen-binding molecule (containing an antigen-binding domain, an FcRn binding domain and two or more binding domains to a sugar chain receptor) to the sugar chain receptor; at the same time, for promoting uptake of an antigen-binding molecule or an antigen (binding to the antigen-binding molecule) containing an antigen-binding domain whose binding activity to an antigen has been changed depending upon the ion-concentration condition. The present invention further provides a method for increasing the number of binding domains, which are contained in an antigen-binding molecule (containing an antigen-binding domain, an FcRn binding domain and two or more binding domains to the sugar chain receptor) to the sugar chain receptor; at the same time, for promoting uptake of an antigen-binding molecule or an antigen (binding to the antigen-binding molecule) (in which at least one amino acid of the antigen-binding domain is an amino acid, which changes the binding activity of the antigen-binding domain to an antigen depending upon the calcium-ion concentration condition or pH condition).

In the present invention, as the method "for changing the binding activity of an antigen-binding domain to an antigen depending upon the ion-concentration condition", a plurality of methods described as a method for producing an antigen-binding molecule in the specification may be appropriately used alone or in combination.

<u>Method for increasing the number of antigens to which a single antigen-binding molecule binds</u>

The present invention provides a method for increasing the number of antigens to which a single antigen-binding molecule binds, *in vivo* and *ex-vivo,* comprising increasing the number of binding domains to a sugar chain receptor in an antigen-binding molecule containing an antigen-binding domain, an FcRn binding domain and one or more binding domains to the sugar chain receptor.

In the present invention, the expression "the number of antigens to which a single antigen-binding molecule binds" refers to the number of antigens that an antigen-binding molecule can bind until it is decomposed and cleared. In the present invention, "increasing the number of antigens to which a single antigen-binding molecule can bind" refers to increasing the binding times of an antigen-binding molecule which repeats dissociation from an antigen molecule and association with another antigen molecule. The antigen molecule binding to an antigen-binding molecule may be the same antigen molecule or a different antigen molecule in a reaction system where both molecules are present. In other words, the binding times are the total binding times of an antigen-binding molecule to an antigen in the reaction system. To describe more specifically, provided that a process where an antigen-binding molecule bound to an antigen is taken up into a cell, dissociated from the antigen in endosome and returns outside the cell is regarded as one cycle, the number of cycle repeats until the antigen-binding molecule is decomposed and cleared is referred to the binding times. The antigen-binding molecule of the present invention having a binding activity to a sugar chain receptor in a neutral pH range binds to a sugar chain receptor and is then taken up into the cell expressing the sugar chain receptor by endocytosis. The antigen-binding molecule of the present invention is released from the sugar chain receptor in an acidic range and binds to FcRn (particularly human FcRn) in the acidic range and then returns outside the cell for recycle use. The antigen-binding molecule of the present invention, which is dissociated from an antigen in the acidic range and returns outside a cell for recycle use, can bind again to an antigen. Therefore, whether or not the number of cycles increased can be determined based on whether "uptake into a cell is promoted" or not, or whether "pharmacokinetics is improved" (described later) or not.

In the present invention, as the method "for increasing the number of binding domains to a sugar chain receptor", a plurality of methods described as a method for producing an antigen-binding molecule in the specification may be appropriately used alone or in combination.

Binding of the antigen-binding molecule of the present invention to an antigen can be performed also ex-vivo

and whether or not the number of antigens binding to a single antigen-binding molecule was increased can be confirmed based on whether or not the clearance speed of an antigen present in plasma was promoted (whether or not the speed has been accelerated compared to the case where an antigen-binding molecule is not administered), which is determined by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body, or (2) a method of administering an antigen-binding molecule to a living body; or confirmed by determining whether or not the antigen concentration in plasma has been lowered by the *ex-vivo* method or by administering an antigen-binding molecule. As to the method (1), a method comprising taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body, can be also used.

Whether the number of antigens to which a single antigen-binding molecule binds has been increased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell, which is to be brought into contact with the antigen-binding molecule of the present invention, expresses a sugar chain receptor or not, can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) not only outside a living body but also inside a living body, the expression: bringing the antigen-binding molecule into contact with the cell expressing a sugar chain receptor in the present invention, includes administering the antigen-binding molecule to a living body. The contact time is for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

The present invention provides a method for increasing the number of binding domains, which are contained in an antigen-binding molecule (containing an antigen-binding domain, an FcRn binding domain and two or more binding domains to a sugar chain receptor) to the sugar chain receptor; at the same time, for increasing the number of antigens to which a single antigen-binding molecule (containing an antigen-binding domain whose binding activity to an antigen changes depending upon the ion-concentration condition) can bind, *in-vivo* or *ex-vivo.* The present invention further provides a method for increasing the number of binding domains, which are contained in an antigen-binding molecule (containing an antigen-binding domain, an FcRn binding domain and two or more binding domains to a sugar chain receptor) to the sugar chain receptor; at the same time, for increasing the number of antigens, to which a single antigen-binding molecule can bind, *in-vivo* or *ex-vivo,* comprising bringing an antigen-binding molecule (the number of antigens to which a single antigen-binding molecule can bind has been increased) containing an antigen-binding domain (whose binding activity to the antigen has been changed depending upon the ion-concentration condition) into contact with a cell expressing a sugar chain receptor (binding to a sugar chain receptor-binding domain contained in the antigen-binding molecule). The present invention further provides a method for increasing the number of binding domains to a sugar chain receptor of an antigen-binding molecule (containing an antigen-binding domain, an FcRn binding domain and two or more binding domains to a sugar chain receptor); at the same time, for increasing the number of antigens, to which a single antigen-binding molecule can bind (in which at least one amino acid of the antigen-binding domain is an amino acid, which changes the binding activity to an antigen depending upon the calcium-ion concentration condition or pH condition) *in-vivo* or *ex-vivo*.

In the present invention, as the method for "changing the binding activity of an antigen-binding domain to an antigen depending upon the ion-concentration condition", a plurality of methods described as a method for producing an antigen-binding molecule in the specification may be appropriately used alone or in combination.

Method for enhancing an ability of an antigen-binding molecule to clear an antigen, *in-vivo* or *ex-vivo*

The present invention provides a method for enhancing an ability of an antigen-binding molecule to clear an antigen, *in-vivo* or *ex-vivo*, comprising increasing the number of binding domains to the sugar chain receptor in an antigen-binding molecule containing an antigen-binding domain, an FcRn binding domain and two or more binding domains to the sugar chain receptor.

The expression: the antigen present outside a cell, refers to an antigen present outside the cell expressing a sugar chain receptor. When the cell expressing a sugar chain receptor is present inside a living body, an antigen present outside the cells of a body fluid such as blood, plasma, serum, urine, lymph fluid, saliva and tear can be mentioned as an example (of the antigen present outside a cell). However, as long as the antigen-binding molecule (of the present invention administered to a living body) can bind to an antigen outside the cell expressing a sugar chain receptor, the antigen present outside a cell is not limited to the antigen present in these body fluids. Particularly preferable example of the antigen present outside a cell, an antigen present in plasma. When the cell expressing a sugar chain receptor is present outside a living body, not only a body fluid taken out from a living body, such as blood, plasma, serum, urine, lymph fluid, saliva and tear, but also an antigen present in a culture fluid of the cell can be mentioned as an example of the antigen present outside a cell.

The antigen-binding molecule of the present invention having a binding activity to a sugar chain receptor in a neutral pH range, binds to the sugar chain receptor and then is taken up into the cell expressing the sugar chain receptor by endocytosis. In an acidic range, the antigen-binding molecule (of the present invention) is released from the sugar chain receptor, binds to FcRn (particularly human FcRn) in the acidic range. In this manner, the antigen-binding molecule of the present invention is returned again outside the cell for recycle use. The antigen-binding molecule of the present invention (from which an antigen dissociates in the acidic range) returned outside the cell for recycle use can bind again to an antigen. In this case, the antigen-binding molecule of the present invention dissociating from an antigen in the acidic range is recycled outside the cell can bind again to the antigen. In this case, the antigen dissociated is decomposed in a lysosome within a cell. Therefore, every time the antigen-binding molecule (of the present invention) is recycled, the antigen (which is present outside the cell expressing a sugar chain receptor and bound to the antigen-binding molecule) is decomposed in a lysosome within the cell (expressing a sugar chain receptor). As a result of such decomposition of the antigen, the number of antigens present outside the cell presumably decreases. Whether the number of antigens present outside a cell decreased or not can be evaluated by measuring the amount of antigen present outside a body fluid (such as blood, plasma, serum, urine, lymph fluid, saliva and tear) if a cell (expressing a sugar chain receptor) is present within a living body, and evaluated by measuring the amount of antigen present in not only a body fluid taken out of a living body, such as blood, plasma, serum, urine, lymph fluid, saliva and tear but also a culture fluid of the cell, if the cell (expressing a sugar chain receptor) is present outside a living body.

In the present invention, as the method "for increasing the number of antigens-binding domains to a sugar chain receptor", a plurality of methods described as a method for producing an antigen-binding molecule in the specification may be appropriately used alone or in combination.

Whether or not an ability of an antigen-binding molecule to clear an antigen *in-vivo* or *ex-vivo* was enhanced by a method (provided by the present invention) comprising increasing the number of binding domains to the sugar chain receptor in an antigen-binding molecule (containing an antigen-binding domain, an FcRn binding domain and two or more binding domains to the sugar chain receptor), can be confirmed by determining whether the antigen outside the cell was cleared or not by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body, or (2) a method of administering an antigen-binding molecule to a living body. As to the method (1), a method including taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used. Accordingly, whether or not the number of antigens present outside a cell decreased can be confirmed also by determining whether or not the amount of antigen present in plasma has been decreased compared to the case where the antigen-binding molecule is not administered or by determining whether or not the antigen concentration in plasma has been reduced by the *ex-vivo* method or administration of an antigen-binding molecule.

Whether the amount of antigen in plasma has decreased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

In the present invention, as the cell expressing a sugar chain receptor (binding to a sugar chain receptor-binding

domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell, which is to be brought into contact with the antigen-binding molecule of the present invention, expresses a sugar chain receptor or not can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) not only outside a living body but also inside a living body, the expression: bringing the antigen-binding molecule into contact with the cell expressing a sugar chain receptor in the present invention, includes administering the antigen-binding molecule to a living body. The contact time is for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

The present invention provides a method for increasing the number of binding domains (to a sugar chain receptor contained in an antigen-binding molecule containing an antigen-binding domain, an FcRn binding domain and two or more binding domains to a sugar chain receptor); at the same time for enhancing an ability of an antigen-binding molecule (which contains an antigen-binding domain whose binding ability to the antigen changes depending upon ion-concentration condition) to clear an antigen, *in-vivo* or *ex-vivo*. The present invention further provides a method for increasing the number of binding domains to a sugar chain receptor (contained in an antigen-binding molecule containing an antigen-binding domain, an FcRn binding domain and two or more binding domains to a sugar chain receptor); at the same time, for enhancing an ability of an antigen-binding molecule (which contains an antigen-binding domain whose binding ability to the antigen has been changed depending upon ion-concentration condition) to clear an antigen, *in-vivo* or *ex-vivo*. The present invention further provides a method for increasing the number of binding domains to a sugar chain receptor (contained in an antigen-binding molecule containing an antigen-binding domain, an FcRn binding domain and two or more binding domains to a sugar chain receptor); at the same time, for enhancing an ability of an antigen-binding molecule to clear an antigen, *in-vivo* or *ex-vivo* (in which at least one amino acid of the antigen-binding domain is an amino acid, which changes the binding activity of the antigen-binding domain to an antigen depending upon the calcium-ion concentration condition or pH condition).

In the present invention, as the method for "changing the binding activity of the antigen-binding domain to the antigen depending upon ion-concentration condition ", a plurality of methods described as a method for producing an antigen-binding molecule in the specification may be appropriately used alone or in combination.

<u>Method for improving the pharmacokinetics of an antigen-binding molecule</u>

The present invention provides a method of improving the pharmacokinetics of an antigen-binding molecule, comprising increasing the number of binding domains to a sugar chain receptor in an antigen-binding molecule containing an antigen-binding domain, an FcRn binding domain and two or more binding domains to a sugar chain receptor.

In the present invention, "improvement of pharmacokinetics", "enhancement of pharmacokinetics" or "excellent pharmacokinetics" can be rephrased by "improvement of retentivity in plasma (in blood)", " enhancement of retentivity in plasma (in blood)", "excellent retentivity in plasma (in blood)", "extending retentivity in plasma (in blood)". The meanings of these expressions are all the same.

In the present invention, the concept: "pharmacokinetics is improved" also includes extending the time period during which an antigen binds to an antigen-binding molecule after administration of the antigen-binding molecule. Whether the time period, during which antigen binds to an antigen-binding molecule after administration of the antigen-binding molecule, was extended or not can be determined by measuring concentration of an antigen-binding molecule-unbound antigen (not binding to an antigen-binding molecule) in plasma, more specifically, based on the time until the concentration of the antigen-binding molecule-unbound antigen in plasma increases, or the ratio of the concentration of the antigen-binding molecule-unbound antigen relative to total antigen concentration increases.

The concentration of the antigen-binding molecule-unbound antigen in plasma, or, the ratio of the concentration of an antigen-binding molecule-unbound antigen relative to total antigen concentration can be determined by a method known to those skilled in the art. For example, a measurement method employed in Pharm Res. (2006) 23 (1) 95-103. can be used. When an antigen plays some role *in-vivo*, whether or not the antigen has bound to an antigen-binding molecule (antagonist molecule) which neutralizes the function of the antigen can be evaluated by determining whether or not the function of the antigen has been neutralized. Whether or not the function of the antigen has been neutralized

can be evaluated by measuring any one of *in-vivo* markers which reflects the function of the antigen. Whether or not an antigen has bound to an antigen-binding molecule (agonist molecule), which activates the function of the antigen, can be evaluated by measuring any one of *in-vivo* markers which reflects the function of the antigen.

Measurement, such as measurement of the concentration of an unbound antigen in plasma, measurement of the ratio of the amount of unbound antigen relative to the total antigen amount and measurement of *in-vivo* marker(s), is not particularly limited and preferably performed in a predetermined time after an antigen-binding molecule is administered. In the present invention, expression: a predetermined time after an antigen-binding molecule is administered, is not particularly limited and can be appropriately determined by those skilled in the art in consideration of the properties of the antigen-binding molecule to be administered. For example, one day, 3 days, 7 days, 14 days and 28 days after an antigen-binding molecule is administered are mentioned.

In the present invention, it is preferable that pharmacokinetics in a human is improved. If it is difficult to measure retentivity in plasma in a human, retentivity in plasma in a human can be estimated based on retentivity in plasma of a mouse (for example, normal mouse, human antigen expression transgenic mouse, human FcRn expressing transgenic mouse 32 lineage or 276 lineage (Jackson Laboratories) (Methods Mol Biol. (2010) 602, 93-104) etc., transgenic mouse, etc.) and a monkey (for example, crab-eating monkey).

The present invention provides a method for increasing the number of binding domains to a sugar chain receptor contained in an antigen-binding molecule (containing an antigen-binding domain, an FcRn binding domain and two or more binding domains to a sugar chain receptor); at the same time, for improving the pharmacokinetics of an antigen-binding molecule containing an antigen-binding domain, which changes the binding activity to an antigen depending upon the ion-concentration condition. The present invention further provides a method for increasing the number of binding domains to a sugar chain receptor in an antigen-binding molecule (containing an antigen-binding domain, an FcRn binding domain and two or more binding domains to a sugar chain receptor); at the same time, for improving the pharmacokinetics of an antigen-binding molecule containing an antigen-binding domain, which has changed the binding activity to an antigen depending upon the ion-concentration condition. The present invention further provides a method for increasing the number of binding domains to a sugar chain receptor contained in an antigen-binding molecule (containing an antigen-binding domain, an FcRn binding domain and two or more binding domains to a sugar chain receptor); at the same time, for improving the pharmacokinetics of an antigen-binding molecule containing an antigen-binding domain, whose at least one amino acid is an amino acid, which has changed the binding activity to an antigen depending upon the calcium-ion concentration condition or pH condition.

In the present invention, as a method for "changing the binding activity of an antigen-binding domain to an antigen depending upon the ion-concentration condition" a plurality of methods described as a method for producing an antigen-binding molecule in the specification may be appropriately used alone or in combination.

<u>Method for promoting dissociation of antigen from antigen-binding molecule</u>

The present invention provides a method for promoting dissociation of an antigen (bound to an antigen-binding molecule outside a cell) from the antigen-binding molecule, comprising increasing the number of binding domains to a sugar chain receptor in an antigen-binding molecule containing an antigen-binding domain, an FcRn binding domain and two or more binding domains to a sugar chain receptor.

In the present invention, as a site at which an antigen is dissociated from an antigen-binding molecule, any site may be used as long as the site is within a cell and preferably, a site within early-stage endosome. In the present invention, the expression: "dissociation of an antigen bound to an antigen-binding molecule outside a cell from the antigen-binding molecule within the cell" refers to dissociation of an antigen from an antigen-binding molecule as a result that the antigen (bound to an antigen-binding molecule outside a cell) is brought into contact with a cell expressing a sugar chain receptor (binding to the sugar chain receptor-binding domain contained in the antigen-binding molecule) *in-vivo* or *ex-vivo.* It is not necessary to dissociate all antigens taken up into a cell from an antigen-binding molecule within the cell. It is sufficient if the rate of antigens from which antigen-binding molecules are dissociated (or antigen-binding molecules from which an antigen is dissociated) in the cell is high.

The antigen-binding molecule of the present invention having a binding activity to a sugar chain receptor in a neutral pH range, binds to the sugar chain receptor and then is taken up into the cell expressing the sugar chain receptor by endocytosis. In an acidic range, the antigen-binding molecule of the present invention is released from the sugar chain receptor, binds to FcRn (particularly human FcRn) in the acidic range and is recycled outside the cell. The antigen-binding molecule of the present invention (from which an antigen dissociates in the acidic range) recycled outside the cell can bind again to an antigen. In this case, the antigen dissociated is decomposed in a lysosome within a cell. Therefore, every time the antigen-binding molecule of the present invention is recycled, the antigen present outside the cell expressing a sugar chain receptor (which bound to the antigen-binding molecule of the present invention) is decomposed in a lysosome within the cell (expressing a sugar chain receptor). As a result of the decomposition of the antigen, the number of antigens present outside a cell presumably decreases. Whether the number of antigens decreased or

not can be evaluated by measuring the amount of antigen present outside cells of a body fluid such as blood, plasma, serum, urine, lymph fluid, saliva and tear when a cell expressing a sugar chain receptor is present within a living body; and evaluated by measuring the amount of antigen present in not only a body fluid taken out of a living body, such as blood, plasma, serum, urine, lymph fluid, saliva and tear but also a culture fluid of the cell, if the cell (expressing a sugar chain receptor) is present outside a living body.

Whether dissociation of an antigen (bound to an antigen-binding molecule outside a cell) from the antigen-binding molecule has been promoted or not by a method of increasing the number of binding domains to a sugar chain receptor in an antigen-binding molecule (containing an antigen-binding domain, an FcRn binding domain and two or more binding domains to a sugar chain receptor) can be confirmed by determining whether the dissociation of an antigen (bound to an antigen-binding molecule outside the cell) from the antigen-binding molecule in the cell has been promoted or not, for example, by (1) a method (called an *ex-vivo* method), comprising taking out plasma containing an antigen-binding molecule and an antigen (binding to the antigen-binding molecule) once out of a living body, bringing the plasma into contact with a cell expressing a sugar chain receptor for a predetermined period, taking the plasma outside the cell (called re-secretion or recycling) for recycle use, and returning the plasma containing a free antigen-binding molecule (not bound to the antigen) into the living body; or (2) a method of administering an antigen-binding molecule to a living body. As to the method (1), a method comprising taking out the plasma containing an antigen (binding to the antigen-binding molecule) once out of a living body, bringing the plasma into contact with an antigen-binding molecule and a cell expressing a sugar chain receptor for a predetermined time, and returning the plasma into the living body can be also used. Accordingly, whether or not the number of antigens present outside a cell decreased can be confirmed also by determining whether or not the amount of antigen present in plasma has decreased compared to the case where the antigen-binding molecule is not administered or by determining whether or not the concentration of the antigen in plasma has been reduced by the *ex-vivo* method or administration of an antigen-binding molecule.

Whether the amount of antigen in plasma decreased or not can be confirmed by determining whether or not the clearance speed of an antigen in the plasma (which is determined by the above (1) and (2) methods) has been promoted compared to the clearance speed of an antigen in the plasma, which is determined by the same method as in the above except that a human natural IgG (particularly human natural IgG1) is used in place of the antigen-binding molecule.

In the present invention, as the cell expressing a sugar chain receptor (binding to a sugar chain receptor-binding domain and contained in an antigen-binding molecule), any cell can be used as long as a desired sugar chain receptor is expressed in the cell. The cell is not limited to a specific cell. To specify the cell expressing a desired sugar chain receptor, database known in the art, such as Human Protein Atlas (http://www.proteinatlas.org/), can be used. Furthermore, whether the cell, which is to be brought into contact with the antigen-binding molecule of the present invention expresses a sugar chain receptor or not can be confirmed by a technique for determining expression of a gene encoding a desired sugar chain receptor and an immunological technique using an antibody binding to a desired sugar chain receptor. These techniques are known in the art. Since the cell expressing a sugar chain receptor can be brought into contact with an antigen-binding molecule and an antigen (binding to the antigen-binding molecule) not only outside a living body but also inside a living body, the expression: bringing the antigen-binding molecule into contact with the cell expressing a sugar chain receptor in the present invention, includes administering the antigen-binding molecule to a living body. The contact time is for example, one minute to several weeks, 30 minutes to one week, one hour to 3 days, and 2 hours to one day; in other words, the time required for an antigen-binding molecule or an antigen (binding to the antigen-binding molecule) to be taken up into a cell by endocytosis is appropriately employed.

For example, as the cell expressing an asialoglycoprotein receptor as a sugar chain receptor, a liver cell can be used. Furthermore, as the cell expressing a mannose receptor as a sugar chain receptor, a wide variety of cells including blood cells can be used.

The present invention provides a method for increasing the number of binding domains to a sugar chain receptor contained in an antigen-binding molecule (containing an antigen-binding domain, an FcRn binding domain and two or more binding domains to a sugar chain receptor); at the same time, for promoting dissociation of an antigen (bound to an antigen-binding molecule outside a cell) from the antigen-binding molecule within the cell containing an antigen-binding domain, which changes the binding activity to an antigen depending upon the ion-concentration condition. The present invention further provides a method for increasing the number of binding domains to a sugar chain receptor contained in an antigen-binding molecule (containing an antigen-binding domain, an FcRn binding domain and two or more binding domains to a sugar chain receptor); at the same time, for promoting dissociation of an antigen (bound to an antigen-binding molecule outside a cell) from the antigen-binding molecule within the cell containing an antigen-binding domain, which has changed the binding activity to an antigen depending upon the ion-concentration condition. The present invention further provides a method for increasing the number of binding domains to a sugar chain receptor contained in an antigen-binding molecule (containing an antigen-binding domain, an FcRn binding domain and two or more binding domains to a sugar chain receptor); at the same time, for promoting dissociation of an antigen (bound to

an antigen-binding molecule outside a cell) from the antigen-binding molecule (in which at least one amino acid of the antigen-binding domain is an amino acid, which changes the binding activity to an antigen depending upon the calcium-ion concentration condition or pH condition).

In the present invention, as a method for "changing the binding activity of an antigen-binding domain to an antigen depending upon the ion-concentration condition" a plurality of methods described as a method for producing an antigen-binding molecule in the specification may be appropriately used alone or in combination.

In the specification, an aspect expressed by "... comprising" includes an aspect "essentially consisting of" and an aspect expressed by "... consisting of".

The contents of all patent literatures and reference documents explicitly cited in the specification are incorporated herein by reference.

Examples

The present invention will be described more specifically by way of Examples; however, the present invention is not limited to these Examples.

[Example 1]

Improvement of clearance acceleration effect of antigen by pH-dependent antigen-binding antibody using a sugar chain receptorWhen an existing neutralizing antibody against a soluble antigen is administered, an antigen binds to the antibody, with the result that retentivity in plasma is presumably enhanced. Antibody generally has a long half-life period (1 week to 3 weeks); whereas, an antigen generally has a short half-life period (1 day or less). Because of this, the antigen bounds to an antibody in plasma acquires a significantly long half-life period compared to an antigen present alone. Consequently, if an existing neutralizing antibody is administered, the antigen concentration in plasma increases. Such a phenomenon has been reported in various documents dealt with neutralizing antibodies targeting a soluble antigen. Examples of the cases include IL-6 (J Immunotoxicol. 2005, 3, 131-9.), amyloid beta (mAbs, 2010, 2: 5, 1-13), MCP-1 (ARTHRITIS & RHEUMATISM 2006, 54, 2387-92), hepcidin (AAPS J. 2010, 4, 646-57.), sIL-6 receptor (Blood. 2008 Nov 15; 112 (10): 3959-64.). It is reported that a total antigen concentration in plasma increases by approximately 10 fold to 1000 fold (the rate of increase varies depending upon the antigen) from a base line by administration of an existing neutralization activity. The term "total antigen concentration in plasma" used herein refers to the total concentration of antigens present in plasma, more specifically, a total of an antibody-binding antigen concentration and an antibody-unbound antigen concentration. In the case of such an antibody drug targeting a soluble antigen, an increase of the total antigen concentration in plasma is unfavorable. This is because in order to neutralize a soluble antigen, the antibody concentration in plasma at least higher than the total antigen concentration in plasma is required. More specifically, a 10-fold to 1000-fold increase of the total antigen concentration in plasma means that the antibody concentration in plasma (more specifically antibody dose) for neutralizing the antigen must be 10 fold to 1000 fold as high as that of the case where the total antigen concentration in plasma does not increase. In contrast, if the total antigen concentration in plasma can be reduced by 10 fold to 1000 fold compared to that of an existing neutralizing antibody, the dose of the antibody can be reduced by the same fold. As described above, an antibody capable of clearing soluble antigens from plasma, thereby reducing the total antigen concentration in plasma is extremely useful beyond an existing neutralizing antibody.

Regarding pH-Dependent human IL-6 receptor-binding antibody

H54/L28-IgG1, which is constituted of H54 (SEQ ID NO: 26) and L28 (SEQ ID NO: 27) (described in WO 2009/125825) is a humanized anti-IL-6 receptor antibody. GL1-IgG1 (Fv4-IgG1 described in WO 2009/125825), which is constituted of VH3-IgG1 (SEQ ID NO: 28) and VL3-CK (SEQ ID NO: 29), is a humanized anti-IL-6 receptor antibody prepared by adding a property of binding to a soluble human IL-6 receptor in a pH-dependent manner (binds at pH 7.4 and dissociates at pH 5.8) compared to H54/L28-IgG1. In an *in-vivo* test using mice (described in WO 2009/125825), it was demonstrated that clearance of a soluble human IL-6 receptor can be significantly accelerated in a group in which a mixture of GL1-IgG1 and a soluble human IL-6 receptor (as an antigen) was administered, compared to a group in which a mixture of H54/L28-IgG1 and a soluble human IL-6 receptor (as an antigen) was administered.

A soluble human IL-6 receptor bound to an antibody (which is capable of binding to a conventional soluble human IL-6 receptor) is recycled together with the antibody into plasma by means of FcRn. In contrast, the antibody binding to a soluble human IL-6 receptor in a pH-dependent manner dissociates the soluble human IL-6 receptor bound to the antibody under the acidic condition within endosome. The soluble human IL-6 receptor dissociated is decomposed by lysosome. Thus, clearance of the soluble human IL-6 receptor can be significantly accelerated. Furthermore, the antibody binding to the soluble human IL-6 receptor in a pH-dependent manner is recycled by FcRn into plasma. The

antibody recycled can bind to a soluble human IL-6 receptor again. By repeating this process, a single antibody molecule can repeatedly bind to a soluble human IL-6 receptor (Figure 1).

Regarding Binding of IgG antibody to FcRn

An IgG antibody acquires long retentivity in plasma by binding to an FcRn. Binding of IgG to FcRn is observed only under an acidic condition (pH 6.0) and rarely observed under a neutral condition (pH 7.4). An IgG antibody is nonspecifically taken up into a cell, binds to FcRn under an acidic condition within an endosome, returns to the cell surface and dissociated from FcRn under a neutral condition of plasma. If a mutation is introduced into the Fc region of IgG to abolish binding to FcRn under an acidic condition, IgG cannot be recycled from the endosome to plasma, with the result that retentivity of the antibody in plasma is significantly reduced. A method for improving the retentivity of an IgG antibody in plasma, a method of improving binding to FcRn under an acidic condition has been reported. By improving binding to FcRn under an acidic condition by introducing an amino acid substation into the Fc region of an IgG antibody, a recycle rate from an endosome to plasma increases, with the result that the retentivity in plasma increases.

Improvement of antigen clearance acceleration effect by pH-dependent antigen-binding antibody using a sugar chain receptor

An antibody binding to an antigen in a pH-dependent manner is extremely useful since it can accelerate clearance of soluble antigens and lower a total antigen concentration in plasma and a single antibody molecule has an effect of repeatedly binding to a soluble antigen multiple times. The antigen clearance acceleration effect is further improved by a method comprising adding a sugar chain to an antibody capable of binding to an antigen in a pH-dependent manner (the sugar chain added to the antibody associates with a sugar chain receptor only observed under a neutral condition (pH 7.4) and dissociates under an acidic condition (pH 6.0)); allowing the antibody to bind to a soluble antigen and to take up into a cell depending upon the sugar-chain receptor, dissociating the antibody from the sugar-chain receptor in an endosome, at the same time, from the soluble antigen, binding the antibody to FcRn, and recycling the antibody into plasma again. This method was evaluated (Figure 2).
For the above method to effectively work, it is considered necessary that the sugar chain binds to the sugar chain receptor and the antigen binds to the antibody in a pH-dependent manner; that the antigen-binding molecule binds to the sugar chain receptor as well as the antigen under a neutral condition, with the result that an antibody-antigen complex is taken up into a cell via the sugar chain receptor; and that the antigen-binding molecule needs to dissociate from the sugar chain receptor as well as the antigen under an acidic condition of an endosome.
As the antigen and antibody having such a feature, an antibody against an IL-6 receptor, Fv4-IgG1 (described in WO 2009/125825) is known. This is referred to as GL1-IgG1 in the present invention.
Furthermore, as the sugar chain and sugar chain receptor having such a feature, galactose and an asialoglycoprotein receptor, and mannose and a mannose receptor are conceivable. It is known that the asialoglycoprotein receptor (a sugar chain receptor), which interacts with galactose in a pH-dependent manner, has high binding activity in the neutral pH range but low binding activity in an acidic pH range (J. Biol. Chem. (1989) 274 (50), 35400-35406). Similarly, it is known that the mannose receptor (a sugar chain receptor), which interacts with mannose in a pH-dependent manner, has high binding activity in a neutral pH range but low binding activity in an acidic pH range (J. Biol. Chem. (1994) 269 (45), 28405-28413).

[Example 2]

Preparation of anti-human IL-6 receptor antibody being capable of binding in a pH-dependent manner and having a galactose-ended complex linked sugar chain introduced in variable region Preparation of pH-dependent human IL-6 receptor binding antibody having an N-linked glycosylation sequence
To GL1-IgG1 consisting of VH3-IgG1 (SEQ ID NO: 28) and VL3-CK (SEQ ID NO: 29), a mutation was introduced so as to contain an N-linked glycosylation sequence Asn-X-Ser/Thr, where X represents an amino acid except Pro, Ser/Thr means Ser or Thr. More specifically, VH3-M111 (SEQ ID NO: 31) having a heavy-chain constant region M111 (SEQ ID NO: 30) was prepared by substituting the 297th (EU numbering) Asn of an IgG1 heavy-chain constant region with Ala; and H01-M111 (SEQ ID NO: 32) was prepared by substituting the 75th (Kabat numbering) Lys of a VH3-M111 heavy-chain variable region with Asn. Furthermore, L02-CK (SEQ ID NO: 33) was prepared by substituting the 18th (kabat numbering) Ser of a VL3-CK light-chain variable region with Asn; L03-CK (SEQ ID NO: 34) was prepared by substituting 20th Thr with Asn; L04-CK (SEQ ID NO: 35) was prepared by substituting 24th Gln with Asn; and L06-CK (SEQ ID NO: 36) was prepared by substituting 20th Thr and 24th Gln each with Asn. An amino acid substituent was introduced by a method known in the art and described in Reference Example 1
H54/L28-IgG1 consisting of H54 (SEQ ID NO: 26) and L28 (SEQ ID NO: 27); GL1-IgG1 consisting of VH3-IgG1 (SEQ ID NO: 28) and VL3-CK (SEQ ID NO: 29); GL1-M111 consisting of VH3-M111 (SEQ ID NO: 31) and VL3-CK; GL2-M111 consisting of VH3-M111 and L02-CK (SEQ ID NO: 33); GL3-M111 consisting of VH3-M111 and L03-CK (SEQ ID NO: 34), GL4-M111 consisting of VH3-M111 and L04-CK (SEQ ID NO: 35), GL5-M111 consisting of VH3-M111 and L06-CK (SEQ ID NO: 36); GL6-M111 consisting of H01-M111 (SEQ ID NO: 32) and VL3-CK; GL7-M111 consisting of H01-M111 and L02-CK; GL8-M111 consisting of H01-M111 and L03-CK; GL9-M111 consisting of H01-M111 and L04-CK; GL10-M111 consisting of H01-M111 and L06-CK; and GL5-IgG1 consisting of VH3-IgG1 and L06-CK were expressed and purified in accordance with methods known to those skilled in the art described in Reference Example 2.

In human natural IgG1, an N-linked sugar chain is added to the 297th Asn residue (EU numbering). In this Example, to easily evaluate introduction of an N-linked sugar chain into a variable region, a modified constant region named M111, which was prepared by substituting the 297th Asn with Ala, was used.

Evaluation of Glycosylation by Reducing SDS-PAGE

Glycosylation of an antibody, to which an N-linked glycosylation sequence was introduced, was evaluated by reducing SDS-PAGE. To each of GL1-M111 to GL10-M111 (6 μg aliquots), Tris-Glycine SDS Sample Buffer (2x) (TEFCO) containing a 5% 2-mercaptoethanol (Wako) was added. Thereafter, the mixture was incubated at 70°C for 5 minutes, a sample to be subjected to electrophoresis was prepared. After electrophoresis was performed by a 12% SDS-PAGE mini 15 well (TEFCO) with Precision plus blue standard (Bio-Rad) used as a molecular-weight marker and CBB staining was performed by CBB Stain One (Nacalai tesque). The obtained electrophoretic pattern is shown in Figure 3.

In GL1-M111 in which a sugar chain is added to neither a heavy chain nor a light chain, a heavy chain emerged at the lower end of the 50 kDa molecular-weight marker, a light chain emerged at the upper end of the 25 kDa molecular-weight marker. In GL2-M111 to GL4-M111 in which a single glycosylation site was introduced in a light chain, a band corresponding to the light chain shifted toward high molecular weight side compared to GL1-M111. In GL5-M111 in which two glycosylation sites were introduced in a light chain, a band corresponding to the light chain shifted toward further higher molecular weight side compared to GL2-M111 to GL4-M111. In GL6-M111 in which a single glycosylation site was introduced in a heavy chain, a band corresponding to a heavy chain shifted toward a high molecular weight side compared to GL1-M111.

In GL7-M111 to GL9-M111 prepared by introducing two glycosylation sites in total in a single site of a heavy chain and a sites of a light chain, respectively, band shift of the heavy chain and light chain toward a high molecular weight side was observed compared to GL1-M111. GL10-M111 prepared by introducing three glycosylation sites in total in a single site of a heavy chain and two sites of a light chain, a band shift of a heavy chain towards a high molecular weight side than GL1-M111 and a band shift of the light chain towards a high molecular weight side than GL7-M111 to GL9-M111 were observed.

From these results, it was demonstrated that a plurality of glycosylation sites can be simultaneously introduced to both heavy chain and light chain.

Evaluation of N-linked sugar chain by anion exchange chromatography

Before and after removal of sialic acid by neuraminidase, anion exchange chromatography was performed. Based on a shape change between the chromatograms, whether or not the N-linked sugar chain that bound to is a high-mannose sugar chain and whether or not sialic acid binds to a terminal of a complex sugar chain were evaluated. To samples of GL1 to GL10 prepared with a 50 mmol/L Acetate pH 5.0, neuraminidase (Roche) was added. The mixture was allowed to stand still at 37°C overnight and subjected to anion exchange chromatography performed by TSK-gel DEAE-NPR (Tosoh) in accordance with a two-liquid gradient method using 10 mmol/L Tris-HCl, pH 7.5 as mobile phase A and 10 mmo/L Tris-HCl/150 mmol/L NaCl, pH 7.5 as mobile phase B. Chromatograms are shown in Figure 4.

GL1-M111 (no sugar chain was added) showed a single peak before the neuraminidase treatment and no change was observed in the chromatogram after the neuraminidase treatment. In contrast, GL2-M111 to GL10-M111 (a sugar chain was added), heterogeneous peaks were observed before the neuraminidase treatment but the number of detection peaks decreased after the neuraminidase treatment. This means that sialic acid was added to a sugar chain terminal of each of GL2-M111 to GL10-M111, more specifically means that N-linked sugar chain added to GL2-M111 to GL10-M111 is not high-mannose sugar chain but a complex sugar chain having a plurality of sialic acids bound thereto. From this, it was confirmed that a complex sugar chain molecule with galactose exposed at a terminal can be prepared by a neuraminidase treatment.

Mass analysis of GL5-M111

To estimate that the N-linked sugar chain was added to GL5-M111 based on mass, mass analysis was performed. After a neuraminidase treatment, GL5-M11 was subjected to a reducing treatment with DTT (Wako) and then subjected to RP-LC/ESI-MS by use of Ultimate3000 (Dionex) and LTQ VELOS (Thermo scientific). Which one of bibranched N-linked sugar chains shown in Table 1 was added was estimated based on the mass and the obtained mass chromatograms are shown in Figure 5.

[Table 1]

| Abbreviation | Sugar-chain structure |
|---|---|
| G0 | 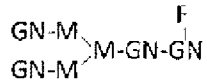 |
| G1 | 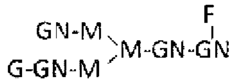 |
| G2 | 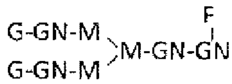 |
| G3 | 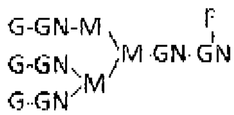 |
| G4 | 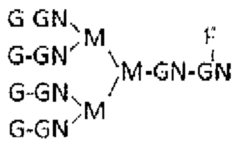 |
| F<br>G<br>M<br>GN | Fucose<br>Galactose<br>Mannose<br>N-Acetylglucosamine |

With an increase of the number of galactose exposed at an N-linked sugar chain terminal, a recognition efficiency via a sugar chain receptor conceivably increases. Then, addition of G2, G3 and G4 among the bibranched N-linked sugar chains was observed. As shown in Figure 5, cases where the two sugar chains added to a light chain consist of G2 and G2, G2 and G3, G3 and G3 and/or G2 and G4 were present.

<u>Inactivation treatment by neuraminidase</u>

Neuraminidase, which was used for removing sialic acid from the N-linked sugar chain terminal and still contained in GL5-M111, was inactivated. After the neuraminidase treatment, GL5-M111 (GL5-M111-SA (-)) was purified with Protein A and incubated at 60°C for 10 minutes (heat treated sample).

<u>Evaluation of neuraminidase activity by anion exchange chromatography</u>

To GL5-M111-SA (-) (SA (-) means that sialic acid is removed) and a heat treated sample, to which GL5-M111 (GL5-M111-SA (+)) (SA (+) means that sialic acid remains without removing) (not treated with neuraminidase) was added, were allowed to stand still at 37°C for 6 hours. These samples were analyzed by the same anion exchange chromatography method used for observation of an N-linked sugar chain. The obtained chromatograms are shown in Figure 6.

As shown in Figure 6, in a case where GL5-M111-SA (+) was added to GL5-M111-SA (-), convergence of a hetero peak derived from GL5-M111-SA (+) was observed. In contrast, even if GL5-M111-SA (+) was added to the heat treated sample, convergence of a peak was not observed. From this, it was demonstrated that neuraminidase remaining in a sample can be inactivated by repurification with Protein A followed by a heat treatment.

Preparation of sample by replacing constant region with IgG1

It was shown that a bibranched N-linked sugar chain can be added to a light-chain variable region due to a modification introduced in GL5. Then, GL1-IgG1 and GL5-IgG1 were prepared by replacing a constant region from M111 to IgG1.

Evaluation of glycosylation of IgG1 sample by reducing SDS-PAGE

GL1-IgG1 and GL5-IgG1 were analyzed by reducing SDS-PAGE in the same manner as in the sample having M111 used in combination. The obtained electrophoretic patterns are shown in Figure 7.

As shown in Figure 7, the light chain of GL5-IgG1 made a large shift toward a high molecular weight side compared to the light chain of GL1-IgG1. From this, it was considered that the sugar chain of a constant region has no effect upon glycosylation of the variable region and that an N-linked sugar chain is added to two sites of the light chain also in GL5-IgG1. Similarly, with respect to both GL5-M111 and GL5-IgG1, addition of an N-linked sugar chain to a light chain was observed by anion exchange chromatography and mass analysis (data is not shown).

Evaluation of binding to human IL-6 receptor (hIL6R)

GL1-IgG1-SA, GL1-M111-SA, GL2-M111-SA, GL3-M111-SA, GL4-M111-SA, GL5-M111-SA, GL6-M111-SA, GL7-M111-SA, GL8-M111-SA, GL9-M111-SA and GL10-M111-SA were prepared and treated with neuraminidase in accordance with the method mentioned above to remove a terminal sialic acid. In this manner, GL1-IgG1-SA (-), GL1-M111-SA (-), GL2-M111-SA (-), GL3-M111-SA (-), GL4-M111-SA (-), GL5-M111-SA (-), GL6-M111-SA (-), GL7-M111-SA (-), GL8-M111-SA (-), GL9-M111-SA (-) and GL10-M111-SA (-) having galactose exposed at a terminal, were prepared.

Kinetic analysis on the antigen-antibody reaction between each of GL1-IgG1-SA (-), GL1-M111-SA (-), GL2-M111-SA (-), GL3-M111-SA (-), GL4-M111-SA (-), GL5-M111-SA (-), GL6-M111-SA (-), GL7-M111-SA (-), GL8-M111-SA (-), GL9-M111-SA (-), GL10-M111-SA (-)and a human IL-6 receptor was performed by use of Biacore T100 (GE Healthcare). An appropriate amount of protein A (Invitrogen) was immobilized onto Sensor chip CM5 (GE Healthcare) by the amine coupling method and then a target antibody was allowed to capture. Subsequently, a human IL-6 receptor dilution solution and a running buffer (a blank) were injected at a flow rate of 20μ L/min for 3 minutes to allow the human IL-6 receptor to interact with the antibody captured on the sensor chip. Either one of two types of running buffers pH 7.4 and pH 6.0 containing 10 mmol/L ACES, 150 mmol/L NaCl, and 0.05% (w/v) Tween20 was used and each buffer was employed for dilution of IL-6R. Thereafter, the running buffer was fed at a flow rate of 20 μL/min for 5 minutes. After observation of dissociation of the IL-6 receptor, 10 mmol/L Glycine-HCl (pH 1.5) was injected at a flow rate of 30 μL/min for 30 seconds to regenerate the sensor chip. All samples were measured at 37°C. From the sensorgram obtained by measurement, an association constant, ka (1/Ms) and a dissociation constant, kd (1/s) as kinetics parameters were computationally obtained. Based on the obtained values, $K_D$ (M) of each antibody to a human IL-6 receptor was calculated. Individual parameters were calculated by use of Biacore T100 Evaluation Software (GE Healthcare). The resultant $K_D$ values of individual antibodies determined at pH 7.4 and pH 6.0 are shown in Table 2 below. It was found that significant difference was not observed in $K_D$ value between individual antibodies at each of the two conditions: pH 7.4 and pH 6.0, compared to GL1-IgG1-SA (-).

[Table 2]

| | $K_D$ at pH 6.0 | $K_D$ at pH 7.4 (M) |
|---|---|---|
| GL1-IgG1-SA (-) | 1.5E-07 | 1.8E-09 |
| GL1-M111-SA (-) | 1.5E-07 | 1.8E-09 |
| GL2-M111-SA (-) | 1.1E-07 | 1.6E-09 |
| GL3-M111-SA (-) | 8.0E-08 | 1.3E-09 |
| GL4-M111-SA (-) | 1.4E-07 | 1.7E-09 |
| GL5-M111-SA (-) | 99.9E-08 | 1.5E-09 |
| GL6-M111-SA (-) | 1.1E-07 | 2.6E-09 |
| GL7-M111-SA (-) | 1.5E-07 | 2.4E-09 |
| GL8-M111-SA (-) | 1.1E-07 | 2.0E-09 |

(continued)

| | $K_D$ at pH 6.0 | $K_D$ at pH 7.4 (M) |
|---|---|---|
| GL9-M111-SA (-) | 1.1E-07 | 2.3E-09 |
| GL10-M111-SA (-) | 1.3E-07 | 2.4E-09 |

From these results, GL5-M111, to which an N-linked glycosylation site is introduced at the 18th and 24th positions (Kabat numbering) of a light chain, was selected as a modified antibody, which has a complex sugar chain having a galactose at a terminal effectively introduced therein and retains a binding activity to a human IL-6 receptor and pH dependency.

Then, kinetic analysis of an antigen-antibody reaction between each of GL1-M111-SA (-) and GL5-M111-SA (-) and a human IL-6 receptor was performed by using Biacore T100 (GE Healthcare). In both cases, measurement was performed by using an antibody treated with sialidase as a sample. An appropriate amount of Anti-Human IgG (γ-chain specific) F(ab')2 fragment antibody produced in goat (Sigma) was immobilized onto Sensor chip CM5 (GE Healthcare) in accordance with the amine coupling method and then a target antibody was allowed to capture. Subsequently, a human IL-6 receptor dilution solution and a running buffer (a blank) were injected at a flow rate of 20 μL/min for 3 minutes to allow the human IL-6 receptor to interact with the antibody captured on the sensor chip. Either one of two types of running buffers pH 7.4 and pH 6.0 containing 10 mmol/L ACES, 150 mmol/L NaCl, and 0.05% (w/v) Tween20 was used, and each buffer was employed for dilution of IL-6R. Thereafter, a running buffer was fed at a flow rate 20 μL/min for 5 minutes. After dissociation of the IL-6 receptor was observed, 10 mmol/L Glycine-HCl (pH 1.5) was injected at a flow rate 30 μL/min for 5 seconds. This procedure was repeated five times to regenerate a sensor chip. All samples were measured at 37°C. From the sensorgram obtained by measurement, an association constant, ka (1/Ms) and a dissociation constant kd (1/s) as kinetics parameters were computationally obtained. From the values, $K_D$ (M) of each antibody to a human IL-6 receptor was calculated. Individual parameters were calculated by use of Biacore T100 Evaluation Software (GE Healthcare). The resultant $K_D$ values of individual antibodies determined at each of pH 7.4 and pH 6.0 are shown in Table 3 below. No significant difference was observed in $K_D$ value between the two antibodies at each of the two conditions: pH 7.4 and pH 6.0.

[Table 3]

| | $K_D$ at pH 6.0 (M) | $K_D$ at pH 7.4 (M) |
|---|---|---|
| GL1-M111-SA (-) | 6.6E-08 | 2.0E-09 |
| GL5-M111-SA (-) | 6.7E-08 | 1.9E-09 |

Next, kinetic analysis of antigen-antibody reactions between each of GL1-IgG1-SA (+), H54L28-IgG1-SA (+), GL1-IgG1-SA (-) and GL5-IgG1-SA (-) and a human IL-6 receptor was performed by using Biacore T100 (GE Healthcare). GL1-IgG1-SA (-) and GL5-IgG1-SA (-) were treated with sialidase and then used as measurement samples. An appropriate amount of Anti-Human IgG (γ-chain specific), F(ab')2 fragment antibody produced in goat (Sigma) was immobilized onto Sensor chip CM5 (GE Healthcare) in accordance with the amine coupling method and then a target antibody was allowed to capture. Subsequently, a human IL-6 receptor dilution solution and a running buffer (a blank) were injected at a flow rate of 20 μL/min for 3 minutes to allow the human IL-6 receptor to interact with the antibody captured on the sensor chip. Either one of two types of running buffers pH 7.4 and pH 6.0 containing 10 mmol/L ACES, 150 mmol/L NaCl, and 0.05% (w/v) Tween20 was used and each buffer was employed for dilution of IL-6R. Thereafter, a running buffer was fed at a flow rate 20 μL/min for 5 minutes. After dissociation of the IL-6 receptor was observed, 10 mmol/L Glycine-HCl (pH 1.5) was injected at a flow rate of 30μ L/min for 5 seconds. This procedure was repeated five times to regenerate the sensor chip. All samples were measured at 37°C. From the sensorgram obtained by measurement, an association constant, ka (1/Ms) and a dissociation constant kd (1/s) as kinetics parameters were computationally obtained. From the values, $K_D$ (M) of each antibody to a human IL-6 receptor was calculated. Individual parameters were calculated by use of Biacore T100 Evaluation Software (GE Healthcare). The $K_D$ values of individual antibodies determined at each of the two conditions: pH 7.4 and pH 6.0 are shown in Table 4 below.

[Table 4]

| | $K_D$ at pH 6.0 | $K_D$ at pH 7.4 |
|---|---|---|
| GL1-IgG1-SA (+) | 6.6E-08 | 2.1E-09 |

(continued)

| | $K_D$ at pH 6.0 | $K_D$ at pH 7.4 |
|---|---|---|
| H54/L28-IgG1-SA (+) | 3.4E-09 | 3.9E-09 |
| GL1-IgG1-SA (-) | 6.1E-08 | 2.2E-09 |
| GL5-IgG1-SA (-) | 5.2E-08 | 1.9E-09 |

[Example 3] Investigation of antigen clearance acceleration effect by pH-dependent (binding) anti-human IL-6 receptor antibody having a galactose-ended N-linked sugar chain introduced to a variable region, by using normal mouse

*In-vivo* test using normal mouse

To a normal mouse (C57BL/6J mouse, Charles River Japan), hsIL-6R (soluble human IL-6 receptor prepared in Reference Example 3) was solely administered or hsIL-6R and anti-human IL-6 receptor antibody were simultaneously administered. Thereafter, *in-vivo* kinetics of the hsIL-6R and the anti-human IL-6 receptor antibody were evaluated. A hsIL-6R solution (5 μg/mL) or a mixture solution of the hsIL-6R and the anti-human IL-6 receptor antibody (5 μg/mL and 0.1 mg/mL respectively) was administered once to caudal vein in a dose of 10 mL/kg. At this time, since anti-human IL-6 receptor antibody is excessively present compared to hsIL-6R, it is considered that almost all hsIL-6R molecules bind to the antibody. Blood was sampled 15 minutes, 7 hours, 1 day, 2 days, 3 days, 4 days, 7 days, 14 days, 21 days and 28 days after the administration. The blood sampled was immediately centrifuged at 4°C, 15,000 rpm for 15 minutes to obtain plasma. The plasma separated was stored in a freezer set at-20°C or less until measurement. As the anti-human IL-6 receptor antibody, GL1-M111, GL5-M111, H54/L28-IgG1, GL1-IgG1 and GL5-IgG1 mentioned above were used.

Measurement of anti-human IL-6 receptor antibody concentration in plasma by ELISA

The concentration of an anti-human IL-6 receptor antibody in mouse plasma was measured by ELISA. First, Anti-Human IgG (γ-chain specific) F(ab')2 Fragment of Antibody (SIGMA) was dispensed in Nunc-Immuno Plates, MaxiSoup (Nalge nunc International). The plates were allowed to stand still at 4°C overnight to prepare Anti-Human IgG immobilized plates. Samples having a plasma concentration of 0.8, 0.4, 0.2, 0.1, 0.05, 0.025 and 0.0125 μg/mL were prepared for forming a calibration curve sample and a mouse plasma measurement sample diluted 100 fold or more were prepared. To each (100 μL) of the calibration-curve samples and the plasma measurement sample, 20 ng/mL hsIL-6R (200 μL) was added. The mixture was allowed to stand still at room temperature for one hour, and thereafter dispensed to the Anti-Human IgG immobilized plates and allowed to stand still at room temperature further for one hour. Thereafter, Biotinylated Anti-human IL-6R Antibody (R&D) was reacted at room temperature for one hour, and then, Streptavidin-PolyHRP80 (Stereospecific Detection Technologies) was reached at room temperature for one hour. Then, a chromogenic reaction was performed using TMB One Component HRP Microwell Substrate (BioFX Laboratories) as a substrate and terminated with 1N-Sulfuric acid (Showa Chemical). Thereafter, the absorbance (of the plates) was measured by a micro plate reader at 450 nm. The concentration in the mouse plasma was computationally obtained from the absorbance shown in the calibration curve by use of analytic software SOFTmax PRO (Molecular Devices). A change of the antibody concentration in normal mouse plasma with time after intravenous administration, measured by this method, is shown in Figure 8 and Figure 10.

Measurement of hsIL-6R concentration in plasma by electrochemiluminescence assay

Concentration of hsIL-6R in mouse plasma was measured by electrochemiluminescence assay. Samples having a plasma hsIL-6R concentration of 2000, 1000, 500, 250, 125, 62.5 and 31.25 pg/mL were prepared for forming a calibration curve sample and a mouse plasma measurement sample diluted to 50 fold or more were prepared. Monoclonal Anti-human IL-6R Antibody (R&D) labeled with ruthenium by means of SULFO-TAG NHS Ester (Meso Scale Discovery), Biotinylated Anti-human IL-6R Antibody (R&D), and a WT-IgG1 solution were mixed and reacted at 37°C overnight. At this time, the final concentration of WT-IgG1 (an anti-human IL-6 receptor antibody) constituted of H (WT) (SEQ ID NO: 37) and L (WT) (SEQ ID NO: 38) was set at 333 μg/mL, which is as excessively high as the concentration of anti-human IL-6 receptor antibody contained in a sample, in order that almost all hsIL-6R molecules in the sample bind to WT-IgG1. Thereafter, the reaction mixture was dispensed to MA400 PR Streptavidin Plates (Meso Scale Discovery). After the reaction was further performed at room temperature for one hour and washed, Read Buffer T(×4)(Meso Scale Discovery) was dispensed. Immediately after that, measurement was performed by SECTOR PR 400 reader (Meso Scale Discovery). The concentration of hSIL-6R was computationally obtained based on the value shown in a calibration curve by use of

analytic software SOFTmax PRO (Molecular Devices). A change of hsIL-6R concentration with time in plasma of a normal mouse after intravenous injection, measured by this method, is shown in Figure 9 and Figure 11.

Binding effect of pH-dependent human IL-6 receptor

The *in-vivo* test results of H54/L28-IgG1 and GL1-IgG1 (to which a pH-dependent human IL-6 receptor bound) were compared. As shown in Figure 8, the retentivity values of the both antibodies in plasma were almost equal. However, as shown in Figure 9, it is confirmed that hsIL-6R, which was administered simultaneously with GL1-IgG1 (to which a pH-dependent human IL-6 receptor bound), was cleared faster than hsIL-6R, which was administered simultaneously with H54/L28-IgG1. From this, it was found that hsIL-6R concentration in plasma 4 days after administration by imparting pH-dependent human IL-6 receptor binding ability can be reduced by approximately 17 fold and approximately 34 fold, respectively.

Effect of N-linked glycosylation

The *in-vivo* test results of GL1-M111 or GL1-IgG1 and GL5-M111 or GL5-IgG1 (having an N-linked sugar chain added) were compared. As shown in Figure 8 or Figure 10, it was found that the antibody concentration in plasma of GL5-M111 or GL5-IgG1 (having an N-linked sugar chain added) changes at a slightly lower level compared to GL1-M111 or GL1-IgG1; however, the half-life periods of both antibodies in plasma do not differ. The difference of a change with time in the antibody concentration in plasma was caused by a large distribution volume of the antibody having an N-linked sugar chain added in the tissue. From this, it was considered that clearance of the antibodies does not differ. Next, as shown in Figure 9 or Figure 11, it was confirmed that hsIL-6R, which was administered simultaneously with GL5-M111-SA (-) or GL5-IgG1-SA (-) (having an N-linked sugar chain added) was cleared significantly faster than hsIL-6R, which was administered simultaneously with GL1-M111-SA (-) or GL1-IgG1-SA (-) (having no N-linked sugar chain added). It was found that in GL5-M111-SA (-) and GL5-IgG1-SA (-), the hsIL-6R concentration in plasma after 2 days can be reduced by approximately 6 fold and approximately 27 fold, respectively by adding an N-linked sugar chain. It was found that the concentration of an antibody in plasma changes with time at a lower level by addition of an N-linked sugar chain (as described above); however, a hsIL-6R concentration in plasma reducing effect is far beyond the decrease. This means that administration of an antibody capable of binding to a soluble IL-6 receptor in a pH-dependent manner and having an N-linked sugar chain added, can further accelerate clearance of the soluble IL-6 receptor, compared to administration of the antibody having no N-linked sugar chain added. In other words, the *in-vivo* antigen concentration in plasma can be reduced by administration of such an antibody (*in-vivo*). Furthermore, as shown in Figure 10 or Figure 11, changes of GL1-IgG1-SA (+) and GL1-IgG1-SA (-) antibody concentration in plasma with time are equivalent and clearance of hIL-6R, which was administered simultaneously with GL1-IgG1-SA (+), was equivalent to the clearance of hIL-6R, which was administered simultaneously with GL1-IgG1-SA (-). From this, it was suggested that removal of sialic acid from an N-linked sugar chain (added to the 297th position (EU numbering) of a heavy-chain constant region) does not influence a change with time of the antibody concentration in plasma or clearance of soluble IL-6 receptor.

When a conventional neutralization antibody (such as H54/L28-IgG1-SA (+)) is administered, clearance of an antigen binding to the antibody decreases, and the antigen remains for a longer time in plasma. It is unfavorable that plasma retentivity of the antigen that is desired to be neutralized is prolonged by administration of an antibody. The plasma retentivity of an antigen can be shortened by adding pH-dependent antigen-binding property (associate under a neutral condition and dissociates under an acidic condition) to an antibody. This time, the plasma retentivity of an antigen can be further shortened by addition of an N-linked sugar chain. Furthermore, it was shown that by administering an antibody capable of binding to an antigen in a pH-dependent manner (capable of binding to FcRn under a neutral condition (pH 7.4)), the same clearance as the clearance of an antigen alone can be attained. Up to now, a method attaining the same clearance as clearance of an antigen alone by administering an antibody has not been known. The method found in the investigation is extremely useful as a method for suppressing retentivity of an antigen (which is desired to be neutralized) from extending, by administration of an antibody. Furthermore, in the investigation, an advantage, i.e., acceleration of clearance of an antigen, was found for the first time by addition of an N-linked sugar chain (which is not involved in binding to an antigen) to an antibody in combination with use of an antibody binding to an antigen in a pH-dependent manner. Moreover, the site of the residues, to which an N-linked sugar chain (not involved in binding to an antigen) is added, are not limited to 20th and 24th residues (kabat numbering) of a light-chain variable region of GL5-M111 or GL5-IgG1. From this, it is considered that clearance of an antigen can be effectively accelerated by an antibody having an N-linked sugar chain added thereto, as long as the addition site is not relevant to amino acid substitution, not involved in binding to an antigen and the N-linked sugar chain can be added to the site.

[Example 4] Preparation of anti-human IL-6 receptor pH-dependent binding antibody having a high mannose-ended sugar chain introduced in variable region

Preparation of human IL-6 receptor pH-dependent binding antibody having high mannose sugar chain

GL1-IgG1, which is constituted of VH3-IgG1 (SEQ ID NO: 28) and VL3-CK (SEQ ID NO: 29), and GL5-IgG1, which is constituted of VH3-IgG1 (SEQ ID NO: 28) and L06-CK (SEQ ID NO: 36) were expressed and purified in accordance with the methods (known to those skilled in the art) described in Reference Example 2. In temporarily introducing an expression vector, kifnensien (SIGMA) was added to a cell culture solution so as to obtain a concentration of 10 μg/mL. In this manner, GL1-IgG1_kif+ and GL5-IgG1_kif+ having a high mannose sugar chain were prepared.

Observation of glycosylation of IgG1 sample by reducing SDS-PAGE

Glycosylation of an antibody having an N-linked glycosylation sequence introduced therein was observed by reducing SDS-PAGE. To each of GL1-G1_kif+ (5 μg aliquots) and GL5-G1_kif+ (5 μg), Tris-Glycine SDS Sample Buffer (2x) (TEFCO) containing a 5% 2-mercaptoethanol (Wako) was added. Then, the mixtures were incubated at 70°C for 5 minutes to prepare samples for electrophoresis. Electrophoresis was performed using a 12% SDS-PAGE mini 15 well (TEFCO) and with Precision plus blue standard (Bio-Rad) as a molecular-weight marker, and thereafter, CBB staining was performed with CBB Stain One (Nacalai tesque). The obtained electrophoretic patterns are shown in Figure 12.

As shown in the Figure, the light chain of GL5-IgG1_kif+ made a large shift toward a high molecular weight side compared to the light chain of GL1-IgG1_kif+. From this, it is considered that N-linked sugar chain binds to two sites of the light chain even in the presence of kifnensien.

Mass analysis of GL5-G1 kif+

To estimate an N-linked sugar chain added to GL5-G1_kif+ from mass, mass analysis was performed. GL5-M11 was treated with neuraminidase and reduced by a treatment with DTT (Wako), and RP-LC/ESI-MS was performed by use of Ultimate3000 (Dionex) and LTQ VELOS (Thermo scientific). Which of the high mannose N-linked sugar chains shown in Table 5 was added was estimated from mass. The obtained mass chromatogram is shown in Figure 13. As shown in Figure 13, the antibodies obtained had two sugar chains, which were added to sites of Man6 and Man9, Man7 and Man9, Man8 and Man9, and Man9 ad Man 9 of the light chain.

[Table 5]

| Abbreviation | Sugar-chain structure |
|---|---|
| Man6 | 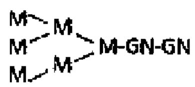 |
| Man7 | 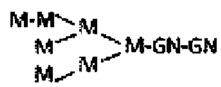 |
| Man8 | 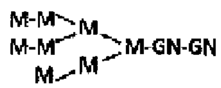 |
| Man9 | 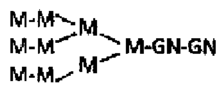 |
| F<br>M<br>GN | Fucose<br>Mannose<br>N-Acetylglucosamine |

Evaluation of binding to human IL-6 receptor (hIL6R)

Using Biacore T100 (GE Healthcare), kinetic analysis of antigen-antibody reactions between each of GL1-IgG1_kif+ and GL5-IgG1_kif+ and a human IL-6 receptor was performed. An appropriate amount of Anti-Human IgG (γ-chain specific) F(ab')2 fragment antibody produced in goat (Sigma) was immobilized onto Sensor chip CM5 (GE Healthcare) by the amine coupling method and then a target antibody was allowed to capture. Subsequently, a human IL-6 receptor dilution solution and a running buffer (a blank) were injected at a flow rate of 20 μL/min for 3 minutes to allow the human IL-6 receptor to interact with the antibody captured on the sensor chip. Either one of two types of running buffers (pH 7.4 and pH 6.0 containing 10 mmol/L ACES, 150 mmol/L NaCl, and 0.05% (w/v) Tween20) was used, and each buffer was employed for dilution of IL-6R. Thereafter, a running buffer was fed at a flow rate 20 μL/min for 5 minutes. After dissociation of the IL-6 receptor was observed, a 10 mmol/L Glycine-HCl (pH 1.5) solution was injected at a flow rate of 30 μL/min for 5 seconds. This procedure was repeated five times to regenerate the sensor chip. All samples were measured at 37°C. From the sensorgram obtained by measurement, an association constant, ka (1/Ms) and a dissociation constant kd (1/s) as kinetics parameters were computationally obtained. From the values, $K_D$ (M) of each antibody to a human IL-6 receptor was calculated. Individual parameters were calculated by use of Biacore T100 Evaluation Software (GE Healthcare). The resultant $K_D$ values of individual antibodies determined at pH 7.4 or pH 6.0 are shown in Table 6 below. A significant difference between $K_D$ values of both antibodies was not observed.

[Table 6]

| | $K_D$ at pH 6.0 (M) | $K_D$ at pH 7.4 (M) |
|---|---|---|
| GL1-IgG1_kif+ | 7.1E-08 | 1.5E-09 |
| GL5-IgG1_kif+ | 6.6E-08 | 1.4E-09 |

[Example 4] Investigation of antigen clearance acceleration effect by a pH-dependent anti-human IL-6 receptor antibody having a high mannose sugar chain introduced in a variable region, in a normal mouse

<u>In-vivo test using normal mouse</u>

To a normal mouse (C57BL/6J mouse, Charles River Japan), hsIL-6R (soluble human IL-6 receptor prepared in Reference Example 3) was solely administered or hsIL-6R and an anti-human IL-6 receptor antibody were simultaneously administered. Thereafter, *in-vivo* kinetics of the hsIL-6R and the anti-human IL-6 receptor antibody were evaluated. A hsIL-6R solution (5 μg/mL) or a mixture solution of the hsIL-6R and the anti-human IL-6 receptor antibody (5 μg/mL and 0.1 mg/mL respectively) was administered once to caudal vein in a dose of 10 mL/kg. At this time, since anti-human IL-6 receptor antibody is excessively present compared to hsIL-6R, it is considered that almost all hsIL-6R molecules bind to the antibody. After the administration, blood was sampled in 15 minutes, 7 hours, on 1 day, 2 days, 3 days, 4 days and 7 days. The blood sampled was immediately centrifuged at 4°C, 15,000 rpm for 15 minutes to obtain plasma. The plasma separated was stored in a freezer set at-20°C or less until measurement. As anti-human IL-6 receptor antibody, GL1-IgG1_kif+ and GL5-IgG1_kif+ mentioned above were used.

<u>Measurement of anti-human IL-6 receptor antibody concentration in plasma by ELISA</u>

The concentration of an anti-human IL-6 receptor antibody in mouse plasma was measured by ELISA. First, Anti-Human IgG (γ-chain specific) F(ab')2 Fragment of Antibody (SIGMA) was dispensed in Nunc-Immuno Plates, MaxiSoup (Nalge nunc International). The mixture was allowed to stand still at 4°C overnight to prepare an Anti-Human IgG immobilized plates. Samples having a plasma concentration of 0.8, 0.4, 0.2, 0.1, 0.05, 0.025 and 0.0125 μg/mL were prepared for forming a calibration curve sample and a mouse plasma measurement sample diluted 100 fold or more were prepared. To each (100 μL) of the calibration-curve samples and plasma measurement sample, 20 ng/mL hsIL-6R (200 μL) was added. The mixture was allowed to stand still at room temperature for one hour. Thereafter, the mixture was dispensed to the Anti-Human IgG immobilized plates and then allowed to stand still at room temperature for one hour. Thereafter, Biotinylated Anti-human IL-6R Antibody (R&D) was reacted at room temperature for one hour, and then, Streptavidin-PolyHRP80 (Stereospecific Detection Technologies) was reacted at room temperature for one hour. Then, a chromogenic reaction was performed using TMB One Component HRP Microwell Substrate (BioFX Laboratories) as a substrate and terminated with 1N-Sulfuric acid (Showa Chemical). Thereafter, the absorbance was measured by a micro plate reader at 450 nm. The concentration in the mouse plasma was computationally obtained from the absorbance shown in the calibration curve by use of analytic software SOFTmax PRO (Molecular Devices). A change of the antibody concentration with time in normal mouse plasma after intravenous administration, measured by this method, is shown in Figure 14.

<u>Measurement of hsIL-6R concentration in plasma by electrochemiluminescence assay</u>

Concentration of hsIL-6R in plasma in a mouse was measured by electrochemiluminescence assay. Samples

of hsIL-6R having a concentration of 2000, 1000, 500, 250, 125, 62.5 and 31.25 pg/mL were prepared for forming a calibration curve sample and a mouse plasma measurement sample diluted to 50 fold or more were prepared. A Monoclonal Anti-human IL-6R Antibody (R&D) labeled with ruthenium by means of SULFO-TAG NHS Ester (Meso Scale Discovery), Biotinylated Anti-human IL-6R Antibody (R&D) and a WT-IgG1 solution were mixed and reacted at 37°C overnight. At this time, the final concentration of WT-IgG1 (constituted of H (WT) (SEQ ID NO: 37) and L (WT) (SEQ ID NO: 38) serving as an anti-human IL-6 receptor antibody) was set at 333 μg/mL, which was as excessively high as the concentration of anti-human IL-6 receptor antibody contained in a sample in order that almost all hsIL-6R molecules in the sample bind to WT-IgG1. Thereafter, the reaction mixture was dispensed to MA400 PR Streptavidin Plates (Meso Scale Discovery). After the reaction was further performed at room temperature for one hour and washed, Read Buffer T(×4)(Meso Scale Discovery) was dispensed. Immediately after that, measurement was performed by SECTOR PR 400 reader (Meso Scale Discovery). The concentration of hSIL-6R was computationally obtained based on the value shown in a calibration curve by use of analytic software SOFTmax PRO (Molecular Devices). A change of hsIL-6R concentration with time in plasma of a normal mouse after intravenous injection measured by use of this method is shown in Figure 15.

Effect of mannose glycosylation

The *in-vivo* test results of GL1-IgG1_kif+ and GL5-IgG1_kif+ (having a mannose sugar chain added) were compared. As shown in Figure 14, it was found that the antibody concentrations in plasma of GL5-IgG1_kif+ (having a mannose sugar chain added) 7 hours after administration decreased by approximately 3.7 fold compared to GL1-IgG1_kif+; however, as shown in Figure 15, hsIL-6R, which was administered simultaneously with GL5-IgG1_kif+ (having a mannose sugar chain added) can reduce the hsIL-6R concentration in plasma by approximately 12.9 fold, 7 hours after the administration compared to hsIL-6R administered simultaneously with GL1-IgG1_kif+ (having no mannose sugar chain added). It was found that the concentration of an antibody in plasma was decreased by adding a mannose sugar chain as described above; however, a hsIL-6R plasma concentration reducing effect is far beyond the decrease. This means that administration of an antibody capable of binding to a soluble IL-6 receptor in a pH-dependent manner and having a mannose sugar chain added can accelerate clearance of a soluble IL-6 receptor, compared to the administration of an antibody having no mannose sugar chain added. In other words, the *in-vivo* antigen concentration in plasma can be reduced by administration of such an antibody (in-vivo). Furthermore, as shown in Figure 15, since clearance of hIL-6R administered simultaneously with GL5-IgG1_kif+ is larger than the clearance of hIL-6R solely administered, it was suggested that the blood concentration of an antigen can be reduced from the blood concentration before the administration, by administering the antibody capable of binding to the antigen in a pH-dependent manner and having a mannose-ended sugar chain added thereto.

[Reference Example 1] Construct of expression vector for IgG antibody having an amino acid substitution

A variant was prepared by use of QuikChange Site-Directed Mutagenesis Kit (Stratagene) in accordance with the method instructed in the accompanying protocol. The obtained plasmid fragment was inserted in an animal cell expression vector to prepare a target H-chain expression vector and L-chain expression vector. The nucleotide sequences of the obtained expression vectors were determined by a method known to those skilled in the art.

[Reference Example 2] Expression and purification of IgG antibody

The expression vector prepared was temporarily introduced into a human fetus kidney cancer cell-derived HEK293H strain (Invitrogen) or FreeStyle 293 cell (Invitrogen) to express the antibody. The resultant culture supernatant was collected and passed through a 0.22 μM-filter MILLEX (R)-GV (Millipore) or a 0.45-μM filter MILLEX (R)-GV (Millipore) to obtain a culture supernatant. From the obtained culture supernatant, the antibody was purified by use of rProtein A Sepharose Fast Flow (GE Healthcare) or Protein G Sepharose 4 Fast Flow (GE Healthcare) in accordance with a method known to those skilled in the art. The concentration of the purified antibody was obtained by measuring absorbance by a spectrophotometer at 280 nm and obtaining the concentration of the antibody from the obtained value, by use of an absorption constant calculated by a PACE method (Protein Science (1995) 4, 2411-2423).

[Reference Example 3] Preparation of soluble human IL-6 receptor (hsIL-6R)

A recombinant human IL-6 receptor serving as an antigen was prepared as follows. A cell strain constantly expressing CHO of a soluble human IL-6 receptor ((hereinafter, hsIL-6R) reported by J. Immunol. 152, 4958-4968 (1994)), consisting of an amino acid sequence having 1st to 357th amino acids from the N terminal side was constructed in accordance with a method known to those skilled in the art and cultured to express hsIL-6R. From the resultant culture

supernatant, hsIL-6R was purified by two steps, i.e., Blue Sepharose 6 FF column chromatography and gel filtration column chromatography. In the final step, a fraction eluted as a main peak was employed as a final purified product.

[Reference Example 4] Acquisition of Ca-dependent binding antibody from a human antibody library by use of phage display technique

Preparation of naive human antibody phage display library

A plurality of human antibody phage display libraries displaying Fab domains each consisting of a human antibody sequence were constructed by polyA RNA prepared from human PBMC or commercially available human polyA RNA, etc. as a template, in accordance with a method shown in Mol. Biol. (2002) 178, 87-100.

Acquisition of Ca-dependent binding antibody fragment from library by beads panning

In the first screening from the human antibody phage display library constructed, only antibody fragments having a binding ability to an antigen were concentrated or concentrated based on Ca-dependent binding ability. When the antibody fragments having a Ca-dependent binding ability were concentrated, the antibody fragments were allowed to bind to an antigen in the presence of Ca ions and then Ca ions were chelated by EDTA. In this manner, phages were eluted. As the antigen, a biotin-labelled human IL-6 receptor was used.

Phages were produced from *Escherichia coli* harboring a phage-display phagemid constructed as mentioned above. The obtained culture fluid was subjected to precipitation with 2.5 M NaCl/10%PEG and then diluted with TBS to obtain a phage library liquid. To the phage library liquid, BSA and $CaCl_2$ were added so as to obtain a final BSA concentration of 4% and a calcium-ion concentration of 1.2 mM. Panning was performed with reference to a general method using an antigen immobilized to magnetic beads (J Immunol. Methods. (J. Immunol. Methods. (2008) 332 (1-2), 2-9, J Immunol. Methods. (2001) 247 (1-2), 191-203, Biotechnol. Prog. (2002) 18 (2), 212-220, Mol. Cell. Proteomics. (2003) 2 (2), 61-69). As the magnetic beads, NeutrAvidin coated beads (Sera-Mag SpeedBeads NeutrAvidin-coated) or Streptavidin coated beads (Dynabeads M-280 Streptavidin) were used.

More specifically, to the phage library liquid prepared, a biotin-labelled antigen (250 pmol) was added to allow the antigen in contact with the phage library liquid at room temperature for 60 minutes. Then, magnetic beads were blocked with BSA and added at room temperature. Binding was performed at room temperature for 15 minutes. The beads were washed once with 1 mL of 1.2 mM $CaCl_2$/TBS (TBS containing 1.2 mM $CaCl_2$). Thereafter, if antigen fragments having a binding ability were concentrated, elution was performed in accordance with a general method. If antibody fragments having a Ca-dependent binding ability were concentrated, the beads were suspended in 2 mM EDTA/TBS (TBS containing 2mM EDTA) to recover the phages. To the phage solution recovered, *Escherichia coli* strain TG1 (10 mL), which reached a logarithm growth phase (OD600 0.4-0.7), was added. A culture was performed while gentry stirring at 37°C for one hour to infect *Escherichia coli* cells with the phage. The *Escherichia coli* cells infected were smeared on a plate of 225 mm × 225 mm. Phage was cultured again starting from culturing of the *Escherichia coli* cells.

In the steps on and after the second panning step, concentration was performed based on a Ca-dependent binding ability. Specifically, to the phage library liquid prepared, biotin-labelled antigen (40 pmol) was added to allow the antigen in contact with the phage library liquid at room temperature for 60 minutes. Then, magnetic beads were blocked with BSA and added at room temperature. Binding was made at room temperature for 15 minutes. The beads were washed once separately with 1 mL of 1.2 mM $CaCl_2$/TBST (TBS containing 1.2 mM $CaCl_2$, 0.1% Tween-20) and 1.2 mM $CaCl_2$/TBS. Thereafter 0.1 mL of 2 mM EDTA/TBS (TBS containing 2mM EDTA) was added to suspend the beads at room temperature. Immediately after that, the beads were separated by use of Magnet Stand to collect a phage solution. The phage solution collected was added to *Escherichia coli* strain TG1 (10 mL), which reached a logarithm growth phase (OD600 0.4-0.7)l, and cultured while gentry stirring at 37°C for one hour to infect *Escherichia coli* cells with the phase. The *Escherichia coli* cells infected were smeared on a plate of 225 mm × 225 mm. Phage was cultured again starting from culturing of the *Escherichia coli* cells. The panning step was repeated twice.

Evaluation by phage ELISA

From a single colony of *Escherichia coli* obtained by the above method, a phage-containing culture supernatant was recovered in accordance with the method (Methods Mol. Biol. (2002) 178, 133-145). To the phage-containing culture supernatant, BSA and $CaCl_2$ were added so as to obtain a final BSA concentration of 4% and a calcium-ion concentration of 1.2 mM. The resultant mixture was subjected to ELISA. A StreptaWell 96 microtiter plate (Roche) was coated with PBS (100 μL) containing the biotin-labelled antigen overnight, washed with PBST (PBS containing 0.1%Tween20) to remove the antigen. Thereafter blocking was performed with 4% BSA-TBS (250 μL) for one hour or more. Then, 4%

BSA-TBS was removed and the culture supernatant prepared was added. The mixture was allowed to stand still at 37°C for one hour. In this way, binding of the phage displaying antibody was made. After washing with 1.2 mM $CaCl_2$/TBST (TBS containing 1.2 mM $CaCl_2$ and 0.1% Tween20), 1.2 mM $CaCl_2$/TBS or 1 mM EDTA/TBS was added. The mixture was allowed to stand still at 37°C for 30 minutes to incubate. After washing with 1.2 mM $CaCl_2$/TBST, HRP-binding anti M13 antibody (Amersham Parmacia Biotech) diluted with TBS containing 4% BSA and a calcium ion at a concentration of 1.2 mM was incubated for one hour. After washing with 1.2 mM $CaCl_2$/TBST, detection was made with a TMB single solution (ZYMED). After the reaction was terminated with sulfuric acid, the absorbance at 450 nm was measured. The antibody fragments determined to have a Ca-dependent binding ability were subjected to nucleotide sequence analysis using a specific primer.

Expression and purification of antibody

A clone, which was determined to have a Ca-dependent binding ability by phage ELISA, was introduced into an animal cell expression plasmid. Expression of an antibody was performed by the following method. A human fetus kidney cell-derived FreeStyle 293-F strain (Invitrogen) was suspended in FreeStyle 293 Expression Medium (Invitrogen). The suspension solution (3 mL) was seeded in each well of a 6 well plate at a cell density of $1.33 \times 10^6$ cells/mL and a plasmid prepared was introduced into the cell by a lipofection method. Culture was made in a $CO_2$ incubator (37°C, 8%$CO_2$, 90 rpm) for 4 days. From the obtained culture supernatant, an antibody was purified by use of rProtein A SepharoseTM Fast Flow (Amersham Biosciences) in accordance with a method known to those skilled in the art. The concentration of the purified antibody was determined based on the absorbance at 280 nm measured by a spectrophotometer. From the obtained measurement value, an antibody concentration was computationally obtained by use of the absorption constant calculated by the PACE method (Protein Science (1995) 4, 2411-2423).

[Reference Example 5] Evaluation of Ca-dependent binding ability of the obtained antibody to human IL-6 receptor

To determine whether the antibody obtained in Reference Example 4, i.e., 6RL#9-IgG1 (heavy chain SEQ ID NO: 7, light chain SEQ ID NO: 39), and FH4-IgG1 (heavy chain SEQ ID NO: 40, light chain SEQ ID NO: 41), have a Ca-dependent binding ability, kinetic analysis of an antigen-antibody reaction was performed by use of Biacore T100 (GE Healthcare). The above heavy-chain variable region was fused with an IgG1 constant region (having a deletion of 2 amino acids at the C terminal of SEQ ID NO: 9) to prepare a heavy chain sequence. Furthermore, a light-chain variable region (SEQ ID NO: 41) was fused with a constant region of light-chain k chain (SEQ ID NO: 42) to prepare a light chain sequence. As an antibody having no Ca dependency, H54/L28-IgG1 (heavy chain SEQ ID NO: 26, light chain SEQ ID NO: 27) described in WO 2009/125825 was used. As a high calcium-ion concentration, 2 mM was used and as a low calcium-ion concentration, 3 $\mu$M was used. As the antigen, a human IL-6 receptor (IL-6R) was used. An appropriate amount of protein A (Invitrogen) was immobilized onto Sensor chip CM4 (GE Healthcare) in accordance with the amine coupling method and then a target antibody was allowed to capture. Either one of two types of running buffers, i.e., 10 mmol/L ACES, 150 mmol/L NaCl, 0.05% (w/v) Tween20, 2 mmol/L $CaCl_2$, (pH 7.4) and 10 mmol/L ACES, 150 mmol/L NaCl, 0.05% (w/v)Tween20, 3 $\mu$Mol/L $CaCl_2$, (pH 7.4) was used. All samples were measured at 37°C. Each buffer was employed for dilution of IL-6R.

With respect to H54L28-IgG1, an IL-6R dilution solution and a running buffer (a blank) were injected at a flow rate 20 $\mu$L/min for 3 minutes and allowed IL-6R to interact with the antibody captured onto a sensor chip. Thereafter, the running buffer was fed at a flow rate of 20 $\mu$L/min for 10 minutes. After dissociation of IL-6R was observed, 10 mmol/L Glycine-HCl, pH 1.5 was injected at a flow rate 30 $\mu$L/min for 30 seconds to regenerate the sensor chip. From the sensorgram obtained by measurement, an association constant, ka (1/Ms) and a dissociation constant kd (1/s) as kinetics parameters were computationally obtained. From the values, $K_D$ (M) of each antibody to a human IL-6 receptor was calculated. Individual parameters were calculated by use of Biacore T100 Evaluation Software (GE Healthcare).

With respect to FH4-IgG1 and 6RL#9-IgG1, an IL-6R dilution solution and the running buffer (a blank) were injected at a flow rate 5 $\mu$L/min for 15 minutes and allowed IL-6R to interact with the antibody captured onto a sensor chip. Thereafter, 10 mmol/L Glycine-HCl, pH 1.5 was injected at a flow rate of 30 $\mu$L/min for 30 seconds to regenerate the sensor chip. From the sensorgram obtained by measurement, an association constant, a dissociation constant$K_D$ (M) was obtained by a steady state affinity model. Individual parameters were calculated by use of Biacore T100 Evaluation Software (GE Healthcare).

The dissociation constant $K_D$ between each antibody and IL-6R obtained by this method in the presence of 2 mM $CaCl_2$ is shown in Table 7. In the case of H54/L28-IgG1, any difference was not observed in binding to IL-6R in different Ca concentrations; however in the cases of FH4-IgG1 and 6RL#9-IgG1, binding significantly decreased under a low Ca concentration condition (Figures 16, 17, 18).

[Table 7]

| | H54/L28-IgG1 | FH4-IgG1 | 6RL#9-IgG1 |
|---|---|---|---|
| $K_D$ (M) | 1.9E-9 | 5.9E-7 | 2.6E-7 |

With respect to H54/L28-IgG1, $K_D$ under a Ca concentration of 3 μM can be calculated in the same manner as under 2 mM Ca concentration. With respect to FH4-IgG1 and 6RL#9-IgG1, if a Ca concentration of 3 μM, binding to IL-6R was not virtually observed. Thus, it is difficult to calculate $K_D$ in accordance with the aforementioned method; however, $K_D$ can be estimated by use of the following Expression 1 (Biacore T100 Software Handbook, BR-1006-48, AE 01/2007).

[Expression 1]

$$Req = C \cdot Rmax/(K_D + C) + RI$$

where

Req (RU): Steady state binding levels
Rmax (RU): Analyte binding capacity of the surface
RI (RU): Bulk refractive index contribution in the sample
C (M): Analyte concentration
$K_D$ (M): Equilibrium dissociation constant

Presumable dissociation constant KD between each antibody and IL-6R was roughly calculated in accordance with Expression 1 in the case of a Ca concentration of 3 μMol/L. The results are shown in Table 8.

[Table 8]

| | H54L28-IgG1 | FH4-IgG1 | 6RL#9-IgG1 |
|---|---|---|---|
| $R_{eq}$ (RU) | | 5 | 10 |
| $R_{max}$ (RU) | | 39 | 72 |
| RI (RU) | | 0 | 0 |
| C (M) | | 5E-06 | 5E-06 |
| $K_D$ (M) | 2.2E-9 | 3.4E-05 | 3.1E-05 |

In Table 8, $R_{eq}$, $R_{max}$, RI and C are estimated from measurement results.

From the results, it was estimated that in FH4-IgG1 and 6RL#9-IgG1, $K_D$ values of them to IL-6R may increase approximately 60 fold and approximately 120 fold, (affinity may decrease 60 fold, 120 fold or more) affinity) respectively by changing the concentration of Ca from 2 mM to 3 μM in terms of $CaCl_2$. KD values of three antibodies: H54/L28-IgG1, FH4-IgG1 and 6RL#9-IgG1 in the presence of 2 mM$CaCl_2$ and 3 μM $CaCl_2$ and Ca dependency of the $K_D$ value are summarized in Table 9.

[Table 9]

| Antibody | H54/L28-IgG1 | FH4-IgG1 | 6RL#9-IgG1 |
|---|---|---|---|
| KD (M) (2 mM $CaCl_2$) | 1.9E-9 | 5.9E-7 | 2.6E-7 |
| KD (M) (3 μM $CaCl_2$) | 2.2E-9 | 3.4E-5 or more | 3.1E-5 or more |
| Ca dependency | Almost equivalent | Approximately 60 fold or more | Approximately 120 fold or more |

[Reference Example 6] Evaluation of binding of calcium ion to obtained antibody

Calcium ion-binding to an antibody was evaluated based on the thermal denaturation mid-temperature (Tm value) measured by differential scanning calorimetry (DSC) (MicroCal VP-Capillary DSC, manufactured by MicroCal).

The thermal denaturation intermediate temperature (Tm value) is used as an index of stability. When a protein is stabilized by binding of a calcium ion, the thermal denaturation intermediate temperature (Tm value) increases compared to the protein to which a calcium ion is not bound (J. Biol. Chem. (2008) 283 (37) 25140-25149). By using this phenomenon, calcium ion-binding to an antibody was evaluated. The antibody purified was dialyzed against a solution containing 20 mM Tris-HCl, 150 mM NaCl and 2 mM $CaCl_2$, pH 7.4, or a solution containing 20 mM Tris-HCl, 150 mM NaCl and 3 μM $CaCl_2$, pH 7.4 by use of EasySEP, (TOMY). A protein solution was prepared with the solution used in dialysis so as to obtain concentration of 0.1 mg/mL and then subjected to DSC measurement by raising temperature from 20°C to 115°C at a rate of 240°C/hr. The thermal denaturation intermediate temperature (Tm value) of a Fab domain of each antibody based on the DSC denaturation curve obtained was calculated and is shown in Table 10.

[Table 10]

| Variable-region sequence | Calcium-ion concentration | | ΔTm [°C] |
|---|---|---|---|
| | 3 μM | 2 mM | 2 mM - 3 μM |
| H54/L28 | 92.87 | 92.87 | 0.00 |
| FH4 | 74.71 | 78.97 | 4.26 |
| 6RL#9 | 77.77 | 78.98 | 1.21 |

From the results of Table 10, it was shown that, in the cases of FH4 and 6RL#9 showing a calcium-dependent binding ability, the Tm value of Fab changes depending upon the calcium-ion concentration; whereas, in H54/L28 showing no calcium-dependent binding ability, the Tm value does not change. A change of the Tm value of Fab shown in FH4 and6RL#9 suggests that a calcium ion binds to these antibodies to stabilize the Fab domain thereof. From this, it was demonstrated that a calcium ion binds to FH4 and 6RL#9; whereas calcium ion is not bound to H54/L28.

[Reference Example 7] Evaluation of the effect of Ca-dependent binding antibody on the retentivity of an antigen in normal mouse plasma

<u>In vivo test using normal mouse</u>

To a normal mouse (C57BL/6J mouse, Charles River Japan) hsIL-6R (soluble human IL-6 receptor prepared in Reference Example 3) was solely administered or hsIL-6R and an anti-human IL-6 receptor antibody were simultaneously administered. Thereafter, *in-vivo* kinetics of the hsIL-6R and the anti-human IL-6 receptor antibody was evaluated. A hsIL-6R solution (5 μg/mL) or a mixture solution of the hsIL-6R and the anti-human IL-6 receptor antibody was administered once to caudal vein in a dose of 10 mL/kg. As the anti-human IL-6 receptor antibody, the aforementioned H54/L28-IgG1, 6RL#9-IgG1 and FH4-IgG1 were used.

The hsIL-6R concentrations of the mixture solutions are all 5 μg/mL, the anti-human IL-6 receptor antibody concentration varies depending upon the antibody. The concentration of H54/L28-IgG1 is 0.1 mg/mL and the concentrations of 6RL#9-IgG1 and FH4-IgG1 are 10 mg/mL. At this time, since anti-human IL-6 receptor antibody is excessively present compared to hsIL-6R, it is considered that almost all hsIL-6R molecules bind to the antibody. Blood was sampled 15 minutes, 7 hours, 1 day, 2 days, 3 days, 4 days, 7 days, 14 days, 21 days, 28 days after the administration. The blood sampled was immediately centrifuged at 4°C, 12,000 rpm for 15 minutes to obtain plasma. The plasma separated was stored in a freezer set at-20°C or less until measurement.

<u>Measurement of anti-human IL-6 receptor antibody concentration in normal mouse plasma by ELISA</u>

The concentration of an anti-human IL-6 receptor antibody in mouse plasma was measured by ELISA. First, Anti-Human IgG (γ-chain specific) F(ab')2 Fragment of Antibody (SIGMA) was dispensed in Nunc-Immuno Plates, MaxiSoup (Nalge nunc International). The mixture was allowed to stand still at 4°C overnight to prepare an Anti-Human IgG immobilized plates. Samples having a plasma concentration of 0.64, 0.32, 0.16, 0.08, 0.04, 0.02, and 0.01 μg/mL were prepared for forming a calibration curve sample and a mouse plasma measurement sample diluted 100 fold or more were prepared, dispensed in Anti-Human IgG immobilized plates and incubated at 25°C for one hour. Thereafter, Biotinylated Anti-human IL-6R Antibody (R&D) was reacted at room temperature for one hour and then, Streptavidin-PolyHRP80 (Stereospecific Detection Technologies) was reacted at 25°C for 0.5 hours. A chromogenic reaction was performed using TMB One Component HRP Microwell Substrate (BioFX Laboratories) as a substrate and terminated with 1N-Sulfuric acid (Showa Chemical). Thereafter, the absorbance at 450 nm was measured by a micro plate reader. The concentration in the mouse plasma was computationally obtained from the absorbance shown in the calibration

curve by use of analytic software SOFTmax PRO (Molecular Devices). Changes of H54/L28-IgG1, 6RL#9-IgG1, FH4-IgG1 antibody concentration with time in normal mouse plasma after intravenous administration measured by this method are shown in Figure 19.

Measurement of hsIL-6R concentration in plasma by electrochemiluminescence assay

Concentration of hsIL-6R in mouse plasma was measured by electrochemiluminescence assay. Samples of hsIL-6R having a concentration of 2000, 1000, 500, 250, 125, 62.5 and 31.25 pg/mL were prepared and a mouse plasma measurement sample diluted to 50 fold or more was prepared. Monoclonal Anti-human IL-6R Antibody (R&D) labeled with ruthenium by means of SULFO-TAG NHS Ester (Meso Scale Discovery), Biotinylated Anti-human IL-6R Antibody (R&D), and a tocilizumab (heavy chain SEQ ID NO: 37, light chain SEQ ID NO: 38) solution were mixed and reacted at 4°C overnight. At this time, 10 mM EDTA was added to the Assay buffer in order to decrease Free Ca concentration in a sample, thereby dissociating almost all hsIL-6R molecules from 6RL#9-IgG1 or FH4-IgG1 and associating with tocilizumab added. Thereafter, the mixture was dispensed to MA400 PR Streptavidin Plates (Meso Scale Discovery). Furthermore, a reaction was performed at 25°C for one hour and Read Buffer T (x4) (Meso Scale Discovery) was dispensed. Immediately after that, measurement was performed by SECTOR PR 400 reader (Meso Scale Discovery). The concentration of hSIL-6R was computationally obtained based on the value shown in a calibration curve by use of analytic software SOFTmax PRO (Molecular Devices). A change of hsIL-6R concentration with time in plasma of a normal mouse after intravenous injection measured by this method is shown in Figure 20.

As a result, in the case of single administration of hsIL-6R, hsIL-6R was cleared at an extremely high speed; whereas, in the simultaneous administration of hsIL-6R and a general antibody H54/L28-IgG1 (no Ca-dependent binding), the clearance speed of hsIL-6R was significantly low. In contrast, in the case of simultaneous administration of 6RL#9-IgG1 or FH4-IgG1 (showing Ca-dependent binding to hsIL-6R (100 fold or more)) and hsIL-6R, the clearance speed was significantly accelerated. In the simultaneous administration cases with 6RL#9-IgG1 and FH4-IgG1, compared to simultaneous administration with H54/L28-IgG1, the hsIL-6R concentration of Day 1 in plasma was successfully reduced 39 fold and 2 fold, respectively. From this, it was confirmed that a calcium-dependent binding antibody can accelerate clearance of an antigen from the plasma.

[Reference Example 8] Identification of calcium ion-binding site in 6RL#9 antibody by X-ray crystal structure analysis

X-ray crystal structure analysis

As shown in Reference Example 6, the thermal denaturation temperature Tm assay suggested that the 6RL#9 antibody bound to calcium ions. However, the site via which the 6RL#9 antibody bound to calcium ions was unpredictable. Thus, a residue responsible for the interaction with calcium ions was identified in the sequence of the 6RL#9 antibody by use of the approach of X-ray crystal structure analysis.

6RL#9 antibody expression and purification

The 6RL#9 antibody expressed for use in X-ray crystal structure analysis was purified. Specifically, animal cells were transiently transfected with plasmids for expression in animal cells prepared so as to permit respective expression of the heavy chain and the light chain of the 6RL#9 antibody as shown in Reference Example 5. The prepared plasmids were transferred by lipofection to 800 mL of a human embryonic kidney cell-derived FreeStyle 293-F line (Invitrogen Corp.) suspended at a final cell density of 1 x $10^6$ cells/mL in FreeStyle 293 Expression Medium (Invitrogen Corp.). The cells transfected with the plasmids were cultured for 5 days in a $CO_2$ incubator (37°C, 8% $CO_2$, 90 rpm). Antibodies were purified from the obtained culture supernatant according to a method generally known to those skilled in the art using rProtein A Sepharose(TM) Fast Flow (Amersham Biosciences, Inc.). The absorbance of the purified antibody solution was measured at 280 nm using a spectrophotometer. The antibody concentration was calculated from the measurement value by use of an extinction coefficient calculated by PACE (Protein Science (1995) 4, 2411-2423).

Purification of Fab fragment from 6RL#9 antibody

The 6RL#9 antibody was concentrated to 21 mg/mL using an ultrafiltration membrane having a molecular weight cutoff of 10000 MWCO. The antibody was diluted to 5 mg/mL with 4 mM L-cysteine, 5 mM EDTA, and a 20 mM sodium phosphate buffer solution (pH 6.5) to prepare 2.5 mL of an antibody sample. After addition of 0.125 mg of papain (Roche Applied Science), the sample was stirred and then left standing at 35°C for 2 hours. The sample thus left standing was further supplemented with one tablet of Protease Inhibitor Cocktail Mini, EDTA-Free (Roche Applied Science) dissolved in 10 mL of a 25 mM MES buffer solution (pH 6), and left standing in ice to terminate the protease reaction

with papain. Next, the sample was added to a 1 mL-size cation-exchange column HiTrap SP HP (GE Healthcare Bio-Sciences Corp.) (equilibrated with a 25 mM MES buffer solution (pH 6)) connected in tandem with a downstream 1 mL-size protein A carrier column HiTrap MabSelect Sure (GE Healthcare Bio-Sciences Corp.). A purified fraction of the 6RL#9 antibody Fab fragment was obtained by elution on a linear gradient of NaCl concentration up to 300 mM in this buffer solution. Next, the obtained purified fraction was concentrated to approximately 0.8 mL using a 5000 MWCO ultrafiltration membrane. The concentrate was added to a gel filtration column Superdex 200 10/300 GL (GE Healthcare Bio-Sciences Corp.) equilibrated with a 100 mM HEPES buffer solution (pH 8) containing 50 mM NaCl. The purified 6RL#9 antibody Fab fragment for crystallization was eluted from the column using this buffer solution. The column operation was all carried out at a low temperature of 6 to 7.5°C.

Crystallization of Fab fragment of 6RL#9 antibody in presence of Ca

Seed crystals of the 6RL#9 Fab fragment were obtained in advance under generally set conditions. Next, the purified 6RL#9 antibody Fab fragment was adjusted to 5 mM by the addition of $CaCl_2$ and concentrated to 12 mg/mL using a 5000 MWCO ultrafiltration membrane. Subsequently, the sample thus concentrated was crystallized by the hanging-drop vapor diffusion method. A 100 mM HEPES buffer solution (pH 7.5) containing 20 to 29% PEG4000 was used as a reservoir solution. The seed crystals were disrupted in a 100 mM HEPES buffer solution (pH 7.5) containing 29% PEG4000 and 5 mM $CaCl_2$ and diluted 100- to 10000-fold, and 0.2 μl of each solution of this dilution series was added to a mixed solution of 0.8 μl of the reservoir solution and 0.8 μl of the concentrated sample on a glass cover to prepare crystallization drops. The crystallization drops were left standing at 20°C for 2 days to 3 days. X-ray diffraction data on the obtained thin plate-like crystals was determined.

Crystallization of Fab fragment of 6RL#9 antibody in absence of Ca

The purified 6RL#9 antibody Fab fragment was concentrated to 15 mg/mL using a 5000 MWCO ultrafiltration membrane. Subsequently, the sample thus concentrated was crystallized by the hanging-drop vapor diffusion method. A 100 mM HEPES buffer solution (pH 7.5) containing 18 to 25% PEG4000 was used as a reservoir solution. Crystals of the 6RL#9 antibody Fab fragment obtained in the presence of Ca were disrupted in a 100 mM HEPES buffer solution (pH 7.5) containing 25% PEG4000 and diluted 100- to 10000-fold, and 0.2 μl of each solution of this dilution series was added to a mixed solution of 0.8 μl of the reservoir solution and 0.8 μl of the concentrated sample on a glass cover to prepare crystallization drops. The crystallization drops were left standing at 20°C for 2 days to 3 days. X-ray diffraction data on the obtained thin plate-like crystals was assayed.

Assay of X-ray diffraction data on crystal of 6RL#9 antibody Fab fragment obtained in presence of Ca

One of the monocrystals (obtained in the presence of Ca) of the 6RL#9 antibody Fab fragment dipped in a 100 mM HEPES buffer solution (pH 7.5) containing 35% PEG4000 and 5 mM $CaCl_2$ was scooped out, together with the external solution, using very small nylon loop pin and frozen in liquid nitrogen. The X-ray diffraction data on the frozen crystal was assayed using beam line BL-17A from Photon Factory, Institute Materials Structure Science, High Energy Accelerator Research Organization (KEK). During the assay, the frozen crystal was left at all times in a nitrogen stream of -178°C to maintain its frozen state. A total of 180 diffraction images were collected, with the crystal rotated by 1° for each image, using a CCD detector Quantum 315r (Area Detector Systems Corporation (ADSC)) equipped with the beam line. The determination of a lattice constant, the indexing of diffraction spots, and the processing of the diffraction date were performed using a program Xia2 (CCP4 Software Suite), XDS Package (Wolfgang Kabsch), and Scala (CCP4 Software Suite). Finally, diffraction intensity data up to a resolution of 2.2 angstroms was obtained. This crystal belonged to the space group $P2_12_12_1$ and had lattice constants a = 45.47 angstroms, b = 79.86 angstroms, c = 116.25 angstroms, $\alpha$ = 90°, $\beta$ = 90°, and $\gamma$ = 90°.

Assay of X-ray diffraction data on crystal of 6RL#9 antibody Fab fragment obtained in absence of Ca

One of the monocrystals (obtained in the absence of Ca) of the 6RL#9 antibody Fab fragment dipped in a 100 mM HEPES buffer solution (pH 7.5) containing 35% PEG4000 was scooped out, together with the external solution, using very small nylon loop pin and frozen in liquid nitrogen. The X-ray diffraction data on the frozen crystal was assayed using beam line BL-5A from Photon Factory, Institute Materials Structure Science, High Energy Accelerator Research Organization (KEK). During the assay, the frozen crystal was left at all times in a nitrogen stream of -178°C to maintain its frozen state. A total of 180 diffraction images were collected, with the crystal rotated by 1° for each image, using a CCD detector Quantum 210r (Area Detector Systems Corporation (ADSC)) equipped with the beam line. The determination of a lattice constant, the indexing of diffraction spots, and the processing of the diffraction date were performed

using a program Xia2 (CCP4 Software Suite), XDS Package (Wolfgang Kabsch), and Scala (CCP4 Software Suite). Finally, diffraction intensity data up to a resolution of 2.3 angstroms was obtained. This crystal, which was the same type of the crystal obtained in the presence of Ca, belonged to the space group $P2_12_12_1$ and had lattice constants a = 45.40 angstroms, b = 79.63 angstroms, c = 116.07 angstroms, $\alpha$ = 90°, $\beta$ = 90°, and $\gamma$ = 90°.

Structural analysis of crystal of 6RL#9 antibody Fab fragment obtained in presence of Ca

The structure of the crystal of the 6RL#9 antibody Fab fragment obtained in the presence of Ca was determined by the molecular replacement method using a program Phaser (CCP4 Software Suite). The number of molecules in the asymmetric unit was presumed to be one from the size of the obtained crystal lattice and the molecular weight of the 6RL#9 antibody Fab fragment. On the basis of homology on the primary sequence, amino acid residues at positions 112 to 220 in the chain A and at positions 116 to 218 in the chain B retrieved from the structure coordinate of PDB code: 1ZA6 were selected as model molecules for search for CL and CH1 regions. Next, amino acid residues at positions 1 to 115 in the chain B retrieved from the structure coordinate of PDB code: 1ZA6 were selected as a model molecule for search for a VH region. Finally, amino acid residues at positions 3 to 147 in the light chain retrieved from the structure coordinate of PDB code: 2A9M were selected as a model molecule for search for a VL region. According to this order, the orientation and position of each model molecule for search in the crystal lattice were determined by rotation and translation functions to obtain an initial structural model of the 6RL#9 antibody Fab fragment. The initial structural model was subjected to rigid body refinement moving each of the VH, VL, CH1, and CL domains to obtain a crystallographic reliability factor R of 46.9% and a Free R value of 48.6% for reflection data of 25-3.0 angstroms. In addition, the model was refined by model correction on a repetition program Coot (Paul Emsley) with reference to electron density maps of coefficients 2Fo-Fc and Fo-Fc calculated through the use of structure refinement using a program Refmac5 (CCP4 Software Suite), an experimentally determined structure factor Fo, a structure factor Fc calculated from the model, and a phase. Final refinement was performed using a program Refmac5 (CCP4 Software Suite) by the incorporation of Ca ion and water molecules into the model on the basis of the electron density maps of coefficients 2Fo-Fc and Fo-Fc. Finally, a crystallographic reliability factor R of 20.0% and a Free R value of 27.9% were obtained for the model of 3440 atoms by use of 21020 reflection data with a resolution of 25-2.2 angstroms.

Assay of X-ray diffraction data on crystal of 6RL#9 antibody Fab fragment obtained in absence of Ca

The structure of the crystal of the 6RL#9 antibody Fab fragment obtained in the absence of Ca was determined using the structure of the crystal, which was the same type thereas, obtained in the presence of Ca. Water and Ca ion molecules were excluded from the structure coordinate of the crystal of the 6RL#9 antibody Fab fragment obtained in the presence of Ca, followed by rigid body refinement moving each of the VH, VL, CH1, and CL domains to obtain a crystallographic reliability factor R of 30.3% and a Free R value of 31.7% for reflection data of 25-3.0 angstroms. In addition, the model was refined by model correction on a repetition program Coot (Paul Emsley) with reference to electron density maps of coefficients 2Fo-Fc and Fo-Fc calculated through the use of structure refinement using a program Refmac5 (CCP4 Software Suite), an experimentally determined structure factor Fo, a structure factor Fc calculated from the model, and a phase. Final refinement was performed using a program Refmac5 (CCP4 Software Suite) by the incorporation of water molecules into the model on the basis of the electron density maps of coefficients 2Fo-Fc and Fo-Fc. Finally, a crystallographic reliability factor R of 20.9% and a Free R value of 27.7% were obtained for the model of 3351 atoms by use of 18357 reflection data with a resolution of 25-2.3 angstroms.

X-ray diffraction data comparison between crystals of 6RL#9 antibody Fab fragment obtained in presence of Ca and in absence of Ca

As a result of structural comparison between the crystals of the 6RL#9 antibody Fab fragment obtained in the presence of Ca and in the absence of Ca, large change was seen in heavy chain CDR3. Figure 21 shows the structure of the heavy chain CDR3 of the 6RL#9 antibody Fab fragment determined by X-ray crystal structure analysis. Specifically, a calcium ion was present at the central portion of the heavy chain CDR3 loop portion in the crystal of the 6RL#9 antibody Fab fragment obtained in the presence of Ca. The calcium ion was considered to interact with amino acid residues 95, 96, and 100a (defined by the Kabat numbering) in the heavy chain CDR3. This suggested that, in the presence of Ca, the heavy chain CDR3 loop, which is important for antigen binding, is stabilized through binding to calcium to take a structure optimum for antigen binding. None of previous reports show that calcium binds to antibody heavy chain CDR3. This structure of antibody heavy chain CDR3 bound with calcium is a novel structure.

[Reference Example 9] Obtainment of antibody binding to IL-6 in Ca-dependent manner from human antibody library using phage display technique

Preparation of naive human antibody phage display library

A human antibody phage display library consisting of a plurality of phages displaying Fab domains having distinct human antibody sequences was constructed according to a method generally known to those skilled in the art using poly-A RNA prepared from human PBMC, commercially available human poly-A RNA, or the like as a template.

Obtainment of antibody fragment binding to antigen in Ca-dependent manner from library by bead panning

The first round of screening of the constructed naive human antibody phage display library was carried out by the enrichment of only antibody fragments having antigen (IL-6)-binding ability. The antigens used were biotin-labeled IL-6. Phages were produced from *Escherichia coli* harboring a phage display phagemid constructed. To the obtained culture fluid of *Escherichia coli* producing phage, 2.5 M NaCl/10%PEG was added to precipitate phages. The precipitate phages were diluted with TBS to obtain a phage library liquid. Then, to the phage library liquid, BSA and $CaCl_2$ were added so as to obtain a final BSA concentration of 4% and a calcium-ion concentration of 1.2 mM. Panning was performed with reference to a general method using an antigen immobilized to magnetic beads (J. Immunol. Methods. (2008) 332 (1-2), 2-9, J. Immunol. Methods. (2001) 247 (1-2), 191-203, Biotechnol. Prog. (2002) 18 (2) 212-20, Mol. Cell Proteomics (2003) 2 (2), 61-9). As the magnetic beads, NeutrAvidin coated beads (Sera-Mag SpeedBeads NeutrAvidin-coated) or Streptavidin coated beads (Dynabeads M-280 Streptavidin) were used.

Specifically, 250 pmol of biotin-labeled antigens was added to the prepared phage library solution and thereby contacted with the phage library solution at room temperature for 60 minutes. After addition of BSA-blocked magnetic beads, the antigen-phage complexes were attached to the magnetic beads at room temperature for 15 minutes. The beads were washed three times with 1.2 mM $CaCl_2$/TBST (TBST containing 1.2 mM $CaCl_2$) and then further washed twice with 1 mL of 1.2 mM $CaCl_2$/TBS (TBS containing 1.2 mM $CaCl_2$). After addition of 0.5 mL of 1 mg/mL trypsin, the beads were suspended at room temperature for 15 minutes, immediately after which the beads were separated using a magnetic stand to recover a phage solution. The recovered phage solution was added to 10 mL of an *E. coli* strain TG1 in a logarithmic growth phase (OD600: 0.4-0.7). The *E. coli* strain was infected by the phages through the gentle spinner culture of the strain at 37°C for 1 hour. The infected *E. coli* was inoculated to a plate of 225 mm x 225 mm. Next, phages were recovered from cultures of the inoculated *E. coli* to prepare a phage library solution.

In the second and subsequent rounds of panning, the phages were enriched with Ca-dependent binding ability as an index. Specifically, 40 pmol of biotin-labeled antigens was added to the prepared phage library solution and thereby contacted with the phage library solution at room temperature for 60 minutes. After addition of BSA-blocked magnetic beads, the antigen-phage complexes were attached to the magnetic beads at room temperature for 15 minutes. The beads were washed with 1 mL of 1.2 mM $CaCl_2$/TBST and 1.2 mM $CaCl_2$/TBS. Then, the beads supplemented with 0.1 mL of 2 mM EDTA/TBS were suspended at room temperature. Immediately thereafter, the beads were separated using a magnetic stand to recover a phage solution. The addition of 5 $\mu$L of 100 mg/mL trypsin to the recovered phage solution cleaved the pIII proteins (helper phage-derived pIII proteins) of non-Fab-displaying phages to cancel the ability of the non-Fab-displaying phages to infect *E. coli.* The phages recovered from the trypsin-treated phage solution were added to 10 mL of an *E. coli* strain TG1 in a logarithmic growth phase (OD600: 0.4-0.7). The *E. coli* strain was infected by the phages through the gentle spinner culture of the strain at 37°C for 1 hour. The infected *E. coli* was inoculated to a plate of 225 mm x 225 mm. Next, phages were recovered from cultures of the inoculated *E. coli* to recover a phage library solution. This panning with Ca-dependent binding ability as an index was performed 3 rounds in total.

Evaluation by phage ELISA

A phage-containing culture supernatant was recovered according to a conventional method (Methods Mol. Biol. (2002) 178, 133-145) from each single colony of the *E. coli* obtained by the above method. After addition of BSA and $CaCl_2$ (final concentration: 4% BSA and 1.2 mM calcium ion), the phage-containing culture supernatant was subjected to ELISA by the following procedures: StreptaWell 96 microtiter plate (F. Hoffmann-La Roche Ltd.) was coated overnight with 100 $\mu$L of PBS containing biotin-labeled antigens. Each well of the plate was washed with PBST to remove unbound antigens. Then, the well was blocked with 250 $\mu$L of 4% BSA-TBS for 1 hour or longer. After removal of 4% BSA-TBS, the prepared culture supernatant was added to each well, and the plate was left standing at 37°C for 1 hour to associate phage-displayed antibodies with the antigens contained in each well. Each well was washed with 1.2 mM $CaCl_2$/TBST, and 1.2 mM $CaCl_2$/TBS or 1 mM EDTA/TBS was added thereto. The plate was left standing at 37°C for 30 minutes for incubation. After washing with 1.2 mM $CaCl_2$/TBST, HRP-conjugated anti-M13 antibodies (Amersham Pharmacia Biotech Inc.) diluted with TBS having 4% BSA and an ionized calcium concentration of 1.2 mM (all were indicated by final

concentrations) were added to each well. The plate was incubated for 1 hour. After washing with 1.2 mM $CaCl_2$/TBST, TMB single solution (ZYMED Laboratories, Inc.) was added to the well. The chromogenic reaction of the solution in each well was terminated by the addition of sulfuric acid. Then, the developed color was assayed on the basis of absorbance at 450 nm.

A 6KC4-1#85 antibody having Ca-dependent IL-6-binding ability was obtained by phage ELISA using 96 isolated clones. The gene of the antibody fragment judged as having Ca-dependent antigen-binding ability as a result of the phage ELISA was amplified as a template using specific primers and then analyzed for its nucleotide sequence. The sequence of the heavy chain variable region of the 6KC4-1#85 antibody is shown in SEQ ID NO: 8, and the sequence of the light chain variable region thereof is shown in SEQ ID NO: 43. A polynucleotide encoding the heavy chain variable region (SEQ ID NO: 8) of the 6KC4-1#85 antibody was linked by PCR to a polynucleotide encoding an IgG1-derived sequence. The resulting DNA fragment was incorporated into vectors for expression in animal cells to construct vectors that permits expression of a heavy chain represented by SEQ ID NO: 44. A polynucleotide encoding the light chain variable region (SEQ ID NO: 43) of the 6KC4-1#85 antibody was linked by PCR to a polynucleotide encoding a natural kappa chain constant region (SEQ ID NO: 42). The resulting DNA fragment encoding the sequence represented by SEQ ID NO: 45 was incorporated into vectors for expression in animal cells. The sequence of the prepared modified form was confirmed by a method generally known to those skilled in the art. The sequence of the prepared modified form was confirmed by a method generally known to those skilled in the art.

Antibody expression and purification

The gene of the clone 6KC4-1#85 judged as having Ca-dependent antigen-binding ability as a result of the phage ELISA was introduced to plasmids for expression in animal cells. Antibody expression was performed by the following method: a human embryonic kidney cell-derived FreeStyle 293-F line (Invitrogen Corp.) was suspended in FreeStyle 293 Expression Medium (Invitrogen Corp.). The suspension having a cell density of 1.33 x $10^6$ cells/mL was inoculated at a concentration of 3 mL/well to a 6-well plate. The prepared plasmids were transferred to the cells by lipofection. The cells were cultured for 4 days in a $CO_2$ incubator (37°C, 8% $CO_2$, 90 rpm). Antibodies were purified from the obtained culture supernatant by a method generally known to those skilled in the art using rProtein A Sepharose(TM) Fast Flow (Amersham Biosciences, Inc.). The absorbance of the purified antibody solution was measured at 280 nm using a spectrophotometer. The antibody concentration was calculated from the obtained measurement value by use of an extinction coefficient calculated by PACE (Protein Science (1995) 4, 2411-2423).

[Reference Example 10] Evaluation of 6KC4-1#85 antibody for its calcium ion binding

The calcium-dependent antigen-binding antibody 6KC4-1#85 obtained from the human antibody library was evaluated for its calcium binding. Whether or not a measured Tm value varied under different ionized calcium concentrations was evaluated by the method described in Reference Example 6.

Table 11 shows the Tm value of the Fab domain of the 6KC4-1#85 antibody. As shown in Table 11, the Tm value of the Fab domain of the 6KC4-1#85 antibody varied depending on calcium ion concentration, demonstrating that the 6KC4-1#85 antibody binds to calcium.

[Table 11]

| Antibody | Calcium-ion concentration | | ΔTm (°C) |
|---|---|---|---|
| | 3 μM | 2 mM | 2 mM - 3 μM |
| 6KC4-1#85 | 71.49 | 75.39 | 3.9 |

Identification of calcium ion-binding site of 6KC4-1#85 antibody

In Example 10, it was shown that 6KC4-1#85 antibody binds to a calcium ion; however, 6KC4-1#85 does not have a calcium-binding motif such as an hVk5-2 sequence. Then, to identify the residue of the 6KC4-1#85 antibody to which a calcium ion binds, the Asp (D) residue present in CDR of the 6KC4-1#85 antibody was substituted with Ala (A) residue, which is not involved in binding or chelating of a calcium ion, to prepare modified heavy chains (6_H1-11 (SEQ ID NO: 46), 6_H1-12 (SEQ ID NO: 47), 6_H1-13 (SEQ ID NO: 48), 6_H1-14 (SEQ ID NO: 49), 6_H1-15 (SEQ ID NO: 50)) and modified light chains (6_L1-5 (SEQ ID NO: 51) and 6_L1-6 (SEQ ID NO: 52)). A modified antibody gene was introduced into an expression vector, which was further introduced into an animal cell. The animal cell was cultured. From the culture fluid, a modified antibody was purified in accordance with the method described in Example 4. Calcium binding of the modified antibody purified was determined by the method described in Example 6. The measurement

results are shown in Table 12.

[Table 12]

| Heavy chain | Light chain | Modified residue | Calcium-ion concentration | | ΔTm (°C) |
|---|---|---|---|---|---|
| | | | 3 μM | 2 mM | 2 mM - 3 μM |
| 6KC4-1#85 | 6KC4-1#85 | Wild type | 71.49 | 75.39 | 3.9 |
| 6H1-11 | 6KC4-1#85 | H-chain position 61 (Kabat numbering) | 71.73 | 75.56 | 3.83 |
| 6H1-12 | 6KC4-1#85 | H-chain position 95 (Kabat numbering) | 72.9 | 73.43 | 0.53 |
| 6H1-13 | 6KC4-1#85 | H-chain position 100a (Kabat numbering) | 70.94 | 76.25 | 5.31 |
| 6H1-14 | 6KC4-1#85 | H-chain position 100g (Kabat numbering) | 73.95 | 75.14 | 1.19 |
| 6H1-15 | 6KC4-1#85 | H-chain position 101 (Kabat numbering) | 65.37 | 66.25 | 0.87 |
| 6KC4-1#85 | 6L1-5 | L-chain position 50 (Kabat numbering) | 71.92 | 76.08 | 4.16 |
| 6KC4-1#85 | 6L1-6 | L-chain position 92 (Kabat numbering) | 72.13 | 78.74 | 6.61 |

As shown in Table 12, 6KC4-1#85 antibody loses a calcium binding ability by substituting position 95, or position 101 (Kabat numbering) of heavy-chain CDR3 of the 6KC4-1#85 antibody with An Ala residue. From this, it is considered that this residue plays an important role in binding calcium. It was elucidated, from the calcium binding ability of a modified 6KC4-1#85 antibody, that the calcium-binding motif present in the vicinity of the base of a loop of heavy chain CDR3 of the 6KC4-1#85 antibody can be also used as a calcium-binding motif in the antigen-binding domain contained in the antigen-binding molecule of the present invention.

[Reference Example 11] Search for human genital cell lineage sequence binding to calcium ion

Acquisition of human genital cell lineage sequence

An antibody containing a human genital cell lineage sequence and capable of binding to a calcium ion has not yet been reported up to present. Then, to determine whether an antibody containing a human genital cell lineage sequence binds to a calcium ion or not, the genital cell lineage sequence of an antibody containing a human genital cell lineage sequence was cloned by using cDNA prepared from a Human Fetal Spleen Poly RNA (Clontech) as a template. The cloned DNA fragment was inserted into an animal cell expression vector. The nucleotide sequence of the obtained expression vector was determined in accordance with a method known to those skilled in the art. The number of the sequences are shown in Table 13. The polynucleotides encoding SEQ ID NO: 53 (Vk1), SEQ ID NO: 54 (Vk2), SEQ ID NO: 55 (Vk3), SEQ ID NO: 56 (Vk4) and SEQ ID NO: 6 (Vk5-2) were each ligated to a constant region (SEQ ID NO: 42) encoding a natural Kappa chain by a PCR method. The resultant DNA fragment was integrated into an animal cell expression vector. Furthermore, polynucleotides encoding SEQ ID NO: 57 (Vk1), SEQ ID NO: 58 (Vk2), SEQ ID NO: 59 (Vk3), SEQ ID NO: 60 (Vk4) and SEQ ID NO: 61 (Vk5) were each ligated to a polynucleotide encoding IgG1 (SEQ ID NO: 9) having 2 amino-acid deletion at the C terminal by a PCR method. The resultant DNA fragment was inserted into an animal cell expression vector. The sequences of the modified forms thus prepared were confirmed by a method known to those skilled in the art.

[Table 13]

| Light chain germline sequence | Heavy chain variable region sequence No. | Light chain variable region sequence No. |
|---|---|---|
| Vk1 | 57 | 53 |
| Vk2 | 58 | 54 |
| Vk3 | 59 | 55 |
| Vk4 | 60 | 56 |
| Vk5 | 61 | 6 |

Expression and purification of antibody

The obtained five DNA fragments having a human genital cell lineage sequence inserted therein were each inserted into an animal cell expression vector, which was then inserted into an animal cell. The antibody was expressed by the following method. A human fetus kidney cell-derived FreeStyle 293-F strain (Invitrogen) was suspended in FreeStyle 293 Expression Medium (Invitrogen). 3 mL of the suspension solution was seeded in each well of a 6 well plate at a cell density of $1.33 \times 10^6$ cells/mL. The plasmid prepared was introduced into the cell by a lipofection method. Culture was performed in a $CO_2$ incubator (37°C, 8%$CO_2$, 90 rpm) for 4 days. From the obtained culture supernatant, an antibody was purified by use of rProtein A SepharoseTM Fast Flow (Amersham Biosciences) in accordance with a method known to those skilled in the art. The absorbance of a solution of the purified antibody was measured by a spectrophotometer. From the obtained measurement value, an antibody concentration was calculated by use of the absorption constant calculated by the PACE method (Protein Science (1995) 4, 2411-2423).

Evaluation of calcium ion-binding activity of antibody containing a human genital cell lineage sequence

The calcium ion-binding activity of the purified antibodies was evaluated. The purified antibodies were each subjected to dialysis (EasySEP, TOMY) treatment using a solution containing 20 mM Tris-HCl, 150 mM NaCl, 2 mM $CaCl_2$ (pH 7.4) or a solution containing 20 mM Tris-HCl, 150 mM NaCl, 3 $\mu$M$CaCl_2$ (pH 7.4) as an external solution. An antibody solution, which was prepared by use of the solution used in dialysis so as to obtain a concentration of approximately 0.1 mg/mL, was used as a test subject, DSC measurement was performed by raising temperature from 20°C up to 115°C at a rate of 240°C/hr. The thermal denaturation intermediate temperature (Tm value) of the Fab domain of each antibody was obtained based on the DSC denaturation curve and shown in Table 14.

[Table 14]

| Light chain germline sequence | Calcium-ion concentration | | ΔTm (°C) |
|---|---|---|---|
| | 3 μM | 2 mM | 2 mM - 3 μM |
| Vk1 | 80.32 | 80.78 | 0.46 |
| Vk2 | 80.67 | 80.61 | -0.06 |
| Vk3 | 81.64 | 81.36 | -0.28 |
| Vk4 | 70.74 | 70.74 | 0 |
| Vk5 | 71.52 | 74.17 | 2.65 |

As a result, the Tm values of Fab domains of the antibodies containing hVk1, hVk2, hVk3 and hVk4 sequences, respectively, did not change even if the calcium-ion concentrations of the solutions containing the Fab domains changed. In contrast, the Tm value of the Fab domain of the antibody containing hVk5 sequence changed depending upon the calcium-ion concentration of the solution of the antibody containing the Fab domain. From this, it was demonstrated that the hVk5 sequence binds to a calcium ion.

[Reference Example 12] Evaluation of human Vk5 (hVk5) sequence

hVk5 Sequence

In the Kabat database, an hVk5-2 sequence alone is registered as the hVk5 sequence. Hereinafter, hVk5 and hVk5-2 are regarded as being identical in the following description.

Construction, expression and purification of hVk5-2 sequence without addition of sugar chain

The hVk5-2 sequence has an N-linked sugar chain, which is added to the amino acid at 20-position (Kabat numbering). Since the sugar chain added to the protein is heterogeneous, it is desirable that a sugar chain is not added in view of homogeneity of a substance. Then, a modified hVk5-2_L65 (SEQ ID NO: 62) was prepared by substituting the Asn (N) residue at position 20 (Kabat numbering) by a Thr (T) residue. An amino acid was substituted in accordance with a method known to those skilled in the art using a QuikChange Site-Directed Mutagenesis Kit (Stratagene). DNA encoding modified hVk5-2_L65 was integrated into an animal expression vector. The animal expression vector was introduced into an animal cell together with an animal expression vector to which a heavy chain i.e., CIM_H (SEQ ID

NO: 63) is integrated so as to express this sequence, in accordance with the method described in Reference Example 4. An antibody having hVk5-2_L65 and CIM_H expressed therein in the animal cell was purified by the method described in Reference Example 4.

Evaluation of physical properties of an antibody containing hVk5-2 sequence without addition of sugar chain

Whether the degree of heterogeneity of the obtained antibody containing the modification sequence hVk5-2_L65 is low or not compared to that of the antibody containing the original hVk5-2 sequence (subjected to modification) was analyzed by ion exchange chromatography. The details of ion exchange chromatography are shown in Table 15. As a result of the analysis, it was shown that the degree of heterogeneity of hVk5-2_L65 (modified at a glycosylation site) is low compared to that of the original hVk5-2 sequence, as shown in Figure 22.

[Table 15]

| | Conditions |
|---|---|
| Column | TOSOH TSKgel DEAE-NPR |
| Mobile phase | A; 10 mM Tris-HCl, 3 μM $CaCl_2$ (pH8.0) |
| | B; 10 mM Tris-HCl, 500 mM NaCl, 3 μM $CaCl_2$ (pH8.0) |
| Gradient schedule | %B = 0 - (5min) - 0 - 2%/1min |
| Column temperature | 40°C |
| Detection | 280 nm |
| Injection amount | 100 μL (5 μg) |

Whether an antibody containing an hVk5-2_L65 sequence having a low degree of heterogeneity binds to a calcium ion or not was evaluated by the method described in Reference Example 6. As a result, as shown in Table 16, the Tm value of a Fab domain of an antibody containing hVk5-2_L65 having a modified glycosylation site varies depending upon a change of the calcium-ion concentration of an antibody solution. More specifically, it was shown that a calcium ion bind to the Fab domain of the antibody containing hVk5-2_L65 (having a modified glycosylation site).

[Table 16]

| Light chain | Glycosylation sequence | Calcium-ion concentration | | ΔTm (°C) 2 mM - 3 μM |
|---|---|---|---|---|
| | | 3 μM | 2 mM | |
| hVk5-2 | Present | 71.52 | 74.17 | 2.65 |
| hVk5-2_L65 | Absent | 71.51 | 73.66 | 2.15 |

[Reference Example 13] Evaluation of binding activity of calcium ion to an antibody molecule containing CDR sequence of hVk5-2 sequence

Preparation, expression and purification of modified antibody containing CDR sequence of hVk5-2 sequence

The hVk5-2_L65 sequence is a sequence in which an amino acid of a glycosylation site present in a framework of human Vk5-2 sequence is modified. Reference Example 12 shows that even if the glycosylation site is modified, a calcium ion can bind. In general, a framework sequence is desirably a genital cell lineage sequence in view of immunogenicity. Then, whether or not the framework sequence of the antibody can be converted to the framework sequence of a genital cell lineage sequence (to which a sugar chain cannot be added), while maintaining the binding activity of a calcium ion to the antibody, was investigated.

Polynucleotides encoding sequences, namely, CaVk1 (SEQ ID NO: 64), CaVk2 (SEQ ID NO: 65), CaVk3 (SEQ ID NO: 66), CaVk4 (SEQ ID NO: 67) (which were prepared by replacing the framework sequence of the hVk5-2 sequence chemically synthesized with hVk1, hVk2, hVk3 and hVk4 sequences, respectively), were each ligated to a polynucleotide encoding a constant region (SEQ ID NO: 42) of a natural Kappa chain by a PCR method to prepare a DNA fragment. These DNA fragments were each integrated into an animal cell expression vector. The sequence of the modified form prepared was determined by a method known to those skilled in the art. The plasmid prepared as mentioned above was

introduced into an animal cell together with a plasmid, in which a polynucleotide encoding CIM_H (SEQ ID NO: 63) was integrated, in accordance with the method described in Reference Example 4. The animal cell was cultured and a desired antibody molecule was expressed. From the culture fluid, the desired antibody molecule was purified.

Evaluation of calcium ion-binding activity of modified antibody containing CDR sequence of hVk5-2 sequence

Whether or not a calcium ion binds to a modified antibody, which contains a framework sequence of a genital cell lineage sequence (hVk1, hVk2, hVk3, hVk4) except an hVk5-2 sequence and a modified antibody containing a CDR sequence of the hVK5-2 sequence, was evaluated by the method described in Reference Example 6. The evaluation results are shown in Table 17. It was shown that the Tm value of the Fab domain of each modified antibody changes depending upon a change of the calcium-ion concentration of an antibody solution. Accordingly, it was demonstrated that antibodies having framework sequences other than the framework sequence of the hVk5-2 sequence can bind to a calcium ion.

[Table 17]

| Germline (light chain framework sequence) | Calcium-ion concentration | | ΔTm (°C) |
|---|---|---|---|
| | 3 μM | 2 mM | 2 mM - 3 μM |
| hVk1 | 77.51 | 79.79 | 2.28 |
| hVk2 | 78.46 | 80.37 | 1.91 |
| hVk3 | 77.27 | 79.54 | 2.27 |
| hVk4 | 80.35 | 81.38 | 1.03 |
| hVk5-2 | 71.52 | 74.17 | 2.65 |

It was further elucidated that the thermal denaturation temperature (Tm value, serving as an index of thermal stability of the F domain) of each of the antibodies modified (so as to contain framework sequences of genital cell lineage sequences (hVk1, hVk2, hVk3, hVk4) except an hVk5-2 sequence) and an antibody modified so as to contain a CDR sequence of an hVK5-2 sequence, is higher than the Tm value of the F domain of an antibody containing the original hVk5-2 sequence. From the result, it was found that the antibodies containing the framework sequences hVk1, hVk2, hVk3, and hVk4 and the antibody containing a CDR sequence of an hVk5-2 sequence have a property of binding to a calcium ion and are excellent molecules in view of thermal stability.

[Reference Example 14] Identification of calcium ion-binding site present in human genital cell lineage hVk5-2 sequence

Designing of mutation site in CDR sequence of hVk5-2 sequence

As described in Reference Example 13, it was shown that an antibody containing a light chain, in which the CDR sequence of the hVk5-2 sequence is introduced into a framework sequence of a different genital cell lineage, can bind to a calcium ion. From the result, it was suggested that the calcium ion-binding site present within hVk5-2 is present in CDR. As an amino acid binding to a calcium ion, more specifically, chelating a calcium ion, an amino acid negatively charged or an amino acid that will serve as an acceptor for a hydrogen bond is mentioned. Then, whether or not an antibody containing a mutated hVk5-2 sequence (prepared by substituting an Asp (D) residue or a Glu (E) residue present in CDR sequence of the hVk5-2 sequence with an Ala (A) residue) binds to a calcium ion was evaluated.

Preparation of an Ala-substituted hVk5-2 sequence and expression and purification of antibody

An antibody molecule containing a light chain, which has a modification of an Asp and/or a Glu residue (present in the CDR sequence of an hVk5-2 sequence) into an Ala residue, was prepared. As described in Reference Example 12, the modified hVk5-2_L65 form (to which no sugar chain is added) maintained a calcium ion-binding ability. From this, it is considered that the modified hVk5-2_L65 form is equivalent to the hVk5-2 sequence in calcium ion-binding ability. In this Reference Example, an amino acid was substituted by using the hVk5-2_L65 form as a template sequence. The modified antibodies prepared are shown in Table 18. Amino acid substitution was performed by use of a QuikChange Site-Directed Mutagenesis Kit (Stratagene), PCR or In fusion Advantage PCR cloning kit (TAKARA) etc., in accordance with a method known to those skilled in the art to construct an expression vector having a modified light chain having an amino acid substitution.

[Table 18]

| Name of light chain modified form | Modified site (Kabat numbering) | SEQ ID NO |
|---|---|---|
| hVk5-2_L65 | Wild type | 62 |
| hVk5-2_L66 | 30 | 68 |
| hVk5-2_L67 | 31 | 69 |
| hVk5-2_L68 | 32 | 70 |
| hVk5-2_L69 | 50 | 71 |
| hVk5-2_L70 | 30, 32 | 72 |
| hVk5-2_L71 | 30, 50 | 73 |
| hVk5-2_L72 | 30, 32, 50 | 74 |
| hVk5-2_L73 | 92 | 75 |

The nucleotide sequences of the obtained expression vectors were determined in accordance with a method known to those skilled in the art. The expression vector of a modified light chain was prepared and temporarily inserted together with an expression vector of heavy chain CIM_H (SEQ ID NO: 63) into a human fetus kidney cancer cell-derived HEK293H strain (Invitrogen) or FreeStyle 293 cell (Invitrogen) to express an antibody. From the obtained culture supernatant, the antibody was purified by use of rProtein A Sepharose Fast Flow (GE Healthcare) in accordance with a method known to those skilled in the art. Absorbance of the purified antibody solution was measured at 280 nm by a spectrophotometer. From the obtained measurement value, the concentration of the antibody was computationally obtained by use of an absorption constant calculated by a PACE method (Protein Science (1995) 4, 2411-2423).

Evaluation of calcium ion-binding activity of antibody having Ala substitution in hVk5-2 sequence

Whether the purified antibody obtained binds to a calcium ion or not was determined by the method described in Reference Example 16. The results are shown in Table 19. Even though an Asp or a Glu residue is substituted with an Ala residue (which cannot be involved in binding or chelating calcium ion) present in the CDR sequence of an hVk5-2 sequence, there was an antibody having a Fab domain whose Tm value does not change even if the calcium-ion concentration of an antibody solution changes. It was shown that the Ala substitution sites (position 32 and position 92 (Kabat numbering)), whose substitution does not change the Tm value, are particularly important for binding between a calcium ion and the antibody.

[Table 19]

| Name of light chain modified form | Modified site (Kabat numbering) | Calcium-ion concentration | | ΔTm (°C) 2 mM-0 mM |
|---|---|---|---|---|
| | | 0 mM | 2 mM | |
| hVk5-2_L65 | Wild type | 71.71 | 73.69 | 1.98 |
| hVk5-2_L66 | 30 | 71.65 | 72.83 | 1.18 |
| hVk5-2_L67 | 31 | 71.52 | 73.30 | 1.78 |
| hVk5-2_L68 | 32 | 73.25 | 74.03 | 0.78 |
| hVk5-2_L69 | 50 | 72.00 | 73.97 | 1.97 |
| hVk5-2_L70 | 30, 32 | 73.42 | 73.60 | 0.18 |
| hVk5-2_L71 | 30, 50 | 71.84 | 72.57 | 0.73 |
| hVk5-2_L72 | 30, 32, 50 | 75.04 | 75.17 | 0.13 |
| hVk5-2_L73 | 92 | 75.23 | 75.04 | -0.19 |

[Reference Example 15] Evaluation of calcium ion-binding activity of antibody containing a hVk1 sequence having a calcium ion-binding motif

Preparation of hVk1 sequence having a calcium ion-binding motif and expression and purification of antibody

From the results of the calcium binding activity of Ala substitution antibody described in Reference Example 14, it was shown that Asp and Glu residues in the CDR sequence of an hVk5-2 sequence are important for calcium binding. Then, only the residues of the position 30, position 31, position 32, position 50 and position 92 (Kabat numbering) were introduced into a variable region sequence of a different genital cell lineage, and then, whether the genital cell lineage can bind to a calcium ion or not was evaluated. To describe more specifically, the position 30, position 31, position 32, position 50 and position 92 (Kabat numbering) residues of the hVk1 sequence (human genital cell lineage sequence) were substituted with the position 30, position 31, position 32, position 50 and position 92 (Kabat numbering) of the hVk5-2 sequence to prepare a modified antibody LfVk1_Ca (SEQ ID NO: 76). More specifically, whether or not an antibody containing an hVk1 sequence to which there five residues alone of the hVk5-2 sequence were introduced can bind to calcium, was evaluated. The modified antibody was prepared in the same manner as in Reference Example 4. The obtained LfVk1_Ca having a light chain modification and LfVk1 (SEQ ID NO: 77) containing a light-chain hVk1 sequence were expressed together with heavy chain CIM_H (SEQ ID NO: 63). The antibodies were expressed and purified in the same manner as in Example 14.

Evaluation of calcium ion-binding activity of antibody having human hVk1 sequence having a calcium ion-binding motif

Whether the purified antibodies obtained as mentioned above bind to a calcium ion or not was determined in the method described in Reference Example 6. The results are shown in Table 20. The Tm value of the Fab domain of the antibody containing LfVk1 having an hVk1 sequence does not change even if the calcium concentration of the antibody solution changes. In contrast, the Tm value of the antibody sequence containing LfVk1_Ca changes by 1°C or more when the calcium concentration of the antibody solution changes. From this, it was demonstrated that an antibody containing LfVk1_Ca binds to calcium. From the above results, it was demonstrated that the entire hVk5-2 CDR sequence is not required, only the residues introduced in constructing the LfVk1_Ca sequence are sufficient in binding to a calcium ion.

[Table 20]

| Name of light chain modified form | Calcium-ion concentration | | ΔTm (°C) |
|---|---|---|---|
| | 3 μM | 2 mM | 2 mM - 3 μM |
| LfVk1 | 83.18 | 83.81 | 0.63 |
| LfVk1_Ca | 79.83 | 82.24 | 2.41 |

[Reference Example 16] Evaluation of calcium binding of hVk5-2 variant sequence

Other than Vk5-2 (SEQ ID NO: 6), Vk5-2 variant 1 (SEQ ID NO: 78) and Vk5-2 variant 2 (SEQ ID NO: 79), which are classified into a Vk5-2 class, were obtained. These variants were evaluated for calcium binding. DNA fragments of Vk5-2, Vk5-2 variant 1 and Vk5-2 variant 2 were each integrated into an animal cell expression vector. The nucleotide sequences of the obtained expression vectors were determined in accordance with a method known to those skilled in the art. The animal cell expression vectors, in which the DNA fragments of Vk5-2, Vk5-2 variant 1 and Vk5-2 variant 2 were separately integrated were each introduced into an animal cell together with an animal expression vector in which a heavy chain, CIM_H (SEQ ID NO: 63) n was integrated such that the heavy chain can be expressed, by the method described in Reference Example 13 to produce an antibody. The obtained antibody was purified. The calcium ion-binding activity of the purified antibody was evaluated. The antibody purified was subjected to dialysis (EasySEP, TOMY) treatment using a solution containing 20 mM Tris-HCl, 150 mM NaCl, 2 mM $CaCl_2$ (pH 7.5) or 20 mM Tris-HCl, 150 mM NaCl (pH 7.5) (calcium-ion concentration is described as 0 mM in Table 21) as an external solution. An antibody solution was prepared with the solution used in dialysis so as to obtain concentration of 0.1 mg/mL and then the antibody solution (a test subject) was subjected to DSC measurement by raising temperature from 20°C to 115°C at a rate of 240°C/hr. The thermal denaturation intermediate temperature (Tm value) of a Fab domain of each antibody obtained was calculated based on the DSC denaturation curve and shown in Table 21.

[Table 21]

| Light chain | Calcium-ion concentration | | ΔTm (°C) |
|---|---|---|---|
| | 0 mM | 2 mM | 2 mM-0 mM |
| Vk5-2 | 71.65 | 74.38 | 2.73 |
| Vk5-2 variant 1 | 65.75 | 72.24 | 6.49 |
| Vk5-2 variant 2 | 66.46 | 72.24 | 5.78 |

As a result, the Tm values of Fab domains containing Vk5-2, Vk5-2 variant 1 and Vk5-2 variant 2, respectively, changed depending upon the calcium-ion concentration of the antibody solution containing the respective Fab domains. From this, it was shown that an antibody having a sequence classified into Vk5-2 binds to a calcium ion. It is elucidated that the calcium-binding motifs present in the sequences of Vk5-2 variant 1 and Vk5-2 variant 2 can be preferably used as a calcium-binding motif changing the binding activity of an antigen-binding domain to an antigen depending upon the ion-concentration condition of the present invention.

## Claims

1. A method for producing an antigen-binding molecule, comprising the following steps:

    (a) a step of providing a polypeptide sequence of an antigen-binding molecule comprising an antigen-binding domain and an FcRn binding domain,
    (b) a step of identifying an amino acid sequence serving as a candidate for a motif for a sugar chain receptor-binding domain in the polypeptide sequence,
    (c) a step of designing a motif for a sugar chain receptor-binding domain comprising an amino acid sequence having at least one amino acid different from the amino acid sequence identified in the step (b),
    (d) a step of preparing a gene encoding a polypeptide of an antigen-binding molecule comprising the motif for the sugar chain receptor-binding domain designed in the step (c), and
    (e) a step of recovering the antigen-binding molecule from a culture fluid of a host cell transformed with the gene obtained in the step (d).

2. The method according to claim 1, further comprising a step of treating the antigen-binding molecule obtained in the step (e) with an enzyme.

3. The production method according to claim 1 or 2, wherein a binding activity of the antigen-binding domain to an

antigen changes depending upon an ion-concentration condition.

4. The method according to claim 3, wherein the ion-concentration condition is a pH condition.

5. The method according to claim 4, wherein the antigen-binding domain has a higher binding activity to an antigen under a neutral pH range condition than a binding activity to the antigen under an acidic pH range condition.

6. The method according to claim 5, wherein the antigen-binding domain having a higher binding activity to the antigen under a neutral pH range condition than a binding activity to the antigen under an acidic pH range condition is provided by substituting at least one amino acid of the antigen-binding domain with an amino acid whose side chain has a pKa of 4.0-8.0 or by inserting at least one amino acid whose side chain has a pKa of 4.0-8.0 into the antigen-binding domain.

7. The method according to claim 3, wherein the ion-concentration condition is a calcium-ion concentration condition.

8. The method according to claim 7, wherein the antigen-binding domain has a higher binding activity to the antigen under a high calcium-ion concentration condition than a binding activity to the antigen under a low calcium-ion concentration condition.

9. The method according to claim 8, wherein the antigen-binding domain having a higher binding activity to the antigen under a high calcium-ion concentration condition than the binding activity to the antigen under a low calcium-ion concentration condition is provided by substituting at least one amino acid of the antigen-binding domain with a calcium-binding motif or by inserting a calcium-binding motif into the antigen-binding domain.

10. The method according to any one of claims 1 to 9, wherein the antigen-binding domain comprises a variable region of an antibody.

11. The method according to any one of claims 1 to 10, wherein the FcRn binding domain comprises an Fc region of an antibody.

12. The method according to claim 11, wherein the antibody is an IgG antibody.

13. The method according to claim 12, wherein the IgG antibody is IgG1, IgG2, IgG3 or IgG4.

14. The method according to any one of claims 1 to 12, wherein a binding activity of the sugar chain receptor-binding domain to a sugar chain receptor changes depending upon an ion-concentration condition.

15. The method according to claim 14, wherein the ion-concentration condition is a pH condition.

16. The method according to any one of claims 1 to 15, wherein a binding activity of the sugar chain receptor-binding domain to the sugar chain receptor under a neutral pH range condition is higher than a binding activity to the sugar chain receptor under an acidic pH range condition.

17. The method according to claim 14, wherein the ion-concentration condition is a calcium-ion concentration condition.

18. The method according to claim 17, wherein a binding activity of the sugar chain receptor-binding domain to the sugar chain receptor under a high calcium-ion concentration condition is higher than a binding activity to the sugar chain receptor under a low calcium-ion concentration condition.

19. The method according to any one of claims 1 to 18, wherein the sugar chain receptor-binding domain is a sugar chain.

20. The method according to claim 19, wherein the sugar chain is an O-linked sugar chain.

21. The method according to claim 19, wherein the sugar chain is an N-linked sugar chain.

22. The method according to claim 21, wherein designing a motif for the sugar chain receptor-binding domain includes designing a motif to which an N-linked sugar chain is added.

**23.** The method according to claim 21 or 22, wherein a terminal of the N-linked sugar chain comprises galactose.

**24.** The method according to claim 23, wherein a terminal of the N-linked sugar chain comprises three or more galactoses.

**25.** The method according to any one of claims 21 to 24, wherein the sugar chain receptor is an asialoglycoprotein receptor.

**26.** The method according to claim 21 or 22, wherein the terminal of the N-linked sugar chain comprises mannose.

**27.** The method according to claim 26, wherein the sugar chain receptor is a mannose receptor.

**28.** An antigen-binding molecule produced by a method according to any one of claims 1 to 27.

**29.** An antigen-binding molecule comprising an FcRn binding domain, an antigen-binding domain whose binding activity to an antigen changes depending upon an ion-concentration condition, and one or more sugar chain receptor-binding domains whose binding activity to a sugar chain receptor changes depending upon an ion-concentration condition.

**30.** The antigen-binding molecule according to claim 29, wherein a binding activity of the antigen-binding domain to the antigen changes depending upon a pH condition.

**31.** The antigen-binding molecule according to claim 30, wherein a binding activity of the antigen-binding domain to the antigen under a neutral pH range condition is higher than a binding activity to the antigen under an acidic pH range condition.

**32.** The antigen-binding molecule according to claim 31, wherein at least one amino acid of the antigen-binding domain includes at least one amino acid whose side chain has a pKa of 4.0-8.0.

**33.** The antigen-binding molecule according to claim 29, wherein a binding activity of the antigen-binding domain to the antigen changes depending upon the calcium-ion concentration condition.

**34.** The antigen-binding molecule according to claim 33, wherein a binding activity of the antigen-binding domain to the antigen under a high calcium-ion concentration condition is higher than a binding activity to the antigen under a low calcium-ion concentration condition.

**35.** The antigen-binding molecule according to claim 34, wherein at least one amino acid of the antigen-binding domain includes a calcium-binding motif.

**36.** The antigen-binding molecule according to any one of claims 29 to 35, wherein the antigen-binding domain comprises a variable region of an antibody.

**37.** The antigen-binding molecule according to any one of claims 29 to 36, wherein the FcRn binding domain comprises an Fc region of an antibody.

**38.** The antigen-binding molecule according to claim 37, wherein the antibody is an IgG antibody.

**39.** The antigen-binding molecule according to claim 38, wherein the IgG antibody is IgG1, IgG2, IgG3 or IgG4.

**40.** The antigen-binding molecule according to any one of claims 29 to 39, wherein a binding activity of the sugar chain receptor-binding domain to a sugar chain receptor changes depending upon an ion-concentration condition.

**41.** The antigen-binding molecule according to claim 40, wherein the ion-concentration condition is a pH condition.

**42.** The antigen-binding molecule according to claim 41, wherein a binding activity of the sugar chain receptor-binding domain to a sugar chain receptor under a neutral pH range condition is higher than a binding activity to the sugar chain receptor under an acidic pH range condition.

**43.** The antigen-binding molecule according to claim 40, wherein the ion-concentration condition is a calcium-ion concentration condition.

**44.** The antigen-binding molecule according to claim 43, wherein a binding activity of the sugar chain receptor-binding domain to a sugar chain receptor under a high calcium-ion concentration condition is higher than a binding activity to the sugar chain receptor under a low calcium-ion concentration condition.

**45.** The antigen-binding molecule according to any one of claims 29 to 44, wherein the sugar chain receptor-binding domain is a sugar chain.

**46.** The antigen-binding molecule according to claim 45, wherein the sugar chain is an O-linked sugar chain or an N-linked sugar chain.

**47.** The antigen-binding molecule according to claim 46, wherein the sugar chain receptor-binding domain includes a motif to which an N-linked sugar chain is bound.

**48.** The antigen-binding molecule according to claim 46 or 47, wherein a terminal of the N-linked sugar chain comprises galactose.

**49.** The antigen-binding molecule according to claim 48, wherein a terminal of the N-linked sugar chain comprises three or more terminal galactoses.

**50.** The antigen-binding molecule according to any one of claims 47 to 49, wherein the sugar chain receptor is an asialoglycoprotein receptor.

**51.** The antigen-binding molecule according to claim 46 or 47, wherein a terminal of the N-linked sugar chain comprises mannose.

**52.** The antigen-binding molecule according to claim 46 or 47, wherein the sugar chain receptor is a mannose receptor.

**53.** The antigen-binding molecule according to any one of claims 28 to 52, wherein the antigen-binding molecule is an antibody.

**54.** The antigen-binding molecule according to any one of claims 28 to 53, wherein the sugar chain receptor-binding domain is contained in the antigen-binding domain.

**55.** The antigen-binding molecule according to any one of claims 28 to 53, wherein the sugar chain receptor-binding domain is contained in the FcRn binding domain.

**56.** A pharmaceutical composition comprising an antigen-binding molecule according to any one of claims 28 to 55.

**57.** A method for allowing a cell expressing a sugar chain receptor to take up an antigen-binding molecule according to any one of claims 28 to 55 into the cell, comprising bringing the antigen-binding molecule into contact with the cell *in-vivo* or *ex-vivo.*

**58.** A method for allowing a cell expressing a sugar chain receptor to take up an antigen bound to an antigen-binding molecule according to any one of claims 28 to 55, comprising bringing the antigen-binding molecule into contact with the cell *in-vivo* or *ex-vivo.*

**59.** A method for increasing the number of antigens to which a single antigen-binding molecule according to any one of claims 28 to 55 binds, comprising bringing the antigen-binding molecule into contact with a cell expressing a sugar chain receptor, *in-vivo* or *ex-vivo.*

**60.** A method for decreasing the number of an antigen being present in an extracellular space, comprising bringing an antigen-binding molecule according to any one of claims 28 to 55 into contact with a cell expressing a sugar chain receptor, *in-vivo* or *ex-vivo.*

**61.** The method according to claim 58, wherein the extracellular space is plasma.

**62.** A method for improving the pharmacokinetics of an antigen-binding molecule according to any one of claims 28 to 55, comprising bringing the antigen-binding molecule into contact with a cell expressing a sugar chain receptor, *in-*

*vivo.*

**63.** A method for promoting dissociation of an antigen bound extracellularly to an antigen-binding molecule according to any one of claims 28 to 55 from the antigen-binding molecule, comprising bringing the antigen-binding molecule into contact with a cell expressing a sugar chain receptor, *in-vivo* or *ex-vivo.*

**64.** A method selected from the following methods:

(i) a method for promoting uptake of an antigen-binding molecule into a cell expressing the sugar chain receptor, *in-vivo* or *ex-vivo;*
(ii) a method for promoting uptake of an antigen bound to an antigen-binding molecule into a cell expressing a sugar chain receptor, *in-vivo* or *ex-vivo;*
(iii) a method for increasing the number of an antigen to which a single antigen-binding molecule binds, *in-vivo* or *ex-vivo;*
(iv) a method for enhancing potency of an antigen-binding molecule to clear an antigen, *in-vivo* or *ex-vivo;*
(v) a method for improving the pharmacokinetics of an antigen-binding molecule; or
(vi) a method for promoting dissociation of an antigen which has bound extracellularly to an antigen-binding molecule from the antigen-binding molecule;

the method comprising, in an antigen-binding molecule comprising an antigen-binding domain, an FcRn binding domain and one or more binding domains to a sugar chain receptor, increasing the number of the binding domains to the sugar chain receptor.

**65.** The method according to claim 64, wherein a binding activity of the antigen-binding molecule to an antigen changes depending upon an ion-concentration condition.

**66.** The method according to claim 64, wherein a binding activity of the antigen-binding domain to an antigen changes depending upon a pH condition.

**67.** The method according to claim 66, wherein a binding activity of the antigen-binding domain to an antigen under a neutral pH range condition is higher than a binding activity to the antigen under an acidic pH range condition.

**68.** The method according to claim 67, wherein at least one amino acid of the antigen-binding domain includes at least one amino acid whose side chain has a pKa of 4.0-8.0.

**69.** The method according to claim 64, wherein a binding activity of the antigen-binding domain to an antigen changes depending upon a calcium-ion concentration condition.

**70.** The method according to claim 69, wherein a binding activity of the antigen-binding domain to an antigen under a high calcium-ion concentration condition is higher than a binding activity to the antigen under a low calcium-ion concentration condition.

**71.** The method according to claim 70, wherein at least one amino acid of the antigen-binding domain includes a calcium-binding motif.

**72.** The method according to any one of claims 64 to 71, wherein the antigen-binding domain comprises a variable region of an antibody.

**73.** The method according to any one of claims 64 to 72, wherein the FcRn binding domain comprises an Fc region of an antibody.

**74.** The method according to claim 73, wherein the antibody is an IgG antibody.

**75.** The method according to claim 74, wherein the IgG antibody is IgG1, IgG2, IgG3 or IgG4.

**76.** The method according to any one of claims 64 to 75, wherein a binding activity of the sugar chain receptor-binding domain to a sugar chain receptor changes depending upon an ion-concentration condition.

**77.** The method according to claim 76, wherein the ion-concentration condition is a pH condition.

**78.** The method according to claim 76, wherein a binding activity of the sugar chain receptor-binding domain to a sugar chain receptor under a neutral pH range condition is higher than a binding activity to the sugar chain receptor under an acidic pH range condition.

**79.** The method according to claim 76, wherein the ion-concentration condition is a calcium-ion concentration condition.

**80.** The method according to claim 79, wherein a binding activity of the sugar chain receptor-binding domain to a sugar chain receptor under a high calcium-ion concentration condition is higher than a binding activity to the sugar chain receptor under a low calcium-ion concentration condition.

**81.** The method according to any one of claims 64 to 80, wherein the sugar chain receptor-binding domain is a sugar chain.

**82.** The method according to claim 81, wherein the sugar chain is an O-linked sugar chain.

**83.** The method according to claim 81, wherein the sugar chain is an N-linked sugar chain.

**84.** The method according to claim 83, wherein the sugar chain receptor-binding domain comprises a motif to which an N-linked sugar chain is bound.

**85.** The method according to claim 83 or 84, wherein a terminal of the N-linked sugar chain comprises galactose.

**86.** The method according to claim 85, wherein a terminal of the N-linked sugar chain comprises three or more terminal galactoses.

**87.** The method according to any one of claims 84 to 86, wherein the sugar chain receptor is an asialoglycoprotein receptor.

**88.** The method according to claim 83 or 84, wherein a terminal of the N-linked sugar chain comprises mannose.

**89.** The method according to claim 83 or 84, wherein the sugar chain receptor is a mannose receptor.

**90.** The method according to any one of claims 64 to 89, wherein the antigen-binding molecule is an antibody.

**91.** The method according to any one of claims 64 to 90, wherein the sugar chain receptor-binding domain is contained in the antigen-binding domain.

**92.** The method according to any one of claims 64 to 90, wherein the sugar chain receptor-binding domain is contained in the FcRn binding domain.

FIG.1

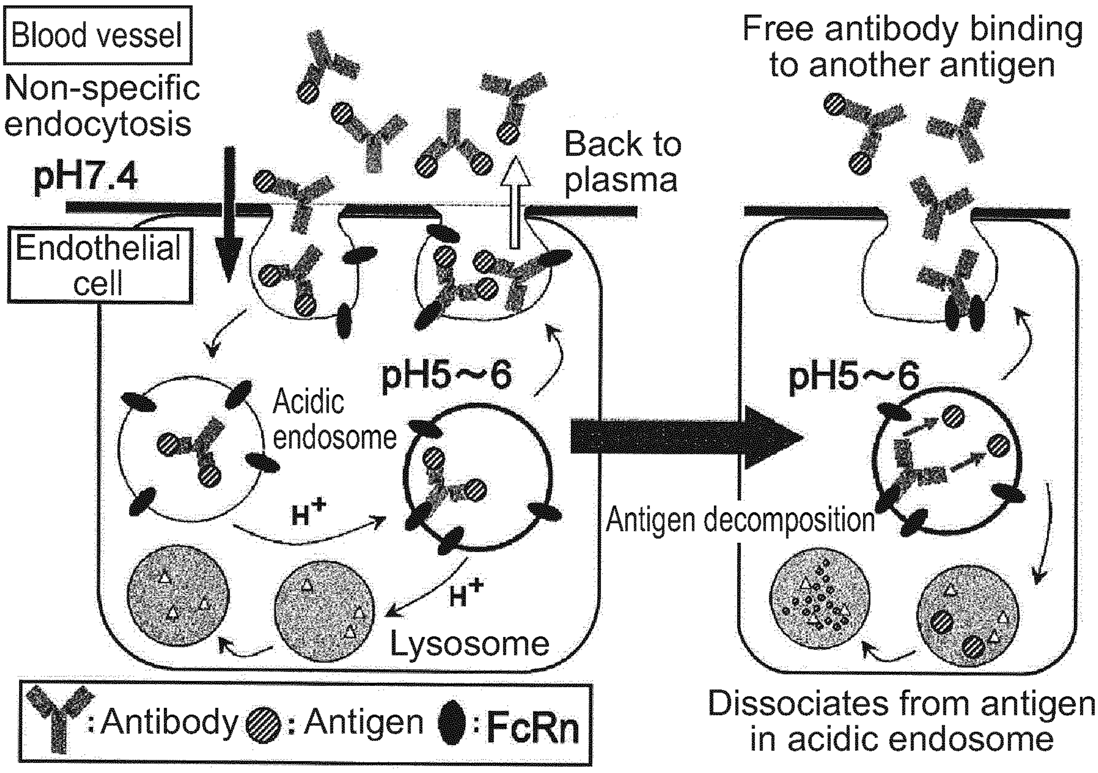
Blood vessel
Non-specific endocytosis
pH7.4
Endothelial cell
Back to plasma
Free antibody binding to another antigen
pH5~6
Acidic endosome
H+
Lysosome
Antigen decomposition
pH5~6
Dissociates from antigen in acidic endosome
: Antibody
: Antigen
: FcRn

FIG.2

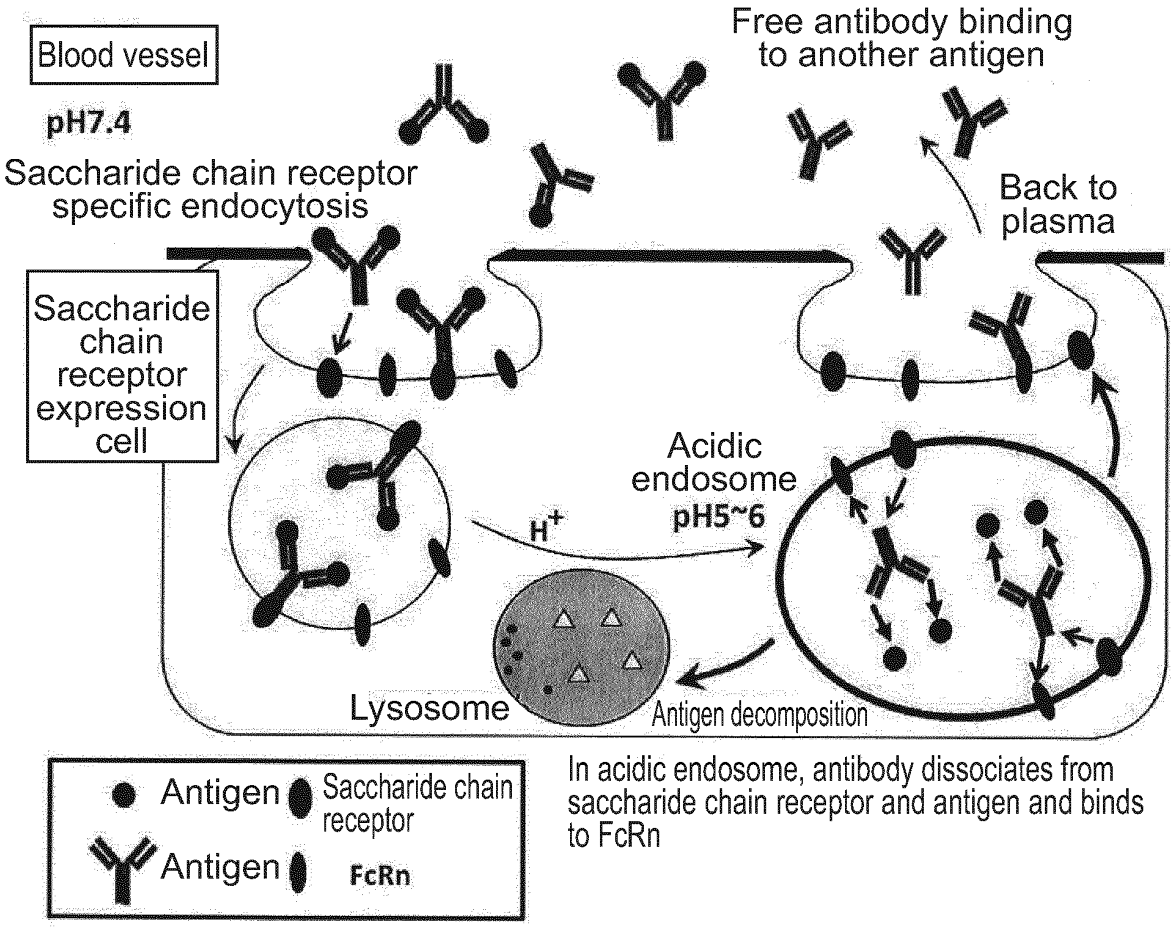
Blood vessel
pH7.4
Saccharide chain receptor specific endocytosis
Free antibody binding to another antigen
Back to plasma
Saccharide chain receptor expression cell
Acidic endosome
pH5~6
$H^+$
Lysosome
Antigen decomposition
Antigen
Saccharide chain receptor
Antigen
FcRn
In acidic endosome, antibody dissociates from saccharide chain receptor and antigen and binds to FcRn

FIG.3

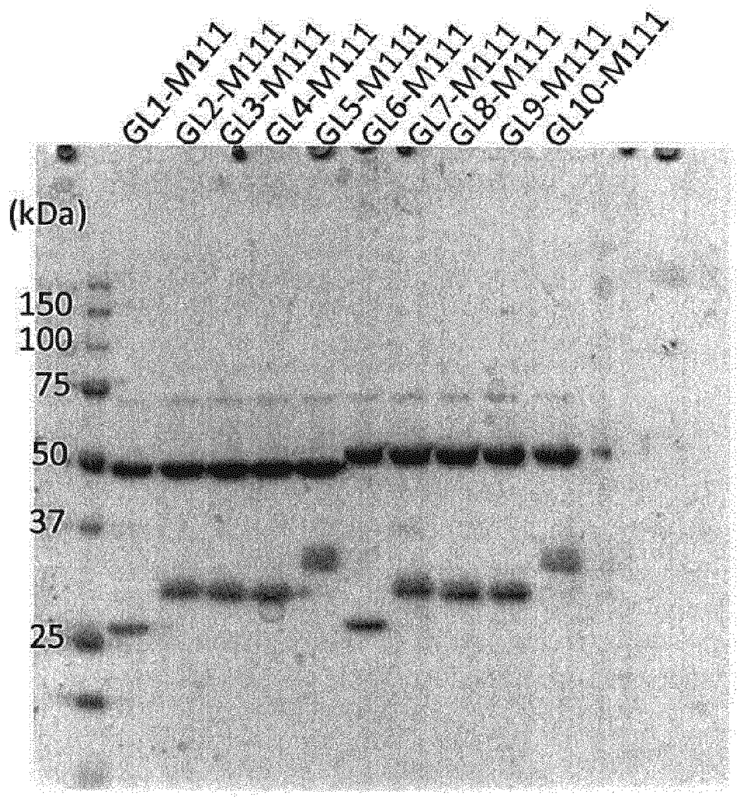
GL1-M111
GL2-M111
GL3-M111
GL4-M111
GL5-M111
GL6-M111
GL7-M111
GL8-M111
GL9-M111
GL10-M111
(kDa)
150
100
75
50
37
25

FIG.4

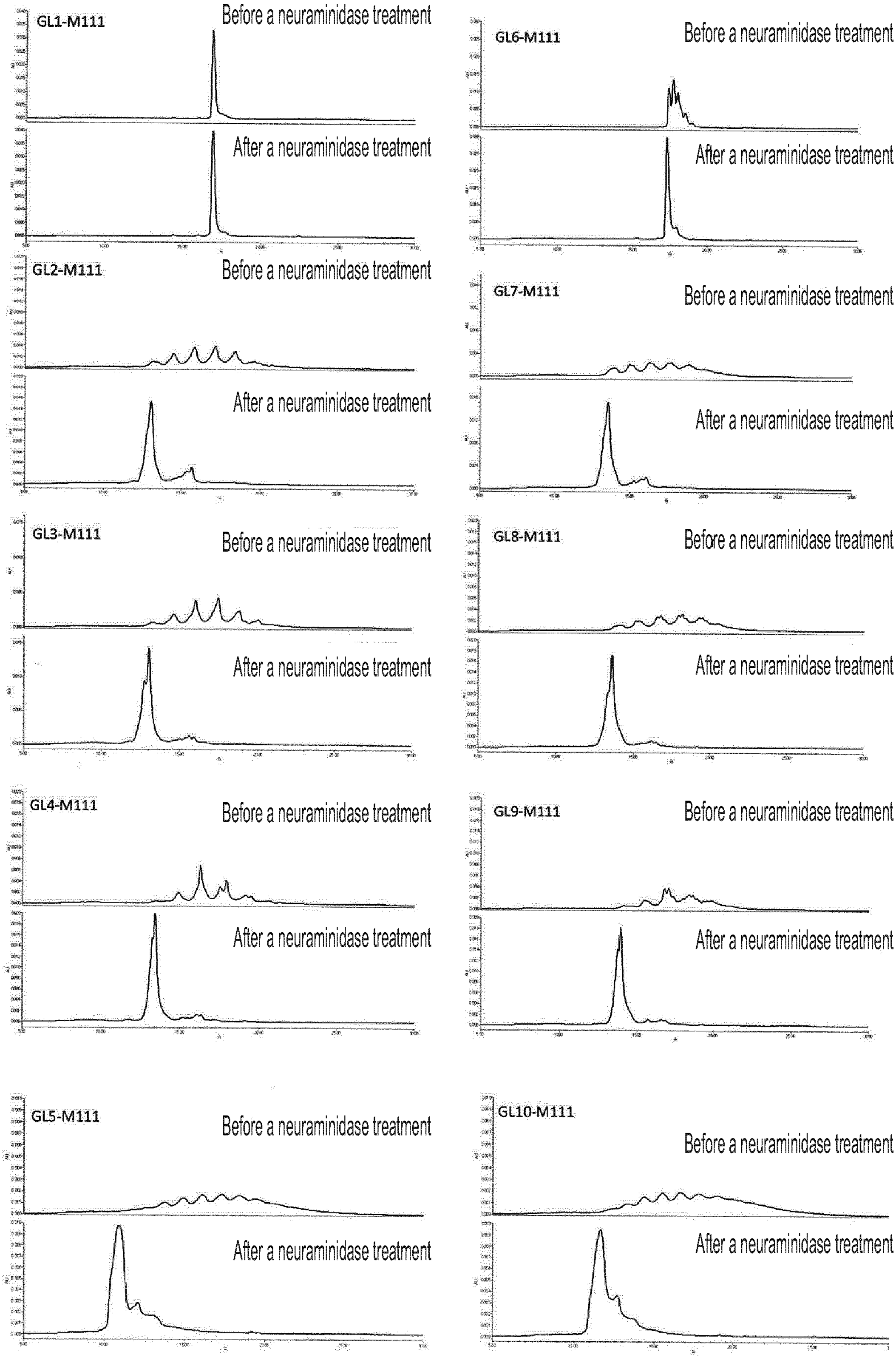

GL1-M111
Before a neuraminidase treatment
After a neuraminidase treatment
GL6-M111
Before a neuraminidase treatment
After a neuraminidase treatment
GL2-M111
Before a neuraminidase treatment
After a neuraminidase treatment
GL7-M111
Before a neuraminidase treatment
After a neuraminidase treatment
GL3-M111
Before a neuraminidase treatment
After a neuraminidase treatment
GL8-M111
Before a neuraminidase treatment
After a neuraminidase treatment
GL4-M111
Before a neuraminidase treatment
After a neuraminidase treatment
GL9-M111
Before a neuraminidase treatment
After a neuraminidase treatment
GL5-M111
Before a neuraminidase treatment
After a neuraminidase treatment
GL10-M111
Before a neuraminidase treatment
After a neuraminidase treatment

FIG.5

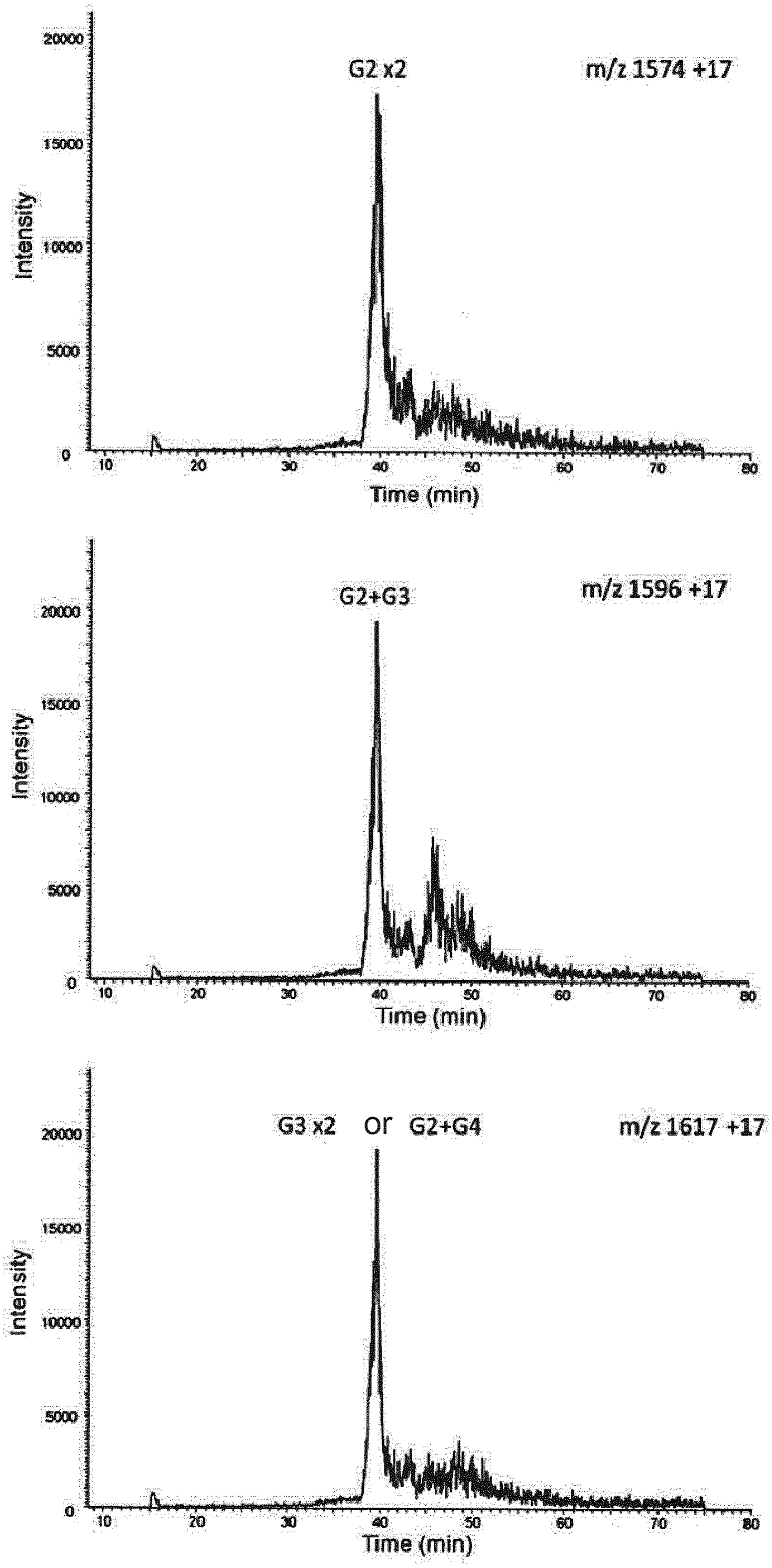
G2 x2
m/z 1574 +17
Intensity
Time (min)
G2+G3
m/z 1596 +17
Intensity
Time (min)
G3 x2 or G2+G4
m/z 1617 +17
Intensity
Time (min)

FIG.6

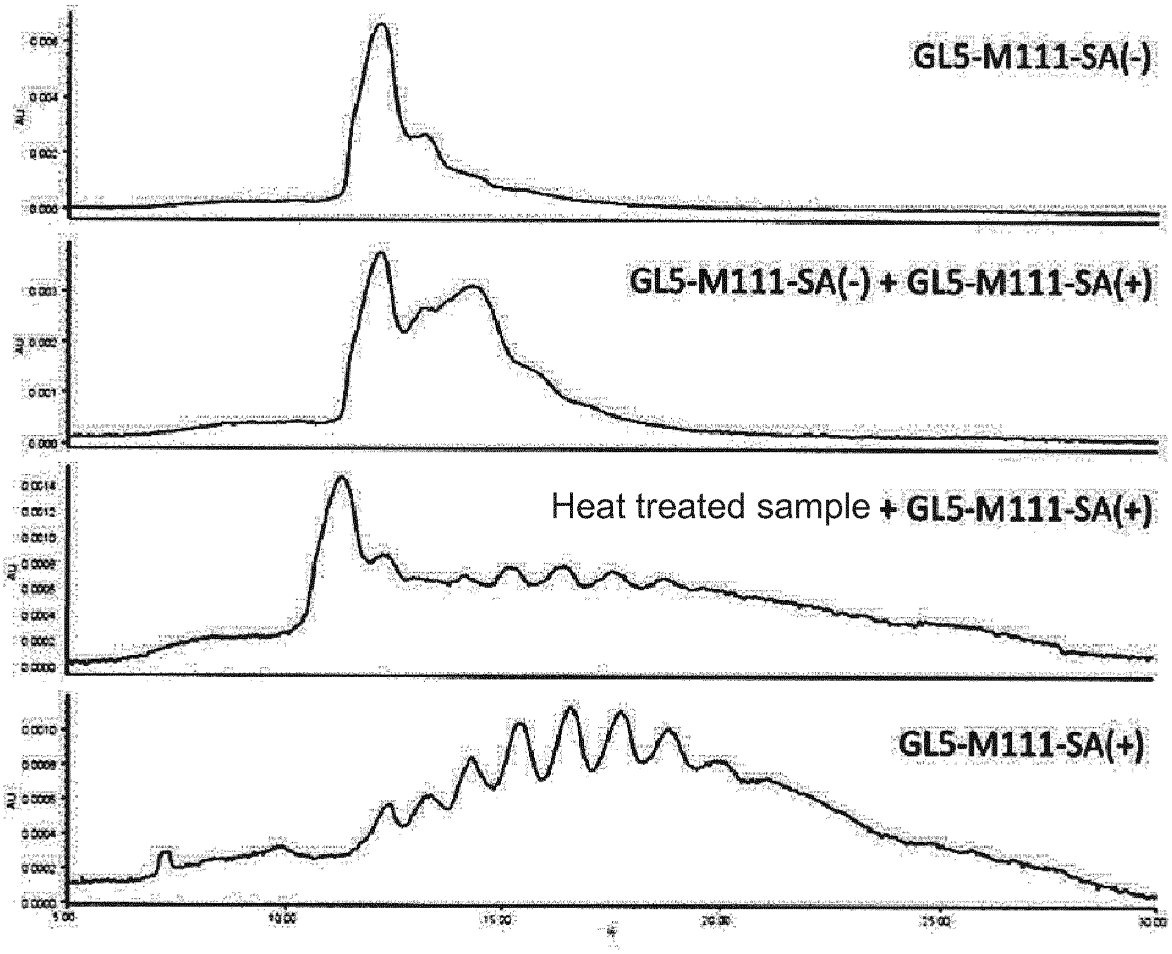
GL5-M111-SA(-)
GL5-M111-SA(-) + GL5-M111-SA(+)
Heat treated sample + GL5-M111-SA(+)
GL5-M111-SA(+)

FIG.7

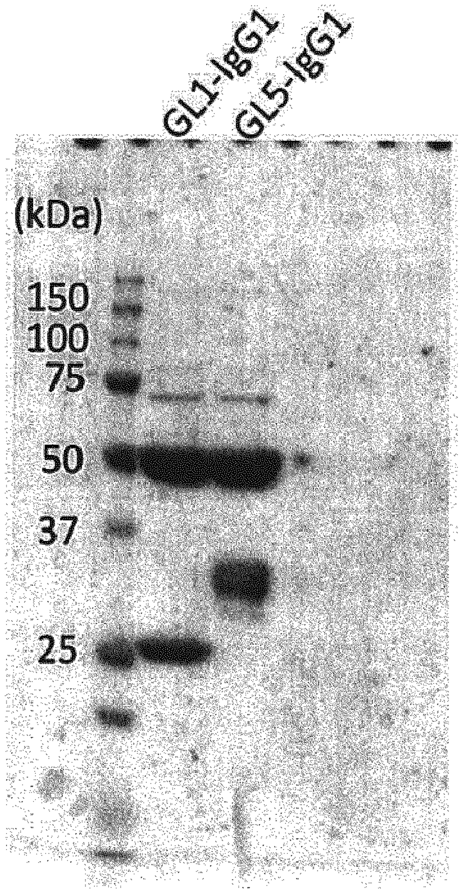

FIG.8

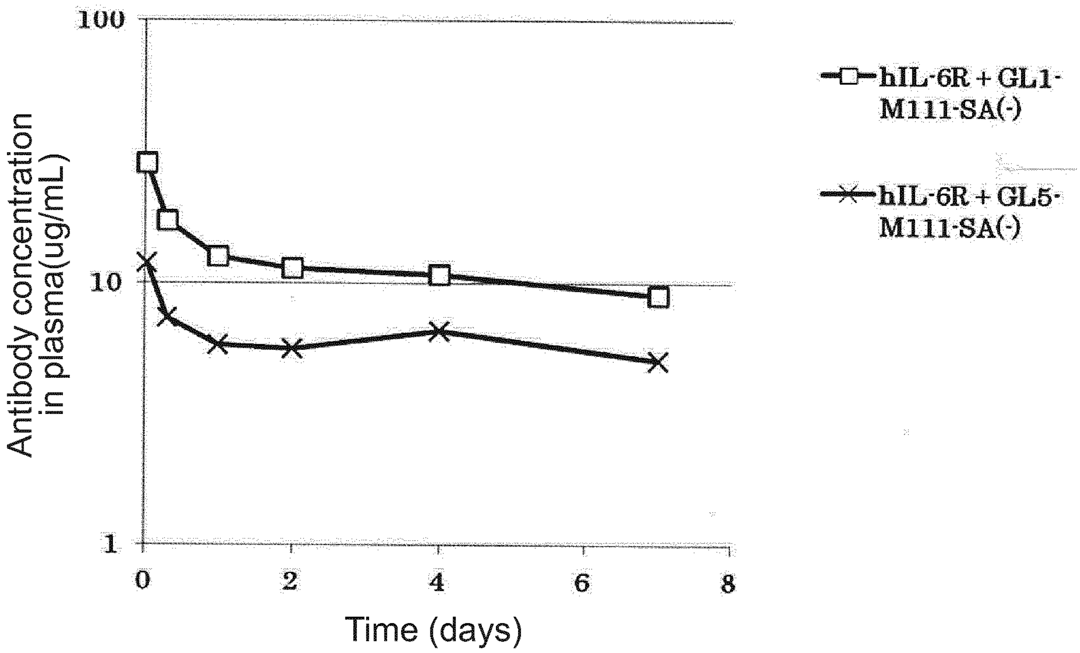
100
10
1
Antibody concentration in plasma(ug/mL)
0
2
4
6
8
Time (days)
hIL-6R + GL1-M111-SA(-)
hIL-6R + GL5-M111-SA(-)

FIG.9

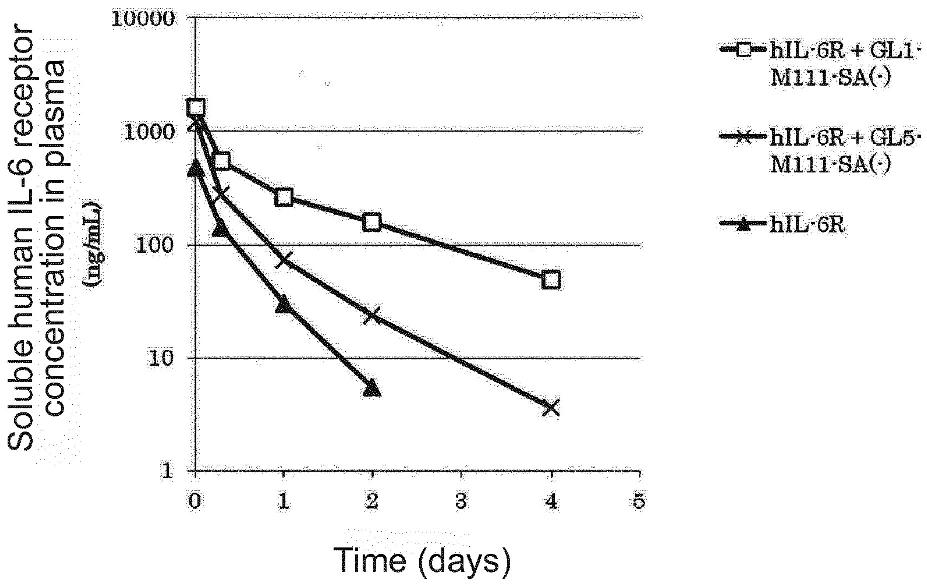
Soluble human IL-6 receptor concentration in plasma (ng/mL)
10000
1000
100
10
1
0
1
2
3
4
5
Time (days)
hIL-6R + GL1-M111-SA(-)
hIL-6R + GL5-M111-SA(-)
hIL-6R

FIG.10

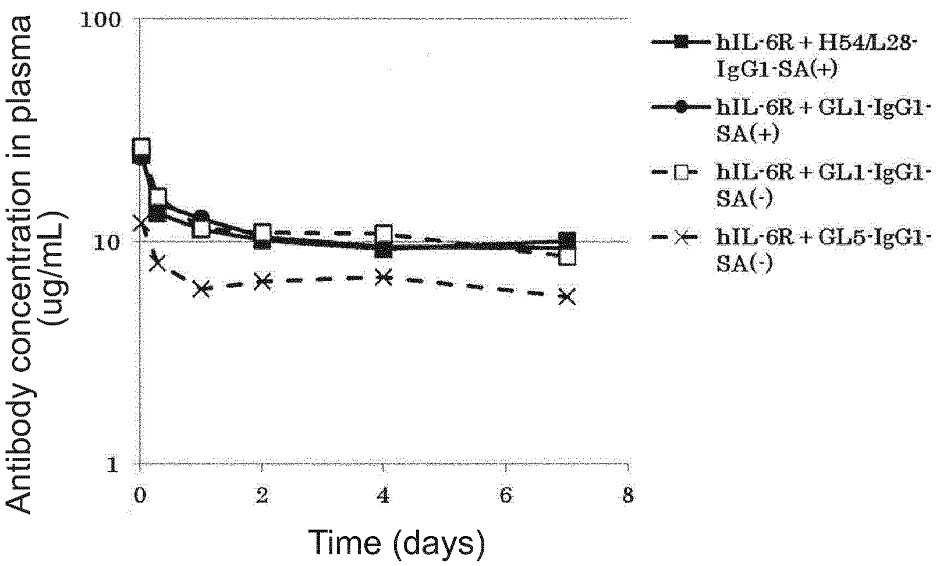
Antibody concentration in plasma (ug/mL)
100
10
1
0
2
4
6
8
Time (days)
hIL-6R + H54/L28-IgG1-SA(+)
hIL-6R + GL1-IgG1-SA(+)
hIL-6R + GL1-IgG1-SA(-)
hIL-6R + GL5-IgG1-SA(-)

FIG.11

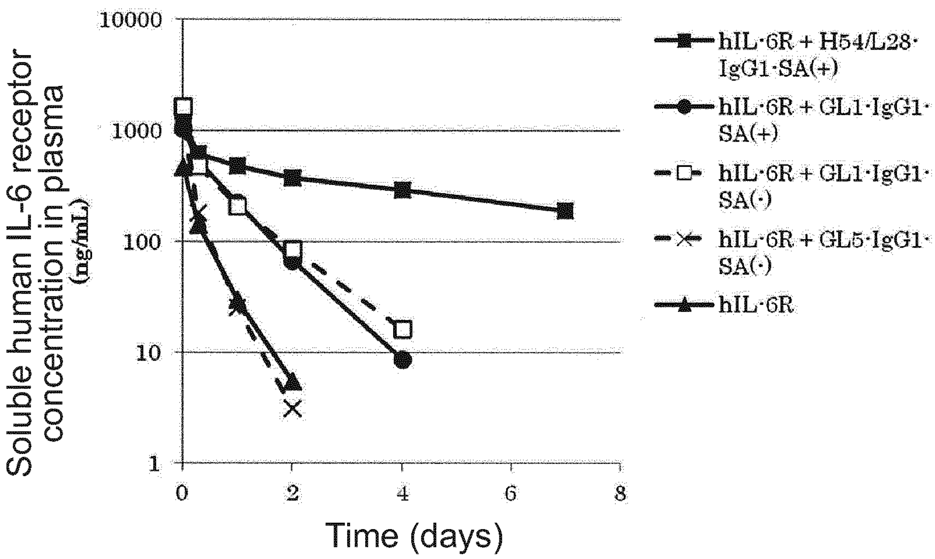
10000
1000
100
10
1
Soluble human IL-6 receptor concentration in plasma (ng/mL)
0
2
4
6
8
Time (days)
hIL-6R + H54/L28-IgG1-SA(+)
hIL-6R + GL1-IgG1-SA(+)
hIL-6R + GL1-IgG1-SA(-)
hIL-6R + GL5-IgG1-SA(-)
hIL-6R

FIG.12

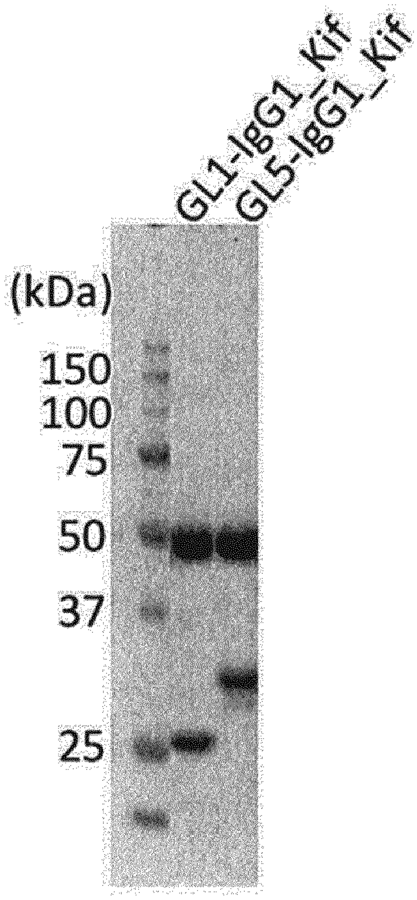

FIG.13

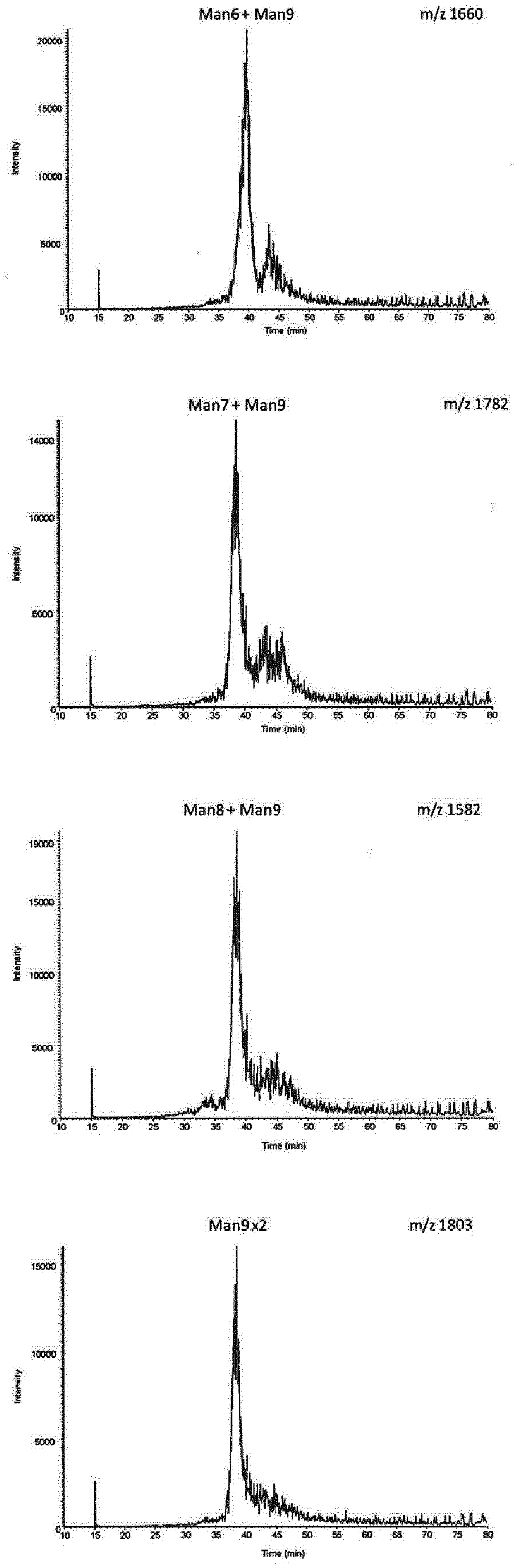
Man6+ Man9
m/z 1660
Intensity
Time (min)
Man7+ Man9
m/z 1782
Intensity
Time (min)
Man8+ Man9
m/z 1582
Intensity
Time (min)
Man9x2
m/z 1803
Intensity
Time (min)

FIG.14

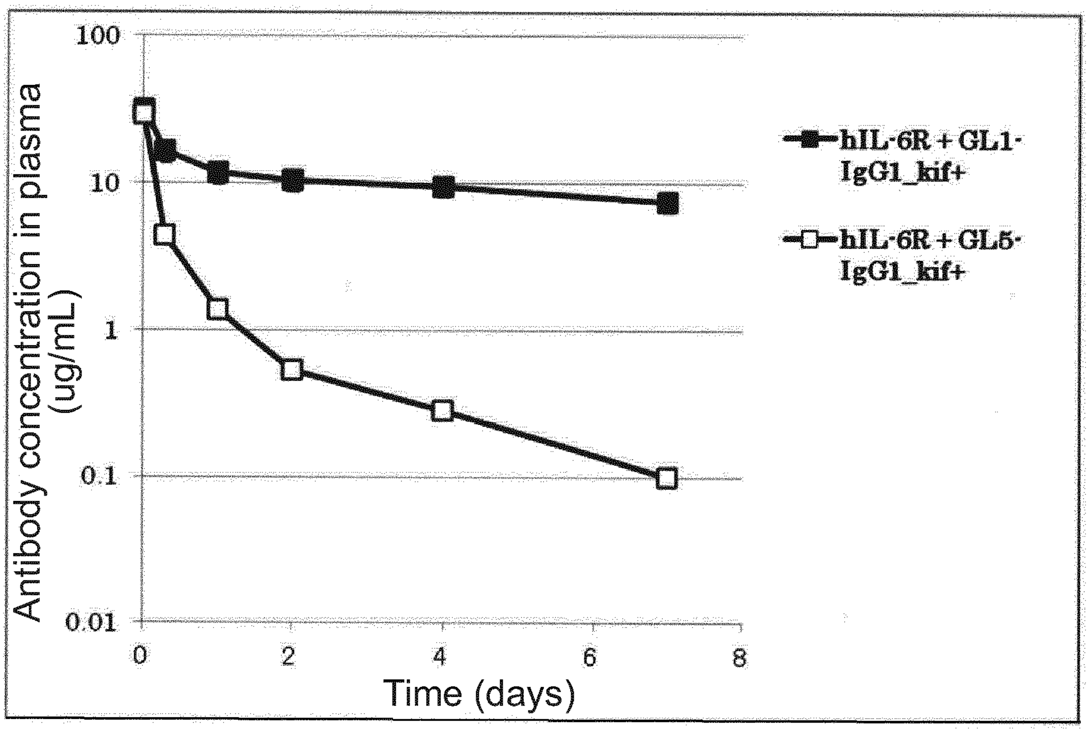
100
10
1
0.1
0.01
Antibody concentration in plasma (ug/mL)
0
2
4
6
8
Time (days)
hIL-6R + GL1-IgG1_kif+
hIL-6R + GL5-IgG1_kif+

FIG.15

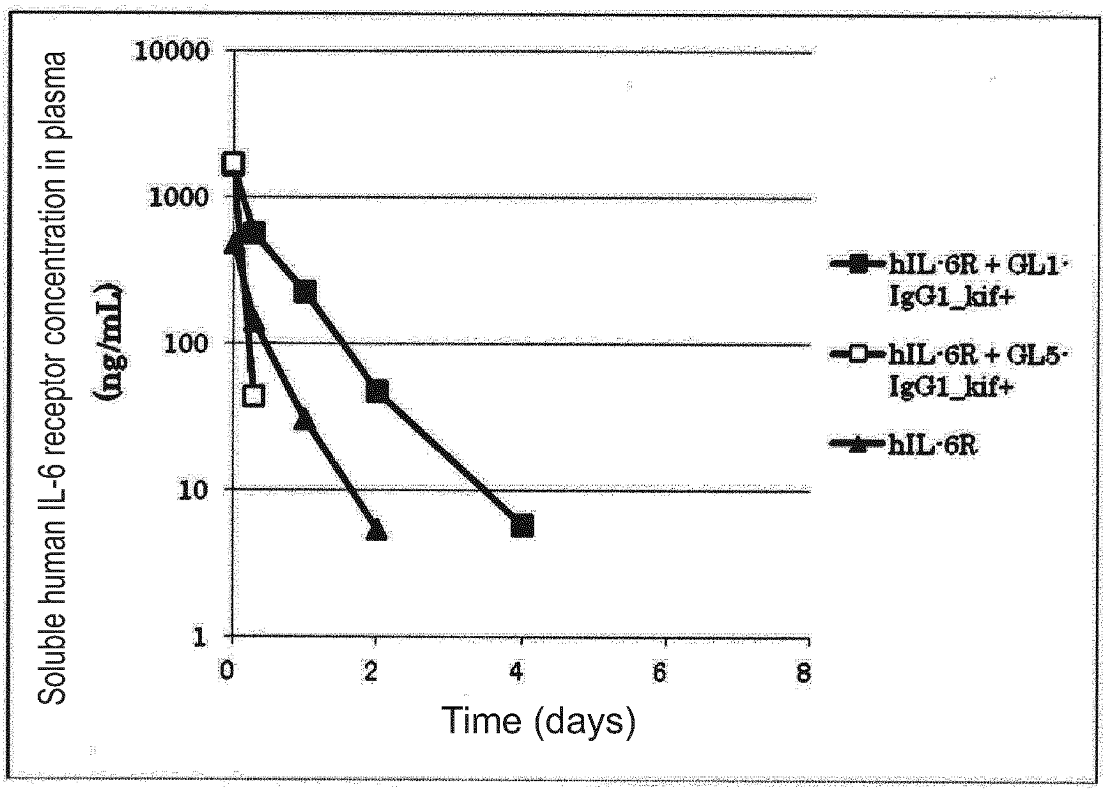
Soluble human IL-6 receptor concentration in plasma (ng/mL)
10000
1000
100
10
1
0
2
4
6
8
Time (days)
hIL-6R + GL1-IgG1_kif+
hIL-6R + GL5-IgG1_kif+
hIL-6R

FIG.16

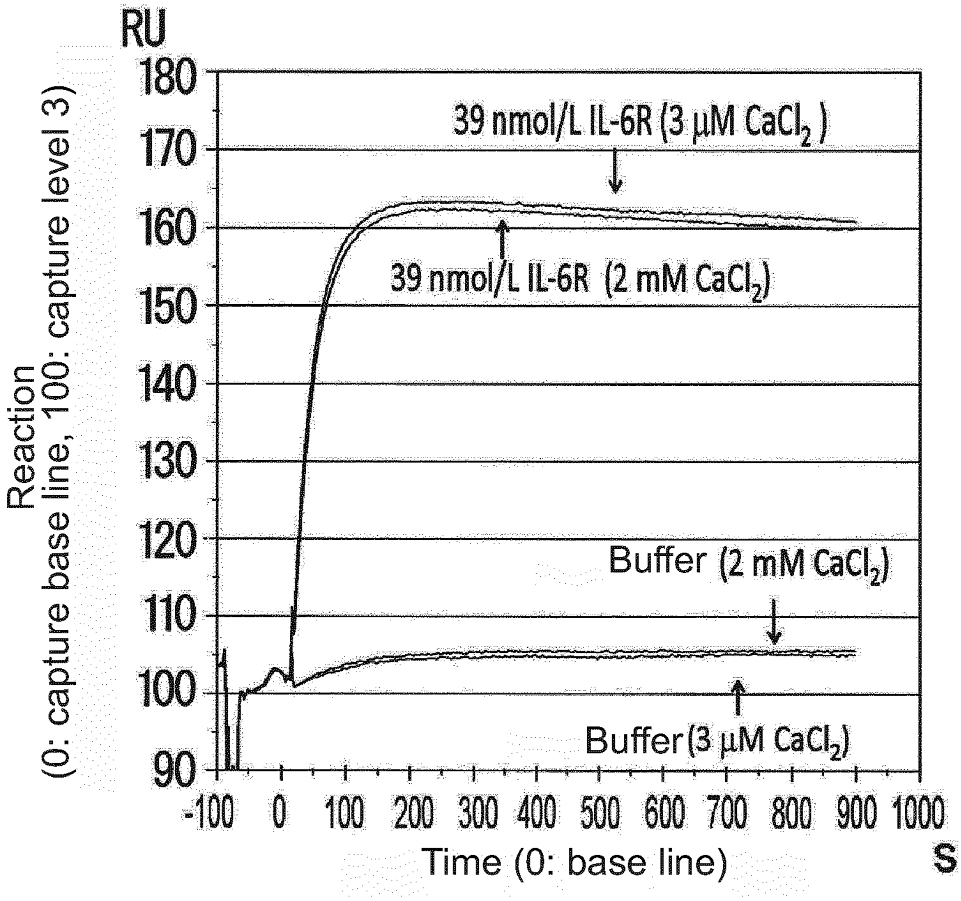
RU
180
170
160
150
140
130
120
110
100
90
Reaction
(0: capture base line, 100: capture level 3)
39 nmol/L IL-6R (3 μM CaCl2 )
39 nmol/L IL-6R (2 mM CaCl2)
Buffer (2 mM CaCl2)
Buffer(3 μM CaCl2)
-100 0 100 200 300 400 500 600 700 800 900 1000
Time (0: base line)
S

FIG.17

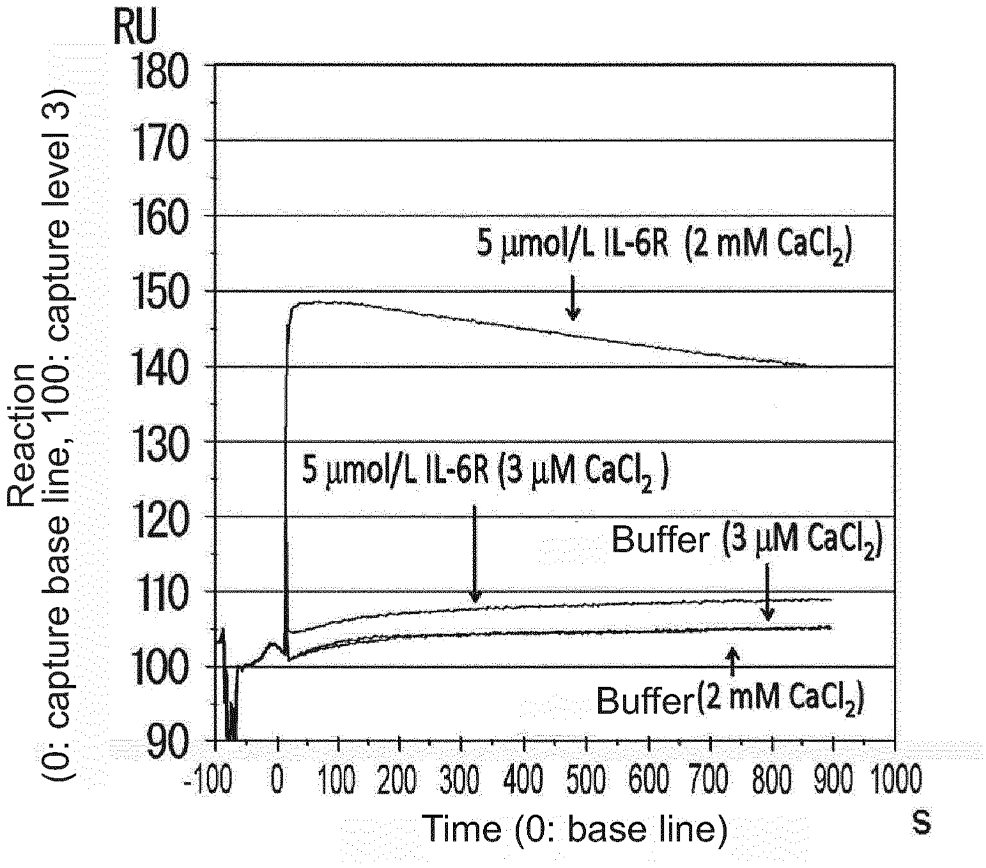
RU
180
170
160
150
140
130
120
110
100
90
Reaction
(0: capture base line, 100: capture level 3)
5 μmol/L IL-6R (2 mM $CaCl_2$)
5 μmol/L IL-6R (3 μM $CaCl_2$ )
Buffer (3 μM $CaCl_2$)
Buffer(2 mM $CaCl_2$)
-100
0
100
200
300
400
500
600
700
800
900
1000
Time (0: base line)
S

FIG.18

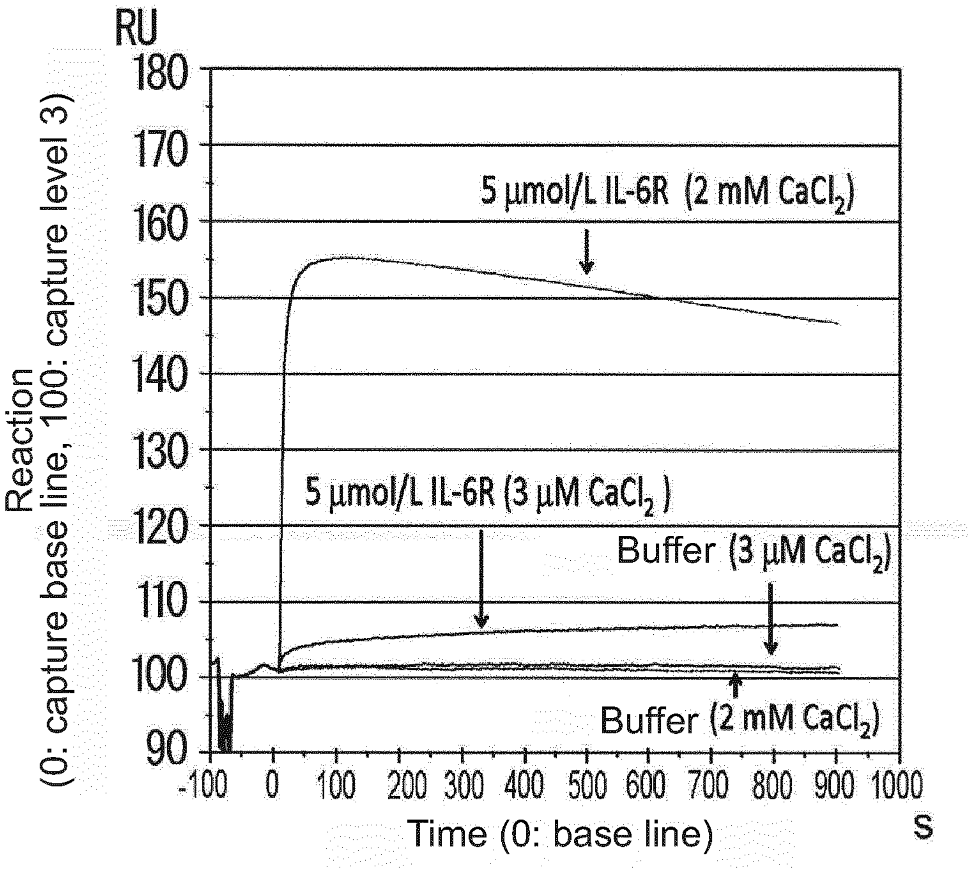
RU
180
170
160
150
140
130
120
110
100
90
Reaction
(0: capture base line, 100: capture level 3)
5 µmol/L IL-6R (2 mM CaCl2)
5 µmol/L IL-6R (3 µM CaCl2 )
Buffer (3 µM CaCl2)
Buffer (2 mM CaCl2)
-100 0 100 200 300 400 500 600 700 800 900 1000
Time (0: base line)
S

FIG.19

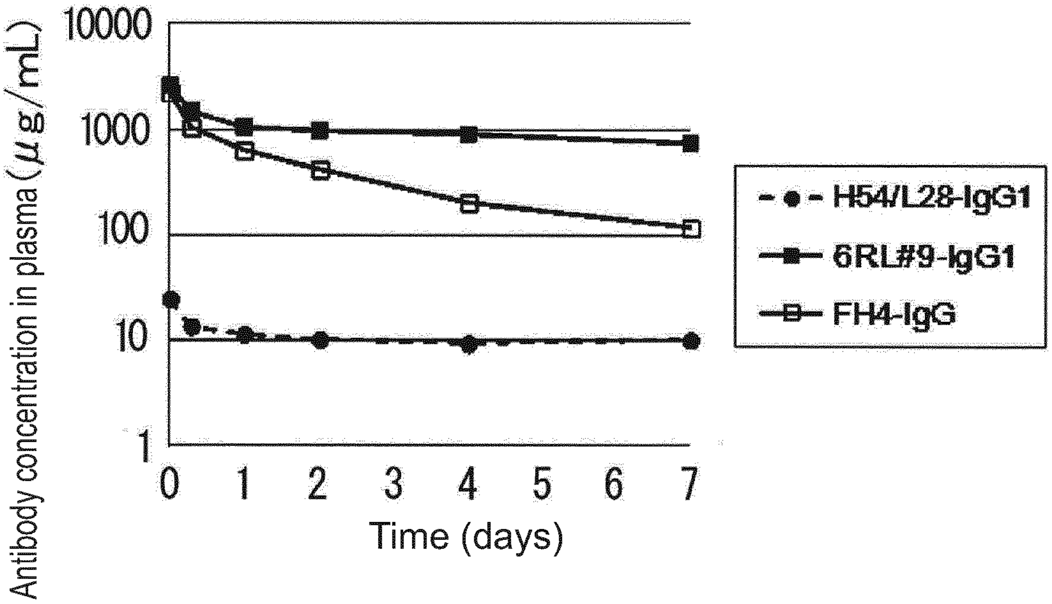
10000
1000
100
10
1
Antibody concentration in plasma (μg/mL)
0
1
2
3
4
5
6
7
Time (days)
H54/L28-IgG1
6RL#9-IgG1
FH4-IgG

FIG.20

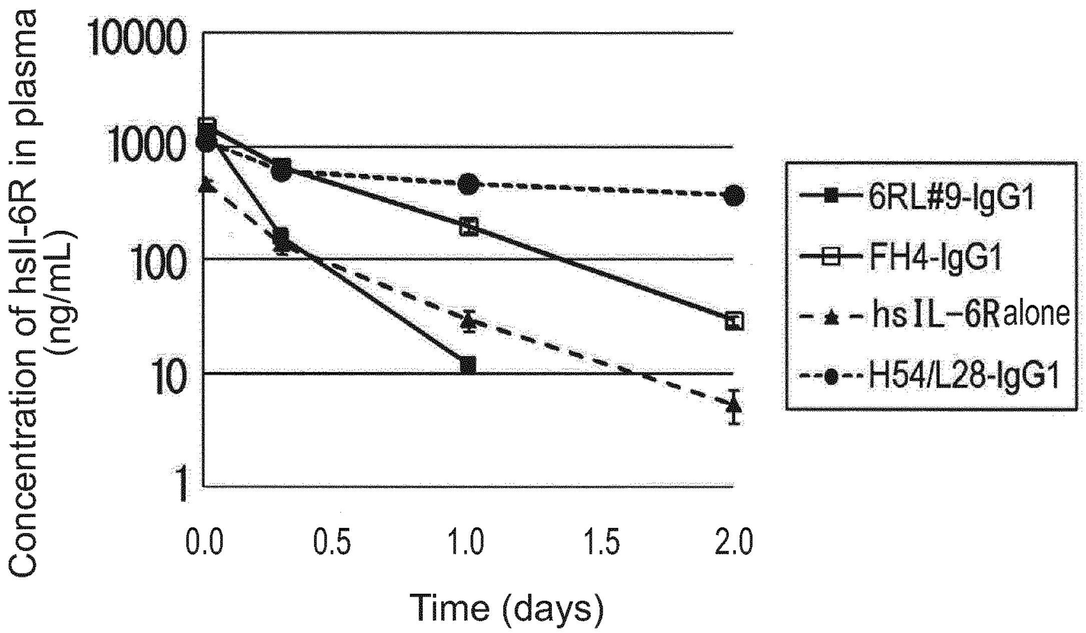
Concentration of hsIl-6R in plasma (ng/mL)
10000
1000
100
10
1
0.0
0.5
1.0
1.5
2.0
Time (days)
6RL#9-IgG1
FH4-IgG1
hsIL-6Ralone
H54/L28-IgG1

FIG.21

(i) Structure of crystal in the presence of calcium ion

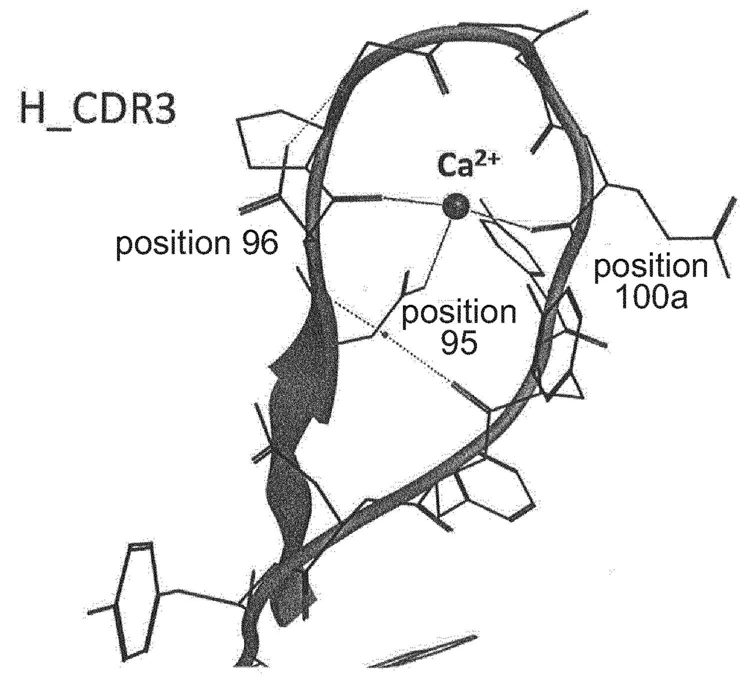


(ii) Structure of crystal in the absence of calcium ion

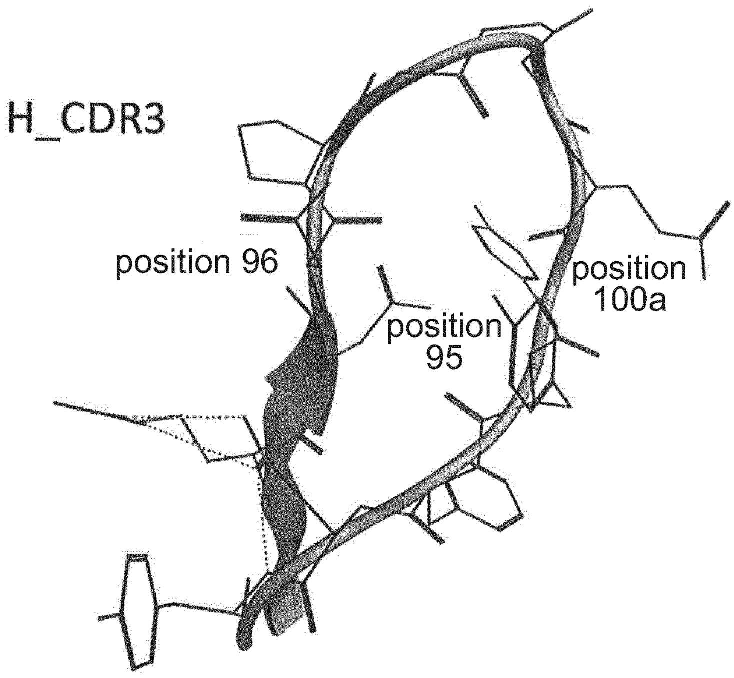

FIG.22

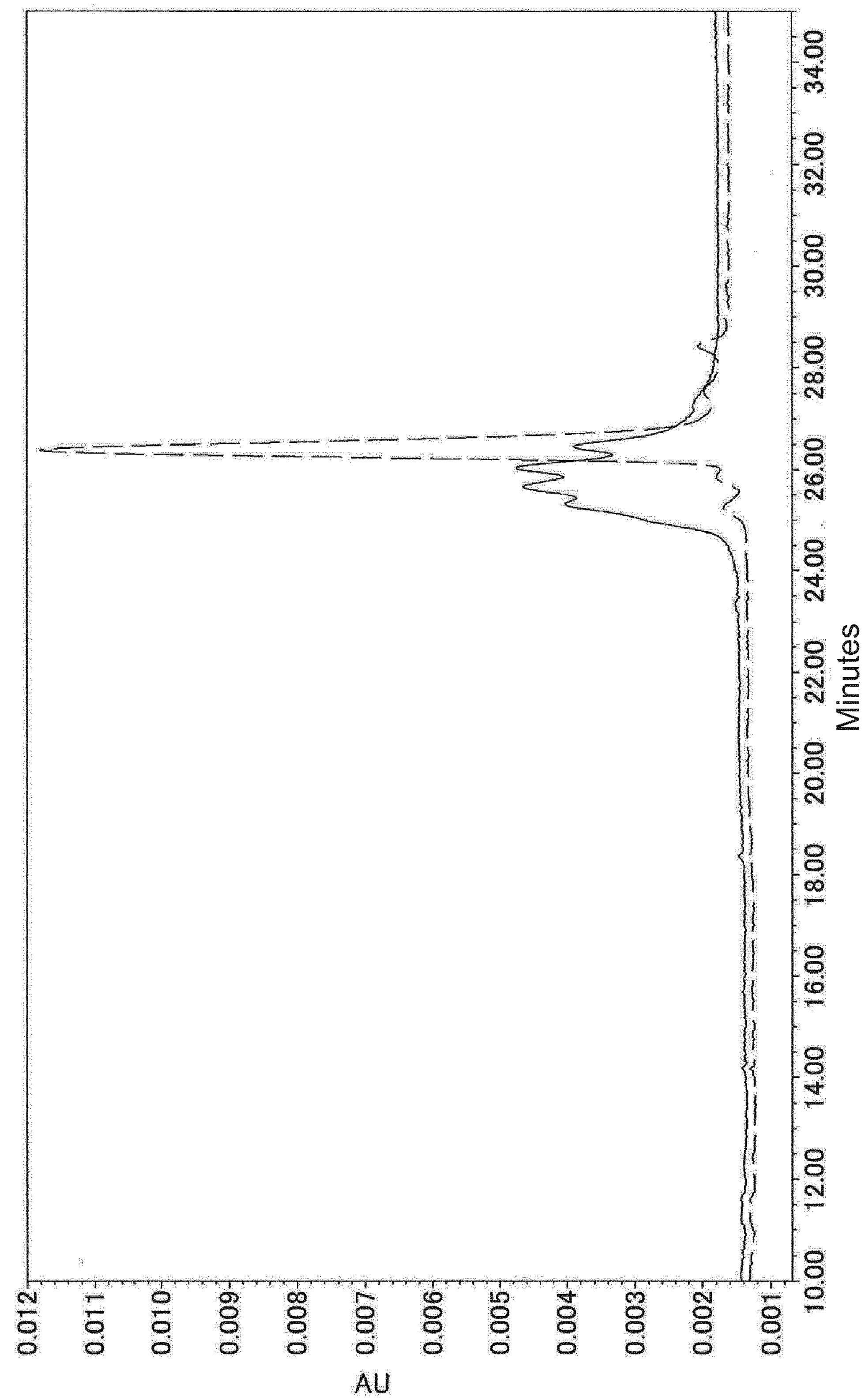
AU
0.012
0.011
0.010
0.009
0.008
0.007
0.006
0.005
0.004
0.003
0.002
0.001
10.00
12.00
14.00
16.00
18.00
20.00
22.00
24.00
26.00
28.00
30.00
32.00
34.00
Minutes

**INTERNATIONAL SEARCH REPORT**

International application No. PCT/JP2012/006453

A. CLASSIFICATION OF SUBJECT MATTER
*C12N15/09*(2006.01)i, *A61K39/00*(2006.01)i, *A61K39/395*(2006.01)i, *C07K16/18* (2006.01)i, *C12P21/02*(2006.01)i, *C12P21/08*(2006.01)i, *C12Q1/02*(2006.01)i

According to International Patent Classification (IPC) or to both national classification and IPC

B. FIELDS SEARCHED

Minimum documentation searched (classification system followed by classification symbols)
C12N15/09, A61K39/00, A61K39/395, C07K16/18, C12P21/02, C12P21/08, C12Q1/02

Documentation searched other than minimum documentation to the extent that such documents are included in the fields searched

Electronic data base consulted during the international search (name of data base and, where practicable, search terms used)
CAPLUS/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br><br><br><br>Y<br><br>A | JP 02-231097 A (The Trustees of Columbia University in the City of New York), 13 September 1990 (13.09.1990), claims & US 6218149 B1 & US 2002/0028486 A1 & EP 359096 A1 | 1,2,10-13, 19-28,36-39, 45-56,64, 72-75,81-92<br>3-8,29-34, 65-70<br>9,14-18,35, 40-44,71, 76-80 |
| Y | WO 2009/125825 A1 (Chugai Pharmaceutical Co., Ltd.), 15 October 2009 (15.10.2009), claims & JP 2012-21004 A & US 2011/0111406 A1 & EP 2275443 A1 | 3-8,29-34, 65-70 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 December, 2012 (11.12.12) | 25 December, 2012 (25.12.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2012/006453

C (Continuation). DOCUMENTS CONSIDERED TO BE RELEVANT

| Category* | Citation of document, with indication, where appropriate, of the relevant passages | Relevant to claim No. |
|---|---|---|
| A | WRIGHT A. et al, Antibody variable region glycosylation: position effects on antigen binding and carbohydrate structure., EMBO J., 1991, Vol.10, No.10, p.2717-2723 | 1-56,64-92 |
| A | WO 2005/116078 A1 (MEDEXGEN INC.), 08 December 2005 (08.12.2005), & US 2006/0247425 A1 & EP 1765872 A | 1-56,64-92 |
| A | WRIGHT A. et al, Effect of glycosylation on antibody function: implications for genetic engineering., Trends Biotechnol., 1997, Vol.15, No.1, p.26-32 | 1-56,64-92 |
| A | US 5714350 A (PROTEIN DESIGN LABS INC.), 03 February 1998 (03.02.1998), (Family: none) | 1-56,64-92 |
| A | Shogo OKA et al., "Idenshi Sosa o Mochiita Tosa no Sakujo Oyobi Kaihen", Protein, nucleic acid and enzyme, 1992, vol.37, no.3, pages 389 to 394 | 1-56,64-92 |
| A | KIM J.J.P. et al, Carbohydrate recognition by the mannose-6-phosphate receptors., Curr. Opin. Struct. Biol., 2009, Vol.19, No.5, p.534-542 | 1-56,64-92 |
| A | Tomoyuki IGAWA et al., "Antibody optimization technologies for developing next generation antibody therapeutics", BIO INDUSTRY, 2011.06, vol.28, no.7, pages 15 to 21 | 1-56,64-92 |

Form PCT/ISA/210 (continuation of second sheet) (July 2009)

## INTERNATIONAL SEARCH REPORT

International application No. PCT/JP2012/006453

**Box No. II Observations where certain claims were found unsearchable (Continuation of item 2 of first sheet)**

This international search report has not been established in respect of certain claims under Article 17(2)(a) for the following reasons:

1. [x] Claims Nos.: 57-63
because they relate to subject matter not required to be searched by this Authority, namely:
The inventions set forth in claims 57-63 of the present application comprise contacting an antigen-binding molecule with a cell *in vivo* and, therefore, pertain to methods for treatment of the human body.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
Since the invention of claim 1 of the present application is disclosed in JP 02-231097 A, there is no special technical feature between the invention of claim 1 and the inventions of claims 2-56 and 64-92 of the present application.
Consequently, the inventions of claims of the present application do not comply with the requirement of unity of invention.

1. [ ] As all required additional search fees were timely paid by the applicant, this international search report covers all searchable claims.
2. [x] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011221400 A
- WO 2009125825 A
- WO 2004044011 A
- WO 2005040229 A
- WO 2002032925 A
- WO 1995001937 A
- WO 2002020565 A
- WO 2003029462 A
- WO 2008016854 A
- WO 2004058821 A
- WO 2003002609 A
- WO 2005037989 A
- WO 2003000883 A
- WO 2004022754 A
- WO 2006006693 A
- WO 2003104453 A
- WO 1994011523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 2008081008 A
- WO 2000042072 A
- WO 2002060919 A
- WO 2004092219 A
- WO 2010045193 A
- US 20090035836 A
- JP 2006141241 A
- EP 125023 A


### Non-patent literature cited in the description


- **CLARK J ROSENSWEIG ; LAURA B FADEN ; MATTHEW C DEWITZ.** *Nature Biotechnology,* 2005, vol. 23, 1073-1078
- **PAVLOU AK ; BELSEY MJ.** *Eur J Pharm Biopharm.,* 2005, vol. 59 (3), 389-96
- **KIM SJ ; PARK Y ; HONG HJ.** *Mol Cells.,* 2005, vol. 20 (1), 17-29
- **HINTON PR ; XIONG JM ; JOHLFS MG ; TANG MT ; KELLER S ; TSURUSHITA N.** *J Immunol.,* 2006, vol. 176 (1), 346-56
- **GHETIE V ; POPOV S ; BORVAK J ; RADU C ; MATESOI D ; MEDESAN C ; OBER RJ ; WARD ES.** *Nat Biotechnol.,* 1997, vol. 15 (7), 637-40
- **RAJPAL A ; BEYAZ N ; HABER L ; CAPPUCCILLI G ; YEE H ; BHATT RR ; TAKEUCHI T ; LERNER RA ; CREA R.** *Proc Natl Acad Sci U S A.,* 2005, vol. 102 (24), 8466-71
- **WU H ; PFARR DS ; JOHNSON S ; BREWAH YA ; WOODS RM ; PATEL NK ; WHITE WI ; YOUNG JF ; KIENER PA.** *J Mol Biol.,* 2007, vol. 368, 652-665
- **HANSON CV ; NISHIYAMA Y ; PAUL S.** *2005,* 2005, vol. 16 (6), 631-6
- **RATHANASWAMI P ; ROALSTAD S ; ROSKOS L ; SU QJ ; LACKIE S ; BABCOOK J.** *Biochem Biophys Res Commun.,* 2005, vol. 334 (4), 1004-13
- **SPIESS M.** *Biochemistry,* 1990, vol. 29 (43), 10009-18
- **WRAGG S. ; DRICKAMER K.** *J Biol Chem,* 1999, vol. 274 (50), 35400-6
- **KIM JJ ; OLSON LJ ; DAHMS NM.** *Curr Opin Struct Biol.,* 2009, vol. 19 (5), 534-42
- **COLOMA MJ ; TRINH RK ; MARTINEZ AR ; MORRISON SL.** *J Immunol,* 1999, vol. 162, 2162-2170
- **IGAWA T ; TSUNODA H ; KURAMOCHI T ; SAMPEI Z ; ISHII S ; HATTORI K.** *MAbs,* 2011, vol. 3, 243-52
- Epitope Mapping Protocols in Methods in Molecular Biology. 1996, vol. 66
- Antibodies A Laboratory Manual. Cold Spring Harbor Laboratory, 1988, 359-420
- *Nat Med.,* 2003, vol. 9 (1), 47-52
- *BioDrugs,* 2006, vol. 20 (3), 151-160
- *EMBO J.,* 1994, vol. 13 (24), 5863-70
- **MULLBERG et al.** *J. Immunol.,* 1994, vol. 152 (10), 4958-4968

- *J. Immunol.,* 1979, vol. 123 (4), 1548-1550
- *Current Topics in Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- **KOHLER ; MILSTEIN et al.** *Methods Enzymol.,* 1981, vol. 73, 3-46
- **VANDAMME et al.** *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002
- *Nucleic Acids Res.,* 1989, vol. 17 (8), 2919-2932
- *Nat. Biotechnol.,* 2005, vol. 23 (9), 1126-1136
- *Bio/Technolog,* 1994, vol. 12 (7), 699-702
- *Cancer Res.,* 1993, vol. 53, 851-856
- **BERNASCONI et al.** *Science,* 2002, vol. 298, 2199-2202
- **GHETIE et al.** *Immunol. Today,* 1997, vol. 18 (12), 592-598
- **RAGHAVAN et al.** *Immunity,* 1994, vol. 1, 303-315
- **YEUNG et al.** *J. Immunol.,* 2009, vol. 182, 7663-7671
- *The Journal of Immunology,* 2009, vol. 182, 7663-7671
- **SPRINGER et al.** *Cell,* 2000, vol. 102, 275-277
- **KAWASAKI ; KRETSINGER.** *Protein Prof.,* 1995, vol. 2, 305-490
- **MONCRIEF et al.** *J. Mol. Evol.,* 1990, vol. 30, 522-562
- **CHAUVAUX et al.** *Biochem. J.,* 1990, vol. 265, 261-265
- **BAIROCH ; COX.** *FEBS Lett.,* 1990, vol. 269, 454-456
- **DAVIS.** *New Biol.,* 1990, vol. 2, 410-419
- **SCHAEFER et al.** *Genomics,* 1995, vol. 25, 638-643
- **ECONOMOU et al.** *EMBO J.,* 1990, vol. 9, 349-354
- **WURZBURG et al.** *Structure,* 2006, vol. 14 (6), 1049-1058
- **H. R. HOOGENBOOM ; G. WINTER.** *J. Mol. Biol.,* 1992, vol. 227, 381-388
- *Bioorg. Med. Chem.,* 2003, vol. 11 (17), 3761-2768
- *Angew. Chem. Int. Ed.,* 2005, vol. 44, 34
- *Chem Soc Rev.,* 2004, vol. 33 (7), 422-430
- *Amino Acids,* 1999, vol. 16 (3-4), 345-379
- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- **HEINRICH et al.** *Biochem. J.,* 1998, vol. 334, 297-314
- **ISHIKAWA et al.** *J. Clin. Exp. Hematopathol.,* 2006, vol. 46 (2), 55-66
- *The Journal of immunology,* 2008, vol. 180 (3), 1686-1693
- *Bioorg. Med. Chem.,* 2011, vol. 19 (8), 2494-2500
- *Bioorg. Med. Chem.,* 2009, vol. 17 (20), 7254-7264
- *Bioorg. Med. Chem.,* 2008, vol. 16 (9), 5216-5231
- *J. Am. Chem. Soc.,* 2004, vol. 126 (33), 10355-10363
- *J. Pept. Sci.,* 2003, vol. 9 (6), 375-385
- *Methods Enzymol.,* 2010, vol. 478, 343-363
- **MILLWARD.** *Biologicals,* 2008, vol. 36, 49-60
- **NEWKIRK.** *Clin. Exp. Immunol.,* 1996, vol. 106, 259-264
- **WRIGHT ; MORRISON.** *J. Exp. Med.,* 1994, vol. 180, 1087-1096
- **ZHOU.** *Biotechnol. Bioeng.,* 2008, vol. 99, 652-665
- **KANDA et al.** *Glycobiology,* 2007, vol. 17, 104-118
- *Biochemistry,* 30 September 2008, vol. 47 (39), 10294-304
- *J Biotechnol.,* 23 February 2009, vol. 139 (4), 318-25
- *Nat Biotechnol.,* February 2006, vol. 24 (2), 210-5
- *J Biol Chem,* 1999, vol. 274 (50), 35400-35406
- *J Biol Chem.,* 11 November 1994, vol. 269 (45), 28405-13
- *Proc Natl Acad Sci U S A. 1,* 2006, vol. 103 (11), 4005-10
- **KABAT et al.** Sequence of Proteins of Immunological Interest. National Institute of Health, 1987
- **CHOTHIA et al.** *Nature,* 1989, vol. 342, 877
- *Nat Biotechnol.,* 2005, vol. 23 (9), 1126-36
- *Protein Engineering,* 1996, vol. 9 (3), 299-305
- *Mol Cell Biol.,* 1988, vol. 8, 466-472
- Current protocols in Molecular Biology. John Wiley & Sons, 1987
- **VICKI GLASER.** *SPECTRUM Biotechnology Applications,* 1993
- **EBERT et al.** *Bio/Technology,* 1994, vol. 12, 699-702
- **MA et al.** *Eur. J. Immunol.,* 1994, vol. 24, 131-8
- **COX KM et al.** *Nat. Biotechnol.,* 2006, 1591-1597

- Strategies for Protein Purification and Characterization: A Laboratory Course Manual. Cold Spring Harbor Laboratory Press, 1996
- *Clin Pharmacol.,* 2008, vol. 48 (4), 406-17
- *Pharm Res.,* 2006, vol. 23 (1), 95-103
- *Methods Mol Biol.,* 2010, vol. 602, 93-104
- *J Immunotoxicol.,* 2005, vol. 3, 131-9
- *mAbs,* 2010, vol. 2 (5), 1-13
- *ARTHRITIS & RHEUMATISM,* 2006, vol. 54, 2387-92
- *AAPS J.,* 2010, vol. 4, 646-57
- *Blood.,* 15 November 2008, vol. 112 (10), 3959-64
- *J. Biol. Chem.,* 1989, vol. 274 (50), 35400-35406
- *J. Biol. Chem.,* 1994, vol. 269 (45), 28405-28413
- *Protein Science,* 1995, vol. 4, 2411-2423
- *J. Immunol.,* 1994, vol. 152, 4958-4968
- *Mol. Biol.,* 2002, vol. 178, 87-100
- *J Immunol. Methods. (J. Immunol. Methods.,* 2008, vol. 332 (1-2), 2-9
- *J Immunol. Methods.,* 2001, vol. 247 (1-2), 191-203
- *Biotechnol. Prog.,* 2002, vol. 18 (2), 212-220
- *Mol. Cell. Proteomics.,* 2003, vol. 2 (2), 61-69
- *Methods Mol. Biol.,* 2002, vol. 178, 133-145
- *J. Biol. Chem.,* 2008, vol. 283 (37), 25140-25149
- *J. Immunol. Methods.,* 2008, vol. 332 (1-2), 2-9
- *J. Immunol. Methods,* 2001, vol. 247 (1-2), 191-203
- *Biotechnol. Prog.,* 2002, vol. 18 (2), 212-20
- *Mol. Cell Proteomics,* 2003, vol. 2 (2), 61-9
- *Science,* 1995, vol. 4, 2411-2423